# EUROPEAN PATENT APPLICATION

(11) **EP 2 757 160 A2**
(43) Date of publication of application: **23.07.2014**
(21) Application number: 13176946.5
(22) Date of filing: 19.07.2010
(51) Int. Cl.: C12Q 1/68

(54) **Gene expression markers for Crohn's disease**

(30) Priority: 20.07.2009 US 227049 P; 05.02.2010 US 302084 P
(62) Divisional of application: 12182666.3
(71) Applicant: Genentech, Inc., South San Francisco, CA 94080 (US)
(72) Inventor: Abbas, Alexander, R., San Carlos, CA 94070 (US); Clark, Hillary, San Francisco, CA 94131 (US); Diehl, Lauri, Los Altos, CA 94024 (US); Lees, Charles, Linlithgow West Lothian EH49 7E (GB); Noble, Colin,L., Edinburgh Midlothian, EH5 3QF (GB); Satsangi, Jack, Edinburgh Midlothian, EH10 5ET (GB)
(74) Representative: Woolley, Lindsey Claire

(57) **Abstract**

The present invention relates to methods of gene expression profiling for inflammatory bowel disease pathogenesis, in which the differential expression in a test sample from a mammalian subject of one or more IBD markers relative to a control is determined, wherein the differential expression in the test sample is indicative of an IBD in the mammalian subject from which the test sample was obtained.

## Description

### Field of the Invention

The present invention relates to gene expression profiles in inflammatory bowel disease pathogenesis, including use in the detection and diagnosis of inflammatory bowel disease.

### Description of Related Art

Immune related and inflammatory diseases are the manifestation or consequence of fairly complex, often multiple interconnected biological pathways which in normal physiology are critical to respond to insult or injury, initiate repair from insult or injury, and mount innate and acquired defense against foreign organisms. Disease or pathology occurs when these normal physiological pathways cause additional insult or injury either as directly related to the intensity of the response, as a consequence of abnormal regulation or excessive stimulation, as a reaction to self, or as a combination of these.

Though the genesis of these diseases often involves multistep pathways and often multiple different biological systems/pathways, intervention at critical points in one or more of these pathways can have an ameliorative or therapeutic effect. Therapeutic intervention can occur by either antagonism of a detrimental process/pathway or stimulation of a beneficial process/pathway.

Many immune related diseases are known and have been extensively studied. Such diseases include immune-mediated inflammatory diseases, non-immune-mediated inflammatory diseases, infectious diseases, immunodeficiency diseases, neoplasia, *etc.*

The term inflammatory bowel disorder ("IBD") describes a group of chronic inflammatory disorders of unknown causes in which the intestine (bowel) becomes inflamed, often causing recurring cramps or diarrhea. The prevalence of IBD in the US is estimated to be about 200 per 100,000 population. Patients with IBD can be divided into two major groups, those with ulcerative colitis ("UC") and those with Crohn's disease ("CD"). Both UC and CD are chronic relapsing diseases and are complex clinical entities that occur in genetically susceptible individuals who are exposed to as yet poorly defined environmental stimuli. (Bonen and Cho, Gastroenterology. 2003; 124:521-536; Gaya et al. Lancet. 2006;367:1271-1284).

Clinically, IBD is characterized by diverse manifestations often resulting in a chronic, unpredictable course. Bloody diarrhea and abdominal pain are often accompanied by fever and weight loss. Anemia is common, as is severe fatigue. Joint manifestations ranging from arthralgia to acute arthritis as well as abnormalities in liver function are commonly associated with IBD. Patients with IBD also have an increased risk of colon carcinomas compared to the general population. During acute "attacks" of IBD, work and other normal activity are usually impossible, and often a patient is hospitalized.

Although the cause of IBD remains unknown, several factors such as genetic, infectious and immunologic susceptibility have been implicated. IBD is much more common in Caucasians, especially those of Jewish descent. The chronic inflammatory nature of the condition has prompted an intense search for a possible infectious cause. Although agents have been found which stimulate acute inflammation, none has been found to cause the chronic inflammation associated with IBD. The hypothesis that IBD is an autoimmune disease is supported by the previously mentioned extraintestinal manifestation of IBD as joint arthritis, and the known positive response to IBD by treatment with therapeutic agents such as adrenal glucocorticoids, cyclosporine and azathioprine, which are known to suppress immune response. In addition, the GI tract, more than any other organ of the body, is continuously exposed to potential antigenic substances such as proteins from food, bacterial byproducts (LPS), etc. The subtypes of IBD are UC and CD.

There is sufficient overlap in the diagnostic criteria for UC and CD that it is sometimes impossible to say which a given patient has; however, the type of lesion typically seen is different, as is the localization. UC mostly appears in the colon, proximal to the rectum, and the characteristic lesion is a superficial ulcer of the mucosa; CD can appear anywhere in the bowel, with occasional involvement of stomach, esophagus and duodenum, and the lesions are usually described as extensive linear fissures. CD differs from UC in that the inflammation extends through all layers of the intestinal wall and involves mesentery as well as lymph nodes. CD may affect any part of the alimentary canal from mouth to anus. The disease is often discontinuous, i.e., severely diseased segments of bowel are separated from apparently disease-free areas. In CD, the bowel wall also thickens which can lead to obstructions. In addition, fistulas and fissures are not uncommon.

The current therapy of IBD usually involves the administration of antiinflammatory or immunosuppressive agents, such as sulfasalazine, corticosteroids, 6-mercaptopurine/azathioprine, or cyclosporine, which usually bring only partial results. If anti-inflammatory/immunosuppressive therapies fail, colectomies are the last line of defense. The typical operation for CD not involving the rectum is resection (removal of a diseased segment of bowel) and anastomosis (reconnection) without an ostomy. Sections of the small or large intestine may be removed. About 30% of CD patients will need surgery within the first year after diagnosis. In the subsequent years, the rate is about 5% per year. Unfortunately, CD is characterized by a high rate of recurrence; about 5% of patients need a second surgery each year after initial surgery.

Refining a diagnosis of inflammatory bowel disease involves evaluating the progression status of the diseases using standard classification criteria. The classification systems used in IBD include the Truelove and Witts Index (Truelove S.C. and Witts, L.J. Br Med J. 1955;2:1041-1048), which classifies colitis as mild, moderate, or severe, as well as Lennard-Jones. (Lennard-Jones JE. Scand J Gastroenterol Suppl 1989;170:2-6) and the simple clinical colitis activity index (SCCAI). (Walmsley et. al. Gut. 1998;43:29-32) These systems track such variables as daily bowel movements, rectal bleeding, temperature, heart rate, hemoglobin levels, erythrocyte sedimentation rate, weight, hematocrit score, and the level of serum albumin.

In approximately 10-15% of cases, a definitive diagnosis of ulcerative colitis or Crohn's disease cannot be made and such cases are often referred to as "indeterminate colitis." Two antibody detection tests are available that can help the diagnosis, each of which assays for antibodies in the blood. The antibodies are "perinuclear anti-neutrophil antibody" (pANCA) and "anti-Saccharomyces cervisiae antibody" (ASCA). Most patients with ulcerative colitis have the pANCA antibody but not the ASCA antibody, while most patients with Crohn's disease have the ASCA antibody but not the pANCA antibody. However, these two tests have shortcomings as some patients have neither antibody and some Crohn's disease patients may have only the pANCA antibody.For clinical practice, a reliable test that would indicate the presence and/or progression of an IBD based on molecular markers rather than the measurement of a multitude of variables would be useful for identifying and/or treating individuals with an IBD. Hypothesis free, linkage and association studies have identified genetic loci that have been associated with UC, notably the MHC region on chromosome 6,(Rioux et al. Am J Hum Genet. 2000;66:1863-1870; Stokkers et al. Gut. 1999; 45:395-401; Van Heel et al. Hum Mol Genet. 2004;13:763-770) the IBD2 locus on chromosome 12 (Parkes et al. Am J Hum Genet. 2000;67:1605-1610; Satsangi et al. Nat Genet. 1996;14:199-202) and the IBD5 locus on chromosome 5. (Giallourakis et. al. Am J. Hum Genet. 2003;73:205-211; Palmieri et. al Aliment Pharmacol Ther. 2006;23:497-506; Russell et. al. Gut. 2006;55:1114-1123; Waller et. al. Gut. 2006;55:809-814) Following a UK wide linkage scan identifying a putative loci of association for UC on chromosome 7q, further studies have implicated variants in the ABCB1 (MDR1) gene which is involved in cellular detoxification with UC. (Satsangi et. al. Nat Genet. 1996;14:199-202; Brant et.al. Am J Hum Genet. 2003;73:1282-1292; Ho et. al. Gastroenterology. 2005;128:288-296)

A complementary approach towards the identification and understanding of the complex gene-gene and gene- environment relationships that result in the chronic intestinal inflammation observed in inflammatory bowel disease (IBD) is microarray gene expression analysis. Microarrays allow a comprehensive picture of gene expression at the tissue and cellular level, thus helping understand the underlying patho-physiological processes. (Stoughton et. al. Annu Rev Biochem. 2005;74:53-82) Microarray analysis was first applied to patients with IBD in 1997, comparing expression of 96 genes in surgical resections of patients with CD to synovial tissue of patients with rheumatoid arthritis. (Heller et. al. Proc Natl Acad Sci U S A. 1997;94:2150-2155) Further studies using microarray platforms to interrogate surgical specimens from patients with IBD identified an number of novel genes that were differentially regulated when diseased samples were compared to controls. (Dieckgraefe et.al. Physiol Genomics. 2000;4:1-11; Lawrance et. al. Hum Mol Genet. 2001;10:445-456).

Current evidence suggests that the inflammatory bowel diseases, Crohn's disease (CD) and ulcerative colitis (UC) are complex non- Mendelian polygenic disorders with important environmental interactions and stimuli. (Gaya et al. Lancet 2006;367:1271-1284) The finding that variants of the NOD2/CARD15 gene are associated with susceptibility to CD is regarded as a landmark discovery and has catalysed widespread interest in the role of the innate and adaptive immune response in the development of CD. (Hugot et al. Nature 2001;411:599-603; Ogura et al. Nature 2001;411:603-606)

Recently genome wide scans (GWS) have identified a number of genetic variants that are associated with CD. The first genome wide association study was carried out in the Japanese CD population (Yamazaki et al. Hum Mol Genet 2005;14:3499-3506), and subsequent studies have now been undertaken in CD populations in North America and Europe.

Polymorphisms in the IL-23R gene on chromosome 1p31 were observed to be associated with CD initially in a US study (Duerr et al. Science 2006;314:1461-1463), and this has now been widely replicated in Europe catalyzing interest in the IL-23/ Th17 pathway. (The Wellcome Trust Case Control Consortium, Nature 2007;447:661-678) In the past 2 years, a number of genome-wide association studies (GWAS) in populations of European descent and a subsequent meta-analysis have identified 32 confirmed CD susceptibility genes/loci. (Barrett et al. Nat Genet 2008, Aug;40(8):955-62) These include innate immune genes that are specific to CD; NOD2, originally described in 2001 (Hugot et al. Nature 2001;411(6837):599-603; Ogura et al. Nature 2001;411(6837):603-6**)** and the autophagy genes ATG16L1 and IRGM (The Wellcome Trust Case Control Consortium, Nature 2007;447:661-678), clearly indicating that defects in the intracellular processing of bacteria constitutes a central feature in the pathogenesis of CD. The discovery that germline variants of IL-23R were protective in CD coincided with murine experiments detailing the contribution of IL-23 (rather than IL-12 with which it shares the p40 subunit) to Th17 driven chronic intestinal inflammation. (Duerr et al. Science 2006;314(5804):1461-3; Maloy et al. Mucosal Immunol 2008;1(5):339-49) The meta-analysis and subsequent studies in UC have demonstrated that 3 other IL-23 pathway genes (IL12B, JAK2 and STAT3) are all IBD susceptibility genes. (Barrett et al. Nat Genet 2008, Aug;40(8):955-62)

At present there are no large scale intestinal genome-wide expression studies in CD. There is now an immediate need to explore in detail the function and expression of the novel genetic associations. We have previously applied the technique of genome-wide expression to examine gene profiles in colonic biopsies from patients with UC. (Noble et al. Gut 2008, Oct;57(10):1398-405) Findings included an expression gradient in the healthy adult colon and a change in expression of a number of novel genes as well as established candidate genes such as the alpha defensins 5 and 6. In the healthy adult colon cluster analysis showed differences in gene expression between the right and left colon and the developmental genes HOXA13, HOXB13, GLI1 and GLI3 primarily drove this separation. In UC expression of serum amyloid A1 (SAA1) and the alpha defensins A5&6 were increased, and the increase in DEFA5&6 expression was further characterized to Paneth cell metaplasia by immunohistochemistry and *in-situ* hybridization.

Increasingly intestinal epithelial cells (IEC) are observed to play a critical role in immune homeostasis in the gut. Indeed the discovery of the role of NOD2/CARD15 and other pathogen-associated molecular pattern (PAMP) receptors play in recognizing intestinal pathogens and responding to cellular stress signals have put IECs at the forefront of intestinal immunological defense.(Strober et al., J.Clin.Invest 2007;117(3):514-21.) The IEC response targets the nuclear transcription factor NF-κB, the central regulator of this pathway.

A number of microarray studies have now been carried out in immune cell subsets to try to understand differences in gene expression during activation and inflammation. Genome wide expression from a compendium of six immune cell types has allowed investigators to identify a collection of immune response *in silico* genes that have specific expression signatures in immune cells. (Abbas et al. Genes Immun 2005;6:319-331) These genes have allowed investigators to differentiate signaling pathways in immune cell subsets and to characterize the inflammatory response of genes known to play a role in immune response and genes of unknown function.

Endoscopic pinch mucosal biopsies have allowed investigators to microarray tissue from a larger range of patients encompassing those with less severe disease. Langmann et. al. used microarray technology to analyze 22,283 genes in biopsy specimens from macroscopically non affected areas of the colon and terminal ileum. (Langmann et. al. Gastroenterology. 2004; 127:26-40) Genes which were involved in cellular detoxification and biotransformation (Pregnane X receptor and MDR1) were significantly downregulated in the colon of patients with UC, however, there was no change in the expression of these genes in the biopsies from patients with CD. Costello and colleagues (Costello et. al. PLoS Med. 2005;2:e199) looked at the expression of 33792 sequences in endoscopic sigmoid colon biopsies obtained from healthy controls, patients with CD and UC. A number of sequences representing novel proteins were differentially regulated and *in silico* analysis suggested that these proteins had putative functions related to disease pathogenesis - transcription factors, signaling molecules and cell adhesion.

In a study of patients with UC, Okahara et al. (Aliment Pharmacol Ther. 2005;21:1091-1097) observed that (migration inhibitory factor- related protein 14 (MRP14), growth- related oncogene gamma (GROγ) and serum amyloid A1 (SAA1) were upregulated where as TIMP1 and elfin were down regulated in the inflamed biopsies when compared to the non- inflamed biopsies. When observing 41 chemokines and 21 chemokine receptors, Puleston et al demonstrated that chemokines CXCLs 1-3 and 8 and CCL20 were upregulated in active colonic CD and UC. (Aliment Pharmacol Ther. 2005;21:109-120) Overall these studies illustrate the heterogeneity of early microarray platforms and tissue collection. However, despite these problems differential expression of a number of genes was consistently observed.

Despite the above identified advances in IBD research, there is a great need for additional diagnostic and therapeutic agents capable of detecting IBD in a mammal and for effectively treating this disorder.

All publications mentioned herein are incorporated herein by reference to disclose and describe the methods and/or materials in connection with which the publications are cited. Publications cited herein are cited for their disclosure prior to the filing date of the present application. Nothing here is to be construed as an admission that the inventors are not entitled to antedate the publications by virtue of an earlier priority date or prior date of invention. Further the actual publication dates may be different from those shown and require independent verification.

### Summary of the Invention

The present invention provides polynucleotides and polypeptides that are overexpressed in inflammatory bowel disease (IBD) as compared to normal tissue, and methods of using those polypeptides, and their encoding nucleic acids, for to detect or diagnose the presence of IBD in mammalian subjects and subsequently to treat those subjects in which IBD is detected with suitable IBD therapeutic agents.

The present invention also provides methods for detecting the presence of and determining the progression of IBD, including Crohn's disease (CD).

The invention disclosed herein provides methods and assays examining expression of one or more gene expression markers in a mammalian tissue or cell sample, wherein the expression of one or more such biomarkers is predictive of whether the mammalian subject from which the tissue or cell sample was taken is more likely to have IBD. In various embodiments of the invention, the methods and assays examine the expression of gene expression markers such as those listed in Table 1 and determine whether expression is differentially expressed relative to a control sample.

In one aspect, the invention concerns a method of detecting or diagnosing an IBD in a mammalian subject comprising determining, in a biological sample obtained from the subject, a differential expression level of (i) one or more nucleic acids encoding one or more polypeptides selected from Table 1, or (ii) RNA transcripts or their expression products of one or more genes selected from Table 1, relative to the expression level in a control, wherein the differential level of expression is indicative of the presence of an IBD in the subject from which the test sample was obtained. In all embodiments, the expression level of a nucleic acid encoding a polypeptide shown as any one of SEQ ID NOS: 5, 6, 8, 11, 12, 2, 14, 16, 18, 20, and 22, is determined.

In one embodiment, the methods of diagnosing or detecting the presence of an IBD in a mammalian subject comprise determining that the expression level of (i) one or more nucleic acids encoding one or more polypeptides selected from Table 1; or (ii) RNA transcripts or expression products thereof of one or more genes selected from Table 1 in a test sample obtained from the subject is lower relative to the level of expression in a control, wherein the lower level of expression is indicative of the presence of IBD in the subject from which the test sample was obtained. In all embodiments, the lower expression level of a nucleic acid encoding a polypeptide shown as any one of SEQ ID NOS: 5, 6, 8, 11, 12, 2, 14, and 16, is determined.

In another embodiment, the methods of diagnosing or detecting the presence of IBD in a mammalian subject comprise determining that the expression level of (i) one or more nucleic acids encoding one or more polypeptides selected from Table 1; or (ii) RNA transcripts or expression products thereof of one or more genes selected from Table 1 in a test sample obtained from the subject is higher relative to the level of expression in a control, wherein the higher level of expression is indicative of the presence of IBD in the subject from which the test sample was obtained. In all embodiments, the higher expression level of a nucleic acid encoding a polypeptide shown as any one of SEQ ID NOS: 18, 20, and 22, is determined.

In one aspect, the methods are directed to diagnosing or detecting a flare-up of an IBD in mammalian subject that was previously diagnosed with an IBD and is currently in remission. The subject may have completed treatment for the IBD or is currently undergoing treatment for the IBD. In one embodiment, the methods comprise determining a differential expression level of (i) one or more nucleic acids encoding one or more polypeptides selected from Table 1; or (ii) RNA transcripts or expression products thereof of one or more genes selected from Table 1 in a biological sample obtained from a mammalian subject relative to the expression level of a control, wherein the difference in expression indicates the subject is more likely to have an IBD flareup. In all embodiments, the differential expression level of a nucleic acid encoding a polypeptide shown as any one of SEQ ID NOS: 5, 6, 8, 11, 12, 2, 14, 16, 18, 20, and 22, is determined. In all embodiments, the test sample may be compared to a prior test sample of the mammalian subject, if available, obtained before, after, or at the time of the intial IBD diagnosis.

In all aspects, the mammalian subject preferably is a human patient, such as a human patient diagnosed with or at risk of developing an IBD. The subject may also be an IBD patient who has received prior treatment for an IBD but is at risk of a recurrence of the IBD.

For all aspects of the method of the invention, determining the expression level of one or more genes described herein (or one or more nucleic acids encoding polypeptide(s) expressed by one or more of such genes) may be obtained, for example, by a method of gene expression profiling. The method of gene expression profiling may be, for example, a PCR-based method.

In various embodiments, the diagnosis includes quantification of the expression level of (i) one or more nucleic acids encoding one or more polypeptides selected from Table 1; or (ii) RNA transcripts or expression products thereof of one or more genes selected from Table 1, such as by immunohistochemistry (IHC) and/or fluorescence *in situ* hybridization (FISH).

For all aspects of the invention, the expression levels of the genes may be normalized relative to the expression levels of one or more reference genes, or their expression products.

For all aspects of the invention, the method may further comprise determining evidence of the expression levels of at least two, three, four, five, six, seven, eight, or nine of said genes, or their expression products.

In another aspect, the methods of present invention also contemplate the use of a "panel" of such genes (i.e. IBD markers as disclosed herein) based on the evidence of their level of expression. In some embodiments, the panel of IBD markers will include at least one, two, three, four, five, six, seven, eight, or nine IBD markers. The panel may include an IBD marker that is overexpressed in IBD relative to a control, an IBD marker that is underexpressed in IBD relative to a control, or IBD markers that are both overexpressed and underexpressed in IBD relative to a control. Such panels may be used to screen a mammalian subject for the differential expression of one or more IBD markers in order to make a determination on whether an IBD is present in the subject.

In one embodiment, the IBD markers that make up the panel are selected from Table 1. In a preferred embodiment, the methods of diagnosing or detecting the presence of an IBD in a mammalian subject comprise determining a differential expression level of RNA transcripts or expression products thereof from a panel of IBD markers in a test sample obtained from the subject relative to the level of expression in a control, wherein the differential level of expression is indicative of the presence of an IBD in the subject from which the test sample was obtained. The differential expression in the test sample may be higher and/or lower relative to a control as discussed herein.

For all aspects of the invention, the method may further comprise the step of creating a report summarizing said prediction.

For all aspects, the IBD diagnosed or detected according to the methods of the present invention is Crohn's disease (CD), ulcerative colitis (UC), or both CD and UC.

For all aspects of the invention, the test sample obtained from a mammalian subject may be derived from a colonic tissue biopsy. In a preferred embodiment, the biopsy is a tissue selected from the group consisting of terminal ileum, the ascending colon, the descending colon, and the sigmoid colon. In other preferred embodiments, the biopsy is from an inflamed colonic area or from a non-inflamed colonic area. The inflamed colonic area may be acutely inflamed or chronically inflamed.

For all aspects, determination of expression levels may occur at more than one time. For all aspects of the invention, the determination of expression levels may occur before the patient is subjected to any therapy before and/or after any surgery. In some embodiments, the determining step is indicative of a recurrence of an IBD in the mammalian subject following surgery or indicative of a flare-up of said IBD in said mammalian subject. In a preferred embodiment, the IBD is Crohn's disease.

In another aspect, the present invention concerns methods of treating a mammalian subject in which the presence of an IBD has been detected by the methods described herein. For example, following a determination that a test sample obtained from the mammalian subject exhibits differential expression relative to a control of one or more of the RNA transcripts or the corresponding gene products of an IBD marker described herein, the mammalian subject may be administered an IBD therapeutic agent.

In one embodiment, the methods of treating an IBD in a mammalian subject in need thereof, comprise (a) determining a differential expression level of (i) one or more nucleic acids encoding one or more polypeptides selected from Table 1; or (ii) RNA transcripts or expression products thereof of one or more genes selected from Table 1 in a test sample obtained from the subject relative to the expression level of a control, wherein said differential level of expression is indicative of the presence of an IBD in the subject from which the test sample was obtained; and (b) administering to said subject an effective amount of an IBD therapeutic agent. In all embodiments, the expression level of a nucleic acid encoding a polypeptide shown as any one of SEQ ID NOS: 5, 6, 8, 11, 12, 2, 14, 16, 18, 20, and 22, is determined.

In a preferred embodiment, the methods of treating an IBD comprise (a) determining that the expression level of (i) one or more nucleic acids encoding one or more polypeptides selected from Table 1; or (ii) RNA transcripts or expression products thereof of one or more genes selected from Table 1 in a test sample obtained from the subject is lower relative to the level of expression in a control, wherein the lower level of expression is indicative of the presence of an IBD in the subject from which the test sample was obtained; and (b) administering to said subject an effective amount of an IBD therapeutic agent. In all embodiments, the lower level of expression of a nucleic acid encoding a polypeptide shown as any one of SEQ ID NOS: 5, 6, 8, 11, 12, 2, 14, and 16 is determined.

In another preferred embodiment, the methods of treating an IBD comprise (a) determining that the expression level of (i) one or more nucleic acids encoding one or more polypeptides selected from Table 1; or (ii) RNA transcripts or expression products thereof of one or more genes selected from Table 1 in a test sample obtained from the subject is higher relative to the level of expression in a control, wherein the higher level of expression is indicative of the presence of an IBD in the subject from which the test sample was obtained. In all embodiments, the higher level of expression of a nucleic acid encoding a polypeptide shown as any one of SEQ ID NOS: 18, 20, and 22, is determined.

In some preferred embodiments, the IBD therapeutic agent is one or more of an aminosalicylate, a corticosteroid, and an immunosuppressive agent.

In one aspect, the panel of IBD markers discussed above is useful in methods of treating an IBD in a mammalian subject. In one embodiment, the mammalian subject is screened against the panel of markers and if the presence of an IBD is determined, IBD therapeutic agent(s) may be administered as discussed herein.

In a different aspect the invention concerns a kit comprising one or more of (1) extraction buffer/reagents and protocol; (2) reverse transcription buffer/reagents and protocol; and (3) qPCR buffer/reagents and protocol suitable for performing the methods of this invention. The kit may comprise data retrieval and analysis software.

In one embodiment, the gene whose differential expression is indicative of an IBD is one or more of: CCL23, CXCL13, IRTA1, ATG16L1, ATG4D, ATG3, ATG12, ATG16L2, LC3B, or any combination thereof.

These and further embodiments of the present invention will be apparent to those of ordinary skill in the art.

### Brief Description of Drawings

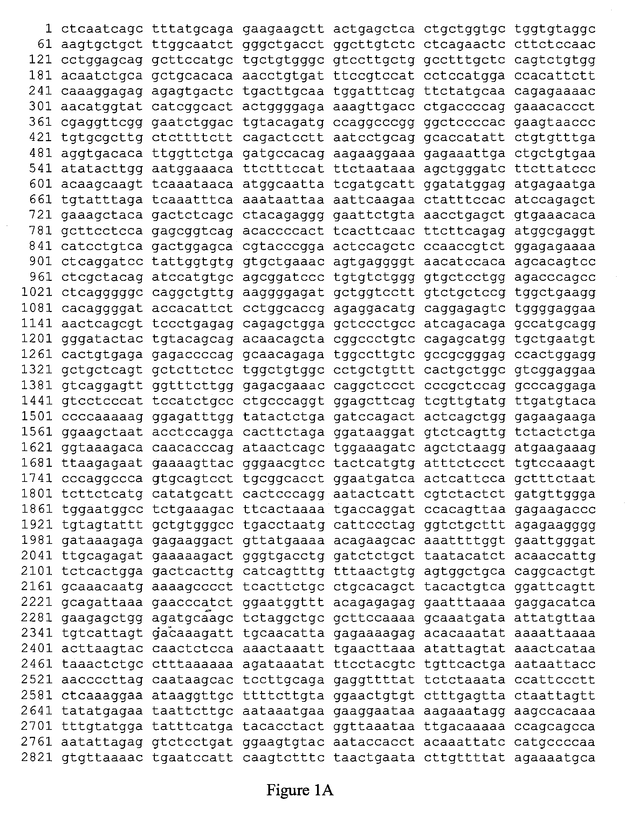

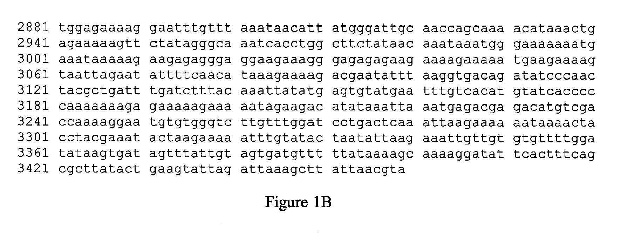

Figure 1 depicts the nucleic acid sequence (SEQ ID NO:1) encoding human IRTA1 polypeptide

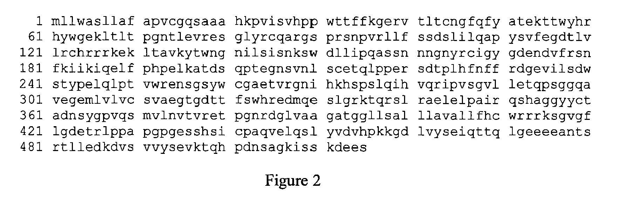

Figure 2 depicts the amino acid sequence (SEQ ID NO:2) encoded by the nucleic acid sequence of Figure 1.

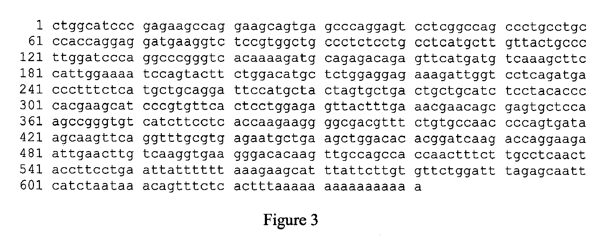

Figure 3 depicts the nucleic acid sequence (SEQ ID NO:3) encoding the CKbeta8-1 transcript of the human CCL23 polypeptide.

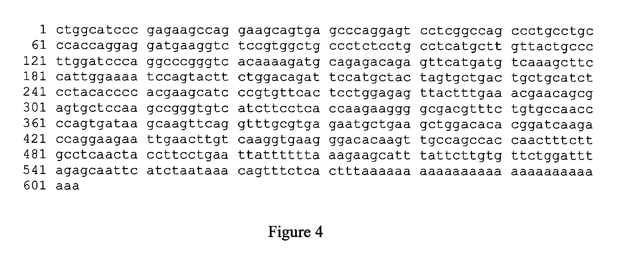

Figure 4 depicts the nucleic acid sequence (SEQ ID NO:4) encoding the CKbeta8 transcript of the human CCL23 polypeptide.

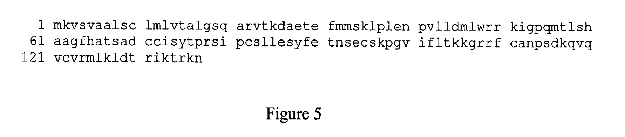

Figure 5 depicts the amino acid sequence (SEQ ID NO:5) encoded by the nucleic acid sequence of Figure 3.

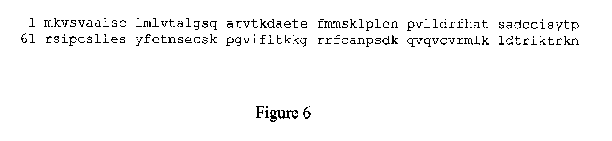

Figure 6 depicts the amino acid sequence (SEQ ID NO:6) encoded by the nucleic acid sequence of Figure 4.

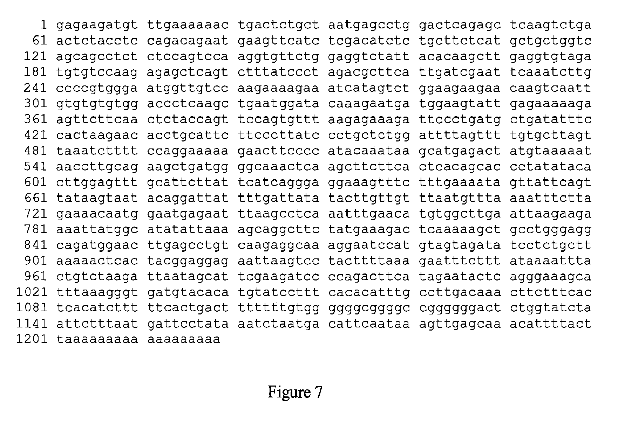

Figure 7 depicts the nucleic acid sequence (SEQ ID NO:7) encoding human CXCL13 polypeptide.

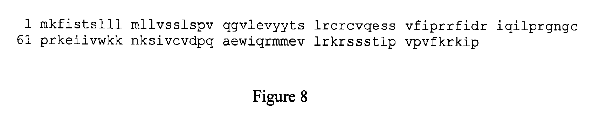

Figure 8 depicts the amino acid sequence (SEQ ID NO:8) encoded by the nucleic acid sequence of Figure 7.

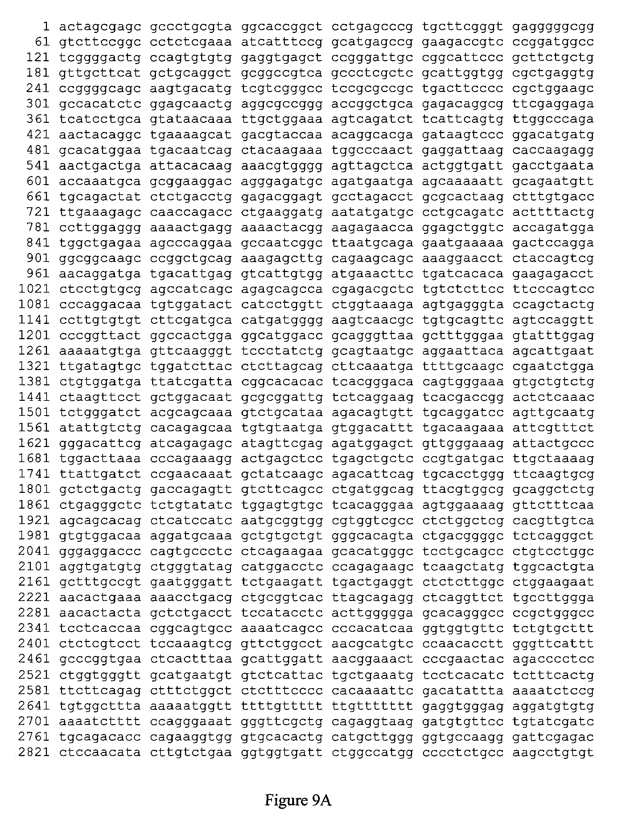

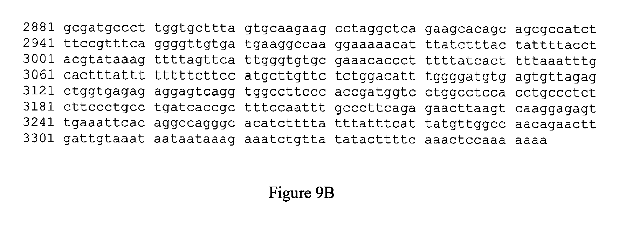

Figure 9 depicts the nucleic acid sequence (SEQ ID NO:9) encoding human ATG16L1 polypeptide (isoform 2).

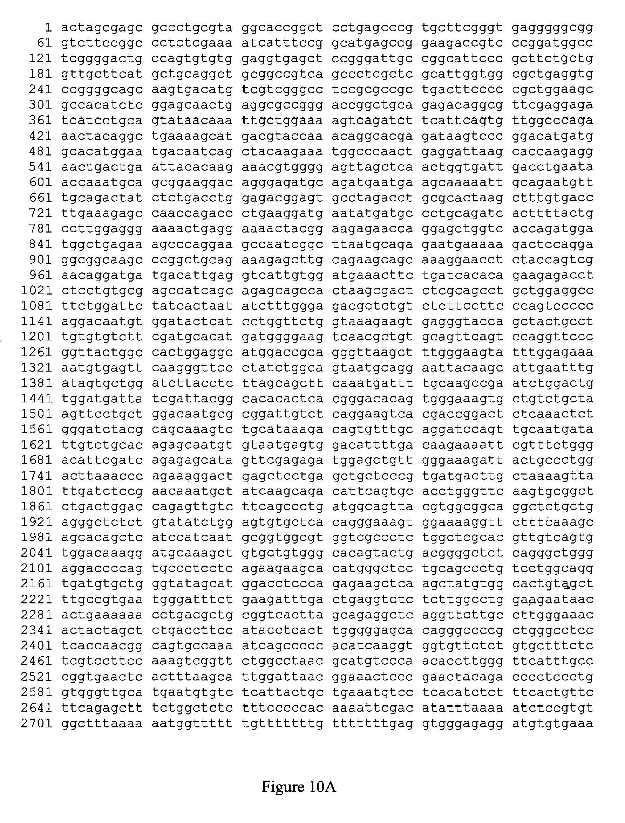

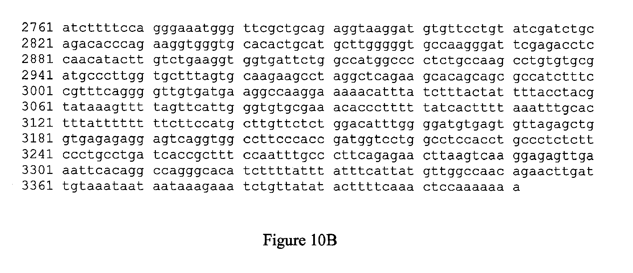

Figure 10 depicts the nucleic acid sequence (SEQ ID NO:10) encoding human ATG16L1 polypeptide (isoform 1).

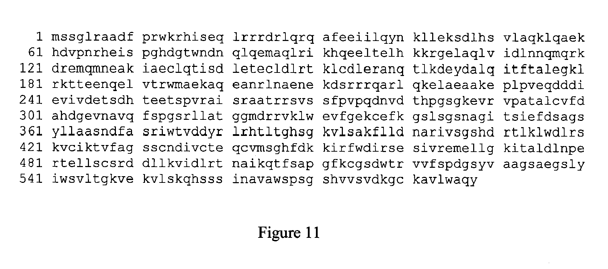

Figure 11 depicts the amino acid sequence (SEQ ID NO:11) encoded by the nucleic acid sequence of Figure 9.

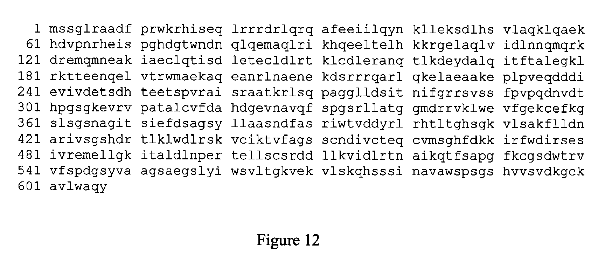

Figure 12 depicts the amino acid sequence (SEQ ID NO:12) encoded by the nucleic acid sequence of Figure 10.

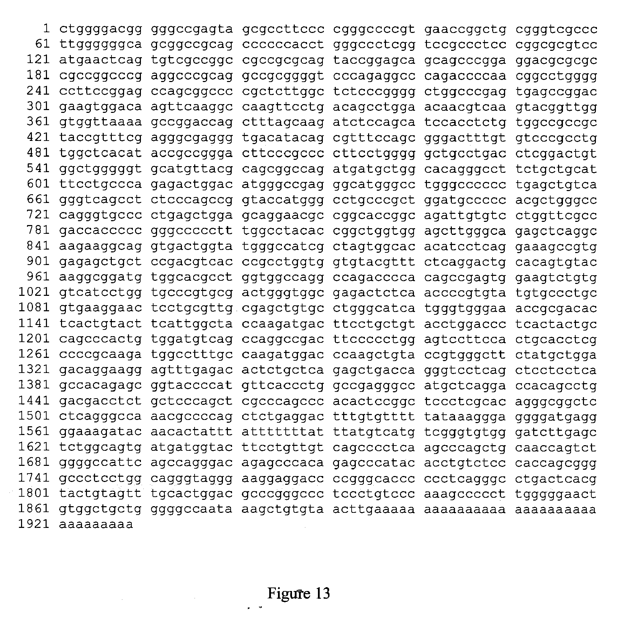

Figure 13 depicts the nucleic acid sequence (SEQ ID NO:13) encoding human ATG4D polypeptide.

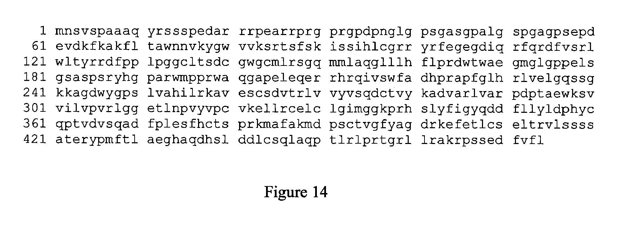

Figure 14 depicts the amino acid sequence (SEQ ID NO:14) encoded by the nucleic acid sequence of Figure 13.

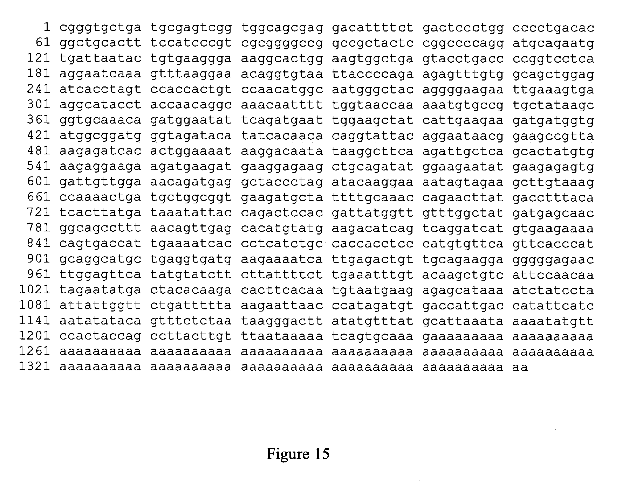

Figure 15 depicts the nucleic acid sequence (SEQ ID NO:15) encoding human ATG3 polypeptide.

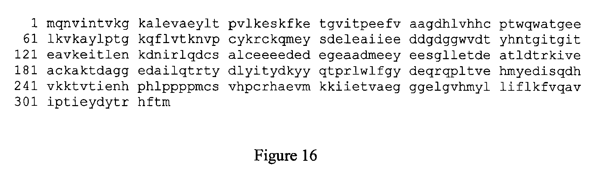

Figure 16 depicts the amino acid sequence (SEQ ID NO:16) encoded by the nucleic acid sequence of Figure 15.

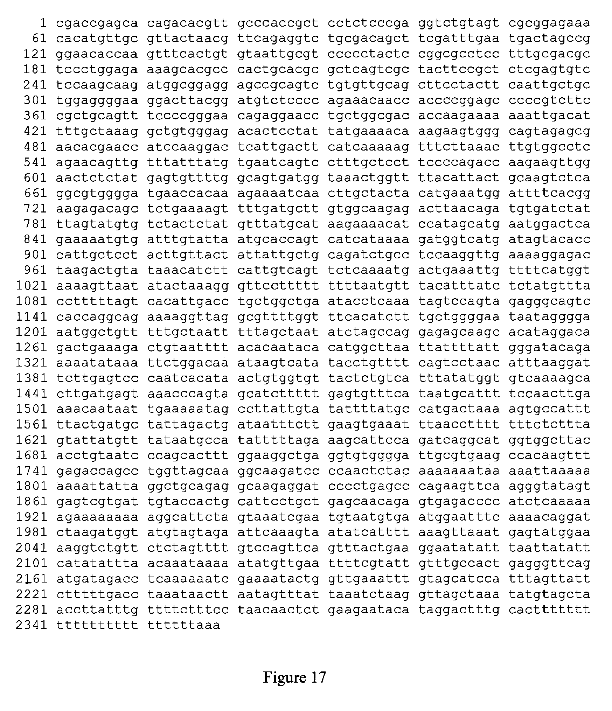

Figure 17 depicts the nucleic acid sequence (SEQ ID NO:17) encoding human ATG12 polypeptide.

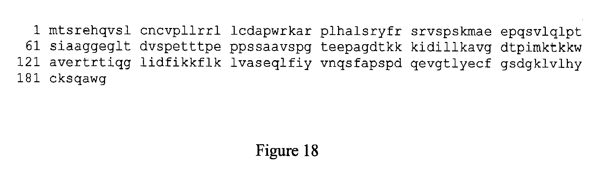

Figure 18 depicts the amino acid sequence (SEQ ID NO:18) encoded by the nucleic acid sequence of Figure 17.

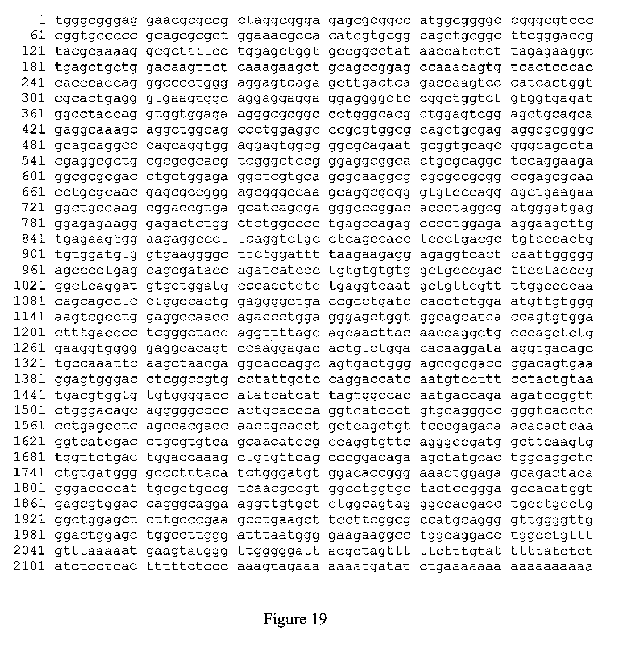

Figure 19 depicts the nucleic acid sequence (SEQ ID NO:19) encoding human ATG16L2 polypeptide.

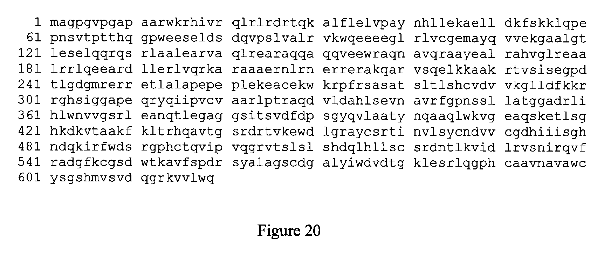

Figure 20 depicts the amino acid sequence (SEQ ID NO:20) encoded by the nucleic acid sequence of Figure 19.

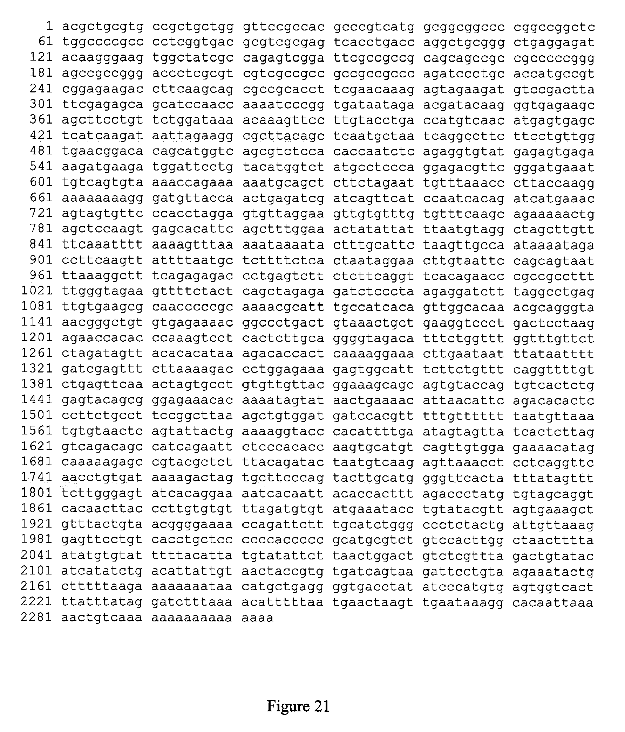

Figure 21 depicts the nucleic acid sequence (SEQ ID NO:21) encoding human LC3B polypeptide.

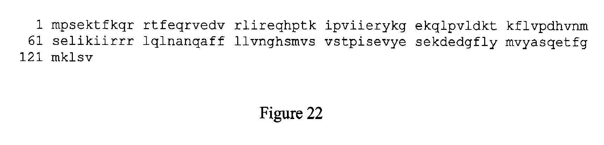

Figure 22 depicts the amino acid sequence (SEQ ID NO:22) encoded by the nucleic acid sequence of Figure 21.

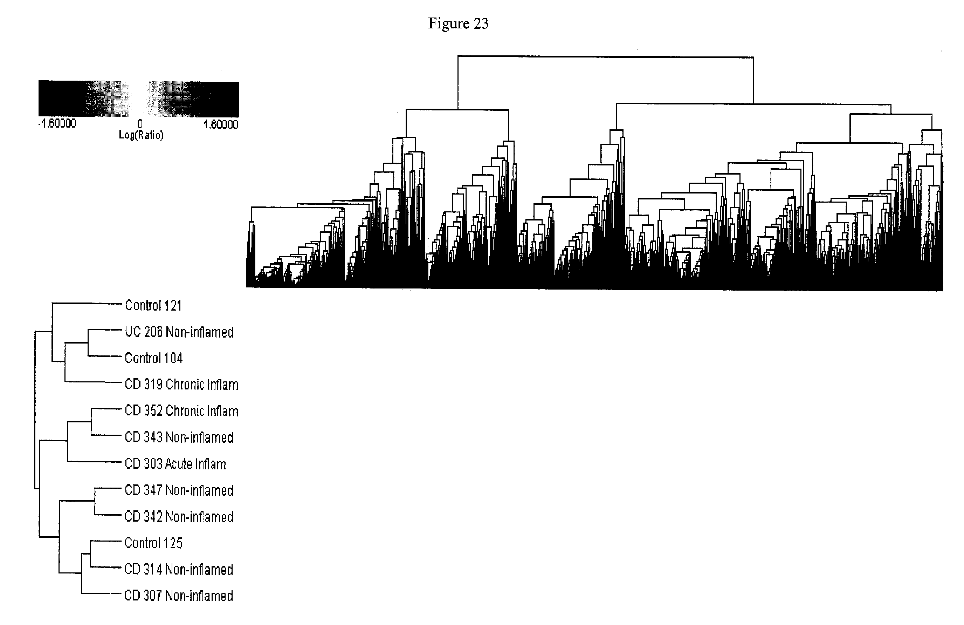

Figure 23 illustrates hierarchical clustering of terminal ileal biopsies from females with Crohn's disease and controls. The data comprises terminal ileal biopsies from 8 patients with CD, three healthy controls with normal terminal ileal pathology and one patient with UC who had normal terminal ileal pathology were clustered. The CD, UC and control patients are annotated with the inflammation status of the biopsy. The degree of upregulation measured in red and downregulation measured in blue can be quantified using the logarithmic key. Two areas appeared to be driving this separation and these have been highlighted in solid line oval- downregulated and dashed line oval - upregulated.

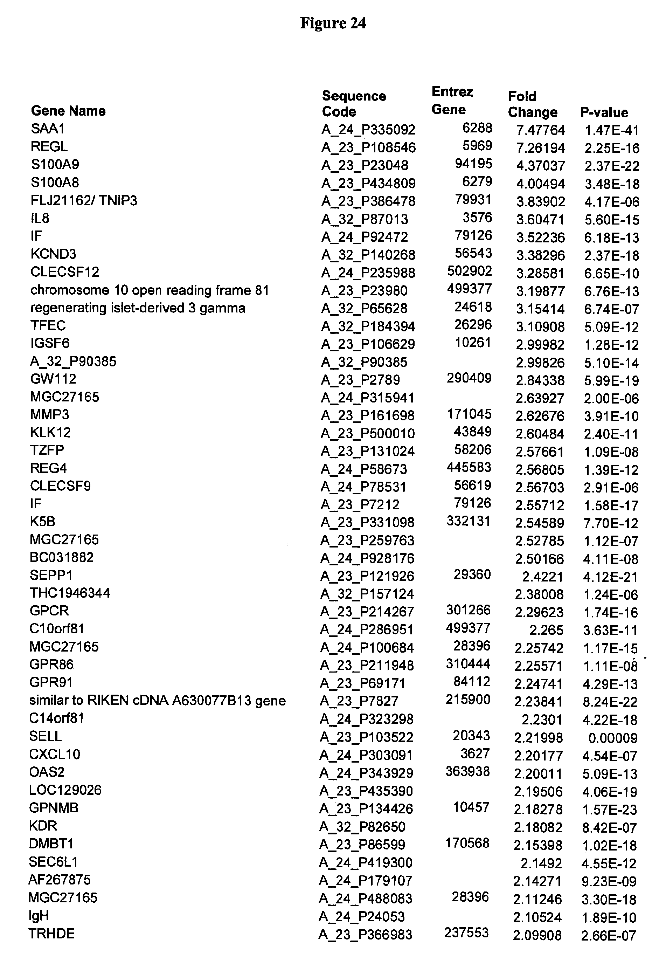

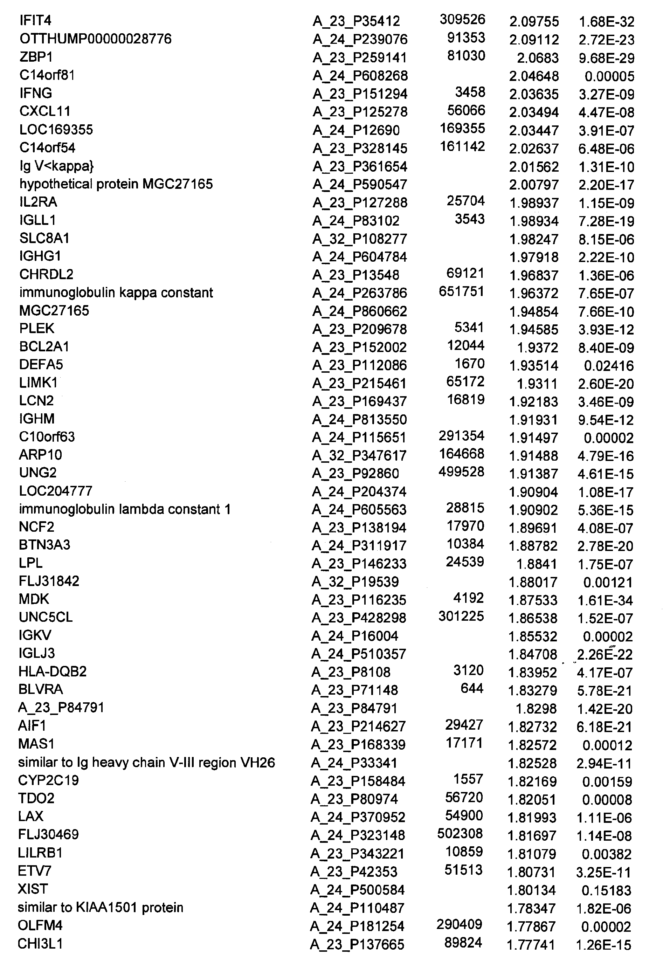

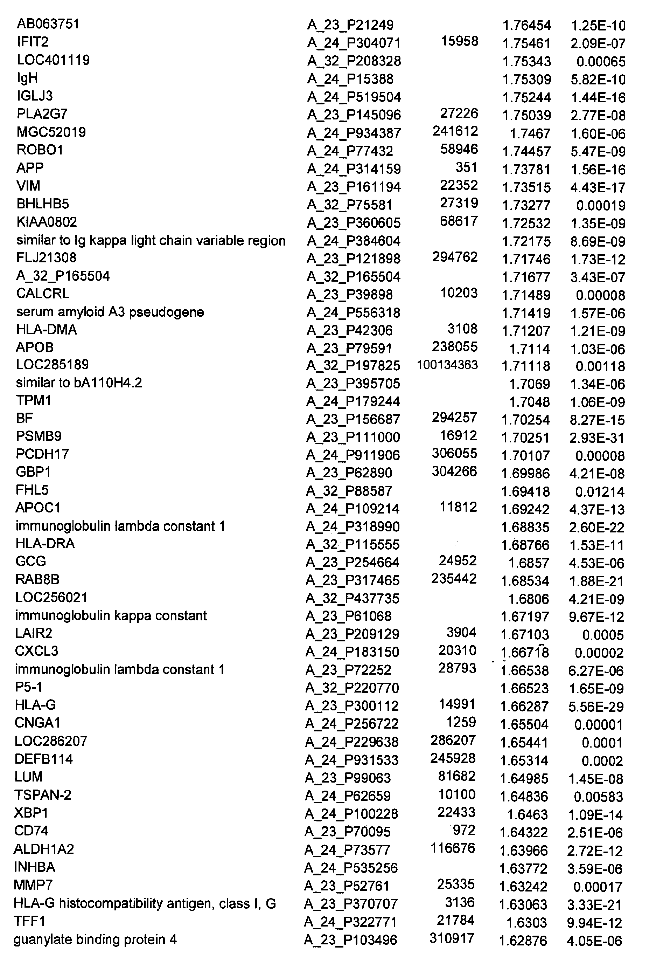

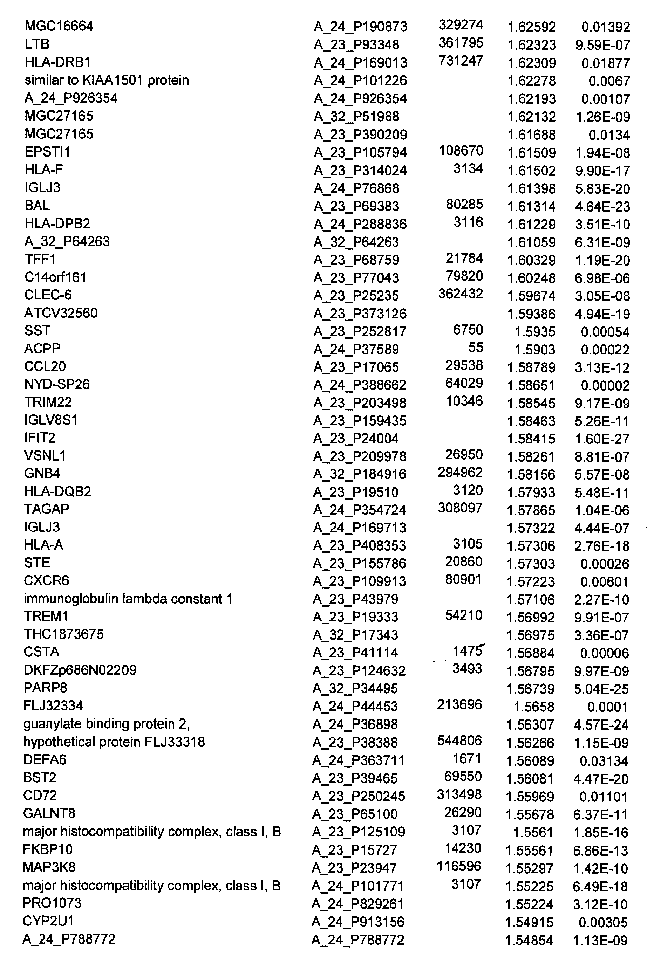

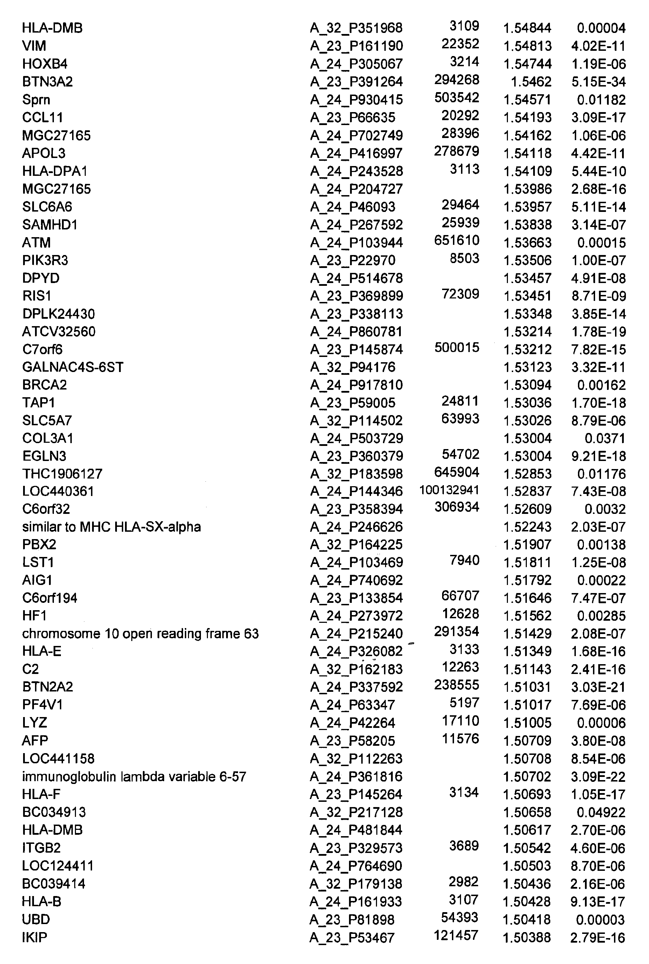

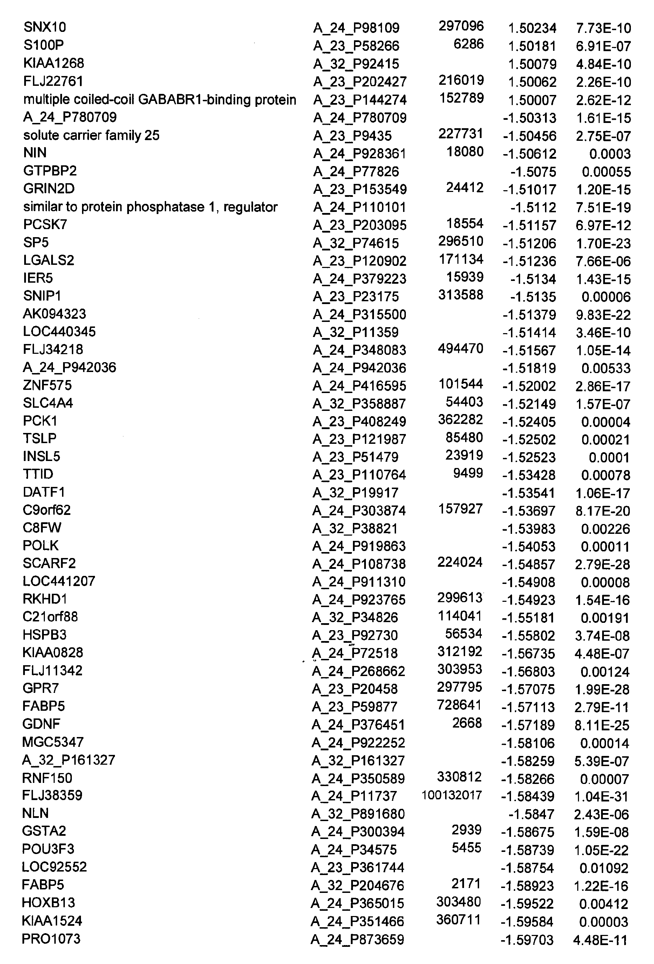

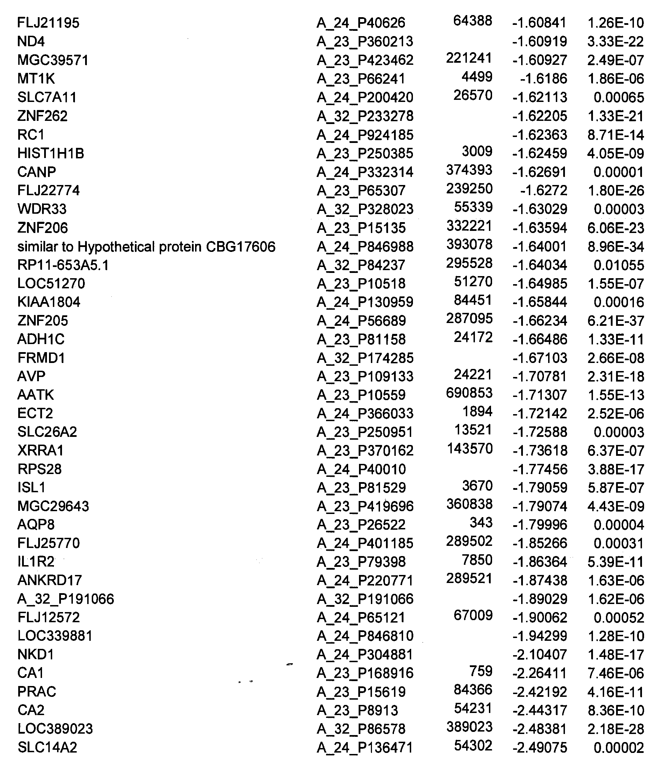

Figure 24 depicts fold changes in gene expression, comparing CD biopsies to controls. Gene Annotation: SAA1-serum amyloid A1, REGL- Rat regenerating islet-derivedlike human homolog, S100A8 & 9-calcium binding protein A8 and A9, TNIP3-TNFAIP3 interacting protein 3, IL-8- Interleukin 8, IF- I factor (complement), KCND3- Potassium voltage-gated channel (Shal-related subfamily) member 3, CLECSF12- C-type (calcium dependent, carbohydrate-recognition domain) lectin, regenerating islet-derived 3 gamma- Pancreatitis-associated protein 2, TFECTranscription factor EC, IGSF6- Immunoglobulin superfamily member 6, A_32_P90385- unknown, GW112- Olfactomedin-4 Precursor (OLM4), MGC27165-Protein containing four immunoglobulin (Ig) domains, MMP3- matrix metalloproteinase 3, KLK12-kallikrein 12, TZFP- testis zinc finger protein, REG4-regenerating islet-derived family, member 4, CLECSF9- C-t ype (calcium dependent, carbohydrate-recognition domain) lectin, superfamily member 9, IF- I factor (complement), AVP- Prepro-arginine vasopressin-neurophysin II, AATK- apoptosisassociated tyrosine kinase, ECT2- epithelial cell transforming sequence 2 oncogene, SLC26A2- solute carrier family 26, XRRA1- X-ray radiation resistance associated 1, RPS28- ribosomal protein S28, ISL1- Insulin gene enhancer protein 1, MGC29643-LY6/PLAUR domain containing 1, AQP8- aquaporin 8, FLJ25770- Hypothetical protein, ANKRD17- ankyrin repeat domain 17, A_32_P191066- Weakly similar to PN0099, FLJ12572- Hypothetical protein, LOC339881- similar to eukaryotic initiation factor 4B, NKD1-naked cuticle homolog 1, CA1 & 2- carbonic anhydrase 1 & 2, PRAC- Prostate, rectum and colon expressed gene protein, LOC389023-hypothetical gene, SLC14A2- solute carrier family 14.

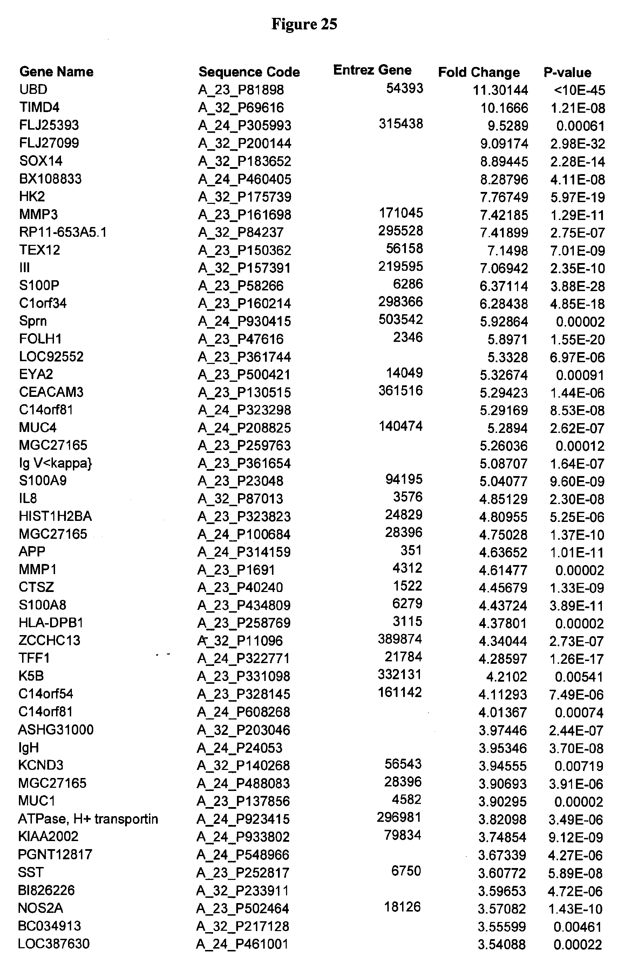

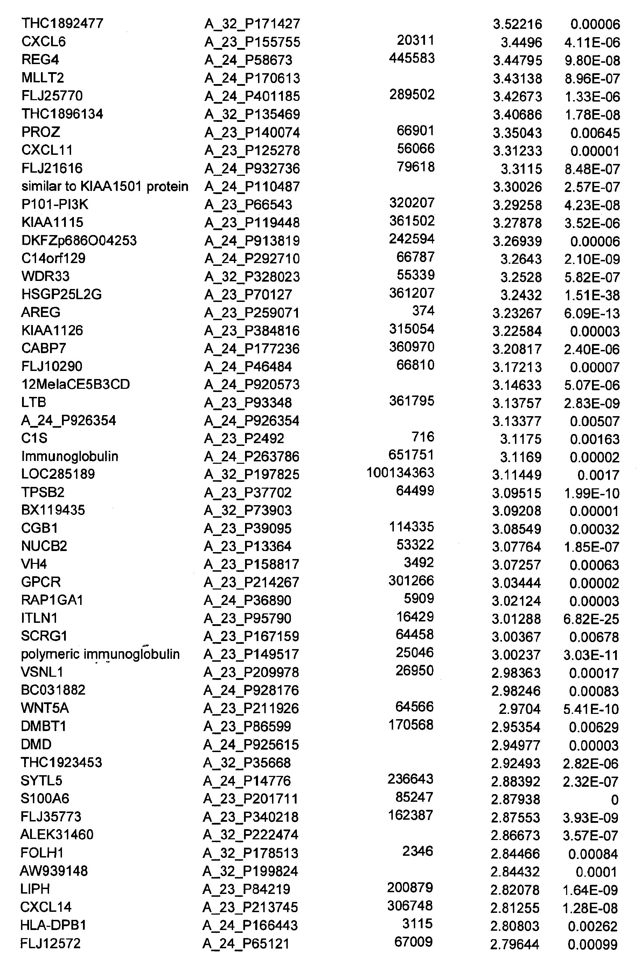

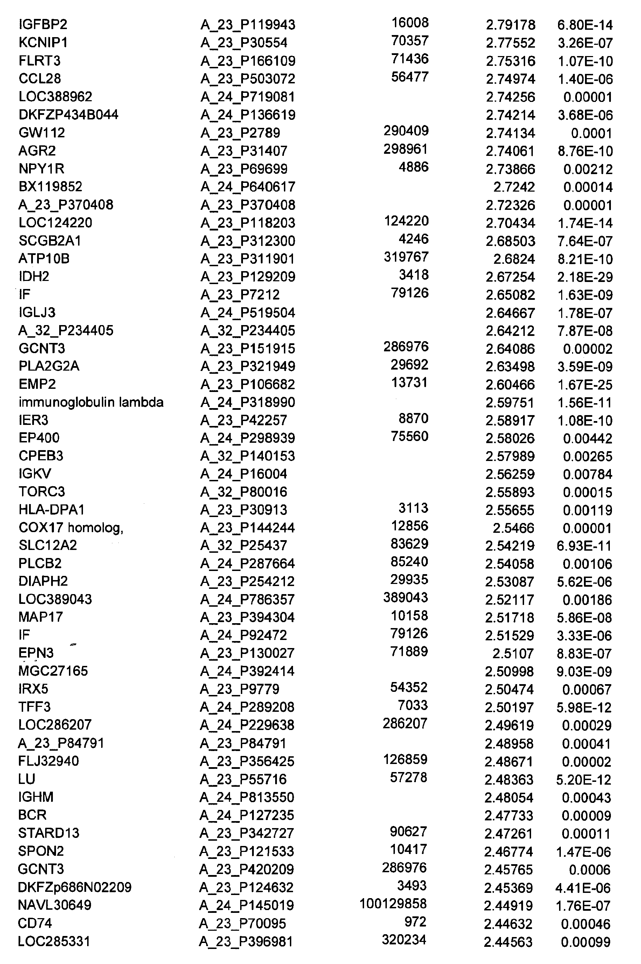

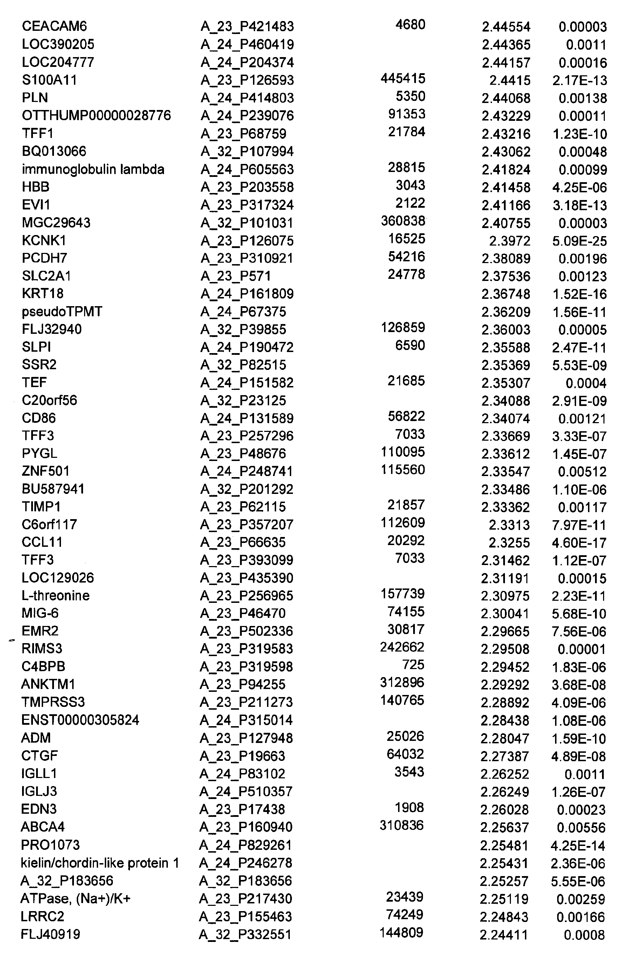

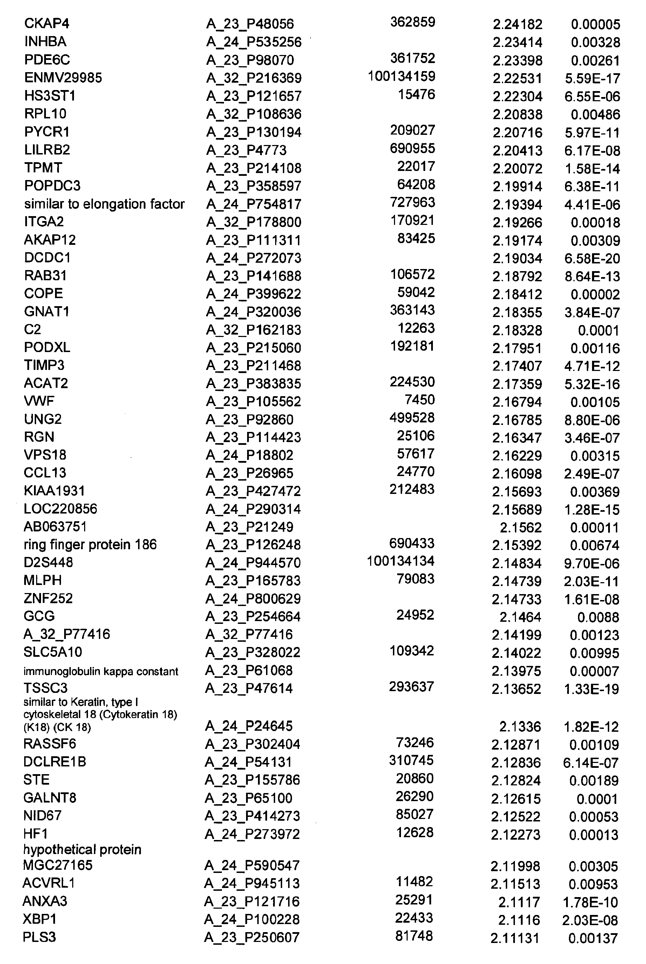

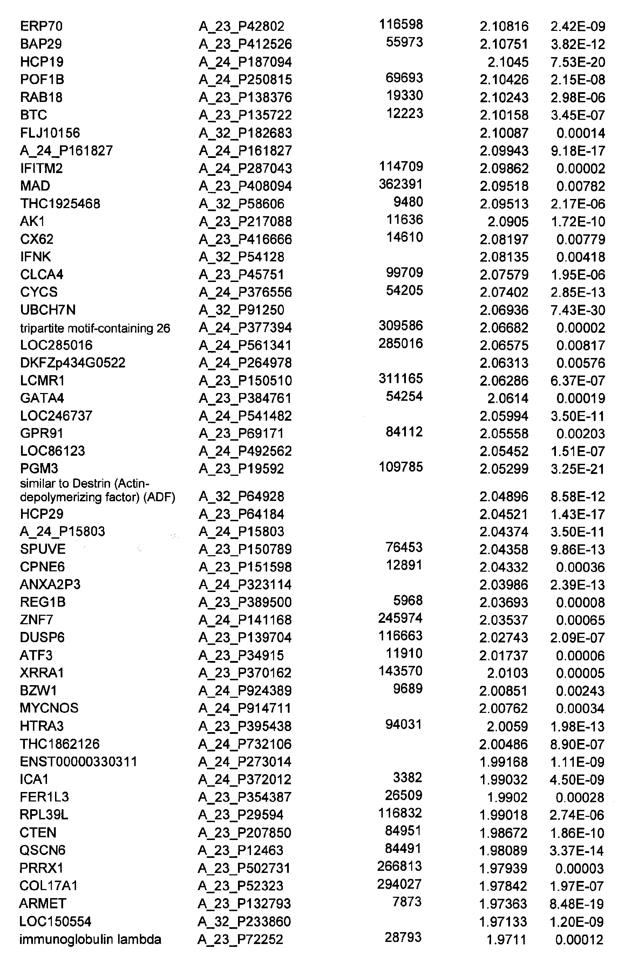

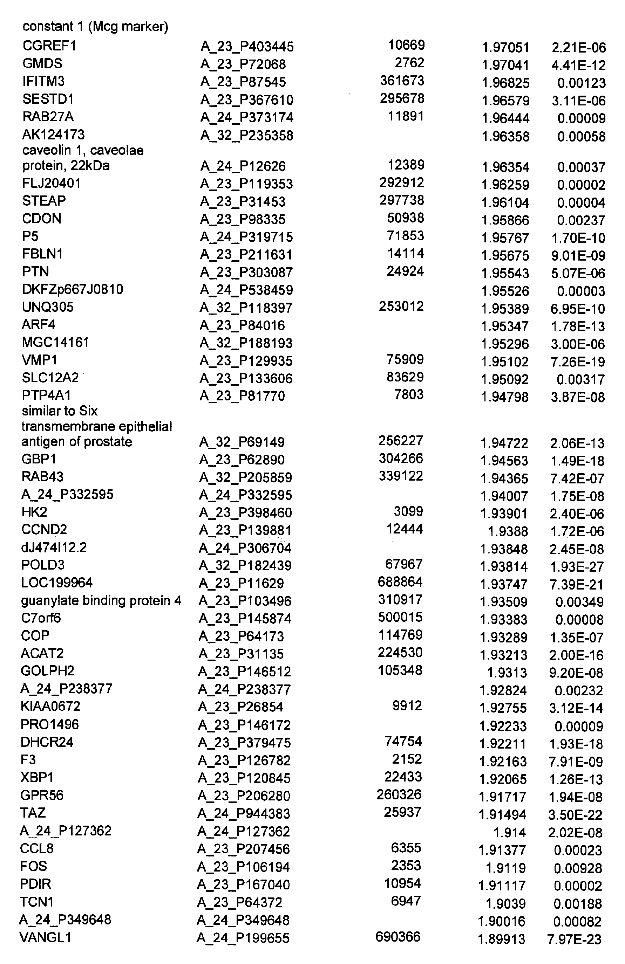

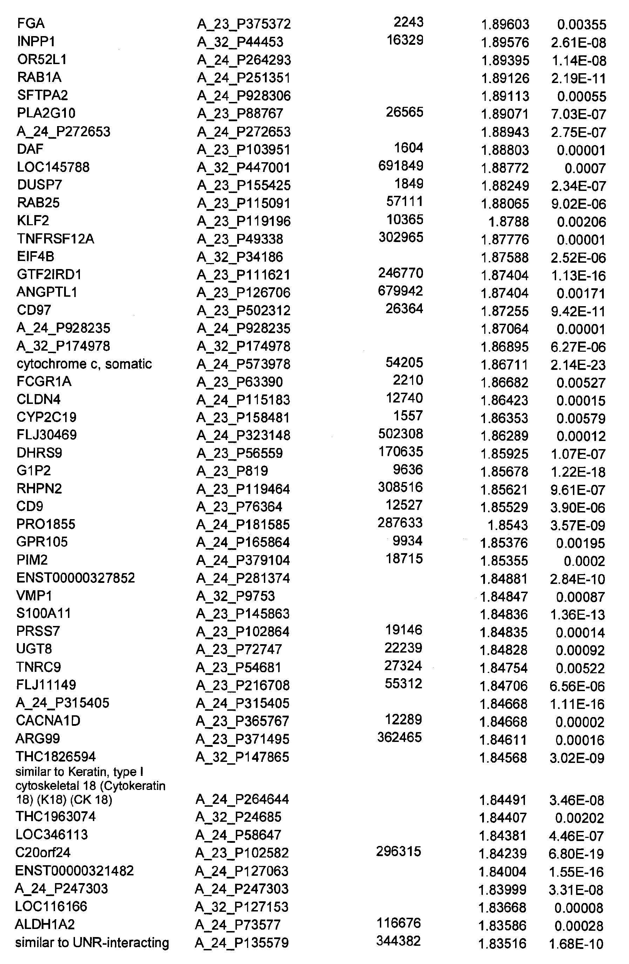

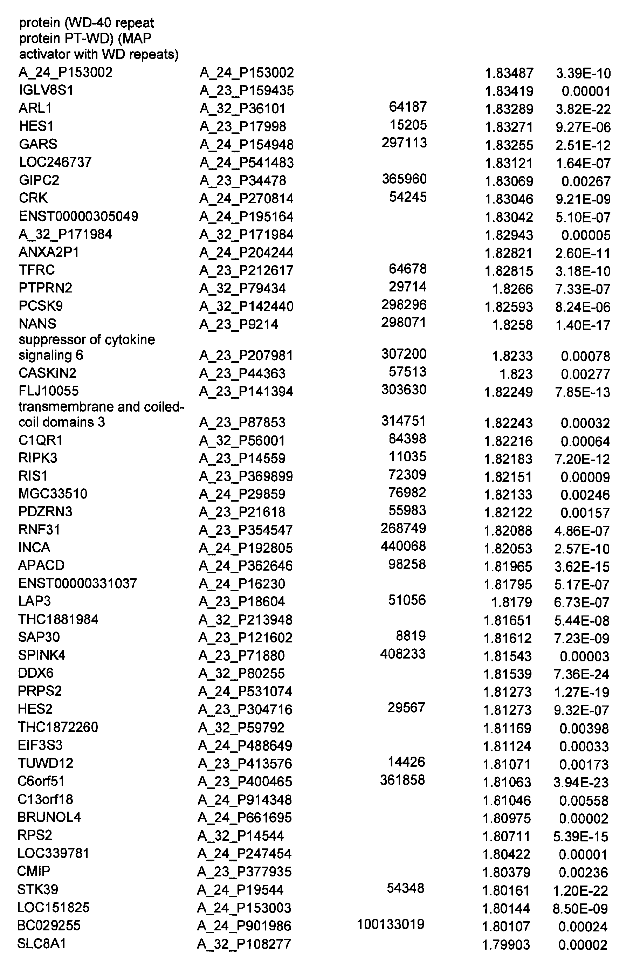

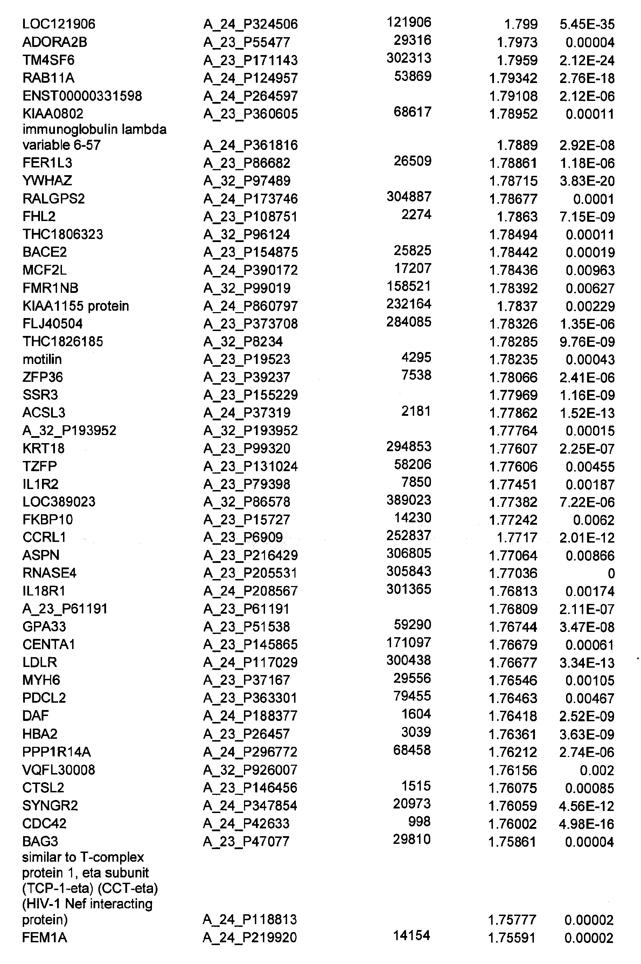

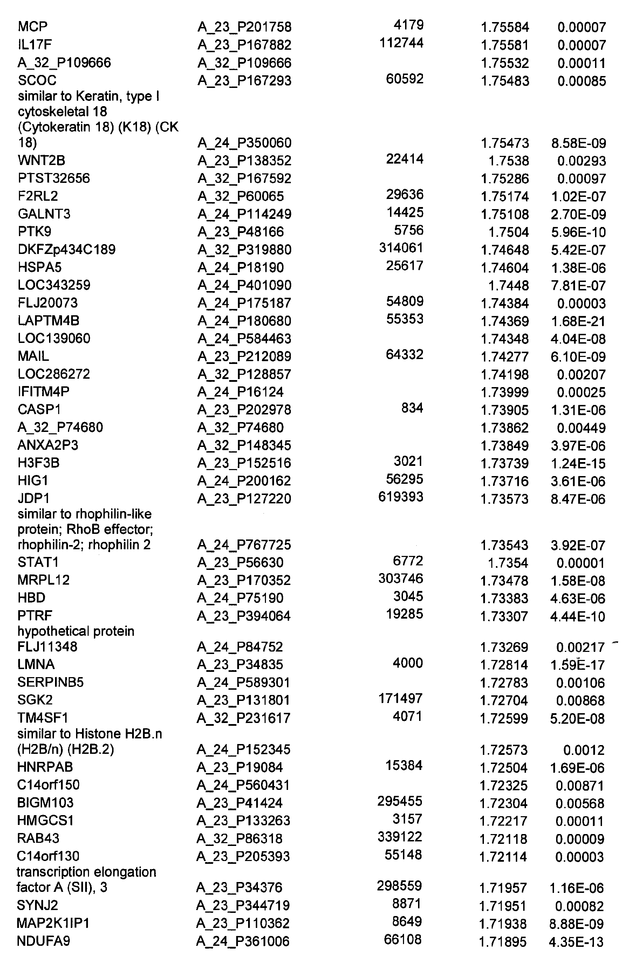

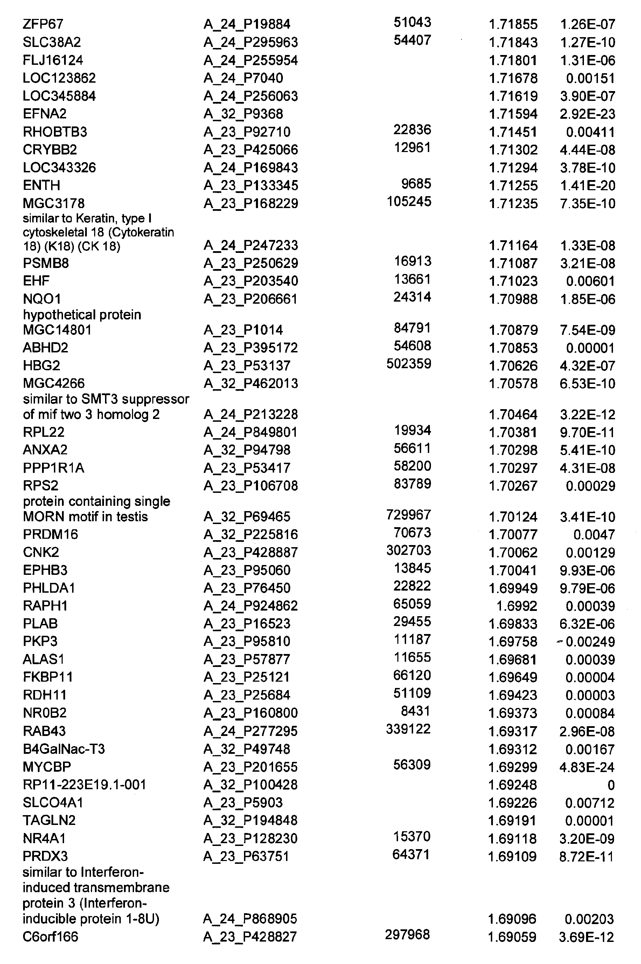

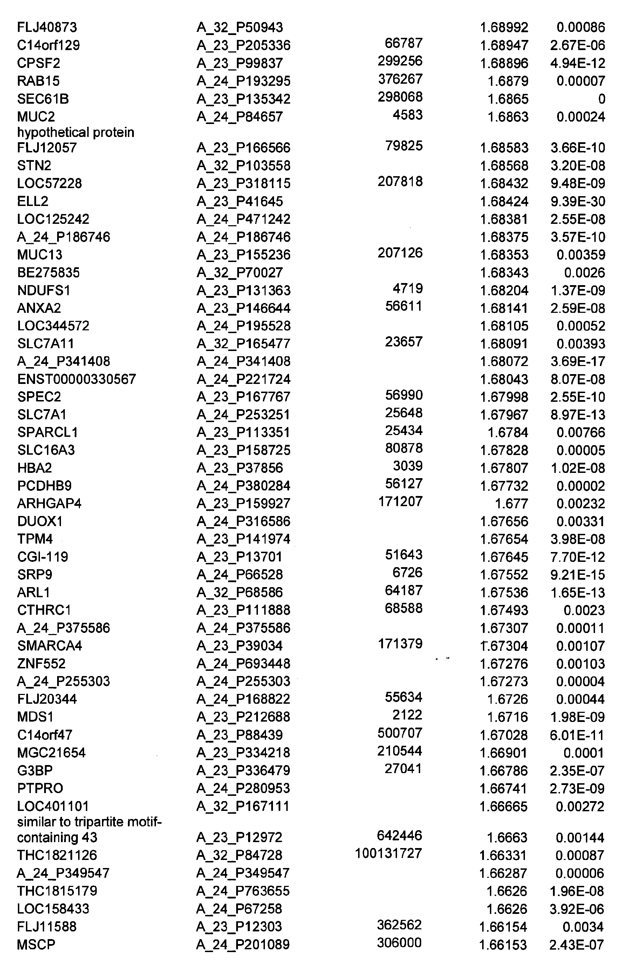

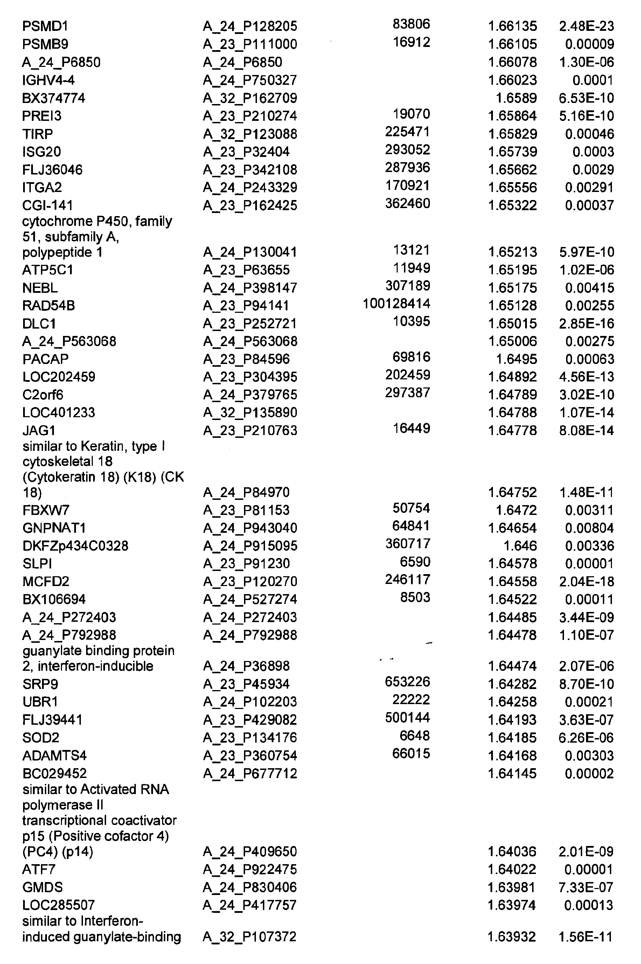

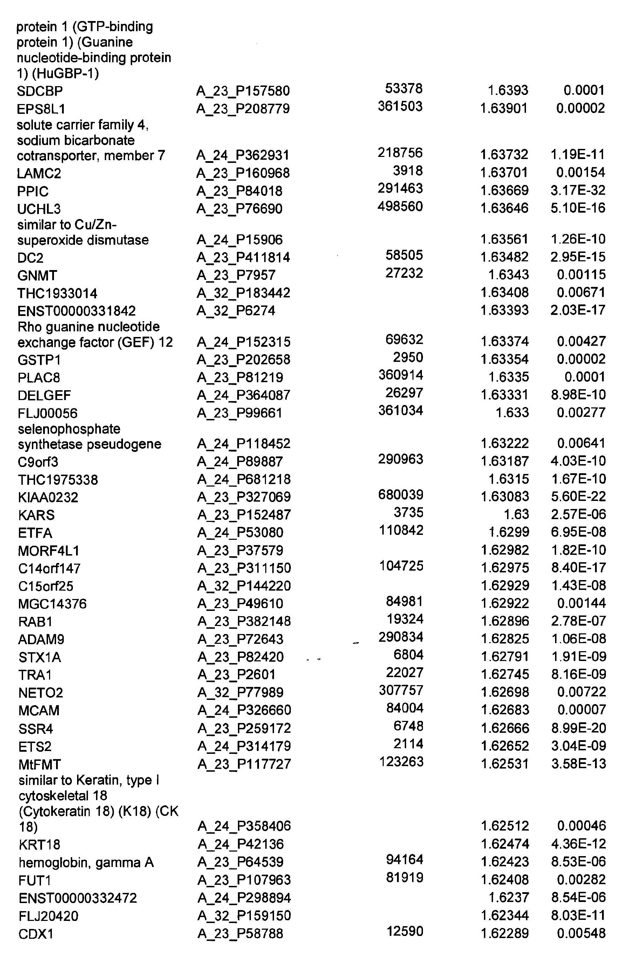

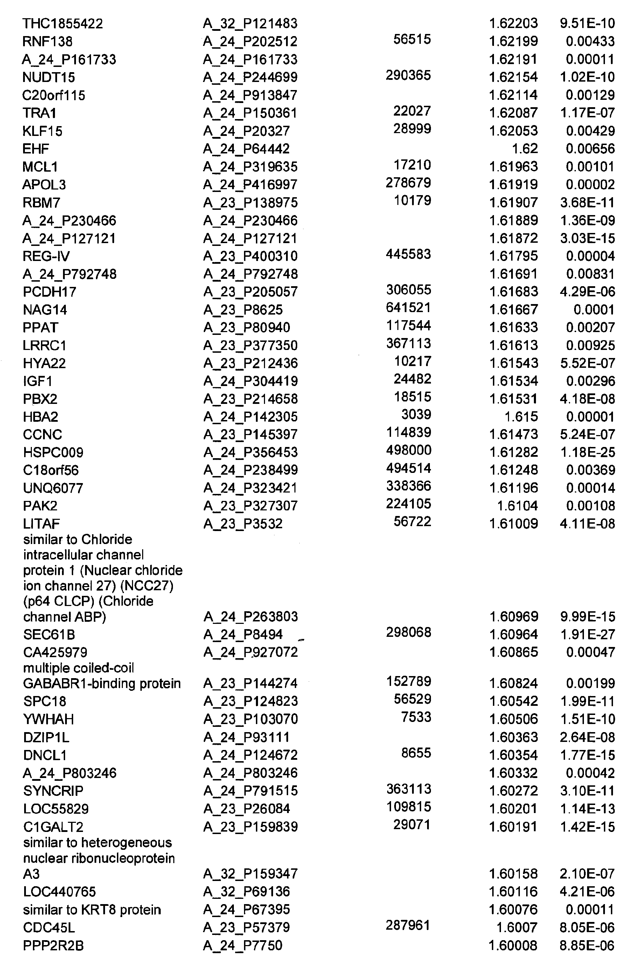

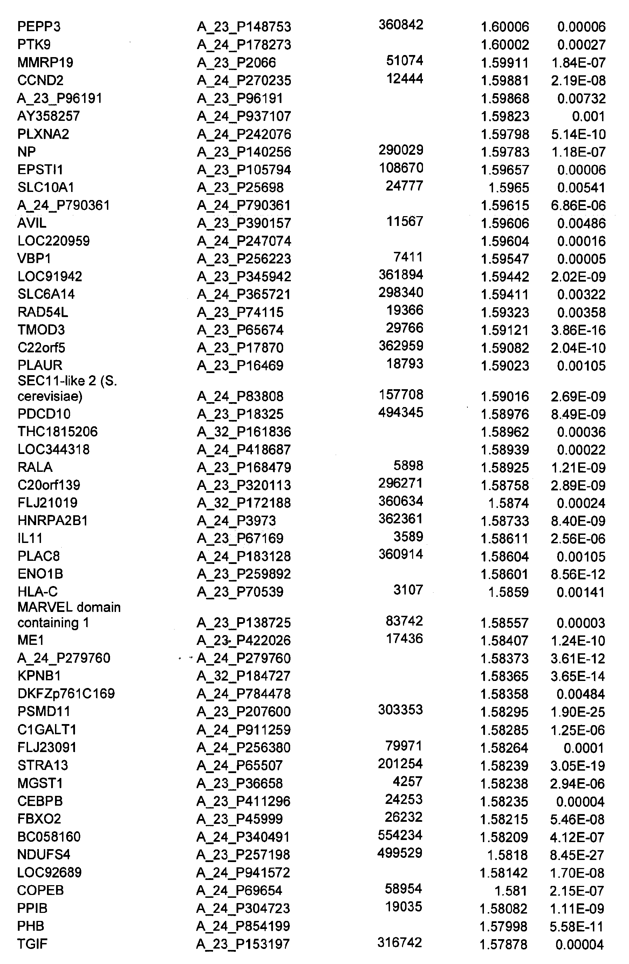

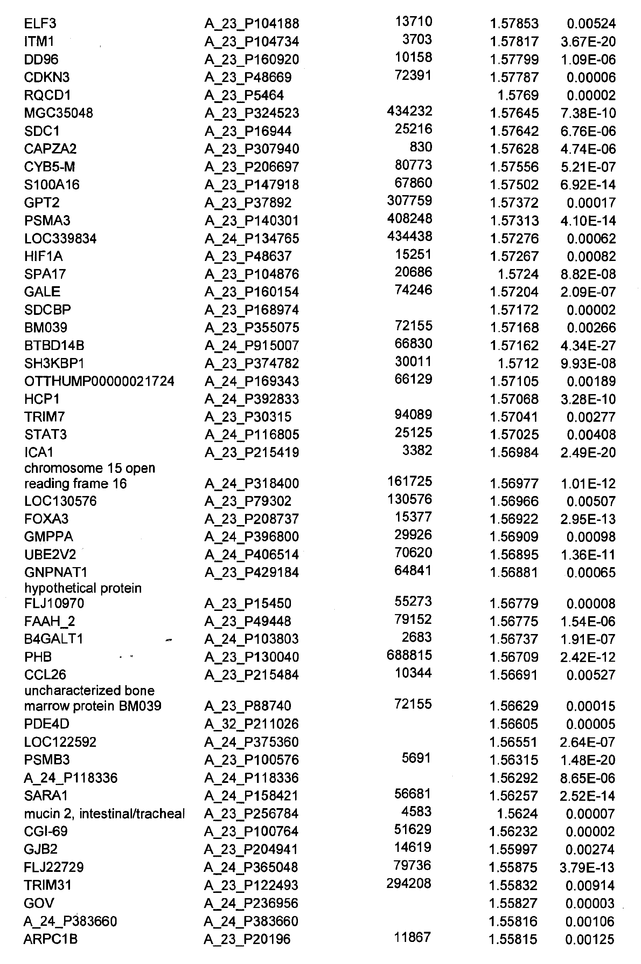

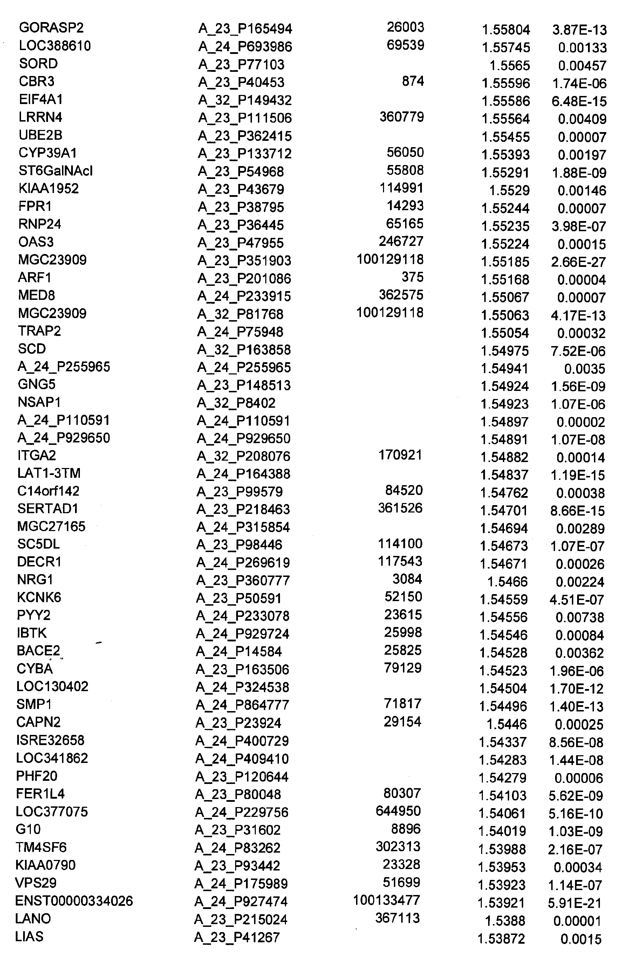

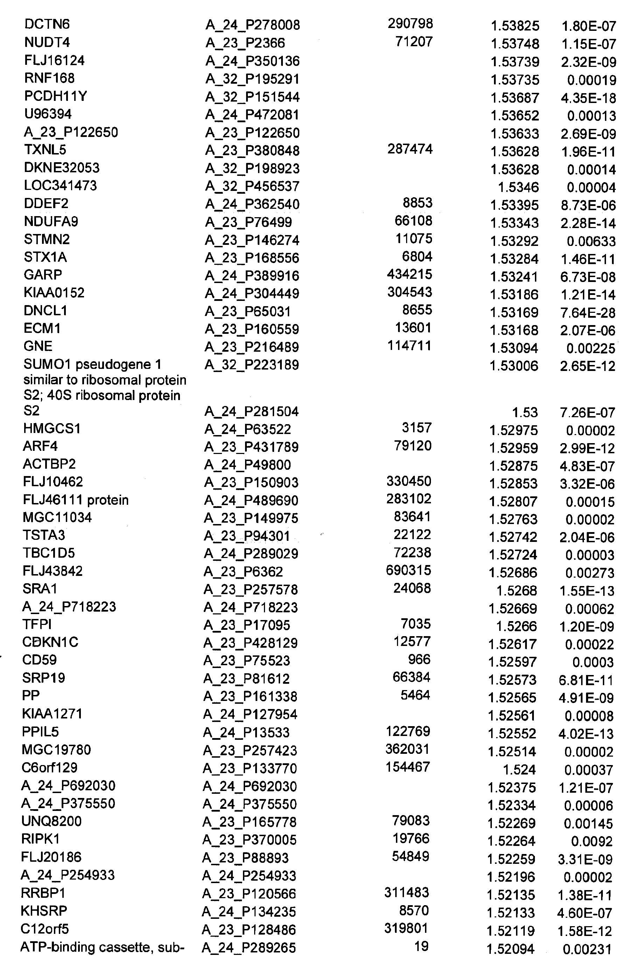

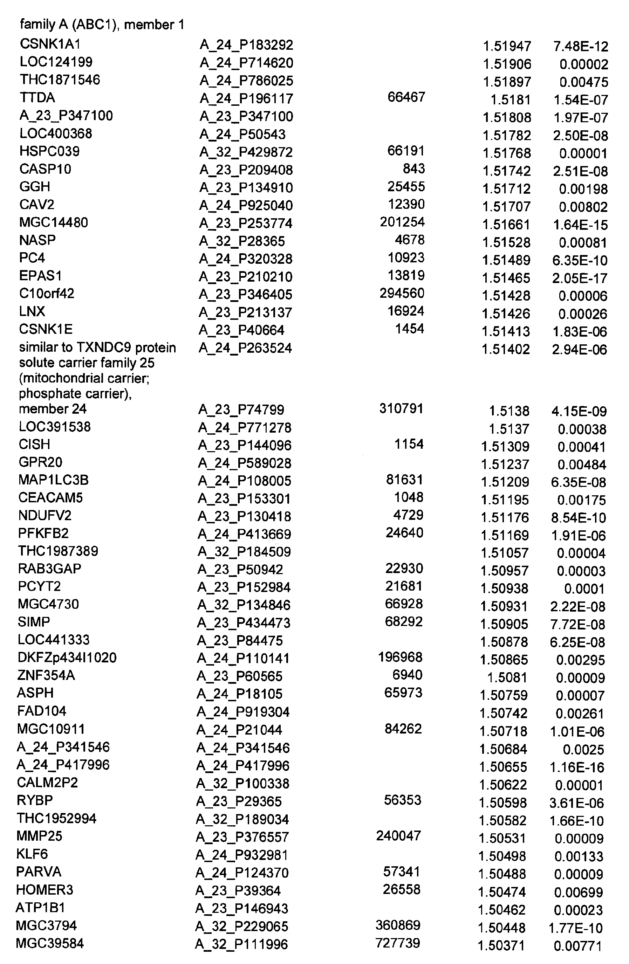

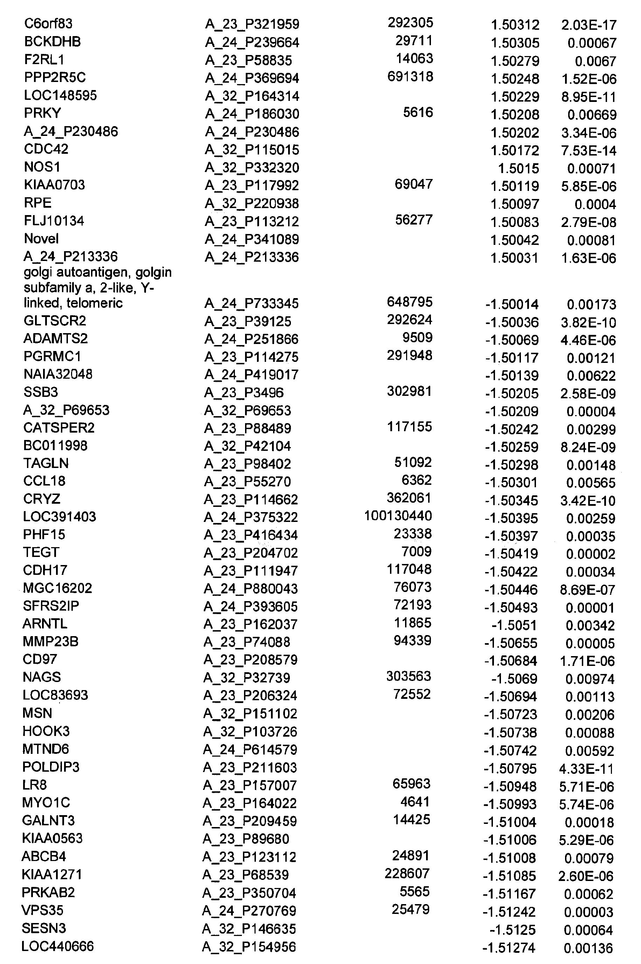

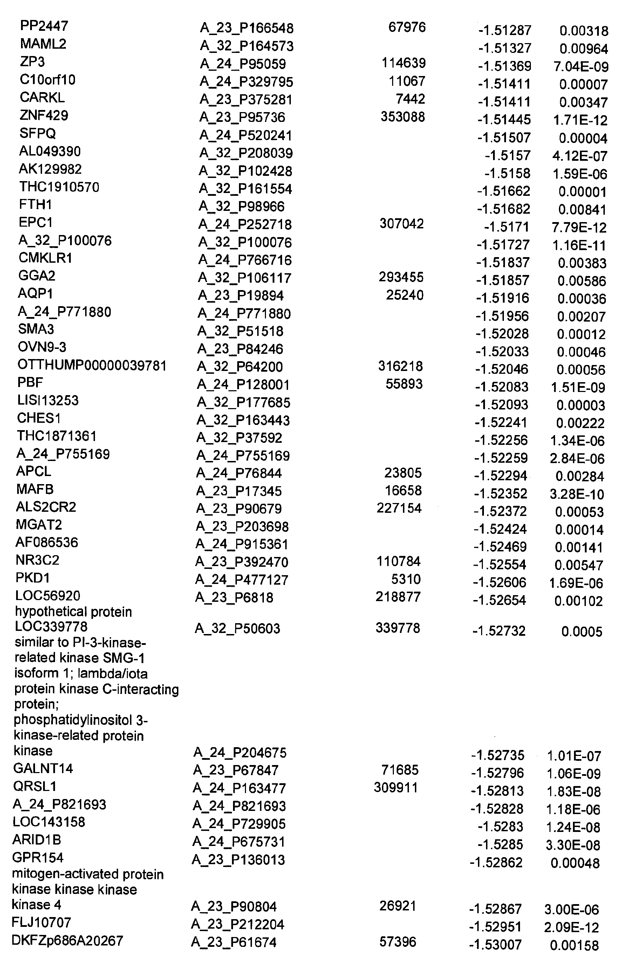

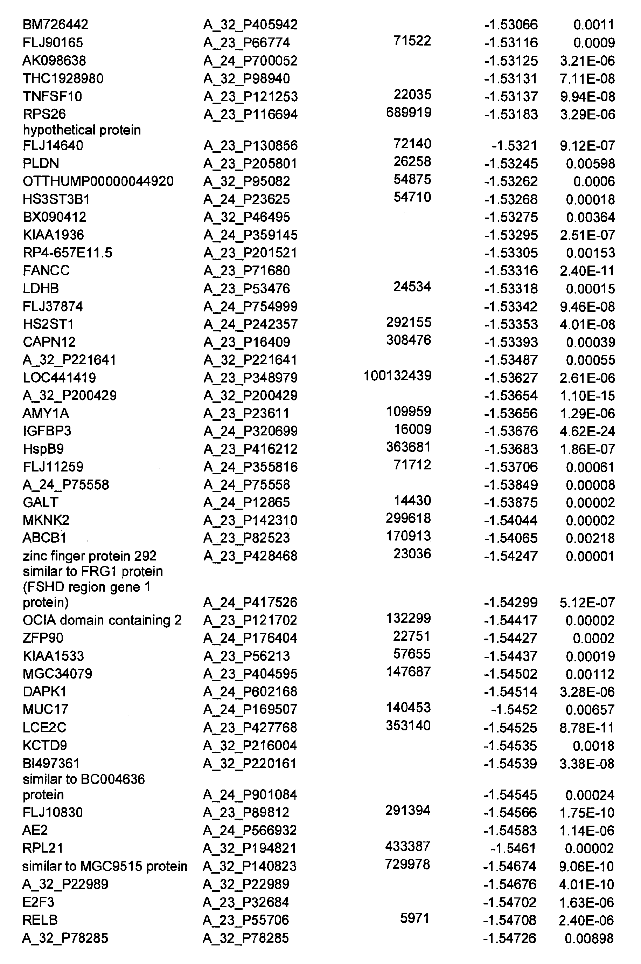

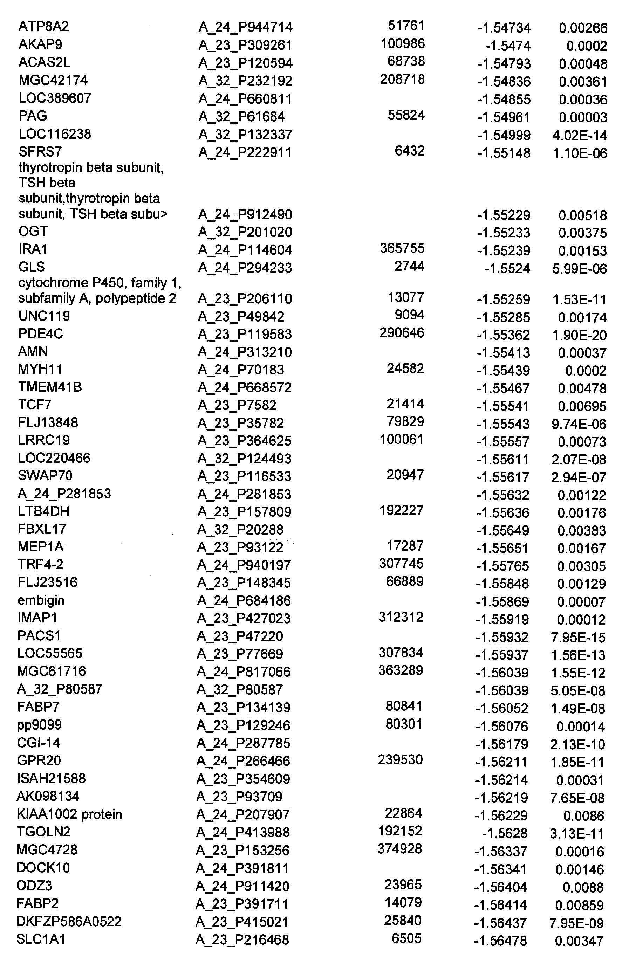

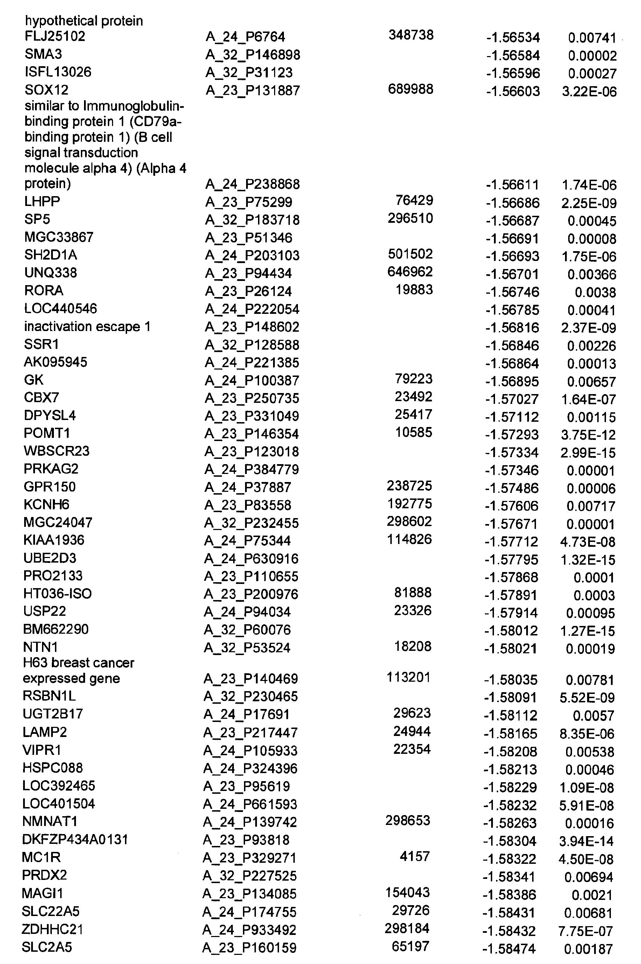

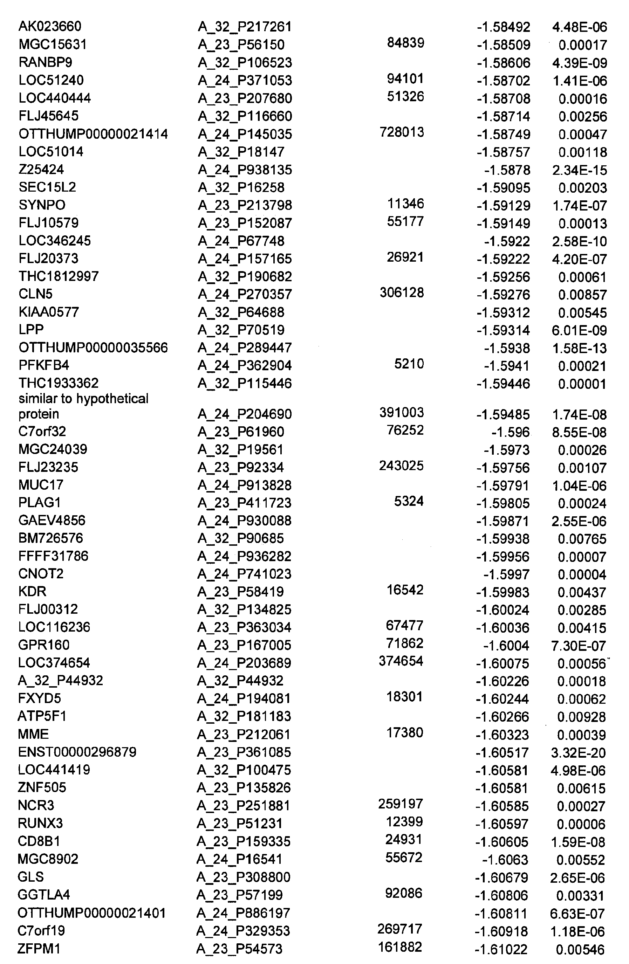

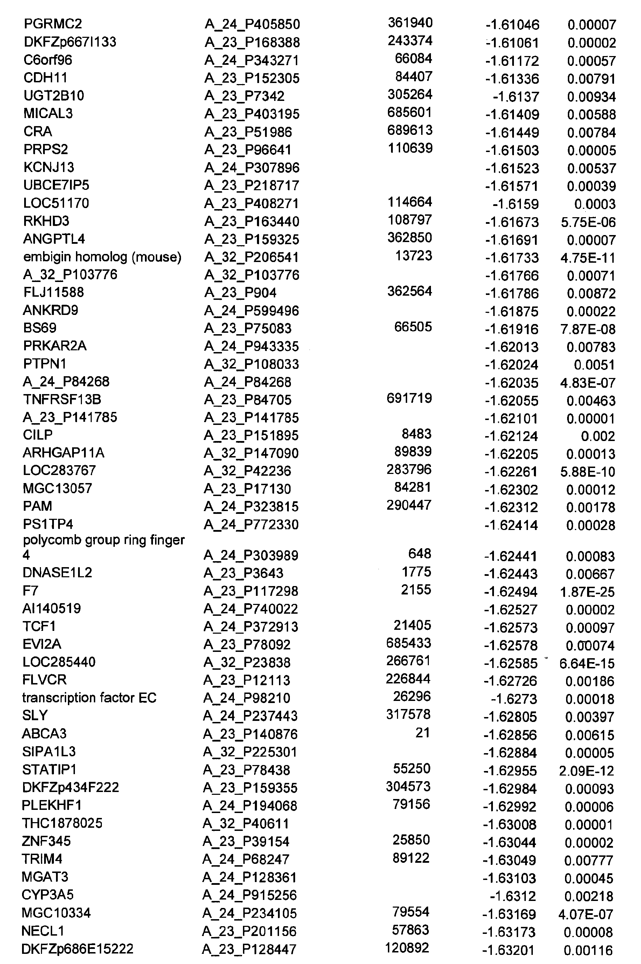

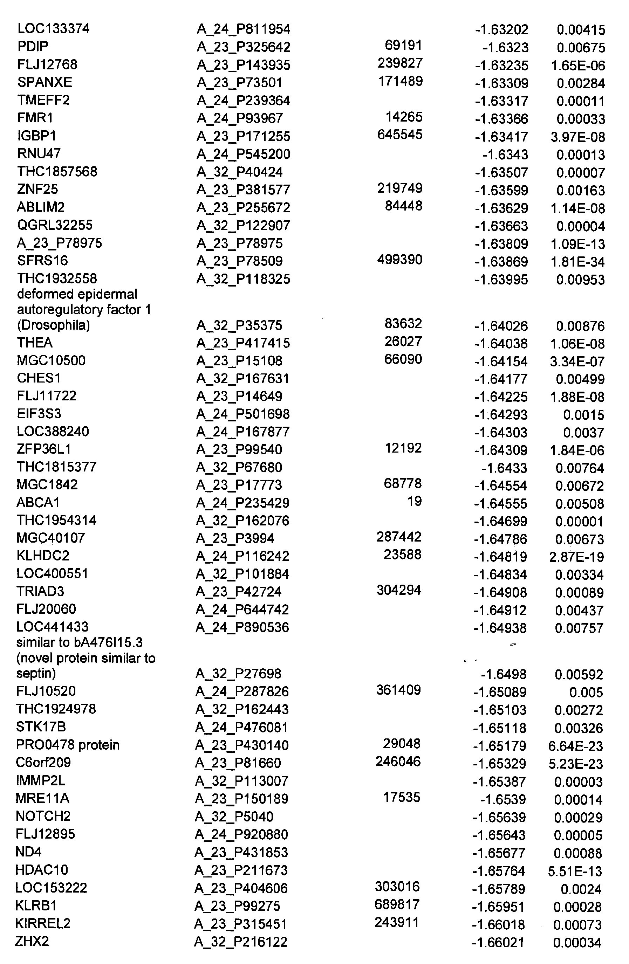

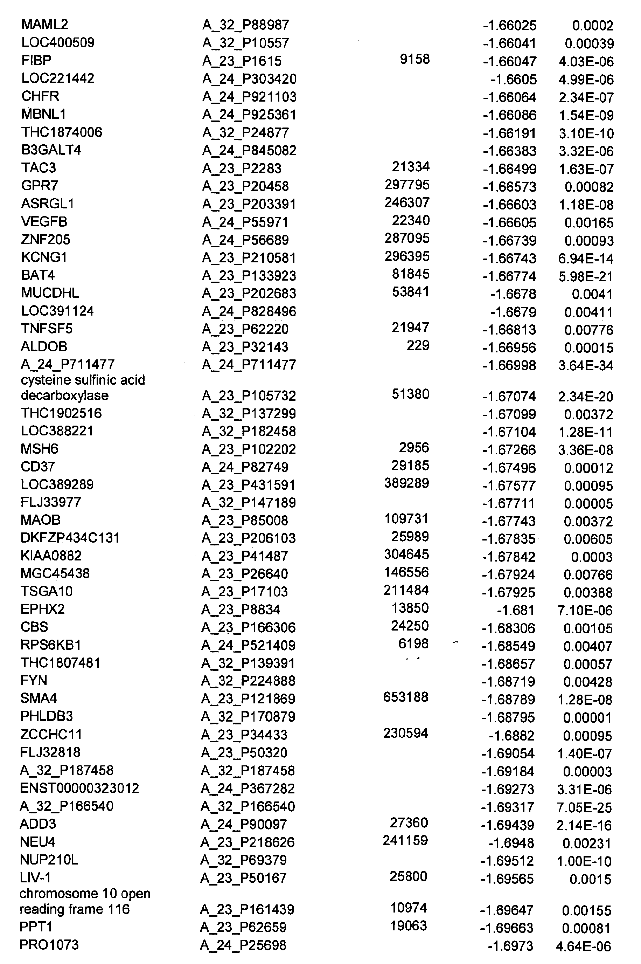

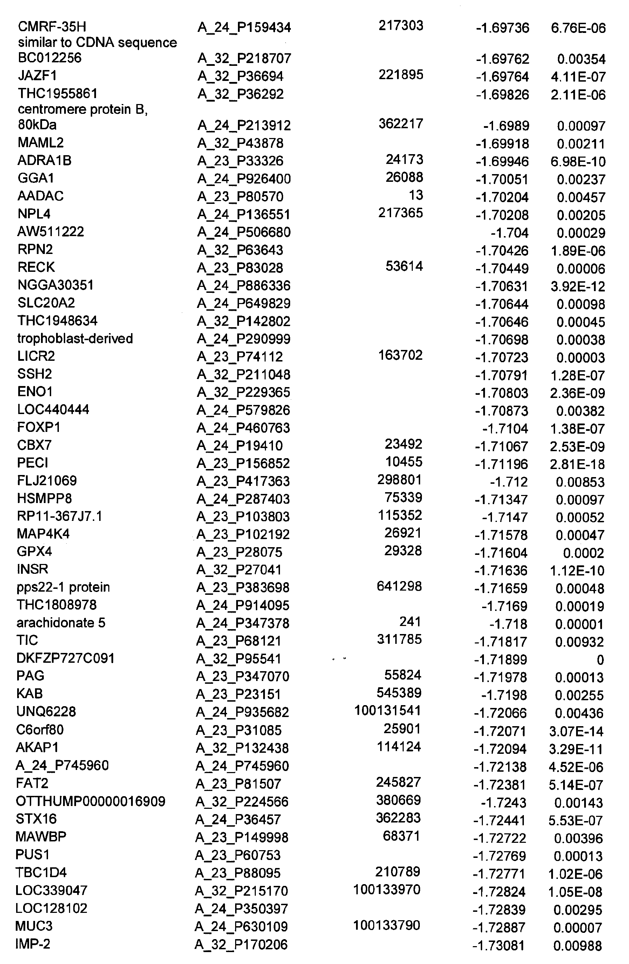

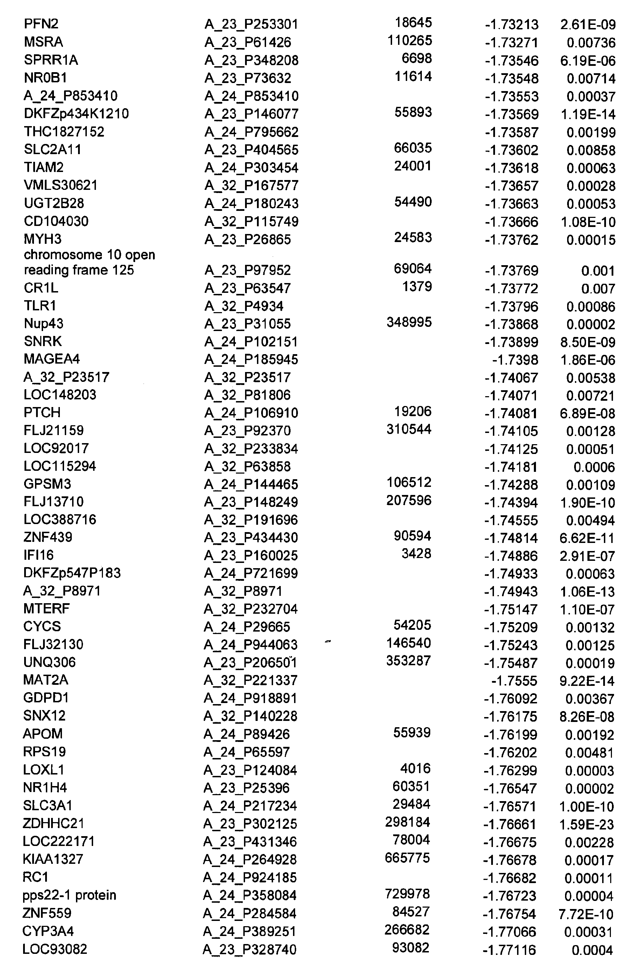

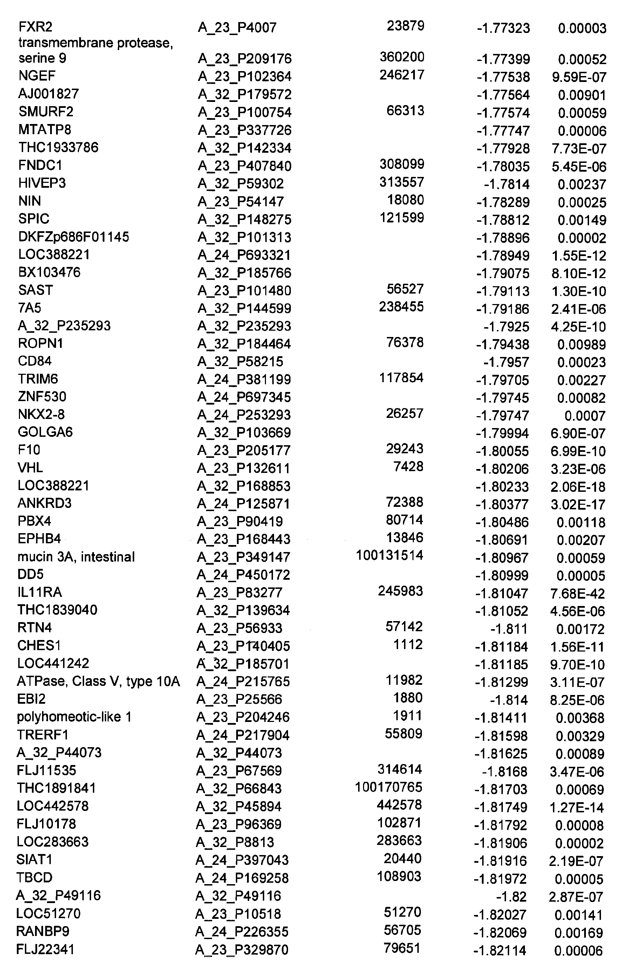

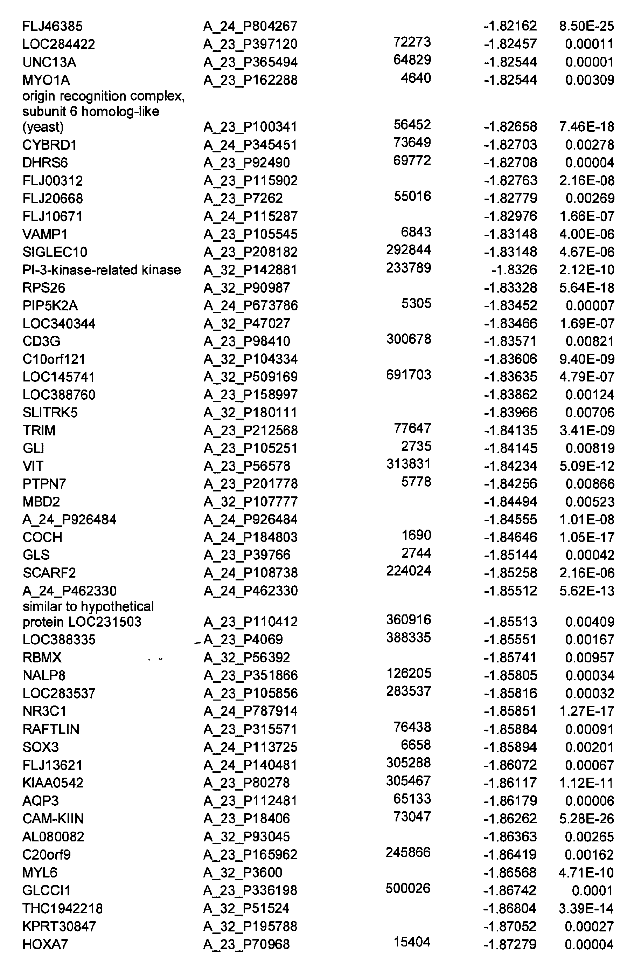

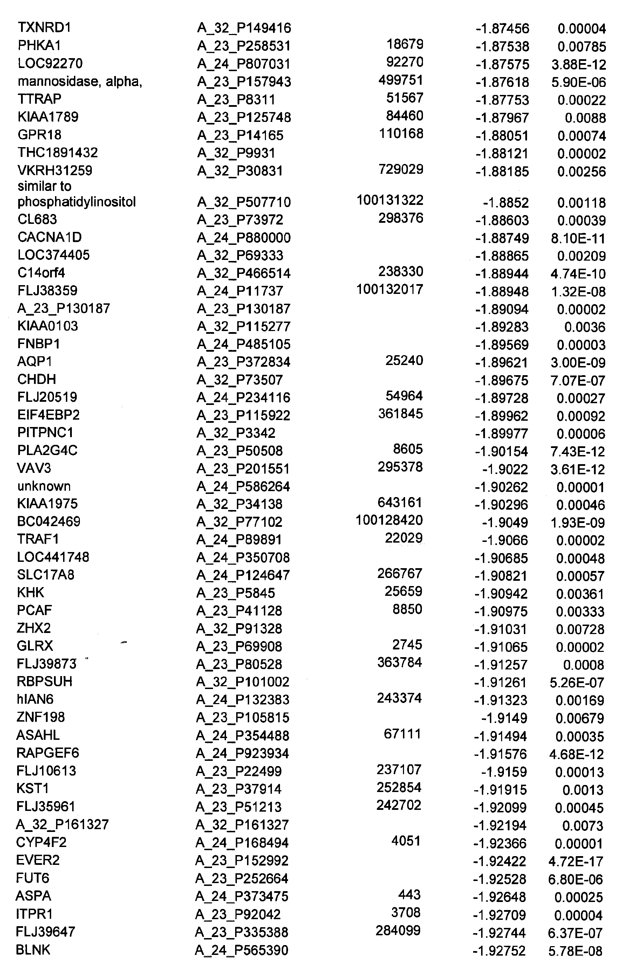

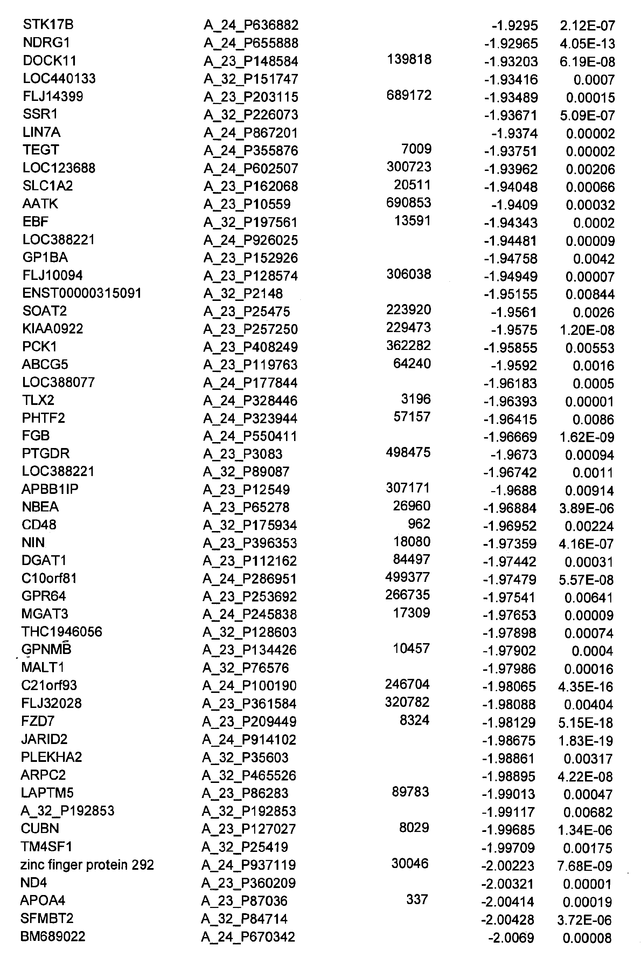

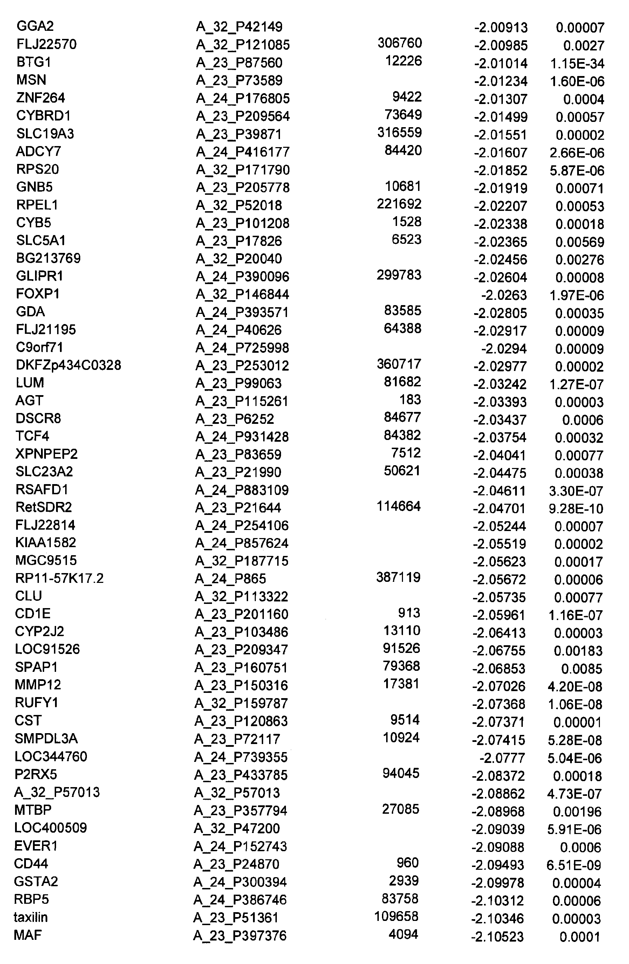

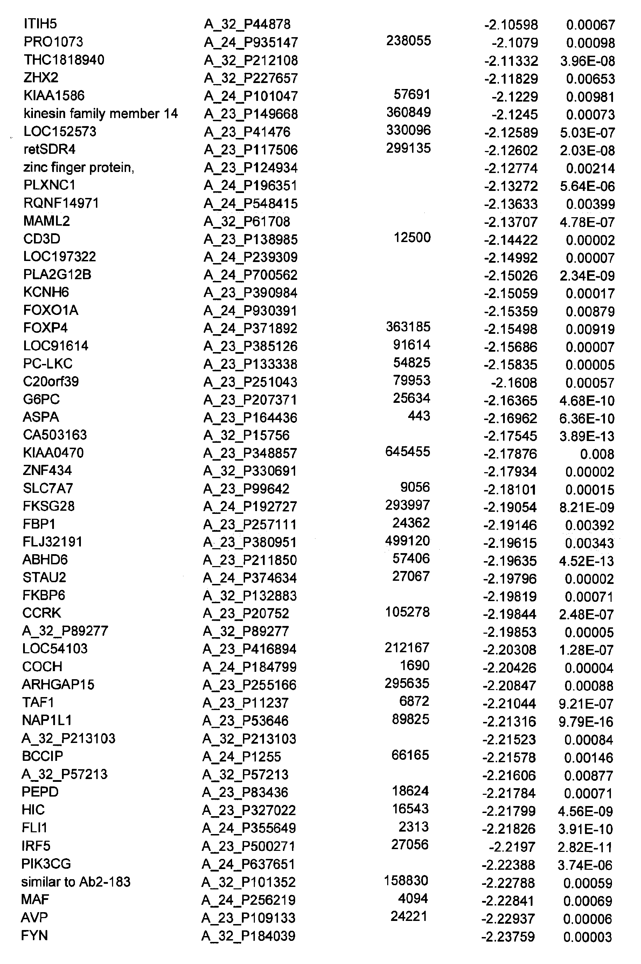

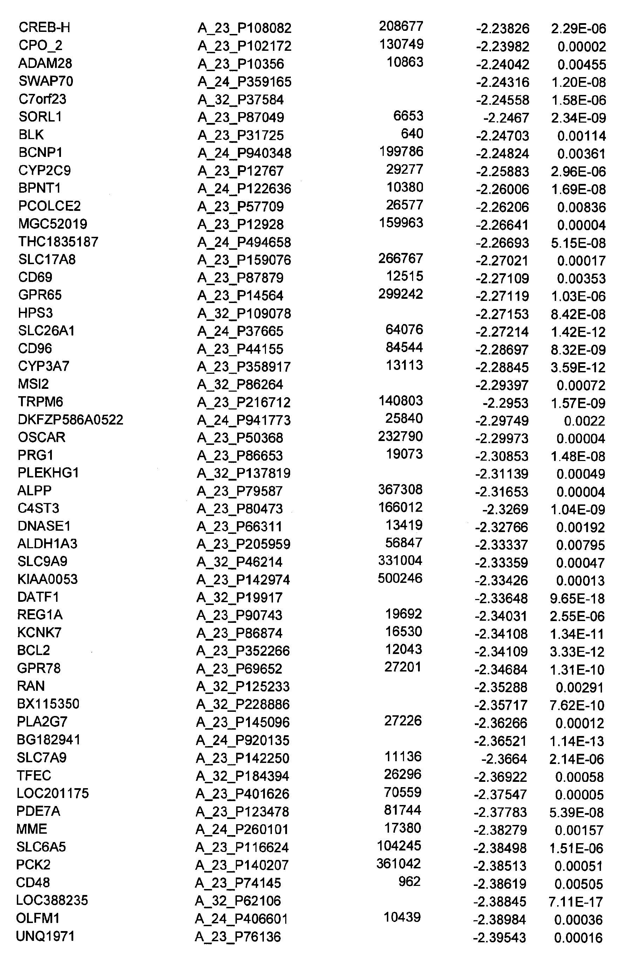

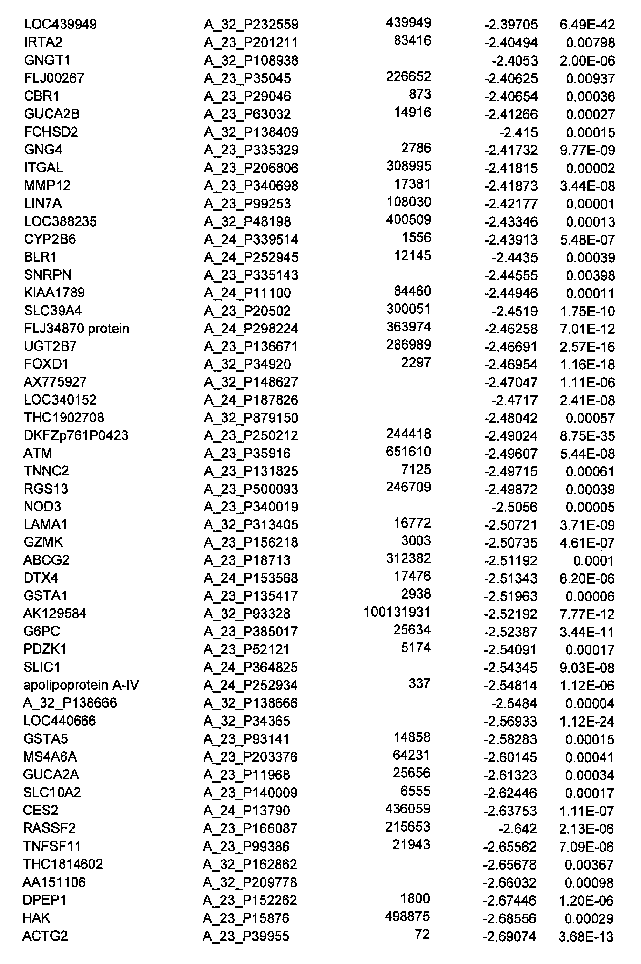

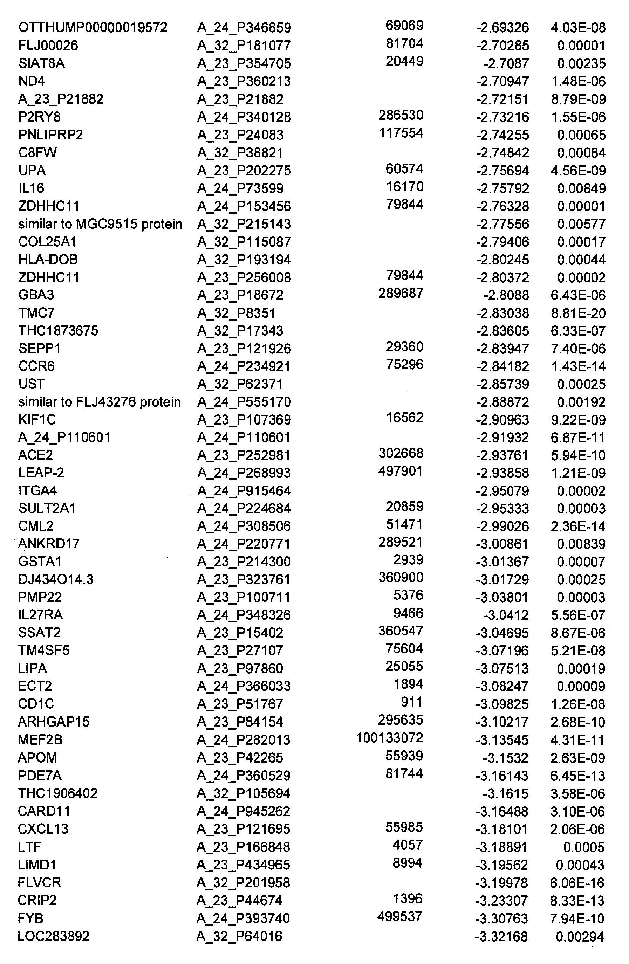

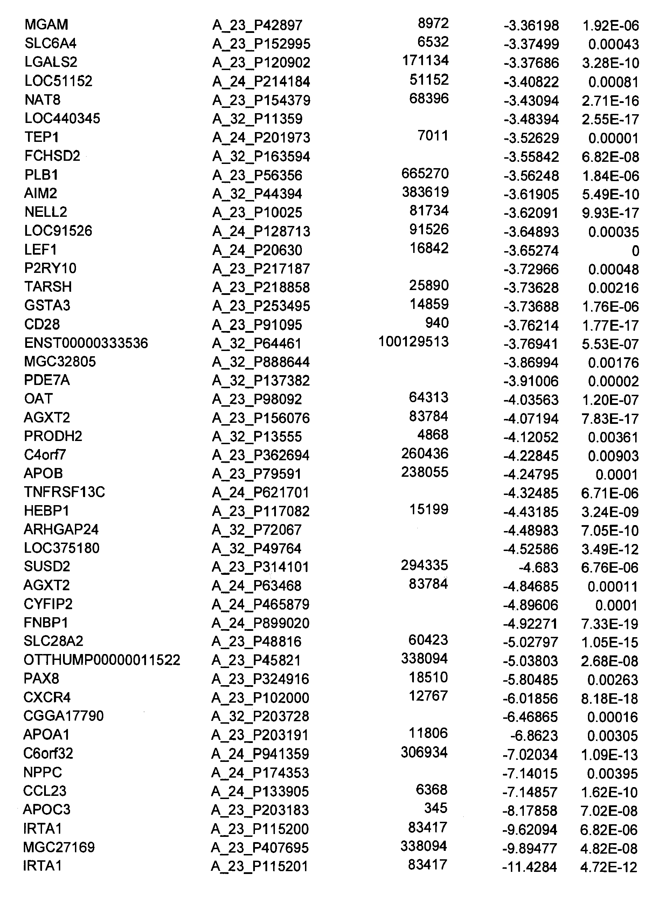

Figure 25 depicts fold changes in gene expression, comparing CD and control biopsies of the terminal ileum. Gene annotation: UBD- Diubiquitin, TIMD4- T-cell immunoglobulin and mucin domain-containing protein 4 Precursor, FLJ25393 & FLJ27099- hypothetical proteins, SOX14- SRY (sex determining region Y)-box 14, BX108833- Soares infant brain 1NIB, HK2-Hexokinase-2, RP11-653A5.1- novel protein, TEX12- testis expressed sequence 12, III-prostate-specific membrane antigen-like protein, S100P- S100 calcium binding protein P, Clorf34- DEME-6 protein, Sprn-shadow of prion protein, FOLH1- folate hydrolase, LOC92552- similar to homologue of MJD, EYA2- Eyesabsent homolog 2, CEACAM3-carcinoembryonic antigen-related cell adhesion molecule 3, C14orf81- hypothetical protein LOC90925, MUC4- mucin 4, TNFRSF13C- Tumor necrosis factor receptor superfamily member 13C, HEBP1-Heme-binding protein, ARHGAP24- Rho GTPase-activating protein 24, LOC375180-Homo sapiens LOC388920, SUSD2- sushi domain containing 2, AGXT2-alanineglyoxylate aminotransferase 2, CYFIP2- Cytoplasmic FMR1 interacting protein 2, FNBP1- Formin binding protein 1, SLC28A2- Solute carrier family 28 member 2, OTTHUMP00000011522- hyporthetical protein MGC27169, PAX8- paired box gene 8, CXCR4- CXC chemokine receptor 4, APOA1- apolipoprotein A-I, C6orf32-chromosome 6 open reading frame 32, NPPC- C-type natriuretic peptide, CCL23-chemokine (C-C motif) ligand 23, APOC3- apolipoprotein C-III, IRTA1-immunoglobulin superfamily receptor translocation associated 1, MGC27169-hypothetical protein.
Figure 26 depicts fold changes in gene expression, comparing non- inflamed CD and control sigmoid colon biopsies.
Figure 27 depicts fold changes in gene expression, comparing inflamed and non- inflamed CD sigmoid colon biopsies.
Figure 28 illustrates expression analysis of the IL-23/ Th17 pathway in Crohn's disease and controls. The IL-23 pathway is depicted along with gene expression of constituent molecules in CD and control biopsies separated by inflammation status. Gene expression is shown as box- whisker plots. The boxes are 25th to the 75th centile. The IL-23 pathway is upregulated in CD biopsies compared to controls and in inflamed CD biopsies compared to non- inflamed CD biopsies.
Figure 29 illustrates the expression analysis of the autophagy pathway in Crohn's disease and controls. The autophagy pathway with gene expression is shown as box-whisker plots. Differential gene expression was observed in 6 of the 20 genes that were examined with ATG16LI, ATG4D and ATG3 being downregulated and ATG12, ATG16L2 and LC3B marginally upregulated. PE-Phosphatidylethanolamine, a lipid which covalently attaches to ATG8/LC3 and mediates its attachment to autophagic membranes.
Figure 30 shows sigmoid colon Crohn's Disease and control biopsies clustered by epithelial cell markers. The colonic biopsies are annotated along the top of the figure: controls (e.g., numbers 1-5, and 7-11), non- inflamed CD (numbers 6, 12, 34, 50-51, and 57), inflamed CD (numbers 15, 45, 49, 52-55, 58, and 60-61), untreated CD ( numbers 42, 46-48, 56, and 59). On the right of the figure the epithelial cell cytokines are annotated. The degree of upregulation measured in red and downregulation measured in blue can be quantified using the logarithmic key.

### Detailed Description of the Invention

### A. Definitions

Unless defined otherwise, technical and scientific terms used herein have the same meaning as commonly understood by one of ordinary skill in the art to which this invention belongs. Singleton et al., Dictionary of Microbiology and Molecular Biology 2nd ed., J. Wiley & Sons (New York, NY 1994), and March, Advanced Organic Chemistry Reactions, Mechanisms and Structure 4th ed., John Wiley & Sons (New York, NY 1992), provide one skilled in the art with a general guide to many of the terms used in the present application.

One skilled in the art will recognize many methods and materials similar or equivalent to those described herein, which could be used in the practice of the present invention. Indeed, the present invention is in no way limited to the methods and materials described. For purposes of the present invention, the following terms are defined below.

The term "inflammatory bowel disease" or "IBD" is used as a collective term for ulcerative colitis and Crohn's disease. Although the two diseases are generally considered as two different entities, their common characteristics, such as patchy necrosis of the surface epithelium, focal accumulations of leukocytes adjacent to glandular crypts, and an increased number of intraepithelial lymphocytes (IEL) and certain macrophage subsets, justify their treatment as a single disease group.

The term "Crohn's disease" or "CD" is used herein to refer to a condition involving chronic inflammation of the gastrointestinal tract. Crohn's-related inflammation usually affects the intestines, but may occur anywhere from the mouth to the anus. CD differs from UC in that the inflammation extends through all layers of the intestinal wall and involves mesentery as well as lymph nodes. The disease is often discontinuous, i.e., severely diseased segments of bowel are separated from apparently disease-free areas. In CD, the bowel wall also thickens which can lead to obstructions, and the development of fistulas and fissures are not uncommon. As used herein, CD may be one or more of several types of CD, including without limitation, ileocolitis (affects the ileum and the large intestine); ileitis (affects the ileum); gastroduodenal CD (inflammation in the stomach and the duodenum); jejunoileitis (spotty patches of inflammation in the jejunum); and Crohn's (granulomatous) colitis (only affects the large intestine).

The term "ulcerative colitis" or "UC" is used herein to refer to a condition involving inflammation of the large intestine and rectum. In patients with UC, there is an inflammatory reaction primarily involving the colonic mucosa. The inflammation is typically uniform and continuous with no intervening areas of normal mucosa. Surface mucosal cells as well as crypt epithelium and submucosa are involved in an inflammatory reaction with neutrophil infiltration. Ultimately, this reaction typically progresses to epithelial damage and loss of epithelial cells resulting in multiple ulcerations, fibrosis, dysplasia and longitudinal retraction of the colon.

The term "inactive" IBD is used herein to mean an IBD that was previously diagnosed in an individual but is currently in remission. This is in contrast to an "active" IBD in which an individual has been diagnosed with and IBD but has not undergone treatment. In addition, the active IBD may be a recurrence of a previously diagnosed and treated IBD that had gone into remission (i.e. become an inactive IBD). Such recurrences may also be referred to herein as "flare-ups" of an IBD. Mammalian subjects having an active autoimmune disease, such as an IBD, may be subject to a flare-up, which is a period of heightened disease activity or a return of corresponding symptoms. Flare-ups may occur in response to severe infection, allegic reactions, physical stress, emotional trauma, surgery, or environmental factors.

The term "modulate" is used herein to mean that the expression of the gene, or level of RNA molecule or equivalent RNA molecules encoding one or more proteins or protein subunits, or activity of one or more proteins or protein subunits is up regulated or down regulated, such that expression, level, or activity is greater than or less than that observed in the absence of the modulator.

The terms "inhibit", "down-regulate", "underexpress" and "reduce" are used interchangeably and mean that the expression of a gene, or level of RNA molecules or equivalent RNA molecules encoding one or more proteins or protein subunits, or activity of one or more proteins or protein subunits, is reduced relative to one or more controls, such as, for example, one or more positive and/or negative controls.

The term "up-regulate" or "overexpress" is used to mean that the expression of a gene, or level of RNA molecules or equivalent RNA molecules encoding one or more proteins or protein subunits, or activity of one or more proteins or protein subunits, is elevated relative to one or more controls, such as, for example, one or more positive and/or negative controls.

The term "diagnosis" is used herein to refer to the identification of a molecular or pathological state, disease or condition, such as the identification of IBD.

The term "prognosis" is used herein to refer to the prediction of the likelihood of IBD development or progression, including autoimmune flare-ups and recurrences following surgery. Prognostic factors are those variables related to the natural history of IBD, which influence the recurrence rates and outcome of patients once they have developed IBD. Clinical parameters that may be associated with a worse prognosis include, for example, an abdominal mass or tenderness, skin rash, swollen joints, mouth ulcers, and borborygmus (gurgling or splashing sound over the intestine). Prognostic factors may be used to categorize patients into subgroups with different baseline recurrence risks.

The "pathology" of an IBD includes all phenomena that compromise the well-being of the patient. IBD pathology is primarily attributed to abnormal activation of the immune system in the intestines that can lead to chronic or acute inflammation in the absence of any known foreign antigen, and subsequent ulceration. Clinically, IBD is characterized by diverse manifestations often resulting in a chronic, unpredictable course. Bloody diarrhea and abdominal pain are often accompanied by fever and weight loss. Anemia is not uncommon, as is severe fatigue. Joint manifestations ranging from arthralgia to acute arthritis as well as abnormalities in liver function are commonly associated with IBD. During acute "attacks" of IBD, work and other normal activity are usually impossible, and often a patient is hospitalized.

The aetiology of these diseases is unknown and the initial lesion has not been clearly defined; however, patchy necrosis of the surface epithelium, focal accumulations of leukocytes adjacent to glandular crypts, and an increased number of intraepithelial lymphocytes and certain macrophage subsets have been described as putative early changes, especially in Crohn's disease.

The term "treatment" refers to both therapeutic treatment and prophylactic or preventative measures for IBD, wherein the object is to prevent or slow down (lessen) the targeted pathologic condition or disorder. Those in need of treatment include those already with an IBD as well as those prone to have an IBD or those in whom the IBD is to be prevented. Once the diagnosis of an IBD has been made by the methods disclosed herein, the goals of therapy are to induce and maintain a remission.

Various agents that are suitable for use as an "IBD therapeutic agent" are known to those of ordinary skill in the art. As described herein, such agents include without limitation, aminosalicylates, corticosteroids, and immunosuppressive agents.

The term "test sample" refers to a sample from a mammalian subject suspected of having an IBD, known to have an IBD, or known to be in remission from an IBD. The test sample may originate from various sources in the mammalian subject including, without limitation, blood, semen, serum, urine, feces, bone marrow, mucosa, tissue, etc. The test sample may originate from a tissue biopsy of the gastrointestinal tract including, without limitation, ascending colon tissue, descending colon tissue, sigmoid colon tissue, ileocolon, and terminal ileum tissue.

The term "control" or "control sample" refers a negative control in which a negative result is expected to help correlate a positive result in the test sample. Controls that are suitable for the present invention include, without limitation, a sample known to have normal levels of gene expression, a sample obtained from a mammalian subject known not to have an IBD, and a sample obtained from a mammalian subject known to be normal. A control may also be a sample obtained from a subject previously diagnosed and treated for an IBD who is currently in remission; and such a control is useful in determining any recurrence of an IBD in a subject who is in remission. In addition, the control may be a sample containing normal cells that have the same origin as cells contained in the test sample. Those of skill in the art will appreciate other controls suitable for use in the present invention.

The term "microarray" refers to an ordered arrangement of hybridizable array elements, preferably polynucleotide probes, on a substrate.

The term "polynucleotide," when used in singular or plural, generally refers to any polyribonucleotide or polydeoxribonucleotide, which may be unmodified RNA or DNA or modified RNA or DNA. Thus, for instance, polynucleotides as defined herein include, without limitation, single- and double-stranded DNA, DNA including single- and double-stranded regions, single- and double-stranded RNA, and RNA including single- and double-stranded regions, hybrid molecules comprising DNA and RNA that may be single-stranded or, more typically, double-stranded or include single- and double-stranded regions. In addition, the term "polynucleotide" as used herein refers to triple-stranded regions comprising RNA or DNA or both RNA and DNA. The strands in such regions may be from the same molecule or from different molecules. The regions may include all of one or more of the molecules, but more typically involve only a region of some of the molecules. One of the molecules of a triple-helical region often is an oligonucleotide. The term "polynucleotide" specifically includes cDNAs. The term includes DNAs (including cDNAs) and RNAs that contain one or more modified bases. Thus, DNAs or RNAs with backbones modified for stability or for other reasons are "polynucleotides" as that term is intended herein. Moreover, DNAs or RNAs comprising unusual bases, such as inosine, or modified bases, such as tritiated bases, are included within the term "polynucleotides" as defined herein. In general, the term "polynucleotide" embraces all chemically, enzymatically and/or metabolically modified forms of unmodified polynucleotides, as well as the chemical forms of DNA and RNA characteristic of viruses and cells, including simple and complex cells.

The term "oligonucleotide" refers to a relatively short polynucleotide, including, without limitation, single-stranded deoxyribonucleotides, single- or double-stranded ribonucleotides, RNA:DNA hybrids and double-stranded DNAs. Oligonucleotides, such as single-stranded DNA probe oligonucleotides, are often synthesized by chemical methods, for example using automated oligonucleotide synthesizers that are commercially available. However, oligonucleotides can be made by a variety of other methods, including *in vitro* recombinant DNA-mediated techniques and by expression of DNAs in cells and organisms.

The terms "differentially expressed gene," "differential gene expression" and their synonyms, which are used interchangeably, refer to a gene whose expression is activated to a higher or lower level in a subject suffering from a disease, specifically an IBD, such as UC or CD, relative to its expression in a normal or control subject. The terms also include genes whose expression is activated to a higher or lower level at different stages of the same disease. It is also understood that a differentially expressed gene may be either activated or inhibited at the nucleic acid level or protein level, or may be subject to alternative splicing to result in a different polypeptide product. Such differences may be evidenced by a change in mRNA levels, surface expression, secretion or other partitioning of a polypeptide, for example. Differential gene expression may include a comparison of expression between two or more genes or their gene products, or a comparison of the ratios of the expression between two or more genes or their gene products, or even a comparison of two differently processed products of the same gene, which differ between normal subjects and subjects suffering from a disease, specifically an IBD, or between various stages of the same disease. Differential expression includes both quantitative, as well as qualitative, differences in the temporal or cellular expression pattern in a gene or its expression products among, for example, normal and diseased cells, or among cells which have undergone different disease events or disease stages. For the purpose of this invention, "differential gene expression" is considered to be present when there is an at least about one-fold, at least about 1.5-fold, at least about 2-fold, at least about 2.5-fold, at least about 3-fold, at least about 3.5 fold, at least about 4-fold, at least about 4.5-fold, at least about 5-fold, at least about 5.5-fold, at least about 6-fold, at least about 7-fold, at least about 8-fold, at least about 9-fold, or at least about 10-fold difference between the expression of a given gene in normal and diseased subjects, or in various stages of disease development in a diseased subject.

The term "over-expression" with regard to an RNA transcript is used to refer to the level of the transcript determined by normalization to the level of reference mRNAs, which might be all transcripts detected in the specimen or a particular reference set of mRNAs.

The phrase "gene amplification" refers to a process by which multiple copies of a gene or gene fragment are formed in a particular cell or cell line. The duplicated region (a stretch of amplified DNA) is often referred to as "amplicon". Usually, the amount of the messenger RNA (mRNA) produced, *i.e.,* the level of gene expression, also increases in the proportion of the number of copies made of the particular gene expressed.

In general, the term "marker" or "biomarker" or refers to an identifiable physical location on a chromosome, such as a restriction endonuclease recognition site or a gene, whose inheritance can be monitored. The marker may be an expressed region of a gene referred to as a "gene expression marker", or some segment of DNA with no known coding function. An "IBD marker" as used herein refers those genes listed in Table 1.

"Stringency" of hybridization reactions is readily determinable by one of ordinary skill in the art, and generally is an empirical calculation dependent upon probe length, washing temperature, and salt concentration. In general, longer probes require higher temperatures for proper annealing, while shorter probes need lower temperatures. Hybridization generally depends on the ability of denatured DNA to reanneal when complementary strands are present in an environment below their melting temperature. The higher the degree of desired homology between the probe and hybridizable sequence, the higher the relative temperature which can be used. As a result, it follows that higher relative temperatures would tend to make the reaction conditions more stringent, while lower temperatures less so. For additional details and explanation of stringency of hybridization reactions, see Ausubel et al., Current Protocols in Molecular Biology, Wiley Interscience Publishers, (1995).

"Stringent conditions" or "high stringency conditions", as defined herein, typically: (1) employ low ionic strength and high temperature for washing, for example 0.015 M sodium chloride/0.0015 M sodium citrate/0.1% sodium dodecyl sulfate at 50°C; (2) employ during hybridization a denaturing agent, such as formamide, for example, 50% (v/v) formamide with 0.1% bovine serum albumin/0.1% Fico11/0.1% polyvinylpyrrolidone/50mM sodium phosphate buffer at pH 6.5 with 750 mM sodium chloride, 75 mM sodium citrate at 42°C; or (3) employ 50% formamide, 5 x SSC (0.75 M NaCl, 0.075 M sodium citrate), 50 mM sodium phosphate (pH 6.8), 0.1% sodium pyrophosphate, 5 x Denhardt's solution, sonicated salmon sperm DNA (50 µg/ml), 0.1% SDS, and 10% dextran sulfate at 42°C, with washes at 42°C in 0.2 x SSC (sodium chloride/sodium citrate), 50% formamide, followed by a high-stringency wash consisting of 0.1 x SSC containing EDTA at 55°C.

"Moderately stringent conditions" may be identified as described by Sambrook et al., Molecular Cloning: A Laboratory Manual, New York: Cold Spring Harbor Press, 1989, and include the use of washing solution and hybridization conditions (e.g., temperature, ionic strength and %SDS) less stringent that those described above. An example of moderately stringent conditions is overnight incubation at 37°C in a solution comprising: 20% formamide, 5 x SSC (150 mM NaCl, 15 mM trisodium citrate), 50 mM sodium phosphate (pH 7.6), 5 x Denhardt's solution, 10% dextran sulfate, and 20 mg/ml denatured sheared salmon sperm DNA, followed by washing the filters in 1 x SSC at about 37-50°C. The skilled artisan will recognize how to adjust the temperature, ionic strength, etc. as necessary to accommodate factors such as probe length and the like.

In the context of the present invention, reference to "at least one," "at least two," "at least five," etc. of the genes listed in any particular gene set means any one or any and all combinations of the genes listed.

The terms "splicing" and "RNA splicing" are used interchangeably and refer to RNA processing that removes introns and joins exons to produce mature mRNA with continuous coding sequence that moves into the cytoplasm of an eukaryotic cell.

In theory, the term "exon" refers to any segment of an interrupted gene that is represented in the mature RNA product (B. Lewin. Genes IV Cell Press, Cambridge Mass. 1990). In theory the term "intron" refers to any segment of DNA that is transcribed but removed from within the transcript by splicing together the exons on either side of it. Operationally, exon sequences occur in the mRNA sequence of a gene as defined by Ref. SEQ ID numbers. Operationally, intron sequences are the intervening sequences within the genomic DNA of a gene, bracketed by exon sequences and having GT and AG splice consensus sequences at their 5' and 3' boundaries.

An "interfering RNA" or "small interfering RNA (siRNA)" is a double stranded RNA molecule usually less than about 30 nucleotides in length that reduces expression of a target gene. Interfering RNAs may be identified and synthesized using known methods (Shi Y., Trends in Genetics 19(1):9-12 (2003), WO/2003056012 and WO2003064621), and siRNA libraries are commercially available, for example from Dharmacon, Lafayette, Colorado.

A "native sequence" polypeptide is one which has the same amino acid sequence as a polypeptide derived from nature, including naturally occurring or allelic variants. Such native sequence polypeptides can be isolated from nature or can be produced by recombinant or synthetic means. Thus, a native sequence polypeptide can have the amino acid sequence of naturally occurring human polypeptide, murine polypeptide, or polypeptide from any other mammalian species.

The term "antibody" herein is used in the broadest sense and specifically covers monoclonal antibodies, polyclonal antibodies, multispecific antibodies (e.g. bispecific antibodies), and antibody fragments, so long as they exhibit the desired biological activity. The present invention particularly contemplates antibodies against one or more of the IBD markers disclosed herein. Such antibodies may be referred to as "anti-IBD marker antibodies".

The term "monoclonal antibody" as used herein refers to an antibody from a population of substantially homogeneous antibodies, *i.e.,* the individual antibodies comprising the population are identical and/or bind the same epitope(s), except for possible variants that may arise during production of the monoclonal antibody, such variants generally being present in minor amounts. Such monoclonal antibody typically includes an antibody comprising a polypeptide sequence that binds a target, wherein the target-binding polypeptide sequence was obtained by a process that includes the selection of a single target binding polypeptide sequence from a plurality of polypeptide sequences.

The monoclonal antibodies herein specifically include "chimeric" antibodies in which a portion of the heavy and/or light chain is identical with or homologous to corresponding sequences in antibodies derived from a particular species or belonging to a particular antibody class or subclass, while the remainder of the chain(s) is identical with or homologous to corresponding sequences in antibodies derived from another species or belonging to another antibody class or subclass, as well as fragments of such antibodies, so long as they exhibit the desired biological activity (U.S. Patent No. 4,816,567; and Morrison et al., Proc. Natl. Acad. Sci. USA, 81:6851-6855 (1984)). Chimeric antibodies of interest herein include "primatized" antibodies comprising variable domain antigen-binding sequences derived from a non-human primate (e.g. Old World Monkey, Ape etc) and human constant region sequences, as well as "humanized" antibodies.

"Humanized" forms of non-human (e.g., rodent) antibodies are chimeric antibodies that contain minimal sequence derived from non-human immunoglobulin. For the most part, humanized antibodies are human immunoglobulins (recipient antibody) in which residues from a hypervariable region of the recipient are replaced by residues from a hypervariable region of a non-human species (donor antibody) such as mouse, rat, rabbit or nonhuman primate having the desired specificity, affinity, and capacity.

An "intact antibody" herein is one which comprises two antigen binding regions, and an Fc region. Preferably, the intact antibody has a functional Fc region.

"Antibody fragments" comprise a portion of an intact antibody, preferably comprising the antigen binding region thereof. Examples of antibody fragments include Fab, Fab', F(ab')₂, and Fv fragments; diabodies; linear antibodies; single-chain antibody molecules; and multispecific antibodies formed from antibody fragment(s).

"Native antibodies" are usually heterotetrameric glycoproteins of about 150,000 daltons, composed of two identical light (L) chains and two identical heavy (H) chains. Each light chain is linked to a heavy chain by one covalent disulfide bond, while the number of disulfide linkages varies among the heavy chains of different immunoglobulin isotypes. Each heavy and light chain also has regularly spaced intrachain disulfide bridges. Each heavy chain has at one end a variable domain (V_{H}) followed by a number of constant domains. Each light chain has a variable domain at one end (V_{L}) and a constant domain at its other end. The constant domain of the light chain is aligned with the first constant domain of the heavy chain, and the light-chain variable domain is aligned with the variable domain of the heavy chain. Particular amino acid residues are believed to form an interface between the light chain and heavy chain variable domains.

The term "variable" refers to the fact that certain portions of the variable domains differ extensively in sequence among antibodies and are used in the binding and specificity of each particular antibody for its particular antigen. However, the variability is not evenly distributed throughout the variable domains of antibodies. It is concentrated in three segments called hypervariable regions both in the light chain and the heavy chain variable domains. The more highly conserved portions of variable domains are called the framework regions (FRs). The variable domains of native heavy and light chains each comprise four FRs, largely adopting a β-sheet configuration, connected by three hypervariable regions, which form loops connecting, and in some cases forming part of, the β-sheet structure. The hypervariable regions in each chain are held together in close proximity by the FRs and, with the hypervariable regions from the other chain, contribute to the formation of the antigen-binding site of antibodies (see Kabat et al., Sequences of Proteins of Immunological Interest, 5th Ed. Public Health Service, National Institutes of Health, Bethesda, MD. (1991)).

The term *"hypervariable region," "HVR,"* or *"HV,"* when used herein refers to the regions of an antibody-variable domain that are hypervariable in sequence and/or form structurally defined loops. Generally, antibodies comprise six HVRs; three in the VH (H1, H2, H3), and three in the VL (L1, L2, L3). In native antibodies, H3 and L3 display the most diversity of the six HVRs, and H3 in particular is believed to play a unique role in conferring fine specificity to antibodies. See, e.g., Xu et al. Immunity 13:37-45 (2000); Johnson and Wu in Methods in Molecular Biology 248:1-25 (Lo, ed., Human Press, Totowa, NJ, 2003)). Indeed, naturally occurring camelid antibodies consisting of a heavy chain only are functional and stable in the absence of light chain. See, e.g., Hamers-Casterman et al., Nature 363:446-448 (1993) and Sheriff et al., Nature Struct. Biol. 3:733-736 (1996).

A number of HVR delineations are in use and are encompassed herein. The Kabat Complementarity Determining Regions (CDRs) are based on sequence variability and are the most commonly used (Kabat et al., Sequences of Proteins of Immunological Interest, 5th Ed. Public Health Service, National Institutes of Health, Bethesda, MD. (1991)). Chothia refers instead to the location of the structural loops (Chothia and Lesk J. Mol. Biol. 196:901-917 (1987)). The AbM HVRs represent a compromise between the Kabat HVRs and Chothia structural loops, and are used by Oxford Molecular's AbM antibody modeling software. The "contact" HVRs are based on an analysis of the available complex crystal structures. The residues from each of these HVRs are noted below.

| Loop | Kabat | AbM | Chothia | Contact |
|---|---|---|---|---|
| L1 | L24-L34 | L24-L34 | L26-L32 | L30-L36 |
| L2 | L50-L56 | L50-L56 | L50-L52 | L46-L55 |
| L3 | L89-L97 | L89-L97 | L91-L96 | L89-L96 |
| H1 | H31-H35B | H26-H35B | H26-H32 | H30-H35B |
| (Kabat Numbering) | | | | |
| H1 | H31-H35 | H26-H35 | H26-H32 | H30-H35 |
| (Chothia Numbering) | | | | |
| H2 | H50-H65 | H50-H58 | H53-H55 | H47-H58 |
| H3 | H95-H102 | H95-H102 | H96-H101 | H93-H101 |

HVRs may comprise "extended HVRs" as follows: 24-36 or 24-34 (L1), 46-56 or 50-56 (L2) and 89-97 or 89-96 (L3) in the VL and 26-35 (H1), 50-65 or 49-65 (H2) and 93-102, 94-102, or 95-102 (H3) in the VH. The variable domain residues are numbered according to Kabat et al., supra, for each of these definitions.

The expression "variable-domain residue-numbering as in Kabat" or "amino-acid-position numbering as in Kabat," and variations thereof, refers to the numbering system used for heavy-chain variable domains or light-chain variable domains of the compilation of antibodies in Kabat *et al., supra.* Using this numbering system, the actual linear amino acid sequence may contain fewer or additional amino acids corresponding to a shortening of, or insertion into, a FR or HVR of the variable domain. For example, a heavy-chain variable domain may include a single amino acid insert (residue 52a according to Kabat) after residue 52 of H2 and inserted residues (e.g. residues 82a, 82b, and 82c, etc. according to Kabat) after heavy-chain FR residue 82. The Kabat numbering of residues may be determined for a given antibody by alignment at regions of homology of the sequence of the antibody with a "standard" Kabat numbered sequence.

Papain digestion of antibodies produces two identical antigen-binding fragments, called "Fab" fragments, each with a single antigen-binding site, and a residual "Fc" fragment, whose name reflects its ability to crystallize readily. Pepsin treatment yields an F(ab')₂ fragment that has two antigen-binding sites and is still capable of cross-linking antigen.

"Fv" is the minimum antibody fragment which contains a complete antigen-recognition and antigen-binding site. This region consists of a dimer of one heavy chain and one light chain variable domain in tight, non-covalent association. It is in this configuration that the three hypervariable regions of each variable domain interact to define an antigen-binding site on the surface of the V_{H}-V_{L} dimer. Collectively, the six hypervariable regions confer antigen-binding specificity to the antibody. However, even a single variable domain (or half of an Fv comprising only three hypervariable regions specific for an antigen) has the ability to recognize and bind antigen, although at a lower affinity than the entire binding site.

The Fab fragment also contains the constant domain of the light chain and the first constant domain (CH1) of the heavy chain. Fab' fragments differ from Fab fragments by the addition of a few residues at the carboxy terminus of the heavy chain CH1 domain including one or more cysteines from the antibody hinge region. Fab'-SH is the designation herein for Fab' in which the cysteine residue(s) of the constant domains bear at least one free thiol group. F(ab')₂ antibody fragments originally were produced as pairs of Fab' fragments which have hinge cysteines between them. Other chemical couplings of antibody fragments are also known.

The "light chains" of antibodies from any vertebrate species can be assigned to one of two clearly distinct types, called kappa (κ) and lambda (λ), based on the amino acid sequences of their constant domains.

The term "Fc region" herein is used to defme a C-terminal region of an immunoglobulin heavy chain, including native sequence Fc regions and variant Fc regions. Although the boundaries of the Fc region of an immunoglobulin heavy chain might vary, the human IgG heavy chain Fc region is usually defined to stretch from an amino acid residue at position Cys226, or from Pro230, to the carboxyl-terminus thereof. The C-terminal lysine (residue 447 according to the EU numbering system) of the Fc region may be removed, for example, during production or purification of the antibody, or by recombinantly engineering the nucleic acid encoding a heavy chain of the antibody. Accordingly, a composition of intact antibodies may comprise antibody populations with all K447 residues removed, antibody populations with no K447 residues removed, and antibody populations having a mixture of antibodies with and without the K447 residue.

Unless indicated otherwise, herein the numbering of the residues in an immunoglobulin heavy chain is that of the EU index as in Kabat et al., Sequences of Proteins of Immunological Interest, 5th Ed. Public Health Service, National Institutes of Health, Bethesda, MD (1991), expressly incorporated herein by reference. The "EU index as in Kabat" refers to the residue numbering of the human IgG1 EU antibody.

A "native sequence Fc region" comprises an amino acid sequence identical to the amino acid sequence of an Fc region found in nature. Native sequence human Fc regions include a native sequence human IgG1 Fc region (non-A and A allotypes); native sequence human IgG2 Fc region; native sequence human IgG3 Fc region; and native sequence human IgG4 Fc region as well as naturally occurring variants thereof.

A "variant Fc region" comprises an amino acid sequence which differs from that of a native sequence Fc region by virtue of at least one amino acid modification, preferably one or more amino acid substitution(s). Preferably, the variant Fc region has at least one amino acid substitution compared to a native sequence Fc region or to the Fc region of a parent polypeptide, e.g. from about one to about ten amino acid substitutions, and preferably from about one to about five amino acid substitutions in a native sequence Fc region or in the Fc region of the parent polypeptide. The variant Fc region herein will preferably possess at least about 80% homology with a native sequence Fc region and/or with an Fc region of a parent polypeptide, and most preferably at least about 90% homology therewith, more preferably at least about 95% homology therewith.

Depending on the amino acid sequence of the constant domain of their heavy chains, intact antibodies can be assigned to different "classes". There are five major classes of intact antibodies: IgA, IgD, IgE, IgG, and IgM, and several of these may be further divided into "subclasses" (isotypes), e.g., IgG1, IgG2, IgG3, IgG4, IgA, and IgA2. The heavy-chain constant domains that correspond to the different classes of antibodies are called α, δ, ε, γ, and µ, respectively. The subunit structures and three-dimensional configurations of different classes of immunoglobulins are well known.

"Single-chain Fv" or "scFv" antibody fragments comprise the V_{H} and V_{L} domains of antibody, wherein these domains are present in a single polypeptide chain. Preferably, the Fv polypeptide further comprises a polypeptide linker between the V_{H} and V_{L} domains which enables the scFv to form the desired structure for antigen binding. For a review of scFv see Plückthun in The Pharmacology of Monoclonal Antibodies, vol. 113, Rosenburg and Moore eds., Springer-Verlag, New York, pp. 269-315 (1994).

The term "diabodies" refers to small antibody fragments with two antigen-binding sites, which fragments comprise a variable heavy domain (V_{H}) connected to a variable light domain (V_{L}) in the same polypeptide chain (V_{H} - V_{L}). By using a linker that is too short to allow pairing between the two domains on the same chain, the domains are forced to pair with the complementary domains of another chain and create two antigen-binding sites. Diabodies are described more fully in, for example, EP 404,097; WO 93/11161; and Hollinger et al., Proc. Natl. Acad. Sci. USA, 90:6444-6448 (1993).

A "naked antibody" is an antibody that is not conjugated to a heterologous molecule, such as a small molecule or radiolabel.

An "isolated" antibody is one which has been identified and separated and/or recovered from a component of its natural environment. Contaminant components of its natural environment are materials which would interfere with diagnostic or therapeutic uses for the antibody, and may include enzymes, hormones, and other proteinaceous or nonproteinaceous solutes. In preferred embodiments, the antibody will be purified (1) to greater than 95% by weight of antibody as determined by the Lowry method, and most preferably more than 99% by weight, (2) to a degree sufficient to obtain at least 15 residues of N-terminal or internal amino acid sequence by use of a spinning cup sequenator, or (3) to homogeneity by SDS-PAGE under reducing or nonreducing conditions using Coomassie blue or, preferably, silver stain. Isolated antibody includes the antibody *in situ* within recombinant cells since at least one component of the antibody's natural environment will not be present. Ordinarily, however, isolated antibody will be prepared by at least one purification step.

An "affinity matured" antibody is one with one or more alterations in one or more hypervariable regions thereof which result an improvement in the affinity of the antibody for antigen, compared to a parent antibody which does not possess those alteration(s). Preferred affinity matured antibodies will have nanomolar or even picomolar affinities for the target antigen. Affinity matured antibodies are produced by procedures known in the art. Marks et al. Bio/Technology 10:779-783 (1992) describes affinity maturation by VH and VL domain shuffling. Random mutagenesis of HVR and/or framework residues is described by: Barbas et al. Proc Nat. Acad. Sci, USA 91:3809-3813 (1994); Schier et al. Gene 169:147-155 (1995); Yelton et al. J. Immunol. 155:1994-2004 (1995); Jackson et al., J. Immunol. 154(7):3310-9 (1995); and Hawkins et al, J. Mol. Biol. 226:889-896 (1992).

An "amino acid sequence variant" antibody herein is an antibody with an amino acid sequence which differs from a main species antibody. Ordinarily, amino acid sequence variants will possess at least about 70% homology with the main species antibody, and preferably, they will be at least about 80%, more preferably at least about 90% homologous with the main species antibody. The amino acid sequence variants possess substitutions, deletions, and/or additions at certain positions within or adjacent to the amino acid sequence of the main species antibody. Examples of amino acid sequence variants herein include an acidic variant (*e.g*. deamidated antibody variant), a basic variant, an antibody with an amino-terminal leader extension (*e.g*. VHS-) on one or two light chains thereof, an antibody with a C-terminal lysine residue on one or two heavy chains thereof, etc., and includes combinations of variations to the amino acid sequences of heavy and/or light chains. The antibody variant of particular interest herein is the antibody comprising an amino-terminal leader extension on one or two light chains thereof, optionally further comprising other amino acid sequence and/or glycosylation differences relative to the main species antibody.

A "glycosylation variant" antibody herein is an antibody with one or more carbohydrate moieities attached thereto which differ from one or more carbohydrate moieties attached to a main species antibody. Examples of glycosylation variants herein include antibody with a G1 or G2 oligosaccharide structure, instead a G0 oligosaccharide structure, attached to an Fc region thereof, antibody with one or two carbohydrate moieties attached to one or two light chains thereof, antibody with no carbohydrate attached to one or two heavy chains of the antibody, etc., and combinations of glycosylation alterations.

Where the antibody has an Fc region, an oligosaccharide structure may be attached to one or two heavy chains of the antibody, e.g. at residue 299 (298, Eu numbering of residues). For pertuzumab, G0 was the predominant oligosaccharide structure, with other oligosaccharide structures such as G0-F, G-1, Man5, Man6, G1-1, G1(1-6), G1(1-3) and G2 being found in lesser amounts in the pertuzumab composition.

Unless indicated otherwise, a "G1 oligosaccharide structure" herein includes G-1, G1-1, G1(1-6) and G1(1-3) structures.

An "amino-terminal leader extension" herein refers to one or more amino acid residues of the amino-terminal leader sequence that are present at the amino-terminus of any one or more heavy or light chains of an antibody. An exemplary amino-terminal leader extension comprises or consists of three amino acid residues, VHS, present on one or both light chains of an antibody variant.

A "deamidated" antibody is one in which one or more asparagine residues thereof has been derivatized, e.g. to an aspartic acid, a succinimide, or an iso-aspartic acid.

### B.1 General Description of the Invention

The practice of the present invention will employ, unless otherwise indicated, conventional techniques of molecular biology (including recombinant techniques), microbiology, cell biology, and biochemistry, which are within the skill of the art. Such techniques are explained fully in the literature, such as, "Molecular Cloning: A Laboratory Manual", 2nd edition (Sambrook et al., 1989); "Oligonucleotide Synthesis" (M.J. Gait, ed., 1984); "Animal Cell Culture" (R.I. Freshney, ed., 1987); "Methods in Enzymology" (Academic Press, Inc.); "Handbook of Experimental Immunology", 4th edition (D.M. Weir & C.C. Blackwell, eds., Blackwell Science Inc., 1987); "Gene Transfer Vectors for Mammalian Cells" (J.M. Miller & M.P. Calos, eds., 1987); "Current Protocols in Molecular Biology" (F.M. Ausubel et al., eds., 1987); and "PCR: The Polymerase Chain Reaction", (Mullis et al., eds., 1994).

As discussed above, the detection or diagnosis of IBD is currently obtained by various classification systems that rely on a number of variables observed in a patient. The present invention is based on the identification of genes that are associated with IBD. Accordingly, the expression levels of such genes can serve as diagnostic markers to identify patients with IBD. As described in the Examples, the differential expression of a number of genes in IBD patients has been observed. Thus, according to the present invention, the genes listed in Table 1 have been identified as differentially expressed in IBD.

**Table 1**

| Gene | Indication(s) | Change in expression | SEQ ID NO nucleic acid | SEQ ID NO amino acid | Figure(s) |
|---|---|---|---|---|---|
| IRTA1 | CD | Decrease | 1 | 2 | 1,2 |
| CCL23 (CKbeta8-1) | CD | Decrease | 3 | 5 | 3,5 |
| CCL23 (CKbeta8) | | | 4 | 6 | 4,6 |
| CXCL13 | CD | Decrease | 7 | 8 | 7,8 |
| ATG16L1 (isoform 2) | CD | Decrease | 9 | 11 | 9,11 |
| ATG16L1 (isoform 1) | | | 10 | 12 | 10,12 |
| ATG4D | CD | Decrease | 13 | 14 | 13,14 |
| ATG3 | CD | Decrease | 15 | 16 | 15,16 |
| ATG12 | CD | Increase | 17 | 18 | 17,18 |
| ATG16L2 | CD | Increase | 19 | 20 | 19,20 |
| LC3B | CD | Increase | 21 | 22 | 21,22 |

### a. Biomarkers of the Invention

The present invention provides numerous gene expression markers or biomarkers for IBD listed in Table 1. In one embodiment of the present invention, the biomarkers are suitable for use in a panel of markers (as described herein). Such panels may include one or more markers from Table 1. Those of ordinary skill in the art will appreciate the various combinations of biomarkers from Table 1 that are suitable for use in the panels described herein.

The genes of Table 1 are considered to be differentially expressed when there is an at least about one-fold, at least about 1.5-fold, at least about 2-fold, at least about 2.5-fold, at least about 3-fold, at least about 3.5 fold, at least about 4-fold, at least about 4.5-fold, at least about 5-fold, at least about 5.5-fold, at least about 6-fold, at least about 7-fold, at least about 8-fold, at least about 9-fold, or at least about 10-fold difference between the expression of a given gene in normal and diseased subjects, or in various stages of disease development in a diseased subject.

In one embodiment of the present invention, a preferred set of IBD markers identified by microarray analysis, includes markers that are upregulated in an IBD. Preferably, the set of upregulated markers includes ATG12, ATG16L2, and LC3B (regulators of the autophagy pathway).

A preferred set of downregulated markers includes immune associated genes IRTA1- a novel surface B-cell receptor, CCL23, CXCL13, and regulators of the autophagy pathway including ATG16L1, ATG4D; and ATG3. IRTA1 is also known as FCRH4; IGFP2; IRTA1; MGC150522; MGC150523; dJ801G22.1; FCRL4. CCL23 is also known as CKb8; MIP3; Ckb-8; MIP-3; MPIF-1; SCYA23; Ckb-8-1; CK-BETA-8; CCL23. CXCL13 is also known as BLC; BCA1; ANGIE; BCA-1; BLR1L; ANGIE2; SCYB13; CXCL13. ATG16L1 is also known as IBD10; WDR30; APG16L; ATG16L; FLJ00045; FLJ10035; FLJ10828; FLJ22677; ATG16L1. ATG4D is also known as APG4D; AUTL4; APG4-D; ATG4D. ATG3 is also known as APG3; APG3L; PC3-96; FLJ22125; MGC15201; APG3-LIKE; DKFZp564M1178; ATG3. ATG12 is also known as APG12; FBR93; APG12L; HAPG12; ATG12. ATG16L2 is also known as WDR80; FLJ00012; ATG16L2. LC3B is also known as LC3B; MAP1A/1BLC3; MAP1LC3B. A panel of biomarkers as described herein may include one of, more than one of, or all of these markers. The panel may include CCL23. Alternatively, the panel may include at least one marker corresponding to a regulator of the autophagy pathway. The panel may further include one or more of IRTA1, CCL23, and CXCL13.

A panel of biomarkers may include one or more of, or all of the markers of Table 1 plus at least one marker from Figure 24, 25, 26, or 27. The panel may include at least one marker from Figure 24, 25, 26, or 27.

Members of lists provided above, as single markers or in any combination, are preferred for use in prognostic and diagnostic assays of the present invention. The IBD markers of the present invention are differentially expressed genes or regions of genes. A differential level of expression of one or more markers in a test sample from a mammalian subject relative to a control can determined from the level of RNA transcripts or expression products detected by one or more of the methods described in further detail below.

Based on evidence of differential expression of RNA transcripts in normal cells and cells from a mammalian subject having IBD, the present invention provides gene markers for IBD. The IBD markers and associated information provided by the present invention allow physicians to make more intelligent treatment decisions, and to customize the treatment of IBD to the needs of individual patients, thereby maximizing the benefit of treatment and minimizing the exposure of patients to unnecessary treatments, which do not provide any significant benefits and often carry serious risks due to toxic side-effects.

Multi-analyte gene expression tests can measure the expression level of one or more genes involved in each of several relevant physiologic processes or component cellular characteristics. In some instances the predictive power of the test, and therefore its utility, can be improved by using the expression values obtained for individual genes to calculate a score which is more highly correlated with outcome than is the expression value of the individual genes. For example, the calculation of a quantitative score (recurrence score) that predicts the likelihood of recurrence in estrogen receptor-positive, node-negative breast cancer is describe in U.S. Published Patent Application No. 20050048542. The equation used to calculate such a recurrence score may group genes in order to maximize the predictive value of the recurrence score. The grouping of genes may be performed at least in part based on knowledge of their contribution to physiologic functions or component cellular characteristics such as discussed above. The formation of groups, in addition, can facilitate the mathematical weighting of the contribution of various expression values to the recurrence score. The weighting of a gene group representing a physiological process or component cellular characteristic can reflect the contribution of that process or characteristic to the pathology of the IBD and clinical outcome. Accordingly, in an important aspect, the present invention also provides specific groups of the genes identified herein, that together are more reliable and powerful predictors of outcome than the individual genes or random combinations of the genes identified.

In addition, based on the determination of a recurrence score, one can choose to partition patients into subgroups at any particular value(s) of the recurrence score, where all patients with values in a given range can be classified as belonging to a particular risk group. Thus, the values chosen will defme subgroups of patients with respectively greater or lesser risk.

The utility of a gene marker in predicting the development or progression of an IBD may not be unique to that marker. An alternative marker having a expression pattern that is closely similar to a particular test marker may be substituted for or used in addition to a test marker and have little impact on the overall predictive utility of the test. The closely similar expression patterns of two genes may result from involvement of both genes in a particular process and/or being under common regulatory control. The present invention specifically includes and contemplates the use of such substitute genes or gene sets in the methods of the present invention.

The markers and associated information provided by the present invention predicting the development and/or progression of an IBD also have utility in screening patients for inclusion in clinical trials that test the efficacy of drug compounds for the treatment of patients with IBD.

The markers and associated information provided by the present invention predicting the presence, development and/or progression of an IBD are useful as criterion for determing whether IBD treatment is appropriate. For example, IBD treatment may be appropriate where the results of the test indicate that an IBD marker is differentially expressed in a test sample from an individual relative to a control sample. The individual may be an individual not known to have an IBD, an individual known to have an IBD, an individual previously diagnosed with an IBD undergoing treatment for the IBD, or an individual previously diagnosed with an IBD and having had surgery to address the IBD. In addition, the present invention contemplates methods of treating an IBD. As described below, the diagnostic methods of the present invention may further comprise the step of administering an IBD therapeutic agent to the mammalian subj ect that provided the test sample in which the differential expression of one or more IBD markers was observed relative to a control. Such methods of treatment would therefore comprise (a) determining the presence of an IBD in a mammalian subject, and (b) administering an IBD therapeutic agent to the mammalian subject.

In another embodiment, the IBD markers and associated information are used to design or produce a reagent that modulates the level or activity of the gene's transcript or its expression product. Said reagents may include but are not limited to an antisense RNA, a small inhibitory RNA (siRNA), a ribozyme, a monoclonal or polyclonal antibody. In a further embodiment, said gene or its transcript, or more particularly, an expression product of said transcript is used in an (screening) assay to identify a drug compound, wherein said drug compounds is used in the development of a drug to treat an IBD.

In various embodiments of the inventions, various technological approaches described below are available for determination of expression levels of the disclosed genes. In particular embodiments, the expression level of each gene may be determined in relation to various features of the expression products of the gene including exons, introns, protein epitopes and protein activity. In other embodiments, the expression level of a gene may be inferred from analysis of the structure of the gene, for example from the analysis of the methylation pattern of gene's promoter(s).

### b. Diagnostic Methods of the Invention

The present invention provides methods of detecting or diagnosing an IBD in a mammalian subject based on differential expression of an IBD marker. In a one embodiment, the methods comprise the use of a panel of IBD markers as discussed above. The panels may include one or more IBD markers selected from Table 1. In one other embodiment, the panel includes ATG16L1 and at least one additional IBD marker selected Table 1.

In some embodiments, the panel of IBD markers will include at least 1 IBD marker, at least two IBD markers, at least three IBD markers, at least 4 IBD markers, at least five IBD markers, at least 6 IBD markers, at least 7 IBD marker, at least 8 IBD markers, or at least 9 IBD markers. In one embodiment, the panel includes markers in increments of five. In another embodiment, the panel includes markers in increments of ten. The panel may include an IBD marker that is overexpressed in IBD relative to a control, an IBD marker that is underexpressed in IBD relative to a control, or IBD markers that are both overexpressed and underexpressed in IBD relative to a control. In a preferred embodiment, the panel includes one or more markers that are upregulated in CD and one or more markers that are downregulated in CD.

In another embodiment, the panels of the present invention may include an IBD marker that is overexpressed in an active IBD relative to a control, underexpressed in an active IBD relative to a control, or IBD markers that are both overexpressed and underexpressed in an active IBD relative to a control. In another embodiment, the panels of the present invention may include an IBD marker that is overexpressed in an inactive IBD relative to a control, underexpressed in an inactive IBD relative to a control, or IBD markers that are both overexpressed and underexpressed in an inactive IBD relative to a control. In a preferred embodiment, the active IBD is CD. In another preferred embodiment, the inactive IBD is CD.

In a preferred embodiment, the methods of diagnosing or detecting the presence of an IBD in a mammalian subject comprise determining a differential expression level of RNA transcripts or expression products thereof from a panel of IBD markers in a test sample obtained from the subject relative to the level of expression in a control, wherein the differential level of expression is indicative of the presence of an IBD in the subject from which the test sample was obtained. The differential expression in the test sample may be higher and/or lower relative to a control as discussed herein.

Differential expression or activity of one or more of the genes provided in the lists above, or the corresponding RNA molecules or encoded proteins in a biological sample obtained from the patient, relative to control, indicates the presence of an IBD in the patient. The control can, for example, be a gene, present in the same cell, which is known to be up-regulated (or down-regulated) in an IBD patient (positive control). Alternatively, or in addition, the control can be the expression level of the same gene in a normal cell of the same cell type (negative control). Expression levels can also be normalized, for example, to the expression levels of housekeeping genes, such as glyceraldehyde-3-phosphate-dehydrogenase (GAPDH) and/or β-actin, or to the expression levels of all genes in the sample tested. In one embodiment, expression of one or more of the above noted genes is deemed positive expression if it is at the median or above, *e.g.* compared to other samples of the same type. The median expression level can be determined essentially contemporaneously with measuring gene expression, or may have been determined previously. These and other methods are well known in the art, and are apparent to those skilled in the art.

Methods for identifying IBD patients are provided herein. Of this patient population, patients with an IBD can be identified by determining the expression level of one or more of the genes, the corresponding RNA molecules or encoded proteins in a biological sample comprising cells obtained from the patient. The biological sample can, for example, be a tissue biopsy as described herein.

The methods of the present invention concern IBD diagnostic assays, and imaging methodologies. In one embodiment, the assays are performed using antibodies as described herein. The invention also provides various immunological assays useful for the detection and quantification of proteins. These assays are performed within various immunological assay formats well known in the art, including but not limited to various types of radioimmunoassays, enzyme-linked immunosorbent assays (ELISA), enzyme-linked immunofluorescent assays (ELIFA), and the like. In addition, immunological imaging methods capable of detecting an IBD characterized by expression of a molecule described herein are also provided by the invention, including but not limited to radioscintigraphic imaging methods using labeled antibodies. Such assays are clinically useful in the detection, monitoring, diagnosis and prognosis of IBD characterized by expression of one or more molecules described herein.

Another aspect of the present invention relates to methods for identifying a cell that expresses a molecule described herein. The expression profile of a molecule(s) described herein make it a diagnostic marker for IBD. Accordingly, the status of the expression of the molecule(s) provides information useful for predicting a variety of factors including susceptibility to advanced stages of disease, rate of progression, and/or sudden and severe onset of symptoms in an active IBD or an inactive IBD, i.e. flare-ups.

In one embodiment, the present invention provides methods of detecting an IBD. A test sample from a mammalian subject and a control sample from a known normal mammal are each contacted with an anti-IBD marker antibody or a fragment thereof. The level of IBD marker expression is measured and a differential level of expression in the test sample relative to the control sample is indicative of an IBD in the mammalian subj ect from which the test sample was obtained. In some embodiments, the level of IBD marker expression in the test sample is determined to be higher than the level of expression in the control, wherein the higher level of expression indicates the presence of an IBD in the subject from which the test sample was obtained. In another embodiments, the level of IBD marker expression in the test sample is determined to be lower than the level of expression in the control, wherein the lower level of expression indicates the presence of an IBD in the subject from which the test sample was obtained.

In another embodiment, the IBD detected by the methods of the present invention is the recurrence or flareup of an IBD in the mammalian subject.

In preferred embodiments, the methods are employed to detect the flare-up of an IBD or a recurrence of an IBD in a mammalian subject previously determined to have an IBD who underwent treatment for the IBD, such as drug therapy or a surgical procedure. Following initial detection of an IBD, additional test samples may be obtained from the mammalian subject found to have an IBD. The additional sample may be obtained hours, days, weeks, or months after the initial sample was taken. Those of skill in the art will appreciate the appropriate schedule for obtaining such additional samples, which may include second, third, fourth, fifth, sixth, etc. test samples. The intial test sample and the additional sample (and alternately a control sample as described herein) are contacted with an anti-IBD marker antibody. The level of IBD marker expression is measured and a differential level of expression in the additional test sample as compared to the initial test sample is indicative of a flare-up in or a recurrence of an IBD in the mammalian subject from which the test sample was obtained.

In one aspect, the methods of the present invention are directed to a determining step. In one embodiment, the determining step comprises measuring the level of expression of one or more IBD markers in a test sample relative to a control. Typically, measuring the level of IBD marker expression, as described herein, involves analyzing a test sample for differential expression of an IBD marker relative to a control by performing one or more of the techniques described herein. The expression level data obtained from a test sample and a control are compared for differential levels of expression. In another embodiment, the determining step further comprises an examination of the test sample and control expression data to assess whether an IBD is present in the subject from which the test sample was obtained.

The methods of the present invention are valuable tools for detecting and IBD marker. Measurement of biomarker expression or protein levels may be performed by using a software program executed by a suitable processor. Suitable software and processors are well known in the art and are commercially available. The program may be embodied in software stored on a tangible medium such as CD-ROM, a floppy disk, a hard drive, a DVD, or a memory associated with the processor, but persons of ordinary skill in the art will readily appreciate that the entire program or parts thereof could alternatively be executed by a device other than a processor, and/or embodied in firmware and/or dedicated hardware in a well known manner.

Following the measurement of one or more IBD markers, the assay results, findings, diagnoses, predictions and/or treatment recommendations are typically recorded and communicated to technicians, physicians and/or patients, for example. In certain embodiments, computers will be used to communicate such information to interested parties, such as, patients and/or the attending physicians. In some embodiments, the assays will be performed or the assay results analyzed in a country or jurisdiction which differs from the country or jurisdiction to which the results or diagnoses are communicated.

To facilitate diagnosis, the level of one or more IBD markers can be displayed on a display device, contained electronically, or in a machine-readable medium, such as but not limited to, analog tapes like those readable by a VCR, CD-ROM, DVD-ROM, USB flash media, among others. Such machine-readable media can also contain additional test results, such as, without limitation, measurements of clinical parameters and traditional laboratory risk factors. Alternatively or additionally, the machine-readable media can also comprise subject information such as medical history and any relevant family history.

The methods of this invention, when practiced for commercial diagnostic purposes generally produce a report or summary of the normalized levels of one or more of the biomarkers described herein. The methods of this invention will produce a report comprising one or more predictions concerning a patient and an IBD.

The methods and reports of this invention can further include storing the report in a database. Alternatively, the method can further create a record in a database for the subject and populate the record with data. In one embodiment the report is a paper report, in another embodiment the report is an auditory report, in another embodiment the report is an electronic record. It is contemplated that the report is provided to a physician and/or the patient. The receiving of the report can further include establishing a network connection to a server computer that includes the data and report and requesting the data and report from the server computer. The methods provided by the present invention may also be automated in whole or in part.

In some embodiments, the determining step comprises the use of a software program executed by a suitable processor for the purpose of (i) measuring the differential level of IBD marker expression in a test sample and a control; and/or (ii) analyzing the data obtained from measuring differential level of IBD marker expression in a test sample and a control. Suitable software and processors are well known in the art and are commercially available. The program may be embodied in software stored on a tangible medium such as CD-ROM, a floppy disk, a hard drive, a DVD, or a memory associated with the processor, but persons of ordinary skill in the art will readily appreciate that the entire program or parts thereof could alternatively be executed by a device other than a processor, and/or embodied in firmware and/or dedicated hardware in a well known manner.

Following the determining step, the measurement results, findings, diagnoses, predictions and/or treatment recommendations are typically recorded and communicated to technicians, physicians and/or patients, for example. In certain embodiments, computers will be used to communicate such information to interested parties, such as, patients and/or the attending physicians. In some embodiments, the assays will be performed or the assay results analyzed in a country or jurisdiction which differs from the country or jurisdiction to which the results or diagnoses are communicated.

In a preferred embodiment, a diagnosis, prediction and/or treatment recommendation based on the level of expression of one or more IBD markers disclosed herein measured in a test subject of having one or more of the IBD markers herein is communicated to the subject as soon as possible after the assay is completed and the diagnosis and/or prediction is generated. The results and/or related information may be communicated to the subject by the subject's treating physician. Alternatively, the results may be communicated directly to a test subject by any means of communication, including writing, electronic forms of communication, such as email, or telephone. Communication may be facilitated by use of a computer, such as in case of email communications. In certain embodiments, the communication containing results of a diagnostic test and/or conclusions drawn from and/or treatment recommendations based on the test, may be generated and delivered automatically to the subject using a combination of computer hardware and software which will be familiar to artisans skilled in telecommunications. One example of a healthcare-oriented communications system is described in U.S. Pat. No. 6,283,761; however, the present invention is not limited to methods which utilize this particular communications system. In certain embodiments of the methods of the invention, all or some of the method steps, including the assaying of samples, diagnosing of diseases, and communicating of assay results or diagnoses, may be carried out in diverse (e.g., foreign) jurisdictions.

The invention provides assays for detecting the differential expression of an IBD marker in tissues associated with the gastrointestinal tract including, without limitation, ascending colon tissue, descending colon tissue, sigmoid colon tissue, and terminal ileum tissue; as well expression in other biological samples such as serum, semen, bone, prostate, urine, cell preparations, and the like. Methods for detecting differential expression of an IBD marker are also well known and include, for example, immunoprecipitation, immunohistochemical analysis, Western blot analysis, molecular binding assays, ELISA, ELIFA and the like. For example, a method of detecting the differential expression of an IBD marker in a biological sample comprises first contacting the sample with an anti-IBD marker antibody, an IBD marker-reactive fragment thereof, or a recombinant protein containing an antigen-binding region of an anit-IBD marker antibody; and then detecting the binding of an IBD marker protein in the sample.

In various embodiments of the inventions, various technological approaches are available for determination of expression levels of the disclosed genes, including, without limitation, RT-PCR, microarrays, serial analysis of gene expression (SAGE) and Gene Expression Analysis by Massively Parallel Signature Sequencing (MPSS), which will be discussed in detail below. In particular embodiments, the expression level of each gene may be determined in relation to various features of the expression products of the gene including exons, introns, protein epitopes and protein activity. In other embodiments, the expression level of a gene may be inferred from analysis of the structure of the gene, for example from the analysis of the methylation pattern of gene's promoter(s).

In one embodiment, the present invention provides a method of diagnosing the presence of an IBD in a mammalian subject by determining that the level of expression of a nucleic acid encoding a polypeptide of Table 1 in a test sample obtained from the subject is different relative to the level of expression in a control, wherein the different level of expression is indicative of the presence of an IBD in the subject from which the test sample was obtained.

In the methods described herein, the determining step may be preceded by the step of obtaining a test sample from the mammalian subject. The determining step may also be preceded by the step of contacting a test sample from the mammalian subject with an agent for the detection of the differential level of expression.

In another embodiment, the present invention provides a method of diagnosing the degree of IBD-associated inflammation in a mammalian subject by determining that the level of expression of a nucleic acid encoding a polypeptide of Table 1 in a test sample obtained from the subject is different relative to the level of expression in a control, wherein the different level of expression is indicative of the degree of IBD-associated inflammation in the the subject from which the test sample was obtained. In another embodiment, the determining step is preceded by the step of obtaining a test sample from the mammalian subject. In one other embodiment, the determining step is preceded by the step of contacting a test sample from the mammalian subject with an agent for the detection of the differential level of expression.

### c. Therapeutic Methods of the Invention

The present invention provides therapeutic methods of treating an IBD in a subject in need that comprise detecting the presence of an IBD in a mammalian subject by the diagnostic methods described herein and then administering to the mammalian subject an IBD therapeutic agent. Those of ordinary skill in the art will appreciate the various IBD therapeutic agents that may be suitable for use in the present invention (see St Clair Jones, Hospital Pharmacist, May 2006, Vol. 13; pages 161-166, hereby incorporated by reference in its entirety). The present invention contemplates methods of IBD treatment in which one or more IBD therapeutic agents are administered to a subject in need. In one embodiment, the IBD therapeutic agent is one or more of an aminosalicylate, a corticosteroid, and an immunosuppressive agent. In a preferred embodiment, the aminosalicylate is one of sulfasalazine, olsalazine, mesalamine, balsalazide, and asacol. In another preferred embodiment, multiple aminosalicylates are co-administered, such as a combination of sulfasalazine and olsalazine. In other preferred embodiments, the corticosteroid may be budesonide, prednisone, prednisolone, methylprednisolone, 6-mercaptopurine (6-MP), azathioprine, methotrexate, and cyclosporin. In other preferred embodiments, the IBD therapeutic agent may an antibiotic, such as ciprofloxacin and/or metronidazole; or an antibody-based agent such as infliximab (Remicade®).

The least toxic IBD therapeutic agents which patients are typically treated with are the aminosalicylates. Sulfasalazine (Azulfidine), typically administered four times a day, consists of an active molecule of aminosalicylate (5-ASA) which is linked by an azo bond to a sulfapyridine. Anaerobic bacteria in the colon split the azo bond to release active 5-ASA. However, at least 20% of patients cannot tolerate sulfapyridine because it is associated with significant side-effects such as reversible sperm abnormalities, dyspepsia or allergic reactions to the sulpha component. These side effects are reduced in patients taking olsalazine. However, neither sulfasalazine nor olsalazine are effective for the treatment of small bowel inflammation. Other formulations of 5-ASA have been developed which are released in the small intestine (e.g. mesalamine and asacol). Normally it takes 6-8 weeks for 5-ASA therapy to show full efficacy. Patients who do not respond to 5-ASA therapy, or who have a more severe disease, are prescribed corticosteroids. However, this is a short term therapy and cannot be used as a maintenance therapy. Clinical remission is achieved with corticosteroids within 2-4 weeks, however the side effects are significant and include Cushing goldface, facial hair, severe mood swings and sleeplessness. The response to sulfasalazine and 5-aminosalicylate preparations is poor in CD, fair to mild in early ulcerative colitis and poor in severe UC. If these agents fail, powerful immunosuppressive agents such as cyclosporine, prednisone, 6-mercaptopurine or azathioprine (converted in the liver to 6-mercaptopurine) are typically tried. For CD patients, the use of corticosteroids and other immunosuppressives must be carefully monitored because of the high risk of intra-abdominal sepsis originating in the fistulas and abscesses common in this disease. Approximately 25% of IBD patients will require surgery (colectomy) during the course of the disease.

Treatment of an IBD may include a surgical procedure, including without limitation, a bowel resection, anastomosis, a colectomy, a proctocolectomy, and an ostomy, or any combination thereof.

In addition to pharmaceutical medicine and surgery, nonconventional treatments for IBD such as nutritional therapy have also been attempted. For example, Flexical®, a semi-elemental formula, has been shown to be as effective as the steroid prednisolone. Sanderson et al., Arch. Dis. Child. 51:123-7 (1987). However, semi-elemental formulas are relatively expensive and are typically unpalatable- thus their use has been restricted. Nutritional therapy incorporating whole proteins has also been attempted to alleviate the symptoms of IBD. Giafer et al., Lancet 335: 816-9 (1990). U.S. Patent No. 5,461,033 describes the use of acidic casein isolated from bovine milk and TGF-2. Beattie et al., Aliment. Pharmacol. Ther. 8: 1-6 (1994) describes the use of casein in infant formula in children with IBD. U.S.P. 5,952,295 describes the use of casein in an enteric formulation for the treatment of IBD. However, while nutrional therapy is non-toxic, it is a palliative treatment and does not treat the underlying cause of the disease.

The present invention contemplates methods of IBD treatment, including for example, in vitro, ex vivo and in vivo therapeutic methods. The invention provides methods useful for treating an IBD in a subject in need upon the detection of an IBD disease state in the subject associated with the expression of one or more IBD markers disclosed herein, such as increased and/or decreased IBD marker expression. In one preferred embodiment, the method comprises (a) determining that the level of expression of (i) one or more nucleic acids encoding one or more polypeptides selected from Table 1; or (ii) RNA transcripts or expression products thereof of one or more genes listed in Table 1 in a test sample obtained from said subject is higher and/or lower relative to the level of expression in a control, wherein said higher and/or lower level of expression is indicative of the presence of an IBD in the subject from which the test sample was obtained; and (b) administering to said subject an effective amount of an IBD therapeutic agent. The determining step (a) may comprise the measurement of the expression of multiple IBD marker

The method of treatment comprises detecting the IBD and administering an effective amount of an IBD therapeutic agent to a subject in need of such treatment. In some embodiments, the IBD disease state is associated with an increased and/or decrease in expression of one or more IBD markers.

In one aspect, the invention provides methods for treating or preventing an IBD, the methods comprising detecting the presence of an IBD in a subject and administering an effective amount of an

IBD therapeutic agent to the subject. It is understood that any suitable IBD therapeutic agent may be used in the methods of treatment, including aminosalicylates, corticosteroids, and immunosuppressive agents as discussed herein.

In any of the methods herein, one may administer to the subject or patient along with a single IBD therapeutic agent discussed herein an effective amount of a second medicament (where the single IBD therapeutic agent herein is a first medicament), which is another active agent that can treat the condition in the subject that requires treatment. For instance, an aminosalicylate may be co-administered with a corticosteroid, an immunsuppressive agent, or another aminosalicylate. The type of such second medicament depends on various factors, including the type of IBD, its severity, the condition and age of the patient, the type and dose of first medicament employed, etc.

Such treatments using first and second medicaments include combined administration (where the two or more agents are included in the same or separate formulations), and separate administration, in which case, administration of the first medicament can occur prior to, and/or following, administration of the second medicament. In general, such second medicaments may be administered within 48 hours after the first medicaments are administered, or within 24 hours, or within 12 hours, or within 3-12 hours after the first medicament, or may be administered over a pre-selected period of time, which is preferably about 1 to 2 days, about 2 to 3 days, about 3 to 4 days, about 4 to 5 days, about 5 to 6 days, or about 6 to 7 days.

The first and second medicaments can be administered concurrently, sequentially, or alternating with the first and second medicament or upon non-responsiveness with other therapy. Thus, the combined administration of a second medicament includes co-administration (concurrent administration), using separate formulations or a single pharmaceutical formulation, and consecutive administration in either order, wherein preferably there is a time period while both (or all) medicaments simultaneously exert their biological activities. All these second medicaments may be used in combination with each other or by themselves with the first medicament, so that the express "second medicament" as used herein does not mean it is the only medicament besides the first medicament, respectively. Thus, the second medicament need not be one medicament, but may constitute or comprise more than one such drug. These second medicaments as set forth herein are generally used in the same dosages and with administration routes as the first medicaments, or about from 1 to 99% of the dosages of the first medicaments. If such second medicaments are used at all, preferably, they are used in lower amounts than if the first medicament were not present, especially in subsequent dosings beyond the initial dosing with the first medicament, so as to eliminate or reduce side effects caused thereby.

Where the methods of the present invention comprise administering one or more IBD therapeutic agent to treat or prevent an IBD, it may be particularly desirable to combine the administering step with a surgical procedure that is also performed to treat or prevent the IBD. The IBD surgical procedures contemplated by the present invention include, without limitation, a bowel resection, anastomosis, a colectomy, a proctocolectomy, and an ostomy, or any combination thereof. For instance, an IBD therapeutic agent described herein may be combined with a colectomy in a treatment scheme, e.g. in treating an IBD. Such combined therapies include and separate administration, in which case, administration of the IBD therapeutic agent can occur prior to, and/or following, the surgical procedure.

Treatment with a combination of one or more IBD therapeutic agents; or a combination of one or more IBD therapeutic agents and a surgical procedure described herein preferably results in an improvement in the signs or symptoms of an IBD. For instance, such therapy may result in an improvement in the subject receiving the IBD therapeutic agent treatment regimen and a surgical procedure, as evidenced by a reduction in the severity of the pathology of the IBD.

The IBD therapeutic agent(s) is/are administered by any suitable means, including parenteral, subcutaneous, intraperitoneal, intrapulmonary, and intranasal, and, if desired for local treatment, intralesional administration. Parenteral infusions include intramuscular, intravenous, intraarterial, intraperitoneal, or subcutaneous administration. Dosing can be by any suitable route, e.g. by injections, such as intravenous or subcutaneous injections, depending in part on whether the administration is brief or chronic.

The IBD therapeutic agent(s) compositions administered according to the methods of the invention will be formulated, dosed, and administered in a fashion consistent with good medical practice. Factors for consideration in this context include the particular disorder being treated, the particular mammal being treated, the clinical condition of the individual patient, the cause of the disorder, the site of delivery of the agent, the method of administration, the scheduling of administration, and other factors known to medical practitioners. The first medicament(s) need not be, but is optionally formulated with one or more additional medicament(s) (e.g. second, third, fourth, etc. medicaments) described herein. The effective amount of such additional medicaments depends on the amount of the first medicament present in the formulation, the type of disorder or treatment, and other factors discussed above. These are generally used in the same dosages and with administration routes as used hereinbefore or about from 1 to 99% of the heretofore employed dosages.

For the prevention or treatment of an IBD, the appropriate dosage of an IBD therapeutic agent (when used alone or in combination with other agents) will depend on the type of disease to be treated, the type of IBD therapeutic agent(s), the severity and course of the disease, whether the IBD therapeutic agent is administered for preventive or therapeutic purposes, previous therapy, the patient's clinical history and response to the IBD therapeutic agent, and the discretion of the attending physician. The IBD therapeutic agent is suitably administered to the patient at one time or over a series of treatments. Depending on the type and severity of the disease, about 1 ug/kg to 15 mg/kg (e.g. 0.1 mg/kg-10 mg/kg) of IBD therapeutic agent is an initial candidate dosage for administration to the patient, whether, for example, by one or more separate administrations, or by continuous infusion. One typical daily dosage might range from about 1 ug/kg to 100 mg/kg or more, depending on the factors mentioned above. For repeated administrations over several days or longer, depending on the condition, the treatment is sustained until a desired suppression of disease symptoms occurs. One exemplary dosage of the IBD therapeutic agent would be in the range from about 0.05 mg/kg to about 10 mg/kg. Thus, one or more doses of about 0.5 mg/kg, 2.0 mg/kg, 4.0 mg/kg or 10 mg/kg (or any combination thereof) may be administered to the patient. Such doses may be administered intermittently, e.g. every week or every three weeks (e.g. such that the patient receives from about two to about twenty, e.g. about six doses of the IBD therapeutic agent). An initial higher loading dose, followed by one or more lower doses may be administered. An exemplary dosing regimen comprises administering an initial loading dose of about 4 mg/kg, followed by a weekly maintenance dose of about 2 mg/kg of the IBD therapeutic agent. However, other dosage regimens may be useful. The progress of this therapy is easily monitored by conventional techniques and assays.

### B.2. Gene Expression Profiling

In general, methods of gene expression profiling can be divided into two large groups: methods based on hybridization analysis of polynucleotides, and other methods based on biochemical detection or sequencing of polynucleotides. The most commonly used methods known in the art for the quantification of mRNA expression in a sample include northern blotting and in situ hybridization (Parker & Barnes, Methods in Molecular Biology 106:247-283 (1999)); RNAse protection assays (Hod, Biotechniques 13:852-854 (1992)); and reverse transcription polymerase chain reaction (RT-PCR) (Weis et al., Trends in Genetics 8:263-264 (1992)). Alternatively, antibodies may be employed that can recognize specific duplexes, including DNA duplexes, RNA duplexes, and DNA-RNA hybrid duplexes or DNA-protein duplexes. Various methods for determining expression of mRNA or protein include, but are not limited to, gene expression profiling, polymerase chain reaction (PCR) including quantitative real time PCR (qRT-PCR), microarray analysis that can be performed by commercially available equipment, following manufacturer's protocols, such as by using the Affymetrix GenChip technology, serial analysis of gene expression (SAGE) (Velculescu et al., Science 270:484-487 (1995); and Velculescu et al., Cell 88:243-51 (1997)), MassARRAY, Gene Expression Analysis by Massively Parallel Signature Sequencing (MPSS) (Brenner et al., Nature Biotechnology 18:630-634 (2000)), proteomics, immunohistochemistry (IHC), etc. Preferably mRNA is quantified. Such mRNA analysis is preferably performed using the technique of polymerase chain reaction (PCR), or by microarray analysis. Where PCR is employed, a preferred form of PCR is quantitative real time PCR (qRT-PCR).

### a. Reverse Transcriptase PCR (RT-PCR)

Of the techniques listed above, the most sensitive and most flexible quantitative method is RT-PCR, which can be used to compare mRNA levels in different sample populations, in normal and test sample tissues, to characterize patterns of gene expression, to discriminate between closely related mRNAs, and to analyze RNA structure.

The first step is the isolation of mRNA from a target sample. The starting material is typically total RNA isolated from colonic tissue biopsies. Thus, RNA can be isolated from a variety of tissues, including without limitation, the terminal ileum, the ascending colon, the descending colon, and the sigmoid colon. In addition, the colonic tissue from which a biopsy is obtained may be from an inflamed and/or a non-inflamed colonic area.

In one embodiment, the mRNA is obtained from a biopsy as defined above wherein the biopsy is obtained from the left colon or from the right colon. As used herein, the "left colon" refers to the sigmoideum and rectosigmoideum and the "right colon" refers to the cecum.

General methods for mRNA extraction are well known in the art and are disclosed in standard textbooks of molecular biology, including Ausubel et al., Current Protocols of Molecular Biology, John Wiley and Sons (1997). In particular, RNA isolation can be performed using purification kit, buffer set and protease from commercial manufacturers, such as Qiagen, according to the manufacturer's instructions. Total RNA from tissue samples can be isolated using RNA Stat-60 (Tel-Test). RNA prepared from a biopsy can be isolated, for example, by cesium chloride density gradient centrifugation.

As RNA cannot serve as a template for PCR, the first step in gene expression profiling by RT-PCR is the reverse transcription of the RNA template into cDNA, followed by its exponential amplification in a PCR reaction. The two most commonly used reverse transcriptases are avilo myeloblastosis virus reverse transcriptase (AMV-RT) and Moloney murine leukemia virus reverse transcriptase (MMLV-RT). The reverse transcription step is typically primed using specific primers, random hexamers, or oligo-dT primers, depending on the circumstances and the goal of expression profiling. For example, extracted RNA can be reverse-transcribed using a GeneAmp RNA PCR kit (Perkin Elmer, CA, USA), following the manufacturer's instructions. The derived cDNA can then be used as a template in the subsequent PCR reaction.

Although the PCR step can use a variety of thermostable DNA-dependent DNA polymerases, it typically employs the Taq DNA polymerase, which has a 5'-3' nuclease activity but lacks a 3'-5' proofreading endonuclease activity. Thus, TaqMan® PCR typically utilizes the 5'-nuclease activity of Taq or Tth polymerase to hydrolyze a hybridization probe bound to its target amplicon, but any enzyme with equivalent 5' nuclease activity can be used. Two oligonucleotide primers are used to generate an amplicon typical of a PCR reaction. A third oligonucleotide, or probe, is designed to detect nucleotide sequence located between the two PCR primers. The probe is non-extendible by Taq DNA polymerase enzyme, and is labeled with a reporter fluorescent dye and a quencher fluorescent dye. Any laser-induced emission from the reporter dye is quenched by the quenching dye when the two dyes are located close together as they are on the probe. During the amplification reaction, the Taq DNA polymerase enzyme cleaves the probe in a template-dependent manner. The resultant probe fragments disassociate in solution, and signal from the released reporter dye is free from the quenching effect of the second fluorophore. One molecule of reporter dye is liberated for each new molecule synthesized, and detection of the unquenched reporter dye provides the basis for quantitative interpretation of the data.

TaqMan® RT-PCR can be performed using commercially available equipment, such as, for example, ABI PRISM 7700TM Sequence Detection SystemTM (Perkin-Elmer-Applied Biosystems, Foster City, CA, USA), or Lightcycler (Roche Molecular Biochemicals, Mannheim, Germany). In a preferred embodiment, the 5' nuclease procedure is run on a real-time quantitative PCR device such as the ABI PRISM 7700TM Sequence Detection SystemTM. The system consists of a thermocycler, laser, charge-coupled device (CCD), camera and computer. The system amplifies samples in a 96-well format on a thermocycler. During amplification, laser-induced fluorescent signal is collected in real-time through fiber optics cables for all 96 wells, and detected at the CCD. The system includes software for running the instrument and for analyzing the data.

5'-Nuclease assay data are initially expressed as Ct, or the threshold cycle. As discussed above, fluorescence values are recorded during every cycle and represent the amount of product amplified to that point in the amplification reaction. The point when the fluorescent signal is first recorded as statistically significant is the threshold cycle (Ct).

To minimize errors and the effect of sample-to-sample variation, RT-PCR is usually performed using an internal standard. The ideal internal standard is expressed at a constant level among different tissues, and is unaffected by the experimental treatment. RNAs most frequently used to normalize patterns of gene expression are mRNAs for the housekeeping genes glyceraldehyde-3-phosphate-dehydrogenase (GAPDH) and β-actin.

A more recent variation of the RT-PCR technique is the real time quantitative PCR, which measures PCR product accumulation through a dual-labeled fluorigenic probe (i.e., TaqMan® probe). Real time PCR is compatible both with quantitative competitive PCR, where internal competitor for each target sequence is used for normalization, and with quantitative comparative PCR using a normalization gene contained within the sample, or a housekeeping gene for RT-PCR. For further details see, e.g. Held et al., Genome Research 6:986-994 (1996).

According to one aspect of the present invention, PCR primers and probes are designed based upon intron sequences present in the gene to be amplified. In this embodiment, the first step in the primer/probe design is the delineation of intron sequences within the genes. This can be done by publicly available software, such as the DNA BLAT software developed by Kent, W.J., Genome Res. 12(4):656-64 (2002), or by the BLAST software including its variations. Subsequent steps follow well established methods of PCR primer and probe design.

In order to avoid non-specific signals, it is important to mask repetitive sequences within the introns when designing the primers and probes. This can be easily accomplished by using the Repeat Masker program available on-line through the Baylor College of Medicine, which screens DNA sequences against a library of repetitive elements and returns a query sequence in which the repetitive elements are masked. The masked intron sequences can then be used to design primer and probe sequences using any commercially or otherwise publicly available primer/probe design packages, such as Primer Express (Applied Biosystems); MGB assay-by-design (Applied Biosystems); Primer3 (Steve Rozen and Helen J. Skaletsky (2000) Primer3 on the WWW for general users and for biologist programmers. In: Krawetz S, Misener S (eds) Bioinformatics Methods and Protocols: Methods in Molecular Biology. Humana Press, Totowa, NJ, pp 365-386).

The most important factors considered in PCR primer design include primer length, melting temperature (Tm), and G/C content, specificity, complementary primer sequences, and 3'-end sequence. In general, optimal PCR primers are generally 17-30 bases in length, and contain about 20-80%, such as, for example, about 50-60% G+C bases. Tm's between 50 and 80 °C, e.g. about 50 to 70 °C are typically preferred.

For further guidelines for PCR primer and probe design see, e.g. Dieffenbach, C.W. et al., "General Concepts for PCR Primer Design" in: PCR Primer, A Laboratory Manual, Cold Spring Harbor Laboratory Press, New York, 1995, pp. 133-155; Innis and Gelfand, "Optimization of PCRs" in: PCR Protocols, A Guide to Methods and Applications, CRC Press, London, 1994, pp. 5-11; and Plasterer, T.N. Primerselect: Primer and probe design. Methods Mol. Biol. 70:520-527 (1997), the entire disclosures of which are hereby expressly incorporated by reference.

Further PCR-based techniques include, for example, differential display (Liang and Pardee, Science 257:967-971 (1992)); amplified fragment length polymorphism (iAFLP) (Kawamoto et al., Genome Res. 12:1305-1312 (1999)); BeadArray™ technology (Illumina, San Diego, CA; Oliphant et al., Discovery of Markers for Disease (Supplement to Biotechniques), June 2002; Ferguson et al., Analytical Chemistry 72:5618 (2000)); BeadsArray for Detection of Gene Expression (BADGE), using the commercially available Luminex100 LabMAP system and multiple color-coded microspheres (Luminex Corp., Austin, TX) in a rapid assay for gene expression (Yang et al., Genome Res. 11:1888-1898 (2001)); and high coverage expression profiling (HiCEP) analysis (Fukumura et al., Nucl. Acids. Res. 31(16) e94 (2003)).

### b. Microarrays

Differential gene expression can also be identified, or confirmed using the microarray technique. Thus, the expression profile of IBD-associated genes can be measured in either fresh or paraffin-embedded tissue, using microarray technology. In this method, polynucleotide sequences of interest (including cDNAs and oligonucleotides) are plated, or arrayed, on a microchip substrate. The arrayed sequences are then hybridized with specific DNA probes from cells or tissues of interest. Just as in the RT-PCR method, the source of mRNA typically is total RNA isolated from biopsy tissue or cell lines derived from cells obtained from a subject having an IBD, and corresponding normal tissues or cell lines. Thus RNA can be isolated from a variety of colonic tissues or colonic tissue-based cell lines.

In a specific embodiment of the microarray technique, PCR amplified inserts of cDNA clones are applied to a substrate in a dense array. Preferably at least 10,000 nucleotide sequences are applied to the substrate. The microarrayed genes, immobilized on the microchip at 10,000 elements each, are suitable for hybridization under stringent conditions. Fluorescently labeled cDNA probes may be generated through incorporation of fluorescent nucleotides by reverse transcription of RNA extracted from tissues of interest. Labeled cDNA probes applied to the chip hybridize with specificity to each spot of DNA on the array. After stringent washing to remove non-specifically bound probes, the chip is scanned by confocal laser microscopy or by another detection method, such as a CCD camera. Quantitation of hybridization of each arrayed element allows for assessment of corresponding mRNA abundance. With dual color fluorescence, separately labeled cDNA probes generated from two sources of RNA are hybridized pairwise to the array. The relative abundance of the transcripts from the two sources corresponding to each specified gene is thus determined simultaneously. The miniaturized scale of the hybridization affords a convenient and rapid evaluation of the expression pattern for large numbers of genes. Such methods have been shown to have the sensitivity required to detect rare transcripts, which are expressed at a few copies per cell, and to reproducibly detect at least approximately two-fold differences in the expression levels (Schena et al., Proc. Natl. Acad. Sci. USA 93(2):106-149 (1996)). Microarray analysis can be performed by commercially available equipment, following manufacturer's protocols, such as by using the Affymetrix GenChip technology, or Incyte's microarray technology, or Agilent's Whole Human Genome microarray technology.

### c. Serial Analysis of Gene Expression (SAGE)

Serial analysis of gene expression (SAGE) is a method that allows the simultaneous and quantitative analysis of a large number of gene transcripts, without the need of providing an individual hybridization probe for each transcript. First, a short sequence tag (about 10-14 bp) is generated that contains sufficient information to uniquely identify a transcript, provided that the tag is obtained from a unique position within each transcript. Then, many transcripts are linked together to form long serial molecules, that can be sequenced, revealing the identity of the multiple tags simultaneously. The expression pattern of any population of transcripts can be quantitatively evaluated by determining the abundance of individual tags, and identifying the gene corresponding to each tag. For more details see, e.g. Velculescu et al., Science 270:484-487 (1995); and Velculescu et al., Cell 88:243-51 (1997).

### d. MassARRAY Technology

In the MassARRAY®-based gene expression profiling method, developed by Sequenom, Inc. (San Diego, CA) following the isolation of RNA and reverse transcription, the obtained cDNA is spiked with a synthetic DNA molecule (competitor), which matches the targeted cDNA region in all positions, except a single base, and serves as an internal standard. The cDNA/competitor mixture is PCR amplified and is subjected to a post-PCR shrimp alkaline phosphatase (SAP) enzyme treatment, which results in the dephosphorylation of the remaining nucleotides. After inactivation of the alkaline phosphatase, the PCR products from the competitor and cDNA are subjected to primer extension, which generates distinct mass signals for the competitor- and cDNA-derives PCR products. After purification, these products are dispensed on a chip array, which is pre-loaded with components needed for analysis with matrix-assisted laser desorption ionization time-of-flight mass spectrometry (MALDI-TOF MS) analysis. The cDNA present in the reaction is then quantified by analyzing the ratios of the peak areas in the mass spectrum generated. For further details see, e.g. Ding and Cantor, Proc. Natl. Acad. Sci. USA 100:3059-3064 (2003).

### e. Gene Expression Analysis by Massively Parallel Signature Sequencing (MPSS)

This method, described by Brenner et al., Nature Biotechnology 18:630-634 (2000), is a sequencing approach that combines non-gel-based signature sequencing with *in vitro* cloning of millions of templates on separate 5 µm diameter microbeads. First, a microbead library of DNA templates is constructed by *in vitro* cloning. This is followed by the assembly of a planar array of the template-containing microbeads in a flow cell at a high density (typically greater than 3 x 10⁶ microbeads/cm²). The free ends of the cloned templates on each microbead are analyzed simultaneously, using a fluorescence-based signature sequencing method that does not require DNA fragment separation. This method has been shown to simultaneously and accurately provide, in a single operation, hundreds of thousands of gene signature sequences from a yeast cDNA library.

The steps of a representative protocol for profiling gene expression using fixed, paraffin-embedded tissues as the RNA source, including mRNA isolation, purification, primer extension and amplification are given in various published journal articles (for example: Godfrey et al. J. Molec. Diagnostics 2: 84-91 (2000); Specht et al., Am. J. Pathol. 158: 419-29 (2001)). Briefly, a representative process starts with cutting about 10 microgram thick sections of paraffin-embedded tissue samples. The mRNA is then extracted, and protein and DNA are removed. General methods for mRNA extraction are well known in the art and are disclosed in standard textbooks of molecular biology, including Ausubel et al., Current Protocols of Molecular Biology, John Wiley and Sons (1997). Methods for RNA extraction from paraffin embedded tissues are disclosed, for example, in Rupp and Locker, Lab Invest. 56:A67 (1987), and De Andrés et al., BioTechniques 18:42044 (1995). In particular, RNA isolation can be performed using purification kit, buffer set and protease from commercial manufacturers, such as Qiagen, according to the manufacturer's instructions. For example, total RNA from cells in culture can be isolated using Qiagen RNeasy mini-columns. Other commercially available RNA isolation kits include MasterPure™ Complete DNA and RNA Purification Kit (EPICENTRE®, Madison, WI), and Paraffin Block RNA Isolation Kit (Ambion, Inc.). Total RNA from tissue samples can be isolated using RNA Stat-60 (Tel-Test). RNA prepared from tissues can be isolated, for example, by cesium chloride density gradient centrifugation. After analysis of the RNA concentration, RNA repair and/or amplification steps may be included, if necessary, and RNA is reverse transcribed using gene specific promoters followed by PCR. Peferably, real time PCR is used, which is compatible both with quantitative competitive PCR, where internal competitor for each target sequence is used for normalization, and with quantitative comparative PCR using a normalization gene contained within the sample, or a housekeeping gene for RT-PCR. For further details see, e.g. "PCR: The Polymerase Chain Reaction", Mullis et al., eds., 1994; and Held et al., Genome Research 6:986-994 (1996). Finally, the data are analyzed to identify the best treatment option(s) available to the patient on the basis of the characteristic gene expression pattern identified in the sample examined.

### f. Immunohistochemistry

Immunohistochemistry methods are also suitable for detecting the expression levels of the IBD markers of the present invention. Thus, antibodies or antisera, preferably polyclonal antisera, and most preferably monoclonal antibodies specific for each marker are used to detect expression. The antibodies can be detected by direct labeling of the antibodies themselves, for example, with radioactive labels, fluorescent labels, hapten labels such as, biotin, or an enzyme such as horse radish peroxidase or alkaline phosphatase. Alternatively, unlabeled primary antibody is used in conjunction with a labeled secondary antibody, comprising antisera, polyclonal antisera or a monoclonal antibody specific for the primary antibody. Immunohistochemistry protocols and kits are well known in the art and are commercially available.

Expression levels can also be determined at the protein level, for example, using various types of immunoassays or proteomics techniques.

In immunoassays, the target diagnostic protein marker is detected by using an antibody specifically binding to the markes. The antibody typically will be labeled with a detectable moiety. Numerous labels are available which can be generally grouped into the following categories:
Radioisotopes, such as 35S, 14C, 125I, 3H, and 131I. The antibody can be labeled with the radioisotope using the techniques described in Current Protocols in Immunology, Volumes 1 and 2, Coligen et al. (1991) Ed. Wiley-Interscience, New York, New York, Pubs. for example and radioactivity can be measured using scintillation counting.

Fluorescent labels such as rare earth chelates (europium chelates) or fluorescein and its derivatives, rhodamine and its derivatives, dansyl, Lissamine, phycoerythrin and Texas Red are available. The fluorescent labels can be conjugated to the antibody using the techniques disclosed in Current Protocols in Immunology, supra, for example. Fluorescence can be quantified using a fluorimeter.

Various enzyme-substrate labels are available and U.S. Patent No. 4,275,149 provides a review of some of these. The enzyme generally catalyzes a chemical alteration of the chromogenic substrate which can be measured using various techniques. For example, the enzyme may catalyze a color change in a substrate, which can be measured spectrophotometrically. Alternatively, the enzyme may alter the fluorescence or chemiluminescence of the substrate. Techniques for quantifying a change in fluorescence are described above. The chemiluminescent substrate becomes electronically excited by a chemical reaction and may then emit light which can be measured (using a chemiluminometer, for example) or donates energy to a fluorescent acceptor. Examples of enzymatic labels include luciferases (e.g., firefly luciferase and bacterial luciferase; U.S. Patent No. 4,737,456), luciferin, 2,3-dihydrophthalazinediones, malate dehydrogenase, urease, peroxidase such as horseradish peroxidase (HRPO), alkaline phosphatase, β-galactosidase, glucoamylase, lysozyme, saccharide oxidases (e.g., glucose oxidase, galactose oxidase, and glucose-6-phosphate dehydrogenase), heterocyclic oxidases (such as uricase and xanthine oxidase), lactoperoxidase, microperoxidase, and the like. Techniques for conjugating enzymes to antibodies are described in O'Sullivan et al. (1981) Methods for the Preparation of Enzyme-Antibody Conjugates for use in Enzyme Immunoassay, in Methods in Enzym. (ed J. Langone & H. Van Vunakis), Academic press, New York 73:147-166.

Examples of enzyme-substrate combinations include, for example: horseradish peroxidase (HRPO) with hydrogen peroxidase as a substrate, wherein the hydrogen peroxidase oxidizes a dye precursor (e.g.,orthophenylene diamine (OPD) or 3,3',5,5'-tetramethyl benzidine hydrochloride (TMB)); alkaline phosphatase (AP) with para-Nitrophenyl phosphate as chromogenic substrate; and β-D-galactosidase (β-D-Gal) with a chromogenic substrate (e.g., p-nitrophenyl-β-D-galactosidase) or fluorogenic substrate 4-methylumbelliferyl-β-D-galactosidase.

Numerous other enzyme-substrate combinations are available to those skilled in the art. For a general review of these, see U.S. Patent Nos. 4,275,149 and 4,318,980.

Sometimes, the label is indirectly conjugated with the antibody. The skilled artisan will be aware of various techniques for achieving this. For example, the antibody can be conjugated with biotin and any of the three broad categories of labels mentioned above can be conjugated with avidin, or vice versa. Biotin binds selectively to avidin and thus, the label can be conjugated with the antibody in this indirect manner. Alternatively, to achieve indirect conjugation of the label with the antibody, the antibody is conjugated with a small hapten (e.g., digoxin) and one of the different types of labels mentioned above is conjugated with an anti-hapten antibody (e.g., anti-digoxin antibody). Thus, indirect conjugation of the label with the antibody can be achieved.

In other versions of immunoassay techniques, the antibody need not be labeled, and the presence thereof can be detected using a labeled antibody which binds to the antibody.

Thus, the diagnostic immunoassays herein may be in any assay format, including, for example, competitive binding assays, direct and indirect sandwich assays, and immunoprecipitation assays. Zola, Monoclonal Antibodies: A Manual of Techniques, pp.147-158 (CRC Press, Inc. 1987).

Competitive binding assays rely on the ability of a labeled standard to compete with the test sample analyze for binding with a limited amount of antibody. The amount of antigen in the test sample is inversely proportional to the amount of standard that becomes bound to the antibodies. To facilitate determining the amount of standard that becomes bound, the antibodies generally are insolubilized before or after the competition, so that the standard and analyze that are bound to the antibodies may conveniently be separated from the standard and analyze which remain unbound.

Sandwich assays involve the use of two antibodies, each capable of binding to a different immunogenic portion, or epitope, of the protein to be detected. In a sandwich assay, the test sample analyze is bound by a first antibody which is immobilized on a solid support, and thereafter a second antibody binds to the analyze, thus forming an insoluble three-part complex. See, e.g.,U.S. Pat No. 4,376,110. The second antibody may itself be labeled with a detectable moiety (direct sandwich assays) or may be measured using an anti-immunoglobulin antibody that is labeled with a detectable moiety (indirect sandwich assay). For example, one type of sandwich assay is an ELISA assay, in which case the detectable moiety is an enzyme.

### g. Proteomics

The term "proteome" is defined as the totality of the proteins present in a sample (e.g. tissue, organism, or cell culture) at a certain point of time. Proteomics includes, among other things, study of the global changes of protein expression in a sample (also referred to as "expression proteomics"). Proteomics typically includes the following steps: (1) separation of individual proteins in a sample by 2-D gel electrophoresis (2-D PAGE); (2) identification of the individual proteins recovered from the gel, e.g. my mass spectrometry or N-terminal sequencing, and (3) analysis of the data using bioinformatics. Proteomics methods are valuable supplements to other methods of gene expression profiling, and can be used, alone or in combination with other methods, to detect the products of the markers of the present invention.

### h. 5'-multiplexed Gene Specific Priming of Reverse Transcription

RT-PCR requires reverse transcription of the test RNA population as a first step. The most commonly used primer for reverse transcription is oligo-dT, which works well when RNA is intact. However, this primer will not be effective when RNA is highly fragmented.

The present invention includes the use of gene specific primers, which are roughly 20 bases in length with a Tm optimum between about 58 °C and 60 °C. These primers will also serve as the reverse primers that drive PCR DNA amplification.

An alternative approach is based on the use of random hexamers as primers for cDNA synthesis. However, we have experimentally demonstrated that the method of using a multiplicity of gene-specific primers is superior over the known approach using random hexamers.

### i. Promoter Methylation Analysis

A number of methods for quantization of RNA transcripts (gene expression analysis) or their protein translation products are discussed herein. The expression level of genes may also be inferred from information regarding chromatin structure, such as for example the methylation status of gene promoters and other regulatory elements and the acetylation status of histones.

In particular, the methylation status of a promoter influences the level of expression of the gene regulated by that promoter. Aberrant methylation of particular gene promoters has been implicated in expression regulation, such as for example silencing of tumor suppressor genes. Thus, examination of the methylation status of a gene's promoter can be utilized as a surrogate for direct quantization of RNA levels.

Several approaches for measuring the methylation status of particular DNA elements have been devised, including methylation-specific PCR (Herman J.G. et al. (1996) Methylation-specific PCR: a novel PCR assay for methylation status of CpG islands. Proc. Natl Acad. Sci. USA. 93, 9821-9826.) and bisulfite DNA sequencing (Frommer M. et al. (1992) A genomic sequencing protocol that yields a positive display of 5-methylcytosine residues in individual DNA strands. Proc. Natl Acad. Sci. USA. 89, 1827-1831.). More recently, microarray-based technologies have been used to characterize promoter methylation status (Chen C.M. (2003) Methylation target array for rapid analysis of CpG island hypermethylation in multiple tissue genomes. Am. J. Pathol. 163, 37-45.).

### j. Coexpression of Genes

A further aspect of the invention is the identification of gene expression clusters. Gene expression clusters can be identified by analysis of expression data using statistical analyses known in the art, including pairwise analysis of correlation based on Pearson correlation coefficients (Pearson K. and Lee A. (1902) Biometrika 2, 357).

In one embodiment, an expression cluster identified herein includes genes upregulated in the left colon.

In another embodiment, an expression cluster identified herein includes genes upregulated in the right colon.

In one other embodiment, an expression cluster identified herein includes genes upregulated in the terminal ileum.

In other embodiments, the expression cluster identified herein includes genes in the

In some embodiments, the expression cluster identified herein includes genes classified under an immune response.

In other embodiments, the expression cluster identified herein includes genes classified under a response to wounding.

### k. Design of Intron-Based PCR Primers and Probes

According to one aspect of the present invention, PCR primers and probes are designed based upon intron sequences present in the gene to be amplified. Accordingly, the first step in the primer/probe design is the delineation of intron sequences within the genes. This can be done by publicly available software, such as the DNA BLAT software developed by Kent, W.J., Genome Res. 12(4):656-64 (2002), or by the BLAST software including its variations. Subsequent steps follow well established methods of PCR primer and probe design.

In order to avoid non-specific signals, it is important to mask repetitive sequences within the introns when designing the primers and probes. This can be easily accomplished by using the Repeat Masker program available on-line through the Baylor College of Medicine, which screens DNA sequences against a library of repetitive elements and returns a query sequence in which the repetitive elements are masked. The masked intron sequences can then be used to design primer and probe sequences using any commercially or otherwise publicly available primer/probe design packages, such as Primer Express (Applied Biosystems); MGB assay-by-design (Applied Biosystems); Primer3 (Steve Rozen and Helen J. Skaletsky (2000) Primer3 on the WWW for general users and for biologist programmers. In: Krawetz S, Misener S (eds) Bioinformatics Methods and Protocols: Methods in Molecular Biology. Humana Press, Totowa, NJ, pp 365-386).

The most important factors considered in PCR primer design include primer length, melting temperature (Tm), and G/C content, specificity, complementary primer sequences, and 3'-end sequence. In general, optimal PCR primers are generally 17-30 bases in length, and contain about 20-80%, such as, for example, about 50-60% G+C bases. Tm's between 50 and 80 °C, e.g. about 50 to 70 °C are typically preferred.

For further guidelines for PCR primer and probe design see, e.g. Dieffenbach, C.W. et al., "General Concepts for PCR Primer Design" in: PCR Primer, A Laboratory Manual, Cold Spring Harbor Laboratory Press, New York, 1995, pp. 133-155; Innis and Gelfand, "Optimization of PCRs" in: PCR Protocols, A Guide to Methods and Applications, CRC Press, London, 1994, pp. 5-11; and Plasterer, T.N. Primerselect: Primer and probe design. Methods Mol. Biol. 70:520-527 (1997), the entire disclosures of which are hereby expressly incorporated by reference.

### I. IBD Gene Set, Assayed Gene Subsequences, and Clinical Application of Gene Expression Data

An important aspect of the present invention is to use the measured expression of certain genes by colonic issue to provide diagnostic information. For this purpose it is necessary to correct for (normalize away) both differences in the amount of RNA assayed and variability in the quality of the RNA used. Therefore, the assay typically measures and incorporates the expression of certain normalizing genes, including well known housekeeping genes, such as GAPDH and Cyp1. Alternatively, normalization can be based on the mean or median signal (Ct) of all of the assayed genes or a large subset thereof (global normalization approach). On a gene-by-gene basis, measured normalized amount of a patient colonic tissue mRNA is compared to the amount found in an appropriate tissue reference set. The number (N) of tissues in this reference set should be sufficiently high to ensure that different reference sets (as a whole) behave essentially the same way. If this condition is met, the identity of the individual colonic tissues present in a particular set will have no significant impact on the relative amounts of the genes assayed. Usually, the tissue reference set consists of at least about 30, preferably at least about 40 different IBD tissue specimens. Unless noted otherwise, normalized expression levels for each mRNA/tested tissue/patient will be expressed as a percentage of the expression level measured in the reference set. More specifically, the reference set of a sufficiently high number (e.g. 40) of IBD samples yields a distribution of normalized levels of each mRNA species. The level measured in a particular sample to be analyzed falls at some percentile within this range, which can be determined by methods well known in the art. Below, unless noted otherwise, reference to expression levels of a gene assume normalized expression relative to the reference set although this is not always explicitly stated.

### m. Production of antibodies

The present invention further provides anti-IBD marker antibodies. Exemplary antibodies include polyclonal, monoclonal, humanized, bispecific, and heteroconjugate antibodies. As discussed herein, the antibodies may be used in the diagnostic methods for IBD, and in some cases in methods of treatment of IBD.

### (1) Polyclonal antibodies

Polyclonal antibodies are preferably raised in animals by multiple subcutaneous (sc) or intraperitoneal (ip) injections of the relevant antigen and an adjuvant. It may be useful to conjugate the relevant antigen to a protein that is immunogenic in the species to be immunized, e.g., keyhole limpet hemocyanin, serum albumin, bovine thyroglobulin, or soybean trypsin inhibitor using a bifunctional or derivatizing agent, for example, maleimidobenzoyl sulfosuccinimide ester (conjugation through cysteine residues), N-hydroxysuccinimide (through lysine residues), glutaraldehyde, succinic anhydride, SOC12, or R1N=C=NR, where R and R1 are different alkyl groups.

Animals are immunized against the antigen, immunogenic conjugates, or derivatives by combining, e.g., 100 µg or 5 µg of the protein or conjugate (for rabbits or mice, respectively) with 3 volumes of Freund's complete adjuvant and injecting the solution intradermally at multiple sites. One month later the animals are boosted with 1/5 to 1/10 the original amount of peptide or conjugate in Freund's complete adjuvant by subcutaneous injection at multiple sites. Seven to 14 days later the animals are bled and the serum is assayed for antibody titer. Animals are boosted until the titer plateaus. Preferably, the animal is boosted with the conjugate of the same antigen, but conjugated to a different protein and/or through a different cross-linking reagent. Conjugates also can be made in recombinant cell culture as protein fusions. Also, aggregating agents such as alum are suitably used to enhance the immune response.

### (2) Monoclonal antibodies

Various methods for making monoclonal antibodies herein are available in the art. For example, the monoclonal antibodies may be made using the hybridoma method first described by Kohler et al., Nature, 256:495 (1975), by recombinant DNA methods (U.S. Patent No. 4,816,567).

In the hybridoma method, a mouse or other appropriate host animal, such as a hamster, is immunized as hereinabove described to elicit lymphocytes that produce or are capable of producing antibodies that will specifically bind to the protein used for immunization. Alternatively, lymphocytes may be immunized *in vitro.* Lymphocytes then are fused with myeloma cells using a suitable fusing agent, such as polyethylene glycol, to form a hybridoma cell (Goding, Monoclonal Antibodies: Principles and Practice, pp.59-103 (Academic Press, 1986)).

The hybridoma cells thus prepared are seeded and grown in a suitable culture medium that preferably contains one or more substances that inhibit the growth or survival of the unfused, parental myeloma cells. For example, if the parental myeloma cells lack the enzyme hypoxanthine guanine phosphoribosyl transferase (HGPRT or HPRT), the culture medium for the hybridomas typically will include hypoxanthine, aminopterin, and thymidine (HAT medium), which substances prevent the growth of HGPRT-deficient cells.

Preferred myeloma cells are those that fuse efficiently, support stable high-level production of antibody by the selected antibody-producing cells, and are sensitive to a medium such as HAT medium. Among these, preferred myeloma cell lines are murine myeloma lines, such as those derived from MOPC-21 and MPC-11 mouse tumors available from the Salk Institute Cell Distribution Center, San Diego, California USA, and SP-2 or X63-Ag8-653 cells available from the American Type Culture Collection, Rockville, Maryland USA. Human myeloma and mouse-human heteromyeloma cell lines also have been described for the production of human monoclonal antibodies (Kozbor, J. Immunol., 133:3001 (1984); and Brodeur et al., Monoclonal Antibody Production Techniques and Applications, pp. 51-63 (Marcel Dekker, Inc., New York, 1987)).

Culture medium in which hybridoma cells are growing is assayed for production of monoclonal antibodies directed against the antigen. Preferably, the binding specificity of monoclonal antibodies produced by hybridoma cells is determined by immunoprecipitation or by an in vitro binding assay, such as radioimmunoassay (RIA) or enzyme-linked immunoabsorbent assay (ELISA).

The binding affinity of the monoclonal antibody can, for example, be determined by the Scatchard analysis of Munson et al., Anal. Biochem., 107:220 (1980).

After hybridoma cells are identified that produce antibodies of the desired specificity, affinity, and/or activity, the clones may be subcloned by limiting dilution procedures and grown by standard methods (Goding, Monoclonal Antibodies: Principles and Practice, pp.59-103 (Academic Press, 1986)). Suitable culture media for this purpose include, for example, D-MEM or RPMI-1640 medium. In addition, the hybridoma cells may be grown in vivo as ascites tumors in an animal.

The monoclonal antibodies secreted by the subclones are suitably separated from the culture medium, ascites fluid, or serum by conventional antibody purification procedures such as, for example, protein A-Sepharose, hydroxylapatite chromatography, gel electrophoresis, dialysis, or affinity chromatography.

DNA encoding the monoclonal antibodies is readily isolated and sequenced using conventional procedures (e.g., by using oligonucleotide probes that are capable of binding specifically to genes encoding the heavy and light chains of murine antibodies). The hybridoma cells serve as a preferred source of such DNA. Once isolated, the DNA may be placed into expression vectors, which are then transfected into host cells such as E. coli cells, simian COS cells, Chinese Hamster Ovary (CHO) cells, or myeloma cells that do not otherwise produce antibody protein, to obtain the synthesis of monoclonal antibodies in the recombinant host cells. Review articles on recombinant expression in bacteria of DNA encoding the antibody include Skerra et al., Curr. Opinion in Immunol., 5:256-262 (1993) and Plückthun, Immunol. Revs., 130:151-188 (1992).

In a further embodiment, monoclonal antibodies or antibody fragments can be isolated from antibody phage libraries generated using the techniques described in McCafferty et al., Nature, 348:552-554 (1990). Clackson et al., Nature, 352:624-628 (1991) and Marks et al., J. Mol. Biol., 222:581-597 (1991) describe the isolation of murine and human antibodies, respectively, using phage libraries. Subsequent publications describe the production of high affinity (nM range) human antibodies by chain shuffling (Marks et al., Bio/Technology, 10:779-783 (1992)), as well as combinatorial infection and in vivo recombination as a strategy for constructing very large phage libraries (Waterhouse et al., Nuc. Acids. Res., 21:2265-2266 (1993)). Thus, these techniques are viable alternatives to traditional monoclonal antibody hybridoma techniques for isolation of monoclonal antibodies.

The DNA also may be modified, for example, by substituting the coding sequence for human heavy chain and light chain constant domains in place of the homologous murine sequences (U.S. Patent No. 4,816,567; and Morrison, et al., Proc. Natl Acad. Sci. USA, 81:6851 (1984)), or by covalently joining to the immunoglobulin coding sequence all or part of the coding sequence for a non-immunoglobulin polypeptide.

Typically such non-immunoglobulin polypeptides are substituted for the constant domains of an antibody, or they are substituted for the variable domains of one antigen-combining site of an antibody to create a chimeric bivalent antibody comprising one antigen-combining site having specificity for an antigen and another antigen-combining site having specificity for a different antigen.

### (3) Humanized antibodies

Methods for humanizing non-human antibodies have been described in the art. Preferably, a humanized antibody has one or more amino acid residues introduced into it from a source which is non-human. These non-human amino acid residues are often referred to as "import" residues, which are typically taken from an "import" variable domain. Humanization can be essentially performed following the method of Winter and co-workers (Jones et al., Nature, 321:522-525 (1986); Riechmann et al., Nature, 332:323-327 (1988); Verhoeyen et al., Science, 239:1534-1536 (1988)), by substituting hypervariable region sequences for the corresponding sequences of a human antibody. Accordingly, such "humanized" antibodies are chimeric antibodies (U.S. Patent No. 4,816,567) wherein substantially less than an intact human variable domain has been substituted by the corresponding sequence from a non-human species. In practice, humanized antibodies are typically human antibodies in which some hypervariable region residues and possibly some FR residues are substituted by residues from analogous sites in rodent antibodies. An example of a humanized antibody used to treat IBD is infliximab (Remicade®), an engineered murine-human chimeric monoclonal antibody. The antibody binds the cytokine TNF-alpha and prevents it from binding its receptors to trigger and sustain an inflammatory response. Infliximab is used to treat both CD and UC.

The choice of human variable domains, both light and heavy, to be used in making the humanized antibodies is very important to reduce antigenicity. According to the so-called "best-fit" method, the sequence of the variable domain of a rodent antibody is screened against the entire library of known human variable-domain sequences. The human sequence which is closest to that of the rodent is then accepted as the human framework region (FR) for the humanized antibody (Sims et al., J. Immunol., 151:2296 (1993); Chothia et al., J. Mol. Biol., 196:901 (1987)). Another method uses a particular framework region derived from the consensus sequence of all human antibodies of a particular subgroup of light or heavy chains. The same framework may be used for several different humanized antibodies (Carter et al., Proc. Natl. Acad. Sci. USA, 89:4285 (1992); Presta et al., J. Immunol., 151:2623 (1993)).

It is further important that antibodies be humanized with retention of high affinity for the antigen and other favorable biological properties. To achieve this goal, according to a preferred method, humanized antibodies are prepared by a process of analysis of the parental sequences and various conceptual humanized products using three-dimensional models of the parental and humanized sequences. Three-dimensional immunoglobulin models are commonly available and are familiar to those skilled in the art. Computer programs are available which illustrate and display probable three-dimensional conformational structures of selected candidate immunoglobulin sequences. Inspection of these displays permits analysis of the likely role of the residues in the functioning of the candidate immunoglobulin sequence, i.e., the analysis of residues that influence the ability of the candidate immunoglobulin to bind its antigen. In this way, FR residues can be selected and combined from the recipient and import sequences so that the desired antibody characteristic, such as increased affinity for the target antigen(s), is achieved. In general, the hypervariable region residues are directly and most substantially involved in influencing antigen binding.

Various forms of the humanized antibody are contemplated. For example, the humanized antibody may be an antibody fragment, such as a Fab, which is optionally conjugated with one or more cytotoxic agent(s) in order to generate an immunoconjugate. Alternatively, the humanized antibody may be an intact antibody, such as an intact IgG1 antibody.

### (4) Human antibodies

As an alternative to humanization, human antibodies can be generated. For example, it is now possible to produce transgenic animals (e.g., mice) that are capable, upon immunization, of producing a full repertoire of human antibodies in the absence of endogenous immunoglobulin production. For example, it has been described that the homozygous deletion of the antibody heavy-chain joining region (JH) gene in chimeric and germ-line mutant mice results in complete inhibition of endogenous antibody production. Transfer of the human germ-line immunoglobulin gene array in such germ-line mutant mice will result in the production of human antibodies upon antigen challenge. See, e.g., Jakobovits et al., Proc. Natl. Acad. Sci. USA, 90:2551 (1993); Jakobovits et al., Nature, 362:255-258 (1993); Bruggermann et al., Year in Immuno., 7:33 (1993); and U.S. Patent Nos. 5,591,669, 5,589,369 and 5,545,807. Alternatively, phage display technology (McCafferty et al., Nature 348:552-553 (1990)) can be used to produce human antibodies and antibody fragments in vitro, from immunoglobulin variable (V) domain gene repertoires from unimmunized donors. According to this technique, antibody V domain genes are cloned in-frame into either a major or minor coat protein gene of a filamentous bacteriophage, such as M13 or fd, and displayed as functional antibody fragments on the surface of the phage particle. Because the filamentous particle contains a single-stranded DNA copy of the phage genome, selections based on the functional properties of the antibody also result in selection of the gene encoding the antibody exhibiting those properties. Thus, the phage mimics some of the properties of the B-cell. Phage display can be performed in a variety of formats; for their review see, e.g., Johnson, Kevin S. and Chiswell, David J., Current Opinion in Structural Biology 3:564-571 (1993). Several sources of V-gene segments can be used for phage display. Clackson et al., Nature, 352:624-628 (1991) isolated a diverse array of anti-oxazolone antibodies from a small random combinatorial library of V genes derived from the spleens of immunized mice. A repertoire of V genes from unimmunized human donors can be constructed and antibodies to a diverse array of antigens (including self-antigens) can be isolated essentially following the techniques described by Marks et al., J. Mol. Biol. 222:581-597 (1991), or Griffith et al., EMBO J. 12:725-734 (1993). See, also, U.S. Patent Nos. 5,565,332 and 5,573,905.

As discussed above, human antibodies may also be generated by *in vitro* activated B cells (see U.S. Patents 5,567,610 and 5,229,275).

### (5) Antibody fragments

Various techniques have been developed for the production of antibody fragments comprising one or more antigen binding regions. Traditionally, these fragments were derived via proteolytic digestion of intact antibodies (see, e.g., Morimoto et al., Journal of Biochemical and Biophysical Methods 24:107-117 (1992); and Brennan et al., Science, 229:81 (1985)). However, these fragments can now be produced directly by recombinant host cells. For example, the antibody fragments can be isolated from the antibody phage libraries discussed above. Alternatively, Fab'-SH fragments can be directly recovered from E. coli and chemically coupled to form F(ab')2 fragments (Carter et al., Bio/Technology 10:163-167 (1992)). According to another approach, F(ab')2 fragments can be isolated directly from recombinant host cell culture. Other techniques for the production of antibody fragments will be apparent to the skilled practitioner. In other embodiments, the antibody of choice is a single chain Fv fragment (scFv). See WO 93/16185; U.S. Patent No. 5,571,894; and U.S. Patent No. 5,587,458. The antibody fragment may also be a Alinear antibody@, e.g., as described in U.S. Patent 5,641,870 for example. Such linear antibody fragments may be monospecific or bispecific.

### (6) Bispecific antibodies

Bispecific antibodies are antibodies that have binding specificities for at least two different epitopes. Exemplary bispecific antibodies may bind to two different epitopes of an IBD marker protein. Bispecific antibodies may also be used to localize agents to cells which express an IBD marker protein.

These antibodies possess an IBD marker-binding arm and an arm which binds an agent (e.g. an aminosalicylate). Bispecific antibodies can be prepared as full length antibodies or antibody fragments (e.g. F(ab')2 bispecific antibodies).

Methods for making bispecific antibodies are known in the art.. Traditional production of full length bispecific antibodies is based on the coexpression of two immunoglobulin heavy chain-light chain pairs, where the two chains have different specificities (Millstein et al., Nature, 305:537-539 (1983)). Because of the random assortment of immunoglobulin heavy and light chains, these hybridomas (quadromas) produce a potential mixture of 10 different antibody molecules, of which only one has the correct bispecific structure. Purification of the correct molecule, which is usually done by affinity chromatography steps, is rather cumbersome, and the product yields are low. Similar procedures are disclosed in WO 93/08829, and in Traunecker et al., EMBO J., 10:3655-3659 (1991).

According to a different approach, antibody variable domains with the desired binding specificities (antibody-antigen combining sites) are fused to immunoglobulin constant domain sequences. The fusion preferably is with an immunoglobulin heavy chain constant domain, comprising at least part of the hinge, CH2, and CH3 regions. It is preferred to have the first heavy-chain constant region (CH1) containing the site necessary for light chain binding, present in at least one of the fusions. DNAs encoding the immunoglobulin heavy chain fusions and, if desired, the immunoglobulin light chain, are inserted into separate expression vectors, and are co-transfected into a suitable host organism. This provides for great flexibility in adjusting the mutual proportions of the three polypeptide fragments in embodiments when unequal ratios of the three polypeptide chains used in the construction provide the optimum yields. It is, however, possible to insert the coding sequences for two or all three polypeptide chains in one expression vector when the expression of at least two polypeptide chains in equal ratios results in high yields or when the ratios are of no particular significance.

In a preferred embodiment of this approach, the bispecific antibodies are composed of a hybrid immunoglobulin heavy chain with a first binding specificity in one arm, and a hybrid immunoglobulin heavy chain-light chain pair (providing a second binding specificity) in the other arm. It was found that this asymmetric structure facilitates the separation of the desired bispecific compound from unwanted immunoglobulin chain combinations, as the presence of an immunoglobulin light chain in only one half of the bispecific molecule provides for a facile way of separation. This approach is disclosed in WO 94/04690. For further details of generating bispecific antibodies see, for example, Suresh et al., Methods in Enzymology, 121:210 (1986).

According to another approach described in U.S. Patent No. 5,731,168, the interface between a pair of antibody molecules can be engineered to maximize the percentage of heterodimers which are recovered from recombinant cell culture. The preferred interface comprises at least a part of the C_{H}3 domain of an antibody constant domain. In this method, one or more small amino acid side chains from the interface of the first antibody molecule are replaced with larger side chains (e.g. tyrosine or tryptophan). Compensatory "cavities" of identical or similar size to the large side chain(s) are created on the interface of the second antibody molecule by replacing large amino acid side chains with smaller ones (e.g. alanine or threonine). This provides a mechanism for increasing the yield of the heterodimer over other unwanted end-products such as homodimers.

Bispecific antibodies include cross-linked or "heteroconjugate" antibodies. For example, one of the antibodies in the heteroconjugate can be coupled to avidin, the other to biotin. Such antibodies have, for example, been proposed to target immune system cells to unwanted cells (U.S. Patent No. 4,676,980), and for treatment of HIV infection (WO 91/00360, WO 92/200373, and EP 03089). Heteroconjugate antibodies may be made using any convenient cross-linking methods. Suitable cross-linking agents are well known in the art, and are disclosed in U.S. Patent No. 4,676,980, along with a number of cross-linking techniques.

Techniques for generating bispecific antibodies from antibody fragments have also been described in the literature. For example, bispecific antibodies can be prepared using chemical linkage. Brennan et al., Science, 229: 81 (1985) describe a procedure wherein intact antibodies are proteolytically cleaved to generate F(ab')₂ fragments. These fragments are reduced in the presence of the dithiol complexing agent sodium arsenite to stabilize vicinal dithiols and prevent intermolecular disulfide formation. The Fab' fragments generated are then converted to thionitrobenzoate (TNB) derivatives. One of the Fab'-TNB derivatives is then reconverted to the Fab'-thiol by reduction with mercaptoethylamine and is mixed with an equimolar amount of the other Fab'-TNB derivative to form the bispecific antibody. The bispecific antibodies produced can be used as agents for the selective immobilization of enzymes.

Various techniques for making and isolating bispecific antibody fragments directly from recombinant cell culture have also been described. For example, bispecific antibodies have been produced using leucine zippers. Kostelny et al., J. Immunol., 148(5):1547-1553 (1992). The leucine zipper peptides from the Fos and Jun proteins were linked to the Fab' portions of two different antibodies by gene fusion. The antibody homodimers were reduced at the hinge region to form monomers and then re-oxidized to form the antibody heterodimers. This method can also be utilized for the production of antibody homodimers. The "diabody" technology described by Hollinger et al., Proc. Natl. Acad. Sci. USA, 90:6444-6448 (1993) has provided an alternative mechanism for making bispecific antibody fragments. The fragments comprise a heavy-chain variable domain (V_{H}) connected to a light-chain variable domain (V_{L}) by a linker which is too short to allow pairing between the two domains on the same chain. Accordingly, the V_{H} and V_{L} domains of one fragment are forced to pair with the complementary V_{L} and V_{H} domains of another fragment, thereby forming two antigen-binding sites. Another strategy for making bispecific antibody fragments by the use of single-chain Fv (sFv) dimers has also been reported. See Gruber et al., J. Immunol., 152:5368 (1994).

Antibodies with more than two valencies are contemplated. For example, trispecific antibodies can be prepared. Tutt et al. J. Immunol. 147: 60 (1991).

### (7) Other amino acid sequence modifications

Amino acid sequence modification(s) of the antibodies described herein are contemplated. For example, it may be desirable to improve the binding affinity and/or other biological properties of the antibody. Amino acid sequence variants of the antibody are prepared by introducing appropriate nucleotide changes into the antibody nucleic acid, or by peptide synthesis. Such modifications include, for example, deletions from, and/or insertions into and/or substitutions of, residues within the amino acid sequences of the antibody. Any combination of deletion, insertion, and substitution is made to arrive at the final construct, provided that the final construct possesses the desired characteristics. The amino acid changes also may alter post-translational processes of the antibody, such as changing the number or position of glycosylation sites.

A useful method for identification of certain residues or regions of the antibody that are preferred locations for mutagenesis is called "alanine scanning mutagenesis" as described by Cunningham and Wells Science, 244:1081-1085 (1989). Here, a residue or group of target residues are identified *(e.g.,* charged residues such as arg, asp, his, lys, and glu) and replaced by a neutral or negatively charged amino acid (most preferably alanine or polyalanine) to affect the interaction of the amino acids with antigen. Those amino acid locations demonstrating functional sensitivity to the substitutions then are refined by introducing further or other variants at, or for, the sites of substitution. Thus, while the site for introducing an amino acid sequence variation is predetermined, the nature of the mutation *per se* need not be predetermined. For example, to analyze the performance of a mutation at a given site, ala scanning or random mutagenesis is conducted at the target codon or region and the expressed antibody variants are screened for the desired activity.

Amino acid sequence insertions include amino- and/or carboxyl-terminal fusions ranging in length from one residue to polypeptides containing a hundred or more residues, as well as intrasequence insertions of single or multiple amino acid residues. Examples of terminal insertions include antibody with an N-terminal methionyl residue or the antibody fused to a cytotoxic polypeptide. Other insertional variants of the antibody molecule include the fusion to the N- or C-terminus of the antibody to an enzyme (e.g. for ADEPT) or a polypeptide which increases the serum half-life of the antibody.

Another type of variant is an amino acid substitution variant. These variants have at least one amino acid residue in the antibody molecule replaced by a different residue. The sites of greatest interest for substitutional mutagenesis include the hypervariable regions, but FR alterations are also contemplated. Conservative substitutions are shown in Table 1 under the heading of "preferred substitutions". If such substitutions result in a change in biological activity, then more substantial changes, denominated "exemplary substitutions" in the following table, or as further described below in reference to amino acid classes, may be introduced and the products screened.

| Original Residue | Exemplary Substitutions | Preferred Substitutions |
|---|---|---|
| Ala (A) | val; leu; ile | val |
| Arg (R) | lys; gln; asn | lys |
| Asn (N) | gln; his; lys; arg | gln |
| Asp (D) | glu | glu |
| Cys (C) | ser | ser |
| Gln (Q) | asn | asn |
| Glu (E) | asp | asp |
| Gly (G) | pro; ala | ala |
| His (H) | asn; gln; lys; arg | arg |
| Ile (I) | leu; val; met; ala; phe; norleucine | leu |
| Leu (L) | norleucine; ile; val; met; ala; phe | ile |
| Lys (K) | arg; gln; asn | arg |
| Met (M) | leu; phe; ile | leu |
| Phe (F) | leu; val; ile; ala; tyr | leu |
| Pro (P) | ala | ala |
| Ser (S) | thr | thr |
| Thr (T) | ser | ser |
| Trp (W) | tyr; phe | tyr |
| Tyr (Y) | trp; phe; thr; ser | phe |
| Val (V) | ile; leu; met; phe; ala; norleucine | leu |

Substantial modifications in the biological properties of the antibody are accomplished by selecting substitutions that differ significantly in their effect on maintaining (a) the structure of the polypeptide backbone in the area of the substitution, for example, as a sheet or helical conformation, (b) the charge or hydrophobicity of the molecule at the target site, or (c) the bulk of the side chain. Amino acids may be grouped according to similarities in the properties of their side chains (in A. L. Lehninger, in Biochemistry, second ed., pp. 73-75, Worth Publishers, New York (1975)): non-polar: Ala (A), Val (V), Leu (L), Ile (I), Pro (P), Phe (F), Trp (W), Met (M); uncharged polar: Gly (G), Ser (S), Thr (T), Cys (C), Tyr (Y), Asn (N), Gln (Q); acidic: Asp (D), Glu (E); and basic: Lys (K), Arg (R), His(H).

Alternatively, naturally occurring residues may be divided into groups based on common side-chain properties: hydrophobic: Norleucine, Met, Ala, Val, Leu, Ile; neutral hydrophilic: Cys, Ser, Thr, Asn, Gln; acidic: Asp, Glu; basic: His, Lys, Arg; residues that influence chain orientation: Gly, Pro; and aromatic: Trp, Tyr, Phe.

Non-conservative substitutions will entail exchanging a member of one of these classes for another class.

Any cysteine residue not involved in maintaining the proper conformation of the antibody also may be substituted, generally with serine, to improve the oxidative stability of the molecule and prevent aberrant crosslinking. Conversely, cysteine bond(s) may be added to the antibody to improve its stability (particularly where the antibody is an antibody fragment such as an Fv fragment).

A particularly preferred type of substitutional variant involves substituting one or more hypervariable region residues of a parent antibody (*e.g*. a humanized or human antibody). Generally, the resulting variant(s) selected for further development will have improved biological properties relative to the parent antibody from which they are generated. A convenient way for generating such substitutional variants involves affinity maturation using phage display. Briefly, several hypervariable region sites *(e.g.* 6-7 sites) are mutated to generate all possible amino substitutions at each site. The antibody variants thus generated are displayed in a monovalent fashion from filamentous phage particles as fusions to the gene III product of M13 packaged within each particle. The phage-displayed variants are then screened for their biological activity (e.g. binding affinity) as herein disclosed. In order to identify candidate hypervariable region sites for modification, alanine scanning mutagenesis can be performed to identify hypervariable region residues contributing significantly to antigen binding. Alternatively, or additionally, it may be beneficial to analyze a crystal structure of the antigen-antibody complex to identify contact points between the antibody and an IBD marker protein. Such contact residues and neighboring residues are candidates for substitution according to the techniques elaborated herein. Once such variants are generated, the panel of variants is subjected to screening as described herein and antibodies with superior properties in one or more relevant assays may be selected for further development.

Engineered antibodies with three or more (preferably four) functional antigen binding sites are also contemplated (U.S. Published Patent Application No. US2002/0004587 A1 Miller et al.*).*

Nucleic acid molecules encoding amino acid sequence variants of the antibody are prepared by a variety of methods known in the art. These methods include, but are not limited to, isolation from a natural source (in the case of naturally occurring amino acid sequence variants) or preparation by oligonucleotide-mediated (or site-directed) mutagenesis, PCR mutagenesis, and cassette mutagenesis of an earlier prepared variant or a non-variant version of the antibody.

### B.3 Determination of inflammation

In one aspect, the identification in a subject of a differentially expressed biomarker described herein may be correlated to a determination of inflammation in the subject. In one embodiment, the expression of a biomarker may be used as a surrogate for inflammation (Sands et al. (2005) Inflamm Bowel Dis. 11(1):S22-S28). In another embodiment, the expression of a biomarker is validated against a determination of inflammation by other techniques. In one other embodiment, the methods of diagnosis and/or treatment of the present invention comprise the step of determinining inflammation in a subject. In another embodiment, the determining step comprises histological evaluation of a test sample obtained from the subject for inflammatory cell infiltrate. In one embodiment, the test sample is a tissue biopsy obtained from the subject.

In another embodiment, the determining step comprises evaluation of a non-tissue biopsy as a test sample from the subject. In one embodiment, the test sample is a biopsy obtained from the fecal material of the subject. In another embodiment, the test sample is blood. In one other embodiment, the determining step comprises a fecal calprotectin or fecal lactoferrin test (Joishy et al. (2008) J Pediatr Gastroenterol Nutr. 48(1):48-54) or a C reactive protein (CRP) blood test (Henriksen et al. (2008) Gut. 57:1518-1523).

### B.4 Kits of the invention

The materials for use in the methods of the present invention are suited for preparation of kits produced in accordance with well known procedures. The invention thus provides kits comprising agents, which may include gene-specific or gene-selective probes and/or primers, for quantitating the expression of the disclosed genes for IBD. Such kits may optionally contain reagents for the extraction of RNA from samples, in particular fixed paraffin-embedded tissue samples and/or reagents for RNA amplification. In addition, the kits may optionally comprise the reagent(s) with an identifying description or label or instructions relating to their use in the methods of the present invention. The kits may comprise containers (including microtiter plates suitable for use in an automated implementation of the method), each with one or more of the various reagents (typically in concentrated form) utilized in the methods, including, for example, pre-fabricated microarrays, buffers, the appropriate nucleotide triphosphates (e.g., dATP, dCTP, dGTP and dTTP; or rATP, rCTP, rGTP and UTP), reverse transcriptase, DNA polymerase, RNA polymerase, and one or more probes and primers of the present invention (e.g., appropriate length poly(T) or random primers linked to a promoter reactive with the RNA polymerase).

### B.5 Reports of the invention

The methods of this invention, when practiced for commercial diagnostic purposes generally produce a report or summary of the normalized expression levels of one or more of the selected genes. The methods of this invention will produce a report comprising a prediction of the clinical outcome of a subject diagnosed with an IBD before and after any surgical procedure to treat the IBD. The methods and reports of this invention can further include storing the report in a database. Alternatively, the method can further create a record in a database for the subject and populate the record with data. In one embodiment the report is a paper report, in another embodiment the report is an auditory report, in another embodiment the report is an electronic record. It is contemplated that the report is provided to a physician and/or the patient. The receiving of the report can further include establishing a network connection to a server computer that includes the data and report and requesting the data and report from the server computer.

The methods provided by the present invention may also be automated in whole or in part.

All aspects of the present invention may also be practiced such that a limited number of additional genes that are co-expressed with the disclosed genes, for example as evidenced by high Pearson correlation coefficients, are included in a prognostic or predictive test in addition to and/or in place of disclosed genes.

Having described the invention, the same will be more readily understood through reference to the following Examples, which is provided by way of illustration, and is not intended to limit the invention in any way.

### Example

### Example 1 - Characterisation of Intestinal Gene Expression Profiles in Crohn's Disease by Genome- wide Microarray Analysis

Genome-wide microarray expression analysis creates a comprehensive picture of gene expression at the cellular level. The aim of this study was to investigate differential intestinal gene expression in patients with Crohn's disease (CD) and controls with sub-analysis of confirmed CD susceptibility genes, associated pathways and cell lineages.

53 CD and 31 control subjects- 23 normal and 8 inflamed non-inflammatory bowel disease patients were studied. Paired endoscopic biopsies were taken from 5 specific anatomical locations for RNA extraction and histology. 41058 expression sequence tags were analyzed using the Agilent platform.

Clustering analysis separated CD and control terminal ileal (TI) biopsies from colonic biopsies and CD and control TI biopsies. In the CD TI biopsies diubiquitin (FC+11.3, p<1x10-45), MMP3 (FC+7.4, p=1.3x10-11), IRTA1 (FC-11.4, p=4.7x102) and CCL23 (FC-7.1, p=1.6x10-10) were differentially expressed compared to controls. In the colon SAA1 (FC+6.3, p=5.3x10-8) was upregulated and TSLP (FC-2.3, p=2.7x10-6) was downregulated comparing non-inflamed CD and control biopsies, and the colonic inflammatory CD signature was characterised by downregulated organic solute carriers-SLC38A4, SLC26A2 and OST alpha. Analysis of the IL-23 pathway revealed IL-23A, JAK2 and STAT3 were upregulated in the CD group compared to controls and in the inflamed compared to non-inflamed CD biopsies. Differential expression was also observed in a number of the autophagy genes, notably ATG16L1.

### Methods

### Patient recruitment

53 patients with CD (Table 2) and 31 control patients who were undergoing colonoscopy were recruited. All CD patients attended the clinic at the Western General Hospital, Edinburgh and the diagnosis of CD adhered to the criteria of Lennard-Jones. (Lennard-Jones JE. Scand J Gastroenterol Suppl 1989;170:2-6) Quiescent CD was classified as Harvey-Bradshaw score of < 3 prior to bowel preparation and normal histology or histology showing only mild chronic inflammation. Active CD was classified as a Harvey-Bradshaw score of 4 or greater prior to bowel preparation and histology showing chronic active inflammation or acute on chronic inflammation.

**Table 2: The Demographics of the Crohn's disease and control patients.**

| | **Crohn's disease** | **Controls** |
|---|---|---|
| Number of patients | 53 | 31 |
| Male/ Female | 26/27 | 11/20 |
| Median age at diagnosis (years) | 28.6 | 43 at time of endoscopy |
| Median duration of follow up (years) | 8.1 | |
| Surgery* | 20 (38%) | |
| Current Smoker | 11 21%) | |
| Family history of IBD | 12 (23%) | |
| Extra-articular symptoms | 13 25%) | |
| 5 ASA Therapy | 21 (40%) | |
| Corticosteroid therapy | 4 (8%) | |
| Immunosuppressant therapy (AZA, 6MP, MTX, MMF) | 13 (25%) | |

| **Disease Group** | | |
|---|---|---|
| New Diagnosis (1) | 7 (13%) | |
| Quiescent disease (2) | 30 (57%) | |
| Active disease (3) | 16 (30%) | |

| **Vienna Classification of disease location at endoscopy** | | |
|---|---|---|
| Ileal disease (L1) | 6 (11%) | |
| Colonic disease (L2) | 28 (53%) | |
| Ileo-colonic disease (L3) | 19 (36%) | |

| **Vienna Classification of disease behavoiur at endoscopy** | | |
|---|---|---|
| Inflammatory (B 1) | 32 (60%) | |
| Stricturing (B2) | 8 (16%) | |
| Penetrating (B3) | 12 (23%) | |

| | | |
|---|---|---|
| * Includes patients who had surgery for luminal complications of Crohn's disease. AZA-azathioprine, 6MP-6 mercaptopurine, MTX-methotrexate, MMFmycophenolate Full phenotypic data were available on 94% of patients at the time of diagnosis and 100% of patients at the time of endoscopy. | | |

Phenotypic data were collected by interview and case-note review. Eleven of the controls were male, 20 were female and they had a median age of 43 at the time of endoscopy. (Noble et al. Gut 2008, Oct;57(10):1398-405) Six of the controls had normal colonoscopies for colon cancer screening, 10 controls had symptoms consistent with irritable bowel syndrome and had a normal colonoscopic investigation and 7 patients had a colonoscopy for an other indication and histologically normal biopsies were obtained. Eight control patients had abnormal inflamed colonic biopsies (1 pseudomembranous colitis, 1 diverticulitis, 1 amoebiasis, 2 microscopic colitis, 1 eosoinophilic infiltrate, 2 scattered lymphoid aggregates and a history of gastroenteritis). For the female TI clustering analysis, one female UC patient with a non-inflamed terminal ileal biopsy was included. Phenotypic data were collected by interview and case-note review. Lothian Local Research Ethics Committee approved the study protocol: REC 04/S 1103/22.

### Biopsy Collection

Paired biopsies were taken from the terminal ileum (TI) and 4 sites in the colon (Table 3). One biopsy was sent for histological examination and the other was snap frozen in liquid nitrogen for RNA extraction. Each biopsy was graded histologically into those with no evidence of inflammation, biopsies with evidence of chronic inflammation and a chronic inflammatory cell infiltrate and those with acute inflammation and an acute inflammatory cell infiltrate.

**Table 3: The location and number of biopsies in Crohn's disease patients and controls**

| | **Crohn's disease** | | **Controls** | |
|---|---|---|---|---|
| **Total number of paired biopsies** | **106** | | **76** | |
| Terminal Ileum | 16 | | 6 | |
| Ascending colon | 25 | | 17 | |
| Descending colon | 32 | | 23 | |
| Sigmoid colon biopsies. | 33 | | 27 | |
| Removed from analysis | 7 | | 3 | |

| | **Inflamed** | **Non-inflamed** | **Inflamed** | **Non-inflamed** |
|---|---|---|---|---|
| Terminal Ileum | 10 | 6 | 1 | 5 |
| Ascending colon | 12 | 8 | 3 | 14 |
| Descending colon | 14 | 16 | 6 | 17 |
| Sigmoid colon biopsies. | 16 | 17 | 8 | 19 |

| | | | | |
|---|---|---|---|---|
| The distribution of log intensities for each sample was plotted and outlier samples (i.e. greater than 2 standard deviations from the mean) were excluded from analysis. | | | | |

### Microarray Analysis

Nucleic acid microarray, often containing thousands of gene sequences, are useful for identifying differentially expressed genes in diseased tissues as compared to their normal counterparts. Using nucleic acid microarrays, test and control mRNA samples from test and control tissue samples are reverse transcribed and labeled to generate cDNA probes. The cDNA probes are then hybridized to an array of nucleic acids immobilized on a solid support. The array is configured such that the sequence and position of each member of the array is known. For example, a selection of genes known to be expressed in certain disease states may be arrayed on a solid support. Hybridization of a labeled probe with a particular array member indicates that the sample from which the probe was derived expresses that gene. If the hybridization signal of a probe from a test (for example, disease tissue) sample is greater than hybridization signal of a probe from a control, normal tissue sample, the gene or genes overexpressed in the disease tissue are identified. The implication of this result is that an overexpressed protein in a disease tissue is useful not only as a diagnostic marker for the presence of the disease condition, but also as a therapeutic target for treatment of the disease condition.

The methodology of hybridization of nucleic acids and microarray technology is well known in the art. In one example, the specific preparation of nucleic acids for hybridization and probes, slides, and hybridization conditions are all detailed in PCT Patent Application Serial No. PCT/US01/10482, filed on March 30, 2001 and which is herein incorporated by reference. The detailed microarray methodology has also previously been reported by Noble et al. (Gut 2008, Oct;57(10):1398-405)

Total RNA was extracted from each biopsy using the micro total RNA isolation from animal tissues protocol *(Qiagen, Yalencia, CA).* 1µg of total RNA was amplified using the Low RNA Input Fluorescent Linear Amplification protocol (*Agilent Technologies, Palo Alto, CA).* A T7 RNA polymerase single round of linear amplification was carried out to incorporate Cyanine-3 and Cyanine-5 label into cRNA. The cRNA was purified using the RNeasy Mini Kit (*Qiagen*)*.* 1 µl of cRNA was quantified using the NanoDrop ND-1000 spectrophotometer (*NanoDrop Technologies, Delaware*)*.* 750ng of Universal Human Reference (*Stratagene, La Jolla, CA*) cRNA labeled with Cyanine-3 and 750ng of the test sample cRNA labeled with Cyanine-5 were fragmented for 30 minutes at 60°C before loading onto the *Agilent* Whole Human Genome microarrays (Agilent Technologies, Palo Alto, CA) which are annotated to represent 33296 genes. The samples were hybridized for 18 hours at 60°C with constant rotation. Microarrays were washed, dried and scanned on the Agilent scanner according to the manufacturer's protocol. Microarray image files were analysed using Agilent's Feature Extraction software version 7.5. The genes were normalized using the *Stratagene* Universal Human Reference. The distribution of log intensities for each sample was plotted and outlier samples (i.e. greater than 2 standard deviations from the mean) were excluded from analysis. The whole data set are available online under the Gene Expression Omnibus of the National Center for Biotechnology Information website.

### Real Time PCR

Real time PCR analysis was undertaken in 5 genes-IL-8, Saar, DEFA5 & 6 and MMP3 on RNA from 15 CD and 6 control TI biopsies. IL-8 and SAA1 were chosen as robust markers of epithelial inflammation and DEFA5 & 6 were selected as there has been considerable interest in their expression in TI CD. Prior to real time PCR analysis 1 RNA amplification cycle was carried out using the MessageAmp™ II aRNA Amplification Kit protocol (*Applied Biosystems, Foster City, CA*)*.* Reverse transcription PCR was then performed on 50ng of RNA using Stratagene model MX4000. TaqMan primers and probes were manufactured in house. **(Table 4)** PRC conditions comprised of 48°C for 30 minutes, 95°C hold for 10 minutes, followed by 40 cycles of 30 second 95°C melt and 1 minute 60°C anneal/extend. Absolute quantification of product was calculated by normalizing to RPL19.

**Table 4: TaqMan primers used for real- time PCR**

| **Gene** | | **Sequence** | **SEQ ID NO:** |
|---|---|---|---|
| **SAA1** | forward | agcgatgccagagagaata | 23 |
| | reverse | ggaagtgattggggtctttg | 24 |
| | Taq | ctttggccatggtgcggagg | 25 |
| **IL8** | forward | actcccagtcttgtcattgc | 26 |
| | reverse | caagtttcaaccagcaagaa | 27 |
| | Taq | tgtgttggtagtgctgtgttgaattacgg, | 28 |
| **DEFA5** | forward | gctacccgtgagtccctct | 29 |
| | reverse | tcttgcactgctttggtttc | 30 |
| | tax | tgtgtgaaatcagtggccgcct | 31 |
| **DEFA6** | forward | agagctttgggctcaacaag | 32 |
| | reverse | atgacagtgcaggtcccata | 33 |
| | Taq | cacttgccattgcagaaggtcctg | 34 |
| **MMP3** | forward | aagggaacttgagcgtgaat | 35 |
| | reverse | gagtgcttccccttctcttg | 36 |
| | Taq | ggcattcaaatgggctgctgc | 37 |

### Data Analysis

Microarray data were analysed using the Rosetta Resolver® software (*Rosetta Inpharmatics, Seattle).* Statistical significance of the microarray data was determined by Student's unpaired *t* test. p < 0.01. Fold change data were calculated using the Rosetta Resolver software. To correct for multiple hypothesis testing a q-value was calculated for each tested feature to estimate significance in terms of the false discovery rate (FDR) rather than the false positive rate. For every differential expression analysis the q-value was calculated and a FDR was calculated using the method proposed by Storey et al. (Proc Natl Acad Sci U S A 2003;100:9440-9445) A FDR of less than 5% was calculated for each of the presented analysis. Hierarchical clustering analysis was undertaken using Pearson correlation method. Gene ontology was analyzed using Ingenuity software (*Ingenuity Systems, Mountain View, CA)* The Mann-Whitney U test was used to analyze the real time PCR data. p < 0.05 was considered significant.

Gene ontology was analysed using Ingenuity software *(Ingenuity Systems, Mountain View, CA).* Hierarchical clustering analysis using a collection of immune response *in silico* genes from a compendium of six immune cell types was undertaken. (Abbas et al. Genes Immun 2005;6(4):319-31) Hierarchical clustering analysis was also undertaken using a set of 14 epithelial cell cytokines-CXCL1, CXCL2 CXCL5, CXCL9, CXCL10, CXCL11, CCL2, CCL4, CCL7, CCL20, IL-8, IL-12A, IL-23A and MDK. (Dwinell et al. Gastroenterology 2001;120(1):49-59; Lee et al. J Immunol 2008;181(9):6536-45; Yang et al. Gastroenterology 1997;113(4):1214-23)

### Results

The aim of the present study was to use microarray expression analysis to describe the transcriptional profiles in the colon and the terminal ileum in patients with CD and controls. In additional to this hypothesis-free scanning, expression of germ line variants identified by GWAS and cell specific lineage analysis were also investigated.

### Unsupervised Hierarchical Clustering Analysis

When all of the CD (n=99) and control biopsies (n=73) were clustered together using unsupervised hierarchical clustering analysis, no separation of the biopsies by either disease status or by the degree of inflammation was observed. When the anatomical location that the biopsies were taken from was considered, 18 TI biopsies clustered together (6 control and 12 CD)(p<0.001).

Figure 23 shows an unsupervised clustering analysis of the TI biopsies initially was confounded by the sex of patients, however when a degree of supervision was introduced and only TI biopsies from female patients and controls were clustered, clustering by disease status was observed.

Gene ontology of the 593 downregulated sequences grouped by biological process revealed a preponderance of genes associated with carboxylitic acid metabolic processes (39 of a total of 464 genes classified by the ontology software to this biological group; OR 3.4, p=7x10⁻¹³), organic acid metabolic processes (38/464; OR 3.1 p=1x10⁻¹²) and lipid metabolic processes (46/620; OR 3.0, p=6.6x10⁻¹²). When the downregulated sequences were grouped by biological function genes grouped under solute/ cation transporter activity (11/50; OR 10.3, p=6.9x10⁻¹⁵), electrochemical potential- driven transporter activity (23/188; OR 5.16, p=2.7x10⁻¹⁴) and solute/ sodium transporter activity (10/46; OR 10.1, p=2.4x10⁻¹³) were disproportionately downregulated. When these groups of genes were combined to encompass all genes involved in transporter activity, there was a significant over representation of this group in the downregulated genes (64/1138; OR 2.3, p=3.6x10⁻⁹).
367 sequences were upregulated in a subset of the CD samples compared to the controls. Ontology of these genes grouping by biological processes showed that genes that grouped into structural molecule activity (22/603; OR 2.62, p=4.5x10⁻⁵) and extracellular matrix structural constituents (6/87; OR 5.5, p=0.0003) were overrepresented. When the genes were grouped by biological function upregulated genes grouped into sequence specific DNA binding (11/430; OR 2.28, p=0.007) and transcription factor activity (20/810; OR 1.7, p=0.043).

### Gene Expression in Crohn's Disease and Controls

When 99 CD biopsies were compared to 73 control biopsies, 259 sequences were upregulated and 87 sequences were downregulated **(****Figure 24****).** Notably upregulated genes in the CD biopsies included the acute phase proteins serum amyloid A1, (SAA1; FC +7.5, p=1.47x10⁻⁴¹), the regenerating C-type lectin family member (REGL; FC +7.3, p=2.3x10⁻¹⁶), the acute phase proteins (S100A9; FC +4.4, p=2.4x10⁻²²) and (S100A8; FC +4.0, p= 3.5x10⁻¹⁸). IL-8 a robust marker of mucosal inflammation was the sixth most upregulated gene (FC +3.6, p=5.6x10⁻¹⁹). Among the most downregulated genes were genes involved in cellular detoxification- (SLC14A2; FC -2.49, p=0.00002), (carbonic anhydrase 2; FC - 2.4, p=8.4x10⁻¹⁰) and (carbonic anhydrase 1; FC -2.3, p=7.5x10⁻⁶).

### Gene Expression in the Terminal Ileum

TI biopsies from 16 patients with CD- 6 non-inflamed biopsies, 7 chronically inflamed biopsies and 3 acutely inflamed biopsies were compared to 6 healthy control TI biopsies. When all of the CD terminal ileal (TI) biopsies were compared to control TI biopsies 1045 sequences had a fold change of greater than 1.5 and 1044 sequences had a fold change of less than -1.5 (p<0.01). **(****Figure 25****).** Interesting upregulated genes in the CD biopsies included diubiquitin (UBD) which is involved in synaptic transmission; FC +11.3, p<1x10⁻⁴⁵, (MMP3; FC +7.4, p=1.3x10⁻¹¹), (IL-8; FC +4.9, p=2.3x10⁻⁸), (trefoil factor 1 (TFF1) which acts in the GI tract to maintain the mucosal surface barrier; FC +4.3, p=1.3x10⁻⁷) and the cytokeratin (keratin 5β; FC +4.2, p=0.005) **(Table 5).** Downregulated genes included immune associated genes (IRTA1- a novel surface B-cell receptor; FC -11.1, p=4.7x10⁻¹²), (CCL23; FC -7.1, p=1.6x10⁻¹⁰), (CXCR4; FC -6.0, p=8.2x10⁻¹⁸), and genes involved in cholesterol metabolism (APOC3; FC -8.2, p=7.0x10⁻⁸) and (APOA1; FC -6.9, p=0.0031).

**Table 5: Expression changes in genes of interest in biopsies from the terminal ileum.**

| **Gene** | **Sequence Code/ Genbank cluster code** | **All CD samples (16) v controls (6). Fold change (FC)** | **p value** | **CD Non-inflamed (6) v non-inflamed controls (6) (FC)** | **p value** | **CD Inflamed (10) v non-inflamed (6) (FC)** | **p value** |
|---|---|---|---|---|---|---|---|
| CXCR4 | A_23_ P102000 | -6.02 | 8.2x10⁻¹⁸ | -2.1 | 5.23x10⁻¹⁰ | +1.73 | 0.0033 |
| IL-8 | A_32_P87013 | +4.85 | 2.30x10⁻⁸ | +1.63 | 0.0017 | +16.9 | 1.26x10⁻¹³ |
| APOA1 | A_23_P203191 | -6.86 | 0.0031 | -1.032 | 0.91 | -12.22 | 0.00003 |
| APOC3 | A_23_P203183 | -8.18 | 7.02x10⁻⁸ | +1.36 | 0.10 | -12.36 | 9.70x10⁻¹⁴ |
| TFF3 | A_23_P257296 | +2.40 | <10⁻⁴⁵ | +2.0 | 1.47x10⁻¹⁶ | +1.72 | 6.1x10⁻²² |
| CD28 | A_23_P91015 | -3.76 | 1.77x10⁻¹⁷ | -4.52 | 1.32x10⁻²² | +1.30 | 0.12 |
| UBD | A_23_P81898 | +11.3 | <10⁻⁴⁵ | +8.48 | 1.32x10⁻³⁴ | +2.50 | 0.00009 |
| IRTA1 | A_23_P115201 | -11.43 | 4.72x10⁻¹² | -1.57 | 0.0001 | -2.93 | 0.0032 |
| CCL23 | A_24_P133905 | -7.14857 | 1.62E-10 | | | | |
| DefA5 | A_23_P112086 | -1.16 | 0.034 | -1.07 | 0.41 | -1.14 | 0.22 |
| DefA6 | A_23_P363711 | -1.085 | 0.11 | -1.11 | 0.34 | +1.04 | 0.70 |

| | | | | | | | |
|---|---|---|---|---|---|---|---|
| Fold changes and p values are shown in a number of different genes in three different experiments. The number of biopsies analyzed in each experiment is shown in brackets. Candidate genes were included in this table if significant consistent changes in expression were observed across more than one experiment. Analysis of DefA5 and DefA6 expression showed no significant changes across the different groups that were examined. | | | | | | | |

### Colonic Gene Expression Analysis

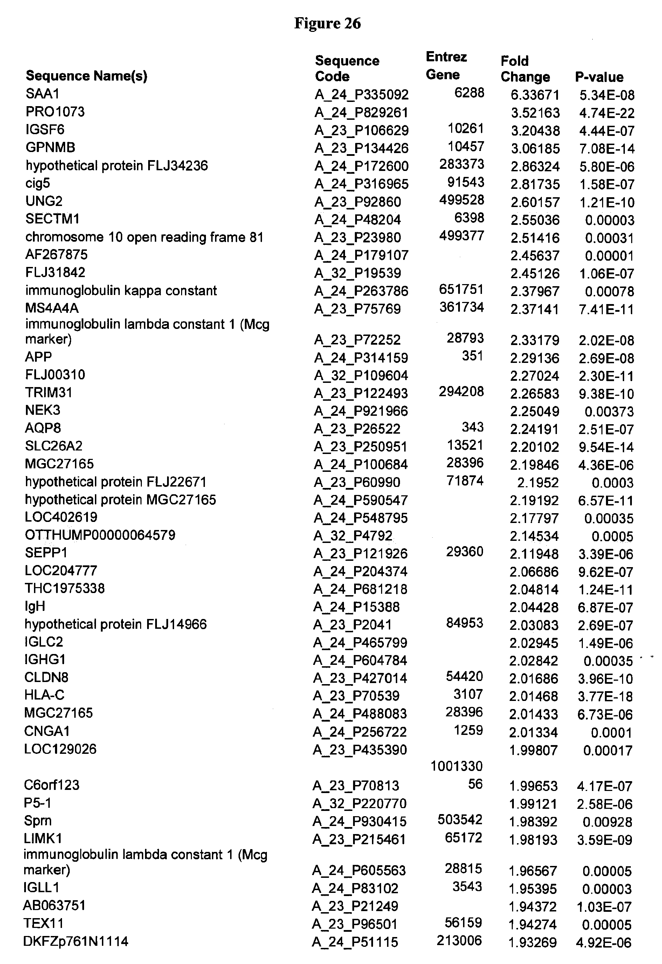

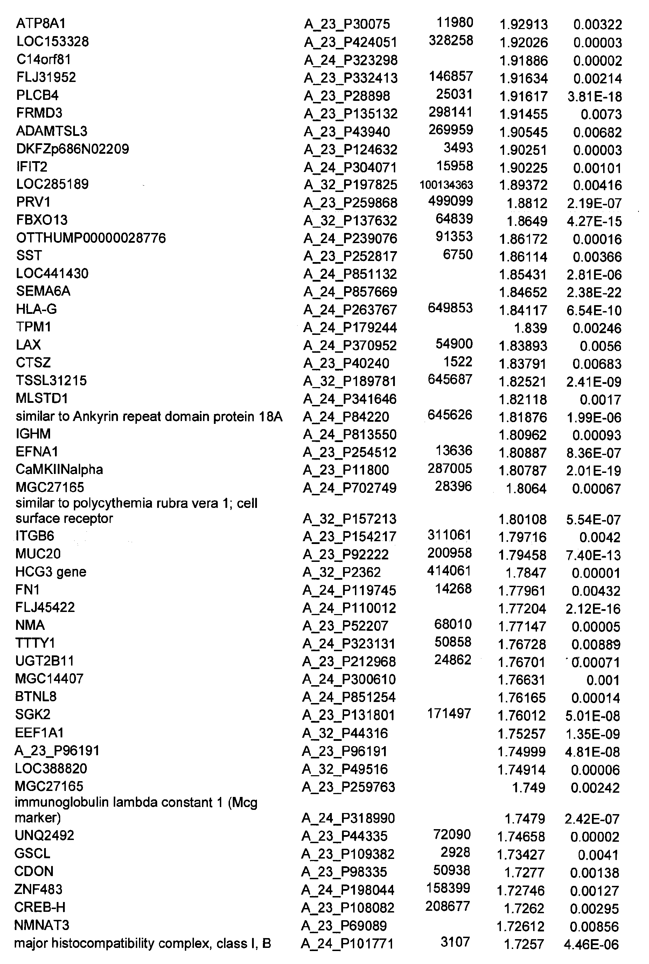

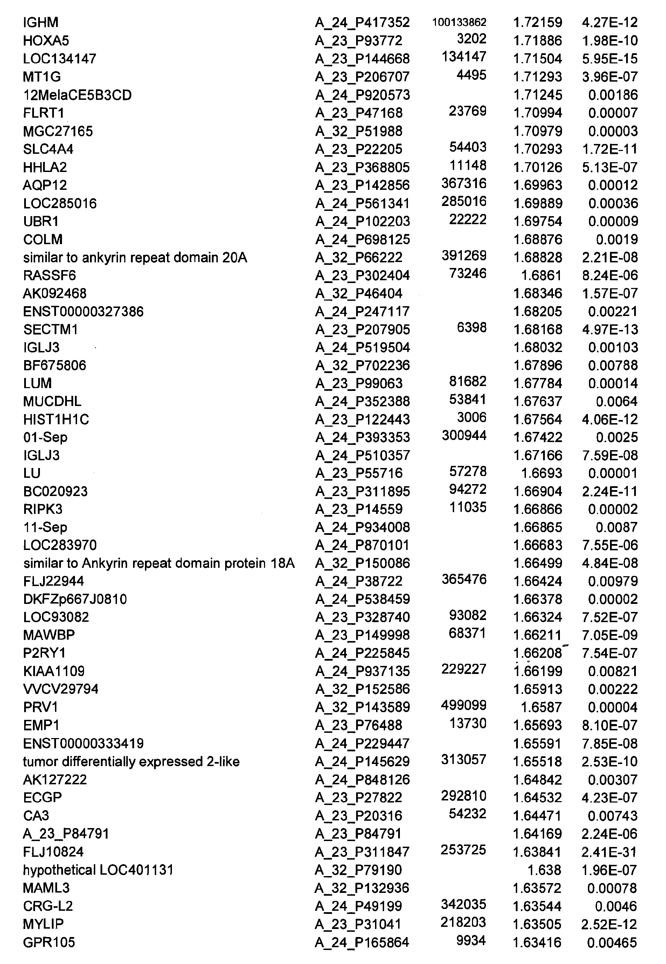

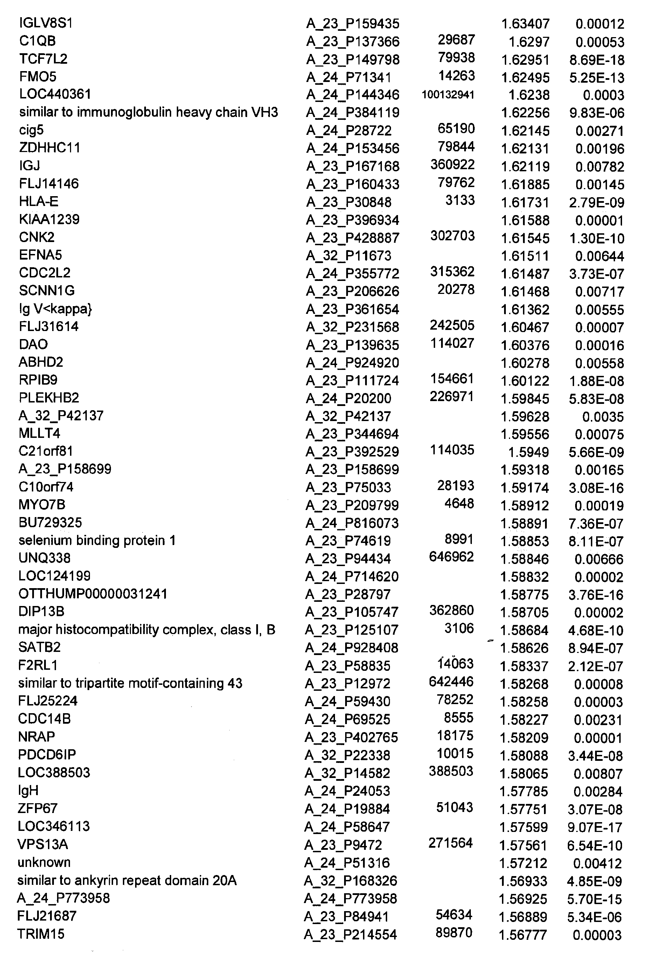

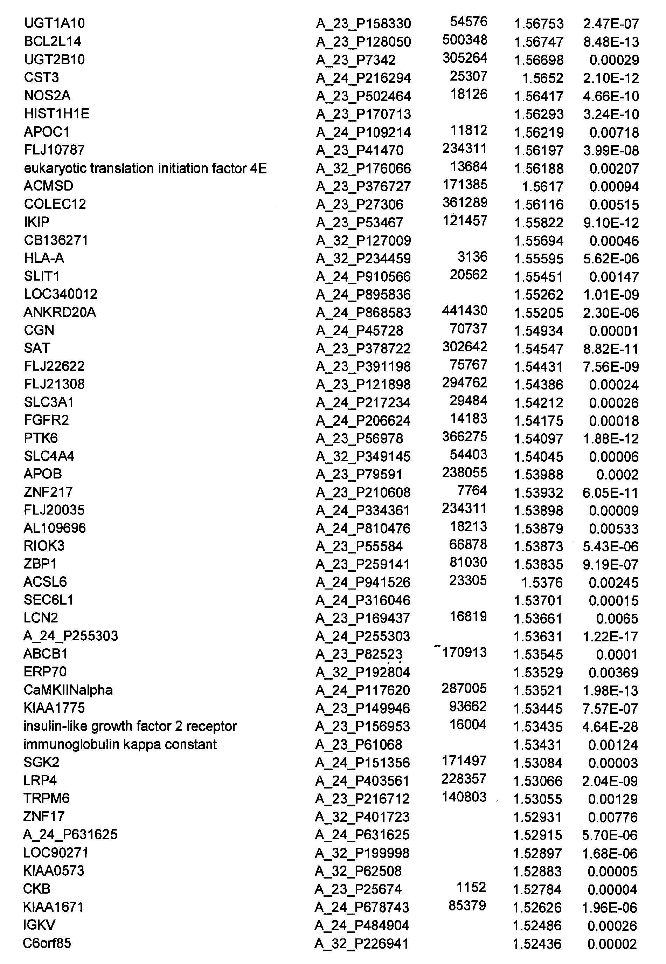

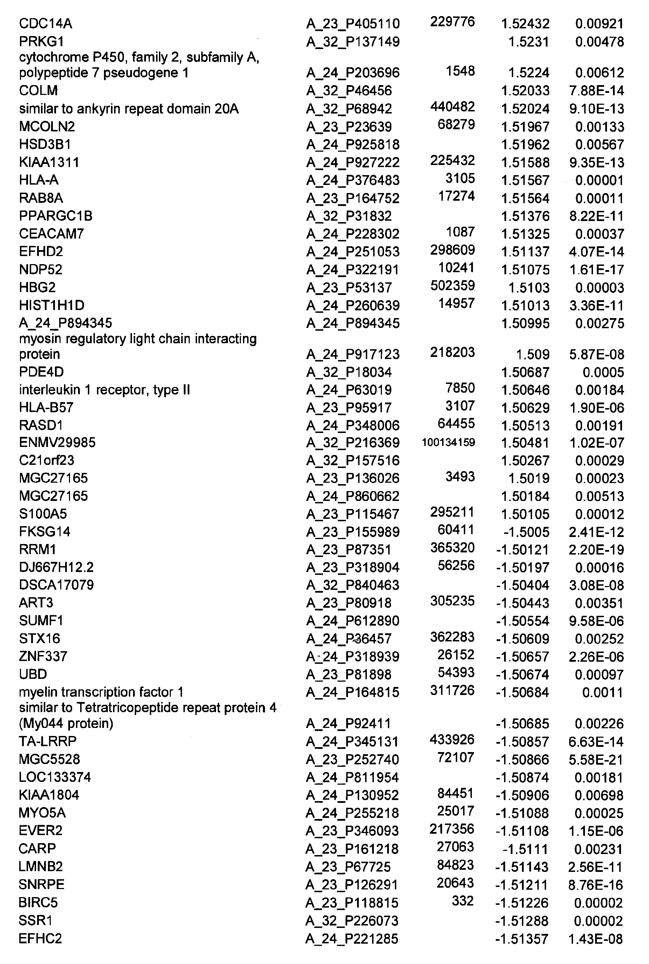

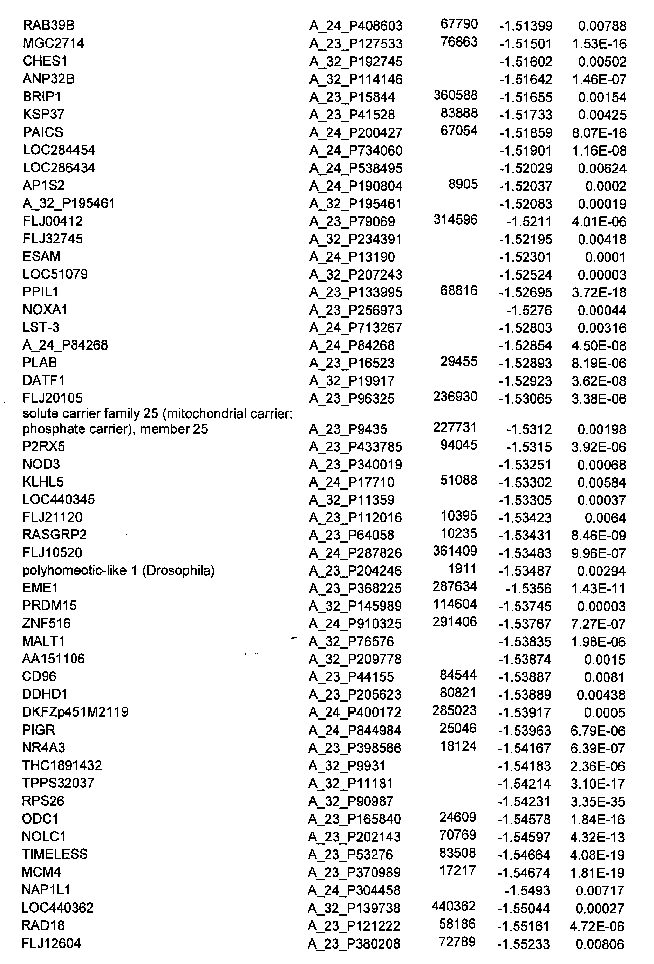

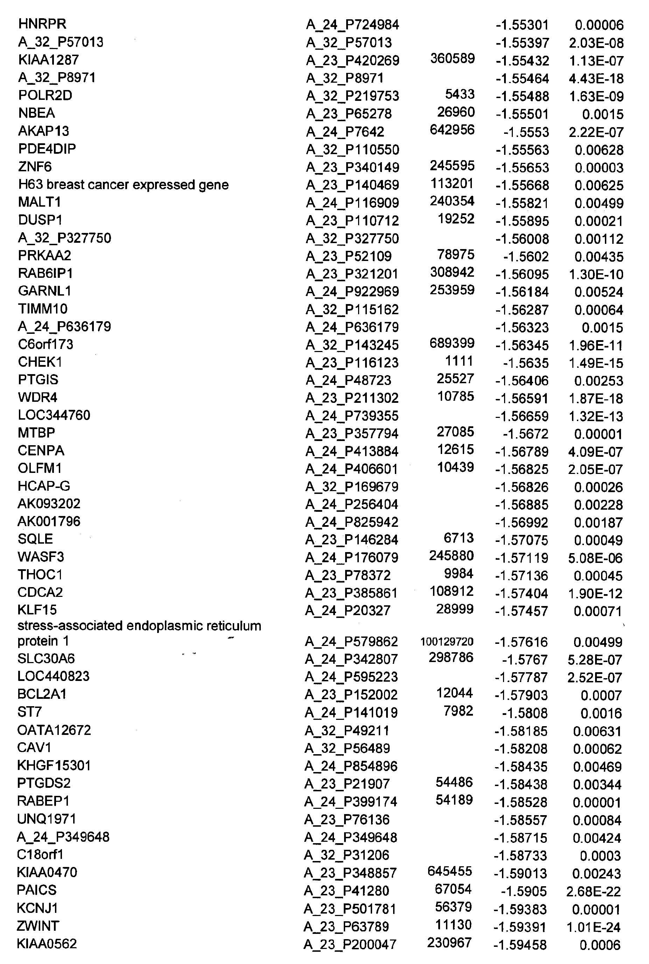

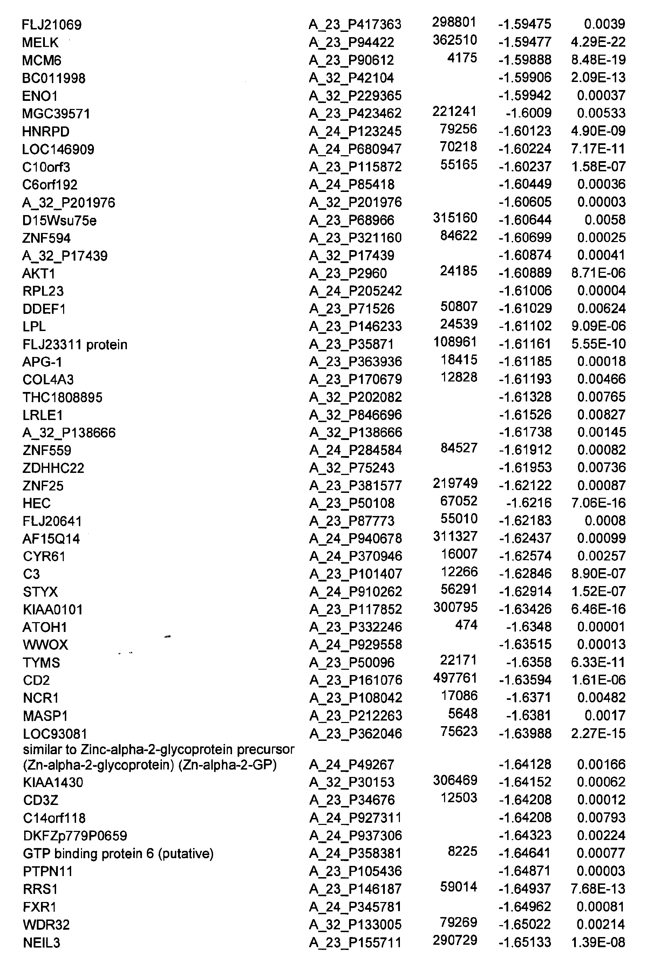

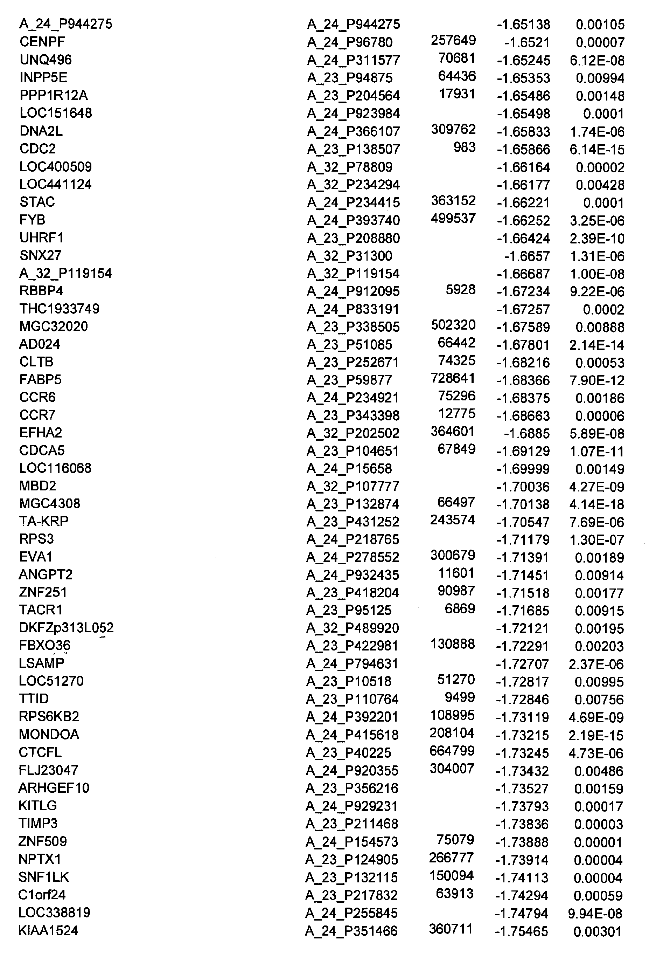

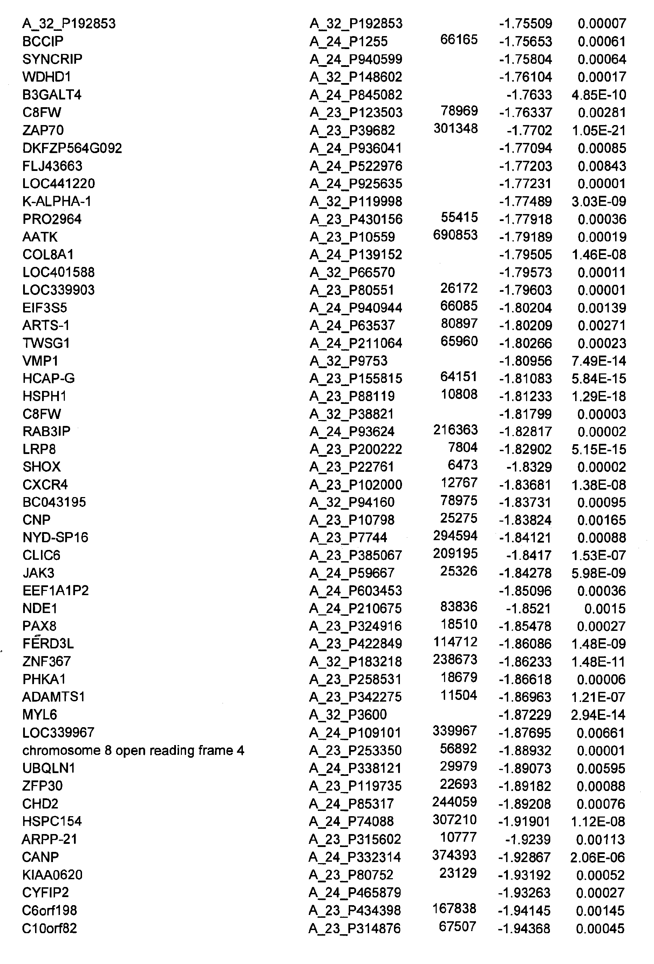

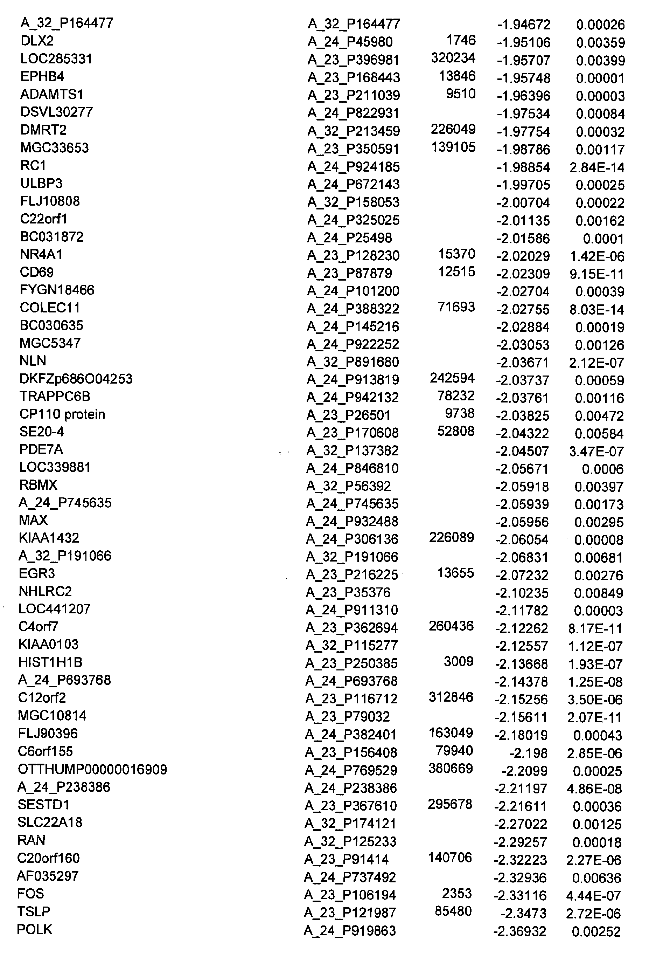

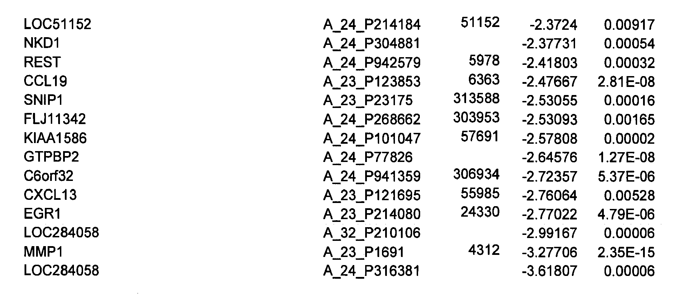

To minimize the effect of differential gene expression related to the anatomical location of the biopsy, sigmoid colon biopsies were used for analysis. (Noble et al. Gut 2008, Oct;57(10):1398-405) To also remove the acute inflammatory expression signature non-inflamed CD biopsies (n=17) were compared to non-inflamed control biopsies (n=18) **(****Figure 26****).** SAA1 remained the most upregulated gene; FC +6.3, p=5.3x10⁻⁸ and in total 279 sequences were upregulated. 349 sequences were downregulated and the most downregulated genes included (MMP1; FC -3.6, p=2.4x10⁻¹⁵), (CXCL13; FC -2.7, p=0.005) and TSLP -thymic stromal lymphoprotein; FC -2.3, p=2.7x10⁻⁶**(Table 6).**

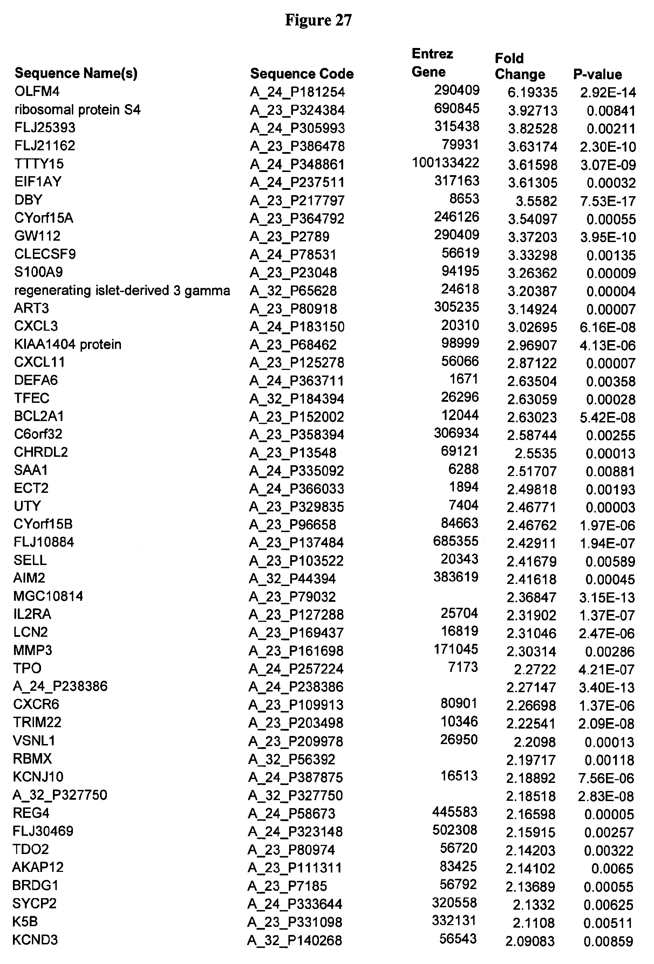

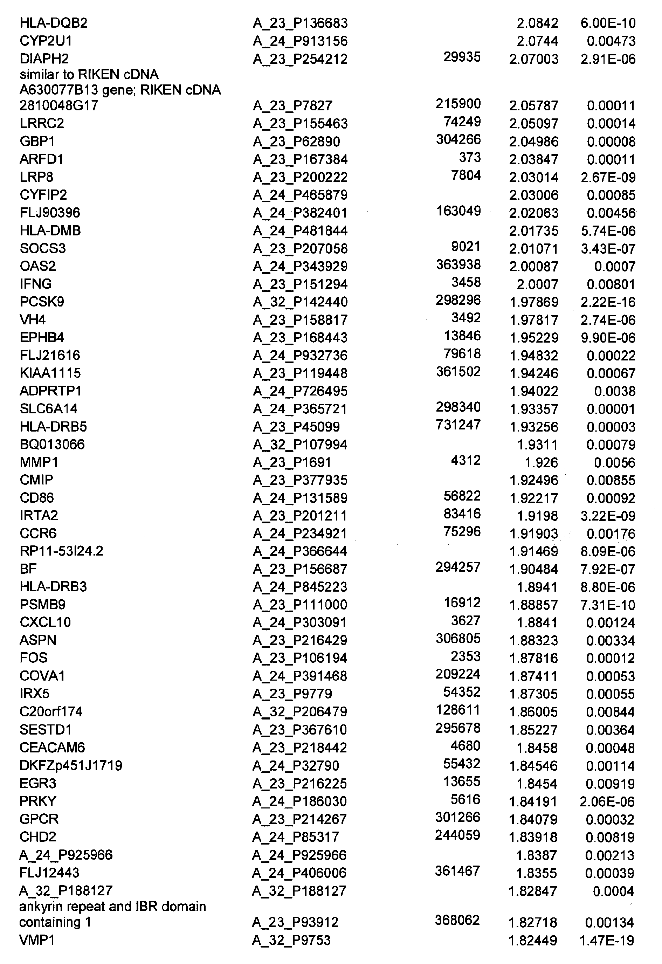

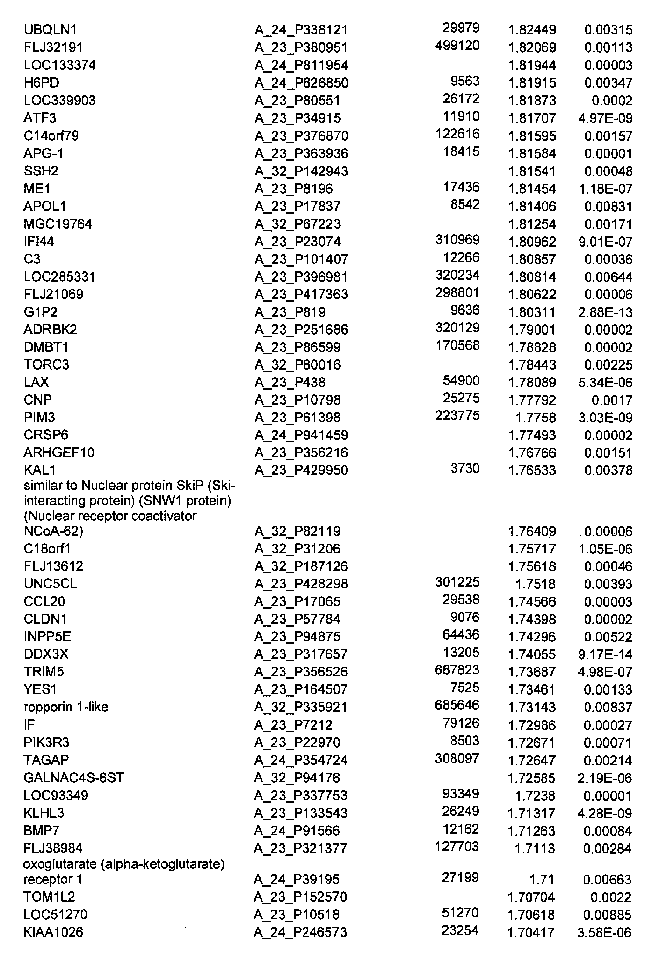

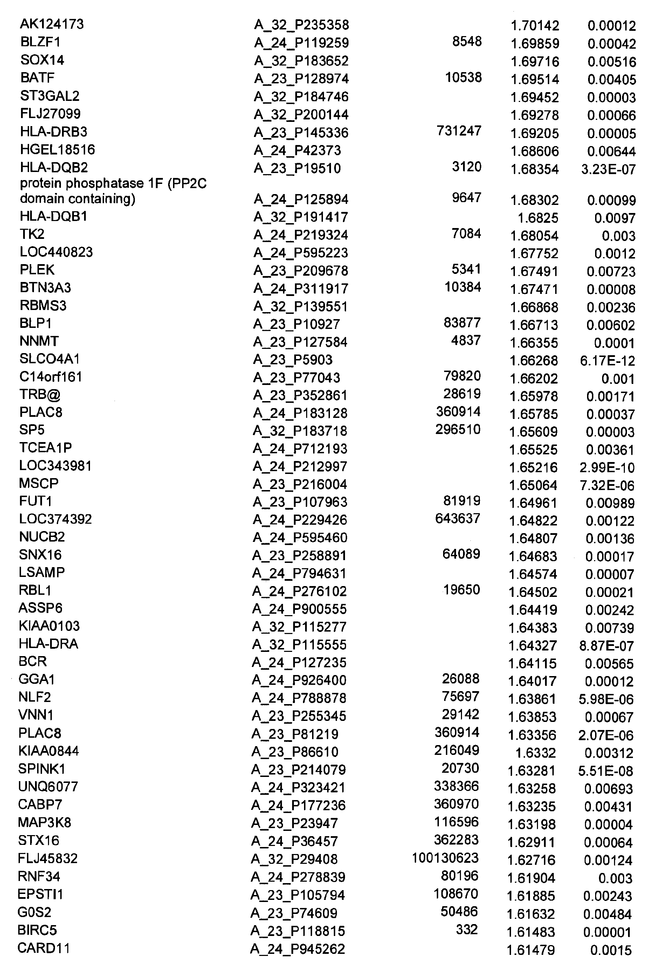

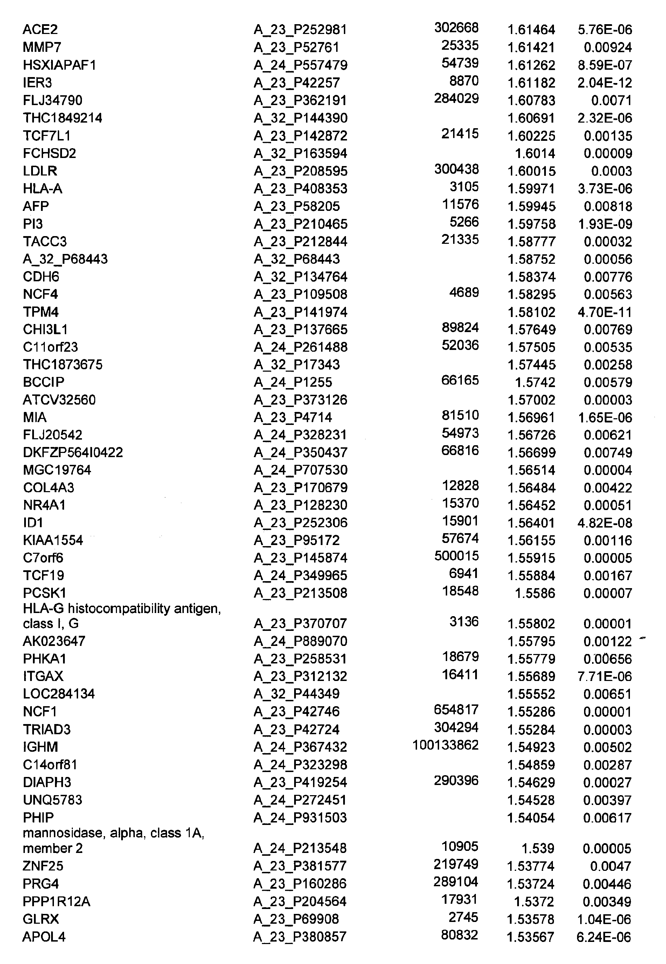

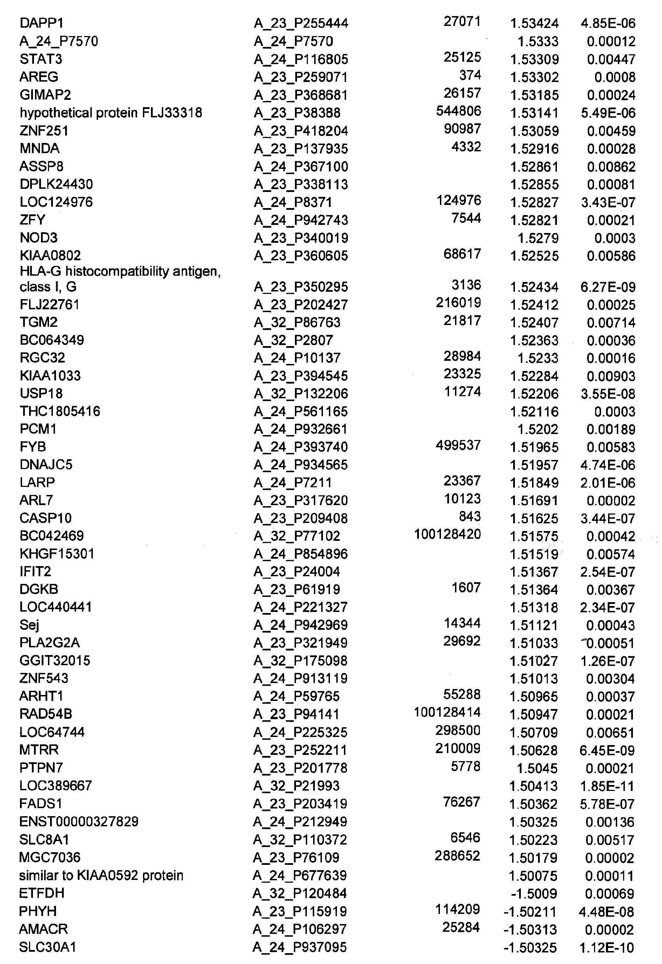

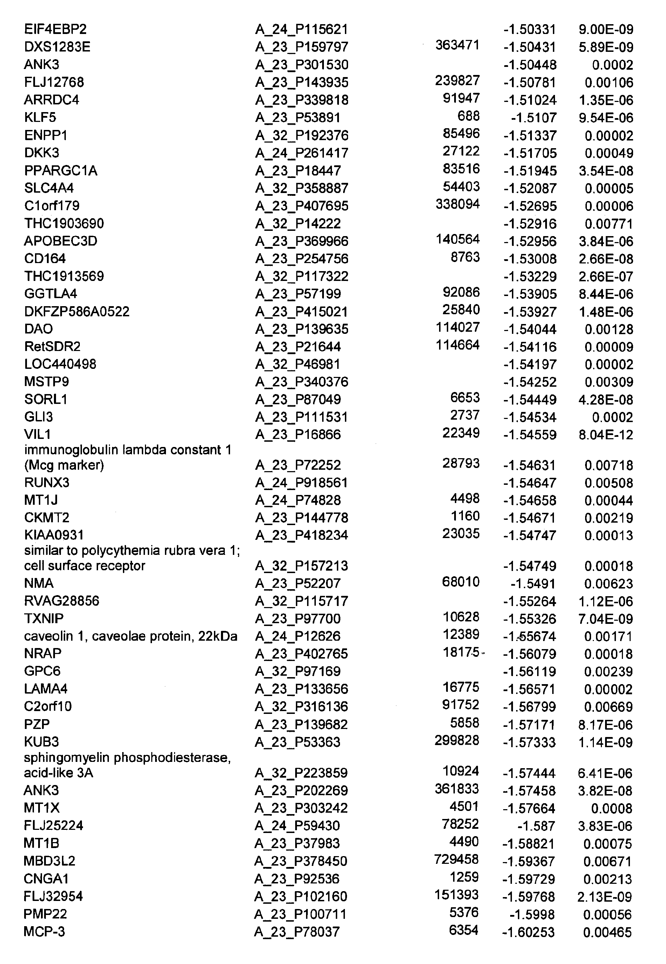

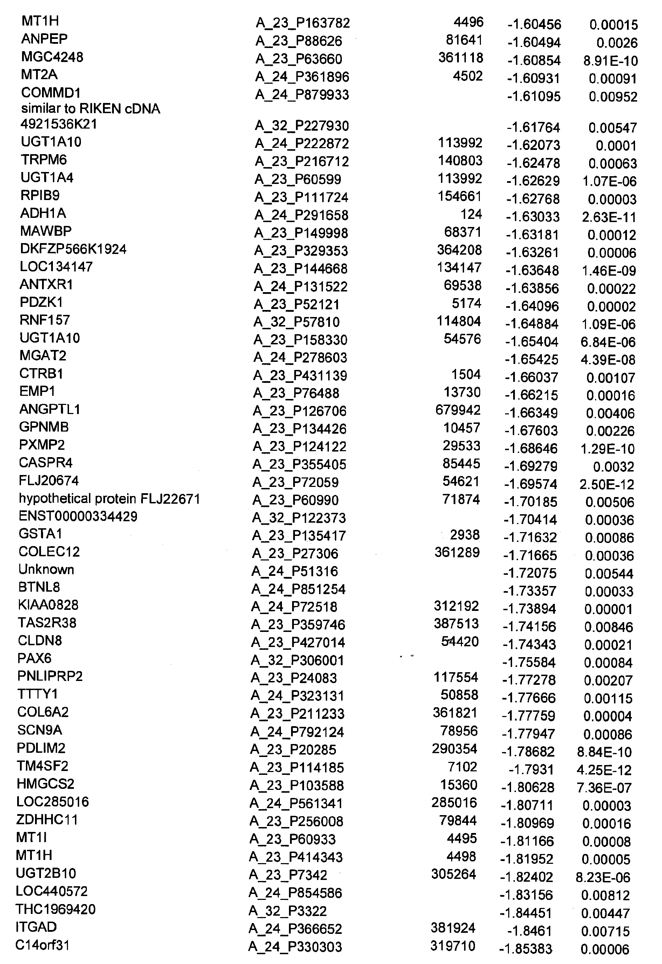

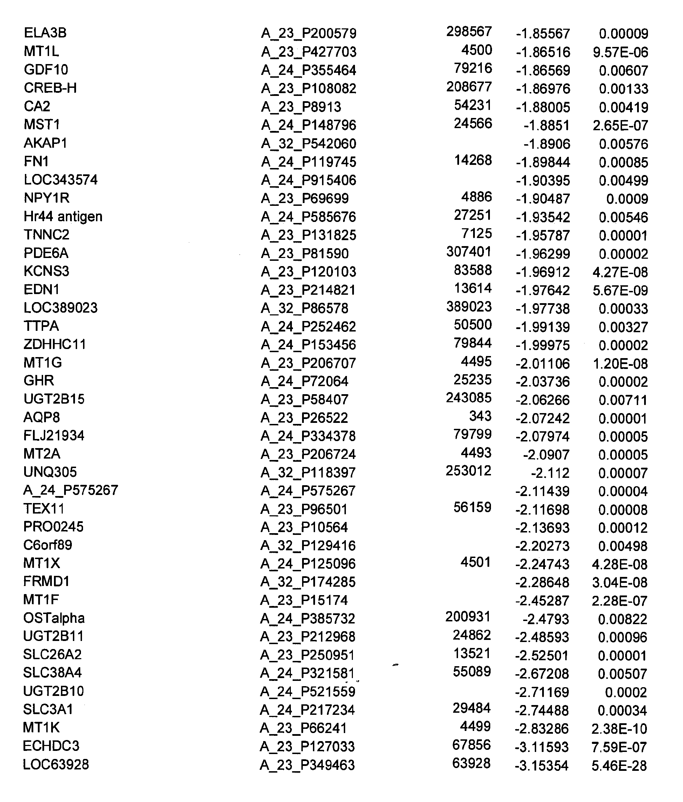

When the acute inflammatory signal was examined in the sigmoid colon and 16 inflamed CD biopsies were compared to 17 non- inflamed CD biopsies, 279 sequences were upregulated and 148 sequences were down regulated **(****Figure 27****).** The most upregulated gene in the inflamed biopsies was OLFM4- an anti-apoptotic molecule that inhibits the capsase cascade and also binds to GRIM19; FC +6.2, p=2.9x10⁻¹⁴. Downregulated genes included organic solute carriers (SLC38A4; FC -2.7, p=0.005), (SLC26A2; FC -2.5, p=0.00001) and (OST alpha; FC -2.5, p=0.008).

### Expression of Genes implicated by GWAS Meta-analysis

Expression of susceptibility genes identified by GWAS meta- analysis by Barrett et al (Nat Genet 2008, Aug;40(8):955-62) were investigated along with further detailed analysis of the IL-23 and autophagy pathways. **(Table 7)** Upregulated genes in the CD biopsies compared to the controls included (NOD2/CARD15; FC +1.23, p=0.000243) (PTGER4- prostaglandin E receptor 4; FC +1.1, p=0.00010) and NKX2.3, a 3 exon homeobox gene; FC +1.37, p=0.001. The cell cycle control gene (CDKAL1; FC - 1.1, p=0.0096) was downregulated in the CD biopsies compared to the controls. No expression data was present for on the *Agilent* chip for IGRM and no differences were observed between disease groups when expression of TNFSF15, PTPN22, ICOSLG, ITLN1, ZNF365, LRRK2 and PTPN2 were examined.

When inflamed and non-inflamed CD sigmoid colon biopsies were compared MST1-Macrophage stimulatory protein; FC -1.58, p=0.0037 and (C11orf30; FC -1.22, p=0.0078) were downregulated in the inflamed biopsies.

**Table 6: Expression changes in genes of interest in biopsies from the colon.**

| **Gene** | **Sequence code** | **All CD (99) v controls (73) All CD Fold change (FC)** | **p value** | **Inflamed (16) v non-inflamed (17) CD sigmoid (FC)** | **p value** | **Inflamed CD sigmoid (16) v inflamed control sigmoid (9) (FC)** | **p value** | **Non-inflamed CD sigmoid (17) v non-inflamed control (18) sigmoid Fold change** | **p value** |
|---|---|---|---|---|---|---|---|---|---|
| SAA1 | A_24_P335092 | +7.5 | 1.5x10⁻⁴¹ | +3.6 | 5.6x10⁻¹⁵ | +8.1 | 1.4x10⁻⁷ | +6.3 | 5.3x10⁻⁸ |
| IL-8 | A_32_P87013 | +7.5 | 1.5x10⁻⁴¹ | +2.5 | 0.0088 | +3.35 | 0.0030 | +1.06 | 0.59 |
| IFNG | A_23_P151294 | +2.1 | 2.3x10⁻⁹ | +2.0 | 0.0080 | +1.29 | 0.50 | +1.37 | 0.18 |
| TSLP | A_23_P121987 | -1.52 | 0.00021 | -1.19 | 0.34 | -1.42 | 0.49 | -2.34 | 2.7x10⁻⁶ |
| MMP3 | A_23_P52761 | +2.63 | 3.9x10⁻¹⁰ | +2.3 | 0.0029 | +7.6 | 3.14x10⁻¹⁰ | -1.50 | 0.015. |
| TNIP3 | A_23_P386478 | +3.84 | 4.2x10⁻⁶ | +3.63 | 2.9x10⁻¹⁰ | +4.41 | 0.00008 | -1.27 | 0.27. |
| TNF | A_23_P376488 | -1.079 | 0.0031 | +1.26 | 0.0044 | -1.13 | 0.15 | -1.10 | 0.13 |
| CXCL13 | A_23_P121695 | -2.76064 | 0.00528 | | | | | | |

| | | | | | | | | | |
|---|---|---|---|---|---|---|---|---|---|
| Fold changes and p values are shown in a number of different genes in four different experiments. The number of biopsies analyzed in each experiment is shown in brackets. Novel genes identified by analysis of the microarray data set and genes with an established role in the pathogenesis of inflammatory bowel disease were investigated. | | | | | | | | | |

**Table 7: Expression of genes identified by Barrett et al (Nat Genet 2008, Aug;40(8):955-62) as being associated with Crohn's disease.**

| **Entrez Gene** | | | **All CD (99) v controls (73)** | | **Inflamed CD sigmoid (16) v non-inflamed CD sigmoid (17)** | |
|---|---|---|---|---|---|---|
| **ID** | **Symbol** | **Agilent ID** | **Fold Change** | **p value** | **Fold Change** | **p value** |
| 3717 | JAK2 | A_23_ P123608 | +1.90 | 9.43E-07 | +1.58 | 0.000031 |
| 55054 | ATG16L1 | A_32_P113508 | -1.16 | 1.96E-05 | +1.06 | 0.549 |
| 3593 | IL-23A/p19 | A_23_P425197 | +2.32 | 0.000099 | +2.11 | 0.000031 |
| 5734 | PTGER4 | A_23_P435394 | +1.11 | 0.000104 | -1.04 | 0.55 |
| 64127 | NOD2 | A_23_P420863 | +1.23 | 0.000243 | +1.24 | 0.1092 |
| 6774 | STAT3 | A_24_P116805 | +2.23 | 0.000353 | +1.66 | 0.0002 |
| 159296 | NKX2-3 | A_23_P52425 | +1.37 | 0.000994 | -1.17 | 0.456 |
| 54901 | CDKAL1 | A_23_P44781 | -1.1 | 0.00964 | -1.14 | 0.0919 |
| 94103 | ORMDL3 | A_23_P38190 | +1.13 | 0.0140 | +1.07 | 0.656 |
| 56946 | C11orf30 | A_23_P380839 | +1.1 | 0.0156 | -1.22 | 0.0077 |
| 9966 | TNFSF15 | A_23_P94754 | +1.08 | 0.0447 | +1.09 | 0.5281 |
| 26191 | PTPN22 | A_23_P201181 | +1.07 | 0.107 | +1.03 | 0.6849 |
| 1235 | CCR6 | A_24_P234921 | +1.21 | 0.144 | +1.84 | 0.0566 |
| 23308 | ICOSLG | A_23_P317667 | +1.1 | 0.161 | -1.10 | 0.857 |
| 55600 | ITLN1 | A_23_P95790 | -1.1 | 0.162 | -1.02 | 0.905 |
| 22891 | ZNF365 | A_23_P86610 | +1.17 | 0.244 | -1.22 | 0.423 |
| 120892 | LRRK2 | A_23_P128447 | +1.25 | 0.413 | +1.37 | 0.135 |
| 5771 | PTPN2 | A_23_P309701 | -1.04 | 0.483 | +1.07 | 0.545 |
| 4485 | MST1 | A_24_P148796 | -1.04 | 0.709 | -1.58 | 0.0036 |
| 149233 | IL-23R | A_23_P7560 | -1.02 | 0.823 | +1.05 | 0.4271 |

| | | | | | | |
|---|---|---|---|---|---|---|
| For each experiment the fold change and p values have been calculated. The number of biopsies analysed in each experiment are shown in brackets. Gene annotation- JAK2-Tyrosine-protein kinase JAK2, ATG16L1-Prostaglandin E2 receptor EP4, NOD2-Nucleotide-binding oligomerization domain-containing protein 2, STAT3-Signal transducer and activator of transcription, NKX2-3- Homeobox protein Nkx-2.3, CDKAL1- CDK5 regulatory subunit-associated protein 1-like 1, ORMDL3- ORM1-like protein 3, C11orf30- Protein EMSY, TNFSF15. Tumor necrosis factor ligand superfamily member 15, PTPN22 & PTPN22- Tyrosine-protein phosphatase non-receptor type 22 and 2, CCR6- C-C chemokine receptor type 6, ICOSLG- ICOS ligand Precursor, ITLN1- Intelectin-1 Precursor, ZNF365- zinc finger protein 365, LRRK2- Leucine-rich repeat serine/threonine-protein kinase 2, MST1-Macrophage stimulatory protein. | | | | | | |

### The IL-23 Pathway

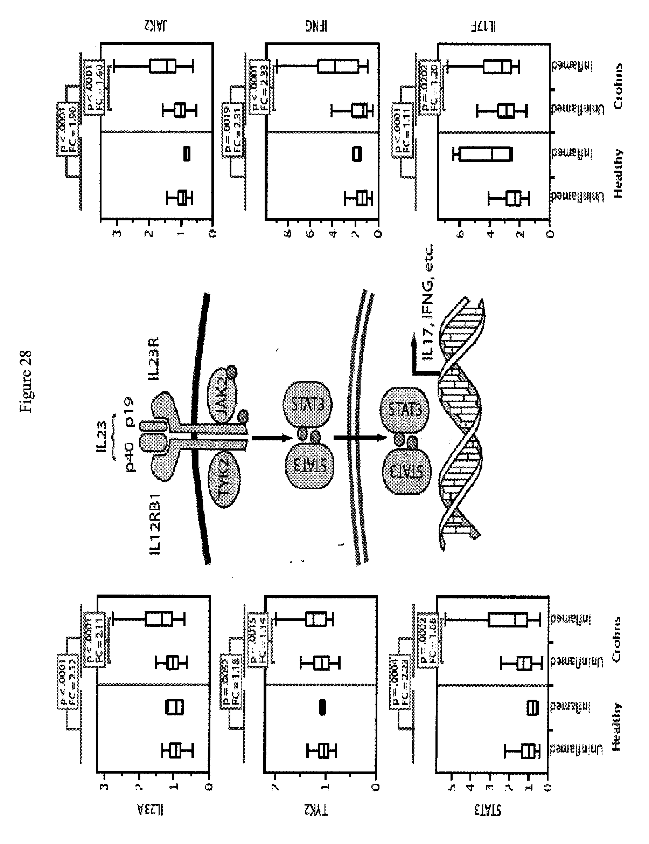

Figure 28 shows that when CD samples were compared to controls (IL-23A/p19; FC +2.32, p=0.000099), (TYK2; FC +1.18, p=0.0052), (JAK2; FC +1.90, p=9.4x10⁻⁷), (STAT3; FC +2.23, p=0.0004), (INFγ; FC +2.31, p=0.0019) and (IL17F; FC +1.11, p<0.0001) were significantly upregulated in the CD biopsies. When inflamed CD biopsies were compared to non-inflamed CD biopsies (IL-23A/p19; FC +2.11, p=0.000031), (TYK2; FC +1.14, p=0.0052), (JAK2; FC +1.90, p=0.00003), (STAT3; FC +1.66, p=0.0002) and (INFγ; FC +2.33, p<0.0001) had increased expression in the inflamed biopsies. No significant changes were observed in IL-23R expression.

### Autophagy Pathway

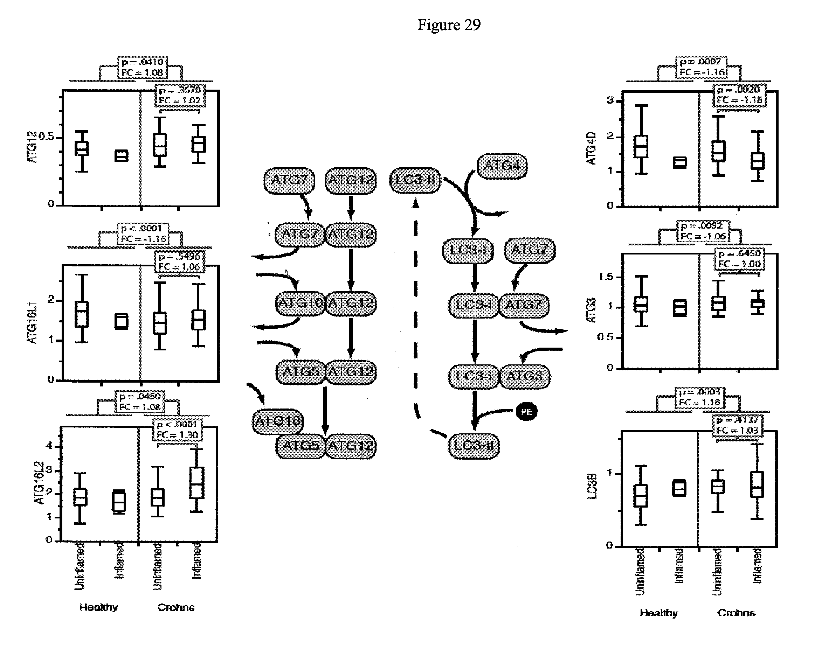

Figure 29 shows the analysis for ATG16LI and 19 other genes and key regulators of the autophagy pathway. ATG16LI was downregulated in the CD biopsies regardless of inflammation status compared to controls; FC -1.16, p=1.96x10⁻⁵ as was (ATG4D; FC -1.14, p=0.0007) and (ATG3; FC - 1.06, p=0.0052). (ATG12; FC +1.1, p=0.041), (ATG16L2; FC +1.1, p=0.045) and (LC3B; FC +1.18, p=0.0003) were marginally upregulated in the CD biopsies compared to the controls.

### Hierarchical Clustering by Specific Probe Subsets: Immune Response in Silico (IRIS) Probes

Using the previously defined *IRIS* probes to detect differential expression, CD and control biopsies from the ascending and descending colon were compared. (Abbas et al. Genes Immun 2005;6(4):319-31) Using the B cell, monocyte and T cell probes we were able to observe separation of the biopsies into CD and control biopsies by unsupervised clustering- B cell probes (p=0.0006, OR 2.74), the monocyte probes (p<0.0001 OR 5.22) and the T cell probes (p=0.0047 OR 2.4) using Chi squared analysis. In the monocyte cluster 2 genes CXCL1 and MMP1 were markedly differentially regulated in the CD biopsies and controls. No TI clustering was observed for any of the examined probes.

### Hierarchical Clustering by Epithelial Cell Markers

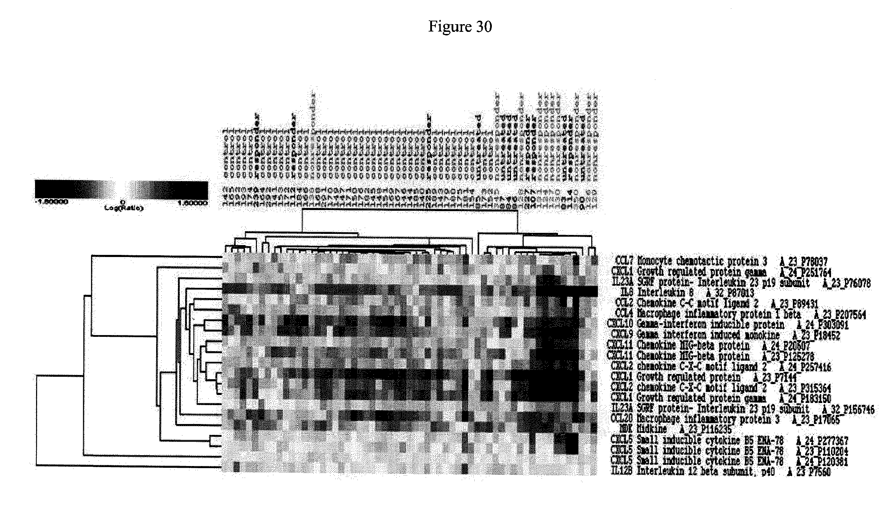

Figure 30 shows an unsupervised clustering analysis using a panel of 14 epithelial cell cytokines, CXCL1, CXCL2 CXCL5, CXCL9, CXCL10, CXCL11, CCL2, CCL4, CCL7, CCL20, IL-8, IL-12A, IL-23A and MDK (Dwinell et al., Gastroenterology 2001; 120(1):49-59; Lee et al., J. Immunol. 2008; 181(9):6536-45; Yang et al., Gastroenteroloty 1997;113 (4):1214-23) showed clear separation between colonic biopsies from CD patients and controls p<0.00001. When TI biopsies were considered this separation was not observed (p=0.052).

### Real Time PCR Confirmation of Microarray Results

In line with the histological classification of the biopsies, and the microarray results significantly higher IL-8 levels were observed in the CD TI biopsies compared to the control TI biopsies (p=0.0045) and in the inflamed CD TI biopsies compared to the non- inflamed CD TI biopsies (p=0.0046)**(Table 8).** Trends were also observed towards SAA1 being more highly expressed in the CD biopsies compared to the controls and in the inflamed compared to the non-inflamed CD TI biopsies. No difference in DEFA5 & 6 expression was observed in the CD TI biopsies compared to the control TI biopsies (p=0.73 and p=0.97 respectively), nor when the inflamed CD TI biopsies were compared to non-inflamed CD TI biopsies (p=0.39 and p=0.69 respectively).

**Table 8: Real time PCR expression in terminal ileal biopsies of patients with Crohn's disease and controls**

| **Genes** | **Median Relative Expression in control TI biopsies (6)** | **Median Relative Expression in CD TI biopsies (15)** | **Median Relative Expression in non- inflamed CD TI biopsies (7)** | **Median Relative Expression v inflamed CD TI biopsies (8)** | **Median Relative Expression in inflamed (8) v non-inflamed (7) CD TI biopsies** |
|---|---|---|---|---|---|
| IL-8 | 8.4 | 65.7 (0.0045) | 20.1 (0.054) | 307 (0.0037) | 307 v 20.1 (0.0046) |
| Def AS | 1.26 | 0.70 (0.73) | 0.51 (0.43) | 0.96 (0.95) | 0.98 v 0.51 (0.39) |
| Def A6 | 0.87 | 1.07 (0.97) | 1.1 (0.74) | 1.04 (0.85) | 1.04 v 1.1 (0.69) |
| SAA1 | 1.7 | 3.52 (0.20) | 2.0 (0.52) | 20.7 (0.14) | 20.7 v 2.0 (0.18) |
| MMP3 | 1.0 | 1.0 (1.0) | 1.0 (1.0) | 1.0 (1.0) | 1.0 v 1.0 (1.0) |

| | | | | | |
|---|---|---|---|---|---|
| The median relative expression of each gene is shown in disease groups along with the p value in brackets. The p values are calculated compared to the control group for each gene analysed. The number of biopsy samples used in each analysis is also shown in brackets. | | | | | |

### DISCUSSION

In this accurately phenotyped data set we have used clustering analysis to interrogate the genome wide expression profiles of patients with CD and controls. For the large number of novel CD susceptibility genes from GWAS where little data are presently available, we have been able to investigate expression profiles in the human colon and TI.

Given current concerns with respect to the reproducibility of microarray expression data it is firstly reassuring that our results are consistent with the findings from a previous microarray study in CD patients where increased expression of the S100 and the REG gene families were observed. (Lawrance et al. Hum Mol Genet 2001;10(5):445-56) Furthermore, in parallel with the results of Costello et al we observed a number of sequences representing novel proteins that were differentially expressed and using ontology and *in silico* analysis we were able characterise genes into functions related to CD pathogenesis. (Costello et al. PLoS Med 2005;2(8):e199)

This is the first study where genome wide expression has been investigated in unpooled TI endoscopic biopsies from CD patients and controls. Clustering analysis allowed us to differentiate between biopsies from CD patients and controls and the observed separation was driven by a cluster of downregulated genes involved in the normal homeostasis of the TI- organic acid and lipid metabolic processes, and solute/ cation transporter activity, and a cluster of genes that were upregulated which grouped into structural molecule activity. The most upregulated gene in the CD compared to the control TI biopsies was diubiquitin or ubiquitin-like protein FAT 10. The family of ubiquitin-like proteins function as part of the ubiquitin proteasome system which is a crucial pathway for protein degradation in eukaryotic cells.(Madsen et al., BMC.Biochem. 2007;8 Suppl 1:S1) The gene is located at the major histocompatibility complex locus in on chromosome 6 (Fan et al., Immunogenetics 1996;44(2):97-103), an established CD susceptibility loci and its expression has been observed to be increased in 90% of hepatocellular carcinomas and in 80% of colon cancers.(Lee et al., Oncogene 1-5-2003;22(17):2592-603) Diubiquitin is a downstream target of p53 and in p53-defective cells its expression is increased resulting in chromosomal instability.(Ren et al., J.Biol.Chem. 21-4-2006;281(16):11413-21; Zhang et al., Oncogene 2006;25(16):2318-27) Overall in this data set diubiquitin was upregulated when all CD biopsies were compared controls by a fold change of 1.5. Furthermore, diubiquitin expression in hepatocellular cancer and colon cancer correlates with increased expression of IFN-gamma and TNFα suggesting a mechanism for carcinogenesis in this pro-inflammatory environment.( Lukasiak et al., Oncogene 9-10-2008;27(46):6068-74)

The differing expression signature observed in the TI biopsies appeared to be primarily inflammation driven, rather than disease specific as the changes were less obvious in the non- inflamed analysis than when the inflamed and non-inflamed CD biopsies were compared. These dysregulated probes could form the basis of a diagnostic expression chip to help diagnose ileal CD and grade its severity.

Another of the notable observations in the TI analysis were data showing no difference in expression of the alpha defensins 5 and 6 (DEFA5&6) in the CD patients and controls regardless of the degree of inflammation in the biopsies. These results were confirmed by real time PCR and are contrary to previous data where reduced DEFA5&6 expression was observed in the TI of CD patients regardless of the degree of inflammation. (Wehkamp et al. Proc Natl Acad Sci U S A 2005;102(50):18129-34)

More recently, Simms et al also showed that expression of DEFA5&6 was down regulated in TI CD biopsies. (Simms et al. Gut 2008;57(7):903-10) However, this downregulation was inflammation specific, probably reflecting a loss of the epithelial layer and a reduction of epithelial and Paneth cells as a consequence of persistent inflammation. In our data set increased expression of DEFA5&6 was observed in the sigmoid colon biopsies of CD patients and this correlated with the degree of inflammation of the biopsies. Previously we have shown that the increase in colonic expression of DEFA5&6 in UC patients is largely mediated by Paneth cell metaplasia and that in the colon unregulated Paneth cell differentiation, and the consequent increase in DEFA5&6 expression, may perpetuate mucosal inflammation. (Noble et al. Gut 2008, Oct;57(10):1398-405)

When the colonic analysis was compared to our previous expression studies in UC there was a 23% homology between the differentially regulated genes in the respective CD and UC analysis compared to controls. (Noble et al. Gut 2008, Oct;57(10):1398-405) The colonic inflammatory expression signature observed in the CD biopsies was also similar to that observed in the UC biopsies and one of the most differentially regulated genes in both of the data sets was serum amyloid A1 (SAA1).

SAA1 is a HLA- associated apolipoprotein acute phase reactant and levels can be elevated in inflammation, trauma and neoplasia. Its transcription is induced by the pro- inflammatory cytokines IL-2, IL-6, TNFα and bacterial LPS, and it is the major factor responsible for the development of secondary AA amyloidosis in chronic immune mediated diseases such as Rheumatoid arthritis or CD. (Gutfeld et al. *J Histochem Cytochem* 2006;**54**(1):63-73) In CD reactive AA amyloidosis is rare and a much more attractive role for SAA1 would be as a marker of disease activity, severity, and potentially because of its induction by TNFα a predictor of response to anti-TNF therapy.

A further interesting change in expression in the colonic CD biopsies reflecting the traditional Th1 and novel Th17 paradigm in CD was the downregulation of thymic stromal lymphopoietin (TSLP) in non-inflamed colonic CD samples compared to non- inflamed controls. TSLP is a cytokine that mediates its effect through dendritic cells to promote the Th2 differentiation of CD4⁺ T cells. (Al Shami et al. *J Exp Med* 2005;**202**(6):829-39) Moreover, mice with an intestinal epithelial cell (IEC) deletion of intrinsic IκB kinase, have reduced TSLP expression and as a consequence have a poor Th2 immune response resulting in a inability to eradicate infection. (Zaph et al. *Nature* 2007;**446**(7135):552-6) These mice also develop severe intestinal inflammation as a result of dendritic cell derived Th1 and Th17 pathway activation and it is intriguing to speculate that in the non- inflamed human CD colon decreased levels of TSLP may perpetuate the subsequent persistent and excessive inflammation.

The identification of IL-23R as a CD susceptibility gene has focused investigation towards the distinct Th17 lineage. (Cho et al. Gastroenterology 2007;133(4):1327-39) We observed that expression of a number of components of this pro-inflammatory pathway- IL-23A, TYK2, STAT3, JAK2, IFNγ and IL-17 were increased in CD compared to controls and that this change was driven by active as opposed to quiescent disease. These convincing genetic and expression data emphasize the importance of this pro-inflammatory pathway in the pathogenesis of CD. Multiple therapeutic targets have been identified in this pathway and clinical trials of a monoclonal antibody against the p40 subunit of IL-23 have produced promising early clinical data. (Sandborn et al. Gastroenterology 2008;135(4):1130-41)

The discovery of ATG16L1 as a CD specific susceptibility gene has strongly implicated the autophagy pathway in the pathogenesis of CD. Autophagy is a highly conserved cellular process where the cell digests part of its own cytoplasm and it functions as a normal physiological response to remove toxic material or intracellular bacteria from the cell. The pathway has also been implicated in the pathogenesis of neurodegenerative diseases such as Alzheimer's and Parkinson's disease. (Lees et al. Inflammatory Bowl Disease Monitor 2009; Vol 9(No 2))

In our data set, 6 of the 20 autophagy genes that we examined were dysregulated emphasizing the importance of this pathway in CD. Recent data have linked the innate immune response and autophagy via Toll-like-receptor (TLR) engagement. (Sanjuan et al. Nature 2007;450(7173):1253-7) TLR induced phagosomes within macrophages triggered ATG5 and ATG7 mediated acidification and enhanced killing of the ingested organisms. These interactions between the innate immune system and the autophagy pathway have provoked investigators to speculate about specific interaction between NOD2/CARD15 and autophagy and this is an area of active investigation. For example, NOD1 and NOD2 have been shown to recruit ATG16LI to the plasma membrane at site of bacterial entry into the cell and that in cells with NOD2 mutations this response is impaired. (Travassos et al., Nat.Immunol. 8-11-2009)

An alternative method for interpreting genome wide expression data is to cluster samples using a subset of genes related to cell lineage. (Abbas et al. Genes Immun 2005;6(4):319-31) We have undertaken this analysis in our samples by separating by genes from key immune cell types and observed clustering of the colonic biopsies. From this we can clearly identify immune cell infiltration in the biopsies and characterize the most differentially expressed genes. These expression signatures can also be used to gain insight into genes of unknown function and provide a resource to investigate immune cell differentiation in health and in different immune mediated diseases.

A final area of interest was in the role of the intestinal epithelial cell (IEC) in the inflammatory process. The fourteen IEC markers we investigated showed good ability to segregate CD patients and controls by clustering analysis with the majority of the chemokines being upregulated in the colonic CD biopsies in an inflammation dependant manner. These results are consistent with previous data from Puleston and colleagues who observed a subset of chemokines-CXCLs 1-3 and CCL20 were upregulated in colonic IBD along with their receptors in a coordinated IEC inflammatory response. (Puleston et al. Aliment Pharmacol Ther 2005;21(2):109-20) The upregulation of these chemokines was significantly more than known leukocyte chemokines emphasizing the central role of the IEC in colonic inflammation.

Further studies carried out in human colonic IBD biopsies, in human colonic cell lines and in human fetal intestinal xenografts have all confirmed the central role of the IEC in mediating, coordinating and perpetuating the pathogenic inflammatory response observed in the colon in both CD and UC. (Dwinell et al. Gastroenterology 2001;120(1):49-59; Banks et al. JPathol 2003;199(1):28-35; Kwon et al. Gut 2002;51(6):818-26)

The strengths of this data are the number of biopsies we analyzed, the lack of pooling of the samples and the rigorous attention to the inflammation status of the biopsies, and their anatomical location. Our data are also consistent with previous expression studies in inflammatory bowel disease and add considerably to the recent genome wide association studies in providing complimentary human colonic and ileal expression data along with detailed analysis of the IL-23 and autophagy pathways.

In conclusion this valuable data set has allowed us to gain novel insight into the pathogenesis of CD at the mucosal level. The data add considerably to the recent genome wide association studies in providing complimentary human colonic and ileal expression data along with detailed analysis of the IL-23 and autophagy pathways. In depth analysis of these exciting new candidate genes along with IEC specific analysis have generated a number of potential therapeutic targets worthy of further investigation.

The following numbered paragraphs (paras.) contain further statements of various aspects of the present invention:-
1. A method of diagnosing the presence of an inflammatory bowel disease (IBD) in a mammalian subject, comprising determining a differential expression level of a nucleic acid encoding a polypeptide shown as any one of SEQ ID NOS: 5, 6, 8, 11, 12, 2, 14, 16, 18, 20, and 22 in a test sample obtained from the subject relative to the expression level of a control, wherein said differential level of expression is indicative of the presence of an IBD in the subject from which the test sample was obtained.
2. The method of para. 1, wherein the differential level of expression is a lower level of expression for a nucleic acid encoding a polypeptide shown as any one of SEQ ID NOS: 5, 6, 8, 11, 12, 2, 14, and 16, wherein the lower level of expression is indicative of the presence of an IBD in the subject from which the test sample was obtained.
3. The method of para. 1, wherein the differential level of expression is a higher level of expression for a nucleic acid encoding a polypeptide shown as any one of SEQ ID NOS: 18, 20, and 22, wherein the higher level of expression is indicative of the presence of an IBD in the subject from which the test sample was obtained.
4. The method of para. 1, 2, or 3 wherein said mammalian subject is a human patient.
5. The method of para. 4 wherein evidence of said expression level is obtained by a method of gene expression profiling.
6. The method of para. 4 wherein said method is a PCR-based method.
7. The method of para. 5 wherein said expression levels are normalized relative to the expression levels of one or more reference genes, or their expression products.
8. The method of para. 1, 2 or 3 comprising determining evidence of the expression levels of at least two of said genes, or their expression products.
9. The method of para. 1, 2 or 3 comprising determining evidence of the expression levels of at least three of said genes, or their expression products.
10. The method of para. 1 or 2 comprising determining evidence of the expression levels of at least four of said genes, or their expression products.
11. The method of para. 1 or 2 comprising determining evidence of the expression levels of at least five of said genes, or their expression products.
12. The method of para. 1, 2, or 3 further comprising the step of creating a report summarizing said IBD detection.
13. The method of para. 1, 2, or 3, wherein said IBD is Crohn's disease.
14. The method of para. 1, 2, or 3, wherein said test sample is from a colonic tissue biopsy.
15. The method of para. 14, wherein said biopsy is from a tissue selected from the group consisting of the terminal ileum, the ascending colon, the descending colon, and the sigmoid colon.
16. The method of para. 14, wherein said biopsy is from an inflamed colonic area.
17. The method of para. 14, wherein said biopsy is from a non-inflamed colonic area.
18. The method of para. 1, 2, or 3, wherein said determining step is indicative of a recurrence of an IBD in said mammalian subject, and wherein said mammalian subject was previously diagnosed with an IBD and treated for said previously diagnosed IBD.
19. The method of para. 18, wherein said treatment comprised surgery.
20. The method of para. 1, 2, or 3, wherein said determining step is indicative of a flare-up of said IBD in said mammalian subject.
21. A method of treating an inflammatory bowel disorder (IBD) in a mammalian subject in need thereof, the method comprising the steps of
   (a) determining a differential expression level of a nucleic acid encoding a polypeptide shown as any one of SEQ ID NOS: 5, 6, 8, 11, 12, 2, 14, 16, and 18, in a test sample obtained from said subject relative to the expression level of a control, wherein said differential level of expression is indicative of the presence of an IBD in the subject from which the test sample was obtained; and
   (b) administering to said subject an effective amount of an IBD therapeutic agent.
22. The method of para. 21, wherein the differential level of expression is a lower level of expression for a nucleic acid encoding a polypeptide shown as any one of SEQ ID NOS: 5, 6, 8, 11, 12, 2, 14, and 16, wherein the lower level of expression is indicative of the presence of an IBD in the subject from which the test sample was obtained.
23. The method of para. 21, wherein the differential level of expression is a higher level of expression for a nucleic acid encoding a polypeptide shown as any one of SEQ ID NOS: 18, 20, and 22, wherein the higher level of expression is indicative of the presence of an IBD in the subject from which the test sample was obtained.
24. The method of para. 21, 22, or 23 wherein said mammalian subject is a human patient.
25. The method of para. 24 wherein evidence of said expression level is obtained by a method of gene expression profiling.
26. The method of para. 24 wherein said method is a PCR-based method.
27. The method of para. 26 wherein said expression levels are normalized relative to the expression levels of one or more reference genes, or their expression products.
28. The method of para. 21, 22, or 23 comprising determining evidence of the expression levels of at least two of said genes, or their expression products.
29. The method of para. 21, 22, or 23 comprising determining evidence of the expression levels of at least three of said genes, or their expression products.
30. The method of para. 21 or 22 comprising determining evidence of the expression levels of at least four of said genes, or their expression products.
31. The method of para. 21 or 22 comprising determining evidence of the expression levels of at least five of said genes, or their expression products.
32. The method of para. 21, 22, or 23 further comprising the step of creating a report summarizing said IBD detection.
33. The method of para. 21, 22, or 23, wherein said IBD is Crohn's disease.
34. The method of para. 21, 22, or 23, wherein said test sample is from a colonic tissue biopsy.
35. The method of para. 34, wherein said biopsy is from a tissue selected from the group consisting of the terminal ileum, the ascending colon, the descending colon, and the sigmoid colon.
36. The method of para. 34, wherein said biopsy is from an inflamed colonic area.
37. The method of para. 34, wherein said biopsy is from a non-inflamed colonic area.
38. The method of para. 21, 22, or 23, wherein said determining step is indicative of a recurrence of an IBD in said mammalian subject, and wherein said mammalian subject was previously diagnosed with an IBD and treated for said previously diagnosed IBD.
39. The method of para. 38, wherein said treatment comprised surgery.
40. The method of para. 21, 22, or 23, wherein said determining step is indicative of a flare-up of said IBD in said mammalian subject.
41. The method of para. 21, 22, or 23, wherein said IBD therapeutic agent is an aminosalicylate.
42. The method of para. 21, 22, or 23, wherein said IBD therapeutic agent is a corticosteroid.
43. The method of para. 21, 22, or 23, wherein said IBD therapeutic agent is an immunosuppressive agent.

## Claims

1. A method of diagnosing the presence of an inflammatory bowel disease (IBD) in a mammalian subject, comprising determining a differential expression level of a nucleic acid encoding a polypeptide that is a regulator of the autophagy pathway in a test sample obtained from the subject relative to the expression level of a control, wherein said differential level of expression is indicative of the presence of an IBD in the subject from which the test sample was obtained.

2. A method of treating an inflammatory bowel disorder (IBD) in a mammalian subject in need thereof, the method comprising the steps of
(a) determining a differential expression level of a nucleic acid encoding a polypeptide that is a regulator of the autophagy pathway in a test sample obtained from said subject relative to the expression level of a control, wherein said differential level of expression is indicative of the presence of an IBD in the subject from which the test sample was obtained; and
(b) administering to said subject an effective amount of an IBD therapeutic agent.

3. The method of claim 1 or 2, wherein the differential level of expression is of a nucleic acid encoding a polypeptide shown as any one of SEQ ID NOS: 16, 18, 20, 22 and 14.

4. The method of claim 1 or 2, wherein the differential level of expression is
(i) a higher level of expression for a nucleic acid encoding a polypeptide shown as any one of SEQ ID NOS: 18, 20, and 22, wherein the higher level of expression is indicative of the presence of an IBD in the subject from which the test sample was obtained; or
(ii) a lower level of expression for a nucleic acid encoding a polypeptide shown as any one of SEQ ID NOS: 16 and 14, wherein the lower level of expression is indicative of the presence of an IBD in the subject from which the test sample was obtained.

5. The method of any one of claims 1 to 4, wherein said mammalian subject is a human patient.

6. The method of claim 5, wherein evidence of said expression level is obtained by a method of gene expression profiling.

7. The method of claim 5, wherein said method is a PCR-based method.

8. The method of claim 6, wherein said expression levels are normalized relative to the expression levels of one or more reference genes, or their expression products.

9. The method of any one of claims 1 to 4, comprising determining evidence of the expression levels of at least two, at least three, at least four, or at least five, of said genes, or their expression products.

10. The method of any one of claims 1 to 4, wherein said IBD is Crohn's disease.

11. The method of any one of claims 1 to 4, wherein said test sample is from a colonic tissue biopsy.

12. The method of claim 11, wherein said biopsy is from (i) a tissue selected from the group consisting of the terminal ileum, the ascending colon, the descending colon, and the sigmoid colon; (ii) an inflamed colonic area; or (iii) a non-inflamed colonic area.

13. The method of any one of claims 1 to 4, wherein said determining step is (i) indicative of a recurrence of an IBD in said mammalian subject, and wherein said mammalian subject was previously diagnosed with an IBD and treated for said previously diagnosed IBD, or (ii) indicative of a flare-up of said IBD in said mammalian subject.

14. The method of claim 13(i), wherein said treatment comprised surgery.

15. The method of claim 2, 3 or 4, wherein said IBD therapeutic agent is (i) an aminosalicylate; (ii) a corticosteroid; or (iii) an immunosuppressive agent.


SEQUENCE LISTING

<110>  Genentech, Inc.

<120>  GENE EXPRESSION MARKERS FOR CROHN'S DISEASE

<130>  LCW/FP6913594

<140>  Unknown, filed herewith.
<141>  2010-07-19

<150>  12182666.3
<150>  2010-07-19

<150>  10737186.6
<151>  2010-07-19

<150>  PCT/US2010/042487
<151>  2010-07-19

<150>  61/302,084
<151>  2010-02-05

<150>  61/227,049
<151>  2009-07-20

<160>  22

<170>  PatentIn version 3.5

<210>  1
<211>  3459
<212>  DNA
<213>  Homo sapiens

<400>  1
ctcaatcagc tttatgcaga gaagaagctt actgagctca ctgctggtgc tggtgtaggc      60

aagtgctgct ttggcaatct gggctgacct ggcttgtctc ctcagaactc cttctccaac     120

cctggagcag gcttccatgc tgctgtgggc gtccttgctg gcctttgctc cagtctgtgg     180

acaatctgca gctgcacaca aacctgtgat ttccgtccat cctccatgga ccacattctt     240

caaaggagag agagtgactc tgacttgcaa tggatttcag ttctatgcaa cagagaaaac     300

aacatggtat catcggcact actggggaga aaagttgacc ctgaccccag gaaacaccct     360

cgaggttcgg gaatctggac tgtacagatg ccaggcccgg ggctccccac gaagtaaccc     420

tgtgcgcttg ctcttttctt cagactcctt aatcctgcag gcaccatatt ctgtgtttga     480

aggtgacaca ttggttctga gatgccacag aagaaggaaa gagaaattga ctgctgtgaa     540

atatacttgg aatggaaaca ttctttccat ttctaataaa agctgggatc ttcttatccc     600

acaagcaagt tcaaataaca atggcaatta tcgatgcatt ggatatggag atgagaatga     660

tgtatttaga tcaaatttca aaataattaa aattcaagaa ctatttccac atccagagct     720

gaaagctaca gactctcagc ctacagaggg gaattctgta aacctgagct gtgaaacaca     780

gcttcctcca gagcggtcag acaccccact tcacttcaac ttcttcagag atggcgaggt     840

catcctgtca gactggagca cgtacccgga actccagctc ccaaccgtct ggagagaaaa 900

ctcaggatcc tattggtgtg gtgctgaaac agtgaggggt aacatccaca agcacagtcc 960

ctcgctacag atccatgtgc agcggatccc tgtgtctggg gtgctcctgg agacccagcc 1020

ctcagggggc caggctgttg aaggggagat gctggtcctt gtctgctccg tggctgaagg 1080

cacaggggat accacattct cctggcaccg agaggacatg caggagagtc tggggaggaa 1140

aactcagcgt tccctgagag cagagctgga gctccctgcc atcagacaga gccatgcagg 1200

gggatactac tgtacagcag acaacagcta cggccctgtc cagagcatgg tgctgaatgt 1260

cactgtgaga gagaccccag gcaacagaga tggccttgtc gccgcgggag ccactggagg 1320

gctgctcagt gctcttctcc tggctgtggc cctgctgttt cactgctggc gtcggaggaa 1380

gtcaggagtt ggtttcttgg gagacgaaac caggctccct cccgctccag gcccaggaga 1440

gtcctcccat tccatctgcc ctgcccaggt ggagcttcag tcgttgtatg ttgatgtaca 1500

ccccaaaaag ggagatttgg tatactctga gatccagact actcagctgg gagaagaaga 1560

ggaagctaat acctccagga cacttctaga ggataaggat gtctcagttg tctactctga 1620

ggtaaagaca caacacccag ataactcagc tggaaagatc agctctaagg atgaagaaag 1680

ttaagagaat gaaaagttac gggaacgtcc tactcatgtg atttctccct tgtccaaagt 1740

cccaggccca gtgcagtcct tgcggcacct ggaatgatca actcattcca gctttctaat 1800

tcttctcatg catatgcatt cactcccagg aatactcatt cgtctactct gatgttggga 1860

tggaatggcc tctgaaagac ttcactaaaa tgaccaggat ccacagttaa gagaagaccc 1920

tgtagtattt gctgtgggcc tgacctaatg cattccctag ggtctgcttt agagaagggg 1980

gataaagaga gagaaggact gttatgaaaa acagaagcac aaattttggt gaattgggat 2040

ttgcagagat gaaaaagact gggtgacctg gatctctgct taatacatct acaaccattg 2100

tctcactgga gactcacttg catcagtttg tttaactgtg agtggctgca caggcactgt 2160

gcaaacaatg aaaagcccct tcacttctgc ctgcacagct tacactgtca ggattcagtt 2220

gcagattaaa gaacccatct ggaatggttt acagagagag gaatttaaaa gaggacatca 2280

gaagagctgg agatgcaagc tctaggctgc gcttccaaaa gcaaatgata attatgttaa 2340

tgtcattagt gacaaagatt tgcaacatta gagaaaagag acacaaatat aaaattaaaa 2400

acttaagtac caactctcca aaactaaatt tgaacttaaa atattagtat aaactcataa 2460

taaactctgc ctttaaaaaa agataaatat ttcctacgtc tgttcactga aataattacc 2520

aaccccttag caataagcac tccttgcaga gaggttttat tctctaaata ccattccctt 2580

ctcaaaggaa ataaggttgc ttttcttgta ggaactgtgt ctttgagtta ctaattagtt 2640

tatatgagaa taattcttgc aataaatgaa gaaggaataa aagaaatagg aagccacaaa 2700

tttgtatgga tatttcatga tacacctact ggttaaataa ttgacaaaaa ccagcagcca 2760

aatattagag gtctcctgat ggaagtgtac aataccacct acaaattatc catgccccaa 2820

gtgttaaaac tgaatccatt caagtctttc taactgaata cttgttttat agaaaatgca 2880

tggagaaaag gaatttgttt aaataacatt atgggattgc aaccagcaaa acataaactg 2940

agaaaaagtt ctatagggca aatcacctgg cttctataac aaataaatgg gaaaaaaatg 3000

aaataaaaag aagagaggga ggaagaaagg gagagagaag aaaagaaaaa tgaagaaaag 3060

taattagaat attttcaaca taaagaaaag acgaatattt aaggtgacag atatcccaac 3120

tacgctgatt tgatctttac aaattatatg agtgtatgaa tttgtcacat gtatcacccc 3180

caaaaaaaga gaaaaagaaa aatagaagac atataaatta aatgagacga gacatgtcga 3240

ccaaaaggaa tgtgtgggtc ttgtttggat cctgactcaa attaagaaaa aataaaacta 3300

cctacgaaat actaagaaaa atttgtatac taatattaag aaattgttgt gtgttttgga 3360

tataagtgat agtttattgt agtgatgttt ttataaaagc aaaaggatat tcactttcag 3420

cgcttatact gaagtattag attaaagctt attaacgta 3459

<210> 2
<211> 515
<212> PRT
<213> Homo sapiens

<400> 2

Met Leu Leu Trp Ala Ser Leu Leu Ala Phe Ala Pro Val Cys Gly Gln
1               5                   10                  15

Ser Ala Ala Ala His Lys Pro Val Ile Ser Val His Pro Pro Trp Thr
            20                  25                  30

Thr Phe Phe Lys Gly Glu Arg Val Thr Leu Thr Cys Asn Gly Phe Gln
        35                  40                  45

Phe Tyr Ala Thr Glu Lys Thr Thr Trp Tyr His Arg His Tyr Trp Gly
    50                  55                  60

Glu Lys Leu Thr Leu Thr Pro Gly Asn Thr Leu Glu Val Arg Glu Ser
65                  70                  75                  80

Gly Leu Tyr Arg Cys Gln Ala Arg Gly Ser Pro Arg Ser Asn Pro Val
                85                  90                  95

Arg Leu Leu Phe Ser Ser Asp Ser Leu Ile Leu Gln Ala Pro Tyr Ser
            100                 105                 110

Val Phe Glu Gly Asp Thr Leu Val Leu Arg Cys His Arg Arg Arg Lys

        115                 120                 125

Glu Lys Leu Thr Ala Val Lys Tyr Thr Trp Asn Gly Asn Ile Leu Ser
    130                 135                 140

Ile Ser Asn Lys Ser Trp Asp Leu Leu Ile Pro Gln Ala Ser Ser Asn
145                 150                 155                 160

Asn Asn Gly Asn Tyr Arg Cys Ile Gly Tyr Gly Asp Glu Asn Asp Val
                165                 170                 175

Phe Arg Ser Asn Phe Lys Ile Ile Lys Ile Gln Glu Leu Phe Pro His
            180                 185                 190

Pro Glu Leu Lys Ala Thr Asp Ser Gln Pro Thr Glu Gly Asn Ser Val
        195                 200                 205

Asn Leu Ser Cys Glu Thr Gln Leu Pro Pro Glu Arg Ser Asp Thr Pro
    210                 215                 220

Leu His Phe Asn Phe Phe Arg Asp Gly Glu Val Ile Leu Ser Asp Trp
225                 230                 235                 240

Ser Thr Tyr Pro Glu Leu Gln Leu Pro Thr Val Trp Arg Glu Asn Ser
                245                 250                 255

Gly Ser Tyr Trp Cys Gly Ala Glu Thr Val Arg Gly Asn Ile His Lys
            260                 265                 270

His Ser Pro Ser Leu Gln Ile His Val Gln Arg Ile Pro Val Ser Gly
        275                 280                 285

Val Leu Leu Glu Thr Gln Pro Ser Gly Gly Gln Ala Val Glu Gly Glu
    290                 295                 300

Met Leu Val Leu Val Cys Ser Val Ala Glu Gly Thr Gly Asp Thr Thr
305                 310                 315                 320

Phe Ser Trp His Arg Glu Asp Met Gln Glu Ser Leu Gly Arg Lys Thr
                325                 330                 335

Gln Arg Ser Leu Arg Ala Glu Leu Glu Leu Pro Ala Ile Arg Gln Ser
            340                 345                 350

His Ala Gly Gly Tyr Tyr Cys Thr Ala Asp Asn Ser Tyr Gly Pro Val
        355                 360                 365

Gln Ser Met Val Leu Asn Val Thr Val Arg Glu Thr Pro Gly Asn Arg
    370                 375                 380

Asp Gly Leu Val Ala Ala Gly Ala Thr Gly Gly Leu Leu Ser Ala Leu
385                 390                 395                 400

Leu Leu Ala Val Ala Leu Leu Phe His Cys Trp Arg Arg Arg Lys Ser
                405                 410                 415

Gly Val Gly Phe Leu Gly Asp Glu Thr Arg Leu Pro Pro Ala Pro Gly
            420                 425                 430

Pro Gly Glu Ser Ser His Ser Ile Cys Pro Ala Gln Val Glu Leu Gln
        435                 440                 445

Ser Leu Tyr Val Asp Val His Pro Lys Lys Gly Asp Leu Val Tyr Ser
    450                 455                 460

Glu Ile Gln Thr Thr Gln Leu Gly Glu Glu Glu Glu Ala Asn Thr Ser
465                 470                 475                 480

Arg Thr Leu Leu Glu Asp Lys Asp Val Ser Val Val Tyr Ser Glu Val
                485                 490                 495

Lys Thr Gln His Pro Asp Asn Ser Ala Gly Lys Ile Ser Ser Lys Asp
            500                 505                 510

Glu Glu Ser
        515

<210>  3
<211>  641
<212>  DNA
<213>  Homo sapiens

<400>  3
ctggcatccc gagaagccag gaagcagtga gcccaggagt cctcggccag ccctgcctgc      60

ccaccaggag gatgaaggtc tccgtggctg ccctctcctg cctcatgctt gttactgccc     120

ttggatccca ggcccgggtc acaaaagatg cagagacaga gttcatgatg tcaaagcttc     180

cattggaaaa tccagtactt ctggacatgc tctggaggag aaagattggt cctcagatga     240

ccctttctca tgctgcagga ttccatgcta ctagtgctga ctgctgcatc tcctacaccc     300

cacgaagcat cccgtgttca ctcctggaga gttactttga aacgaacagc gagtgctcca     360

agccgggtgt catcttcctc accaagaagg ggcgacgttt ctgtgccaac cccagtgata     420

agcaagttca ggtttgcgtg agaatgctga agctggacac acggatcaag accaggaaga     480

attgaacttg tcaaggtgaa gggacacaag ttgccagcca ccaactttct tgcctcaact     540

accttcctga attatttttt aaagaagcat ttattcttgt gttctggatt tagagcaatt 600

catctaataa acagtttctc actttaaaaa aaaaaaaaaa a 641

<210> 4
<211> 603
<212> DNA
<213> Homo sapiens

<400> 4
ctggcatccc gagaagccag gaagcagtga gcccaggagt cctcggccag ccctgcctgc 60

ccaccaggag gatgaaggtc tccgtggctg ccctctcctg cctcatgctt gttactgccc 120

ttggatccca ggcccgggtc acaaaagatg cagagacaga gttcatgatg tcaaagcttc 180

cattggaaaa tccagtactt ctggacagat tccatgctac tagtgctgac tgctgcatct 240

cctacacccc acgaagcatc ccgtgttcac tcctggagag ttactttgaa acgaacagcg 300

agtgctccaa gccgggtgtc atcttcctca ccaagaaggg gcgacgtttc tgtgccaacc 360

ccagtgataa gcaagttcag gtttgcgtga gaatgctgaa gctggacaca cggatcaaga 420

ccaggaagaa ttgaacttgt caaggtgaag ggacacaagt tgccagccac caactttctt 480

gcctcaacta ccttcctgaa ttatttttta aagaagcatt tattcttgtg ttctggattt 540

agagcaattc atctaataaa cagtttctca ctttaaaaaa aaaaaaaaaa aaaaaaaaaa 600

aaa 603

<210> 5
<211> 137
<212> PRT
<213> Homo sapiens

<400> 5

Met Lys Val Ser Val Ala Ala Leu Ser Cys Leu Met Leu Val Thr Ala
1               5                   10                  15

Leu Gly Ser Gln Ala Arg Val Thr Lys Asp Ala Glu Thr Glu Phe Met
            20                  25                  30

Met Ser Lys Leu Pro Leu Glu Asn Pro Val Leu Leu Asp Met Leu Trp
        35                  40                  45

Arg Arg Lys Ile Gly Pro Gln Met Thr Leu Ser His Ala Ala Gly Phe
    50                  55                  60

His Ala Thr Ser Ala Asp Cys Cys Ile Ser Tyr Thr Pro Arg Ser Ile
65                  70                  75                  80

Pro Cys Ser Leu Leu Glu Ser Tyr Phe Glu Thr Asn Ser Glu Cys Ser

85                  90                  95

Lys Pro Gly Val Ile Phe Leu Thr Lys Lys Gly Arg Arg Phe Cys Ala
            100                 105                 110

Asn Pro Ser Asp Lys Gln Val Gln Val Cys Val Arg Met Leu Lys Leu
        115                 120                 125

Asp Thr Arg Ile Lys Thr Arg Lys Asn
    130                 135

<210>  6
<211>  120
<212>  PRT
<213>  Homo sapiens

<400>  6

Met Lys Val Ser Val Ala Ala Leu Ser Cys Leu Met Leu Val Thr Ala
1               5                   10                  15

Leu Gly Ser Gln Ala Arg Val Thr Lys Asp Ala Glu Thr Glu Phe Met
            20                  25                  30

Met Ser Lys Leu Pro Leu Glu Asn Pro Val Leu Leu Asp Arg Phe His
        35                  40                  45

Ala Thr Ser Ala Asp Cys Cys Ile Ser Tyr Thr Pro Arg Ser Ile Pro
    50                  55                  60

Cys Ser Leu Leu Glu Ser Tyr Phe Glu Thr Asn Ser Glu Cys Ser Lys
65                  70                  75                  80

Pro Gly Val Ile Phe Leu Thr Lys Lys Gly Arg Arg Phe Cys Ala Asn
                85                  90                  95

Pro Ser Asp Lys Gln Val Gln Val Cys Val Arg Met Leu Lys Leu Asp
            100                 105                 110

Thr Arg Ile Lys Thr Arg Lys Asn
        115                 120

<210>  7
<211>  1219
<212>  DNA
<213>  Homo sapiens

<400>  7
gagaagatgt ttgaaaaaac tgactctgct aatgagcctg gactcagagc tcaagtctga      60

actctacctc cagacagaat gaagttcatc tcgacatctc tgcttctcat gctgctggtc     120

agcagcctct ctccagtcca aggtgttctg gaggtctatt acacaagctt gaggtgtaga 180

tgtgtccaag agagctcagt ctttatccct agacgcttca ttgatcgaat tcaaatcttg 240

ccccgtggga atggttgtcc aagaaaagaa atcatagtct ggaagaagaa caagtcaatt 300

gtgtgtgtgg accctcaagc tgaatggata caaagaatga tggaagtatt gagaaaaaga 360

agttcttcaa ctctaccagt tccagtgttt aagagaaaga ttccctgatg ctgatatttc 420

cactaagaac acctgcattc ttcccttatc cctgctctgg attttagttt tgtgcttagt 480

taaatctttt ccaggaaaaa gaacttcccc atacaaataa gcatgagact atgtaaaaat 540

aaccttgcag aagctgatgg ggcaaactca agcttcttca ctcacagcac cctatataca 600

cttggagttt gcattcttat tcatcaggga ggaaagtttc tttgaaaata gttattcagt 660

tataagtaat acaggattat tttgattata tacttgttgt ttaatgttta aaatttctta 720

gaaaacaatg gaatgagaat ttaagcctca aatttgaaca tgtggcttga attaagaaga 780

aaattatggc atatattaaa agcaggcttc tatgaaagac tcaaaaagct gcctgggagg 840

cagatggaac ttgagcctgt caagaggcaa aggaatccat gtagtagata tcctctgctt 900

aaaaactcac tacggaggag aattaagtcc tacttttaaa gaatttcttt ataaaattta 960

ctgtctaaga ttaatagcat tcgaagatcc ccagacttca tagaatactc agggaaagca 1020

tttaaagggt gatgtacaca tgtatccttt cacacatttg ccttgacaaa cttctttcac 1080

tcacatcttt ttcactgact ttttttgtgg ggggcggggc cggggggact ctggtatcta 1140

attctttaat gattcctata aatctaatga cattcaataa agttgagcaa acattttact 1200

taaaaaaaaa aaaaaaaaa 1219

<210> 8
<211> 109
<212> PRT
<213> Homo sapiens

<400> 8

Met Lys Phe Ile Ser Thr Ser Leu Leu Leu Met Leu Leu Val Ser Ser
1               5                   10                  15

Leu Ser Pro Val Gln Gly Val Leu Glu Val Tyr Tyr Thr Ser Leu Arg
            20                  25                  30

Cys Arg Cys Val Gln Glu Ser Ser Val Phe Ile Pro Arg Arg Phe Ile
        35                  40                  45

Asp Arg Ile Gln Ile Leu Pro Arg Gly Asn Gly Cys Pro Arg Lys Glu
    50                  55                  60

Ile Ile Val Trp Lys Lys Asn Lys Ser Ile Val Cys Val Asp Pro Gln
65                  70                  75                  80

Ala Glu Trp Ile Gln Arg Met Met Glu Val Leu Arg Lys Arg Ser Ser
                85                  90                  95

Ser Thr Leu Pro Val Pro Val Phe Lys Arg Lys Ile Pro
            100                 105

<210>  9
<211>  3354
<212>  DNA
<213>  Homo sapiens

<400>  9
actagcgagc gccctgcgta ggcaccggct cctgagcccg tgcttcgggt gagggggcgg      60

gtcttccggc cctctcgaaa atcatttccg gcatgagccg gaagaccgtc ccggatggcc     120

tcggggactg ccagtgtgtg gaggtgagct ccgggattgc cggcattccc gcttctgctg     180

gttgcttcat gctgcaggct gcggccgtca gccctcgctc gcattggtgg cgctgaggtg     240

ccggggcagc aagtgacatg tcgtcgggcc tccgcgccgc tgacttcccc cgctggaagc     300

gccacatctc ggagcaactg aggcgccggg accggctgca gagacaggcg ttcgaggaga     360

tcatcctgca gtataacaaa ttgctggaaa agtcagatct tcattcagtg ttggcccaga     420

aactacaggc tgaaaagcat gacgtaccaa acaggcacga gataagtccc ggacatgatg     480

gcacatggaa tgacaatcag ctacaagaaa tggcccaact gaggattaag caccaagagg     540

aactgactga attacacaag aaacgtgggg agttagctca actggtgatt gacctgaata     600

accaaatgca gcggaaggac agggagatgc agatgaatga agcaaaaatt gcagaatgtt     660

tgcagactat ctctgacctg gagacggagt gcctagacct gcgcactaag ctttgtgacc     720

ttgaaagagc caaccagacc ctgaaggatg aatatgatgc cctgcagatc acttttactg     780

ccttggaggg aaaactgagg aaaactacgg aagagaacca ggagctggtc accagatgga     840

tggctgagaa agcccaggaa gccaatcggc ttaatgcaga gaatgaaaaa gactccagga     900

ggcggcaagc ccggctgcag aaagagcttg cagaagcagc aaaggaacct ctaccagtcg     960

aacaggatga tgacattgag gtcattgtgg atgaaacttc tgatcacaca gaagagacct    1020

ctcctgtgcg agccatcagc agagcagcca cgagacgctc tgtctcttcc ttcccagtcc    1080

cccaggacaa tgtggatact catcctggtt ctggtaaaga agtgagggta ccagctactg    1140

ccttgtgtgt cttcgatgca catgatgggg aagtcaacgc tgtgcagttc agtccaggtt    1200

cccggttact ggccactgga ggcatggacc gcagggttaa gctttgggaa gtatttggag    1260

aaaaatgtga gttcaagggt tccctatctg gcagtaatgc aggaattaca agcattgaat    1320

ttgatagtgc tggatcttac ctcttagcag cttcaaatga ttttgcaagc cgaatctgga    1380

ctgtggatga ttatcgatta cggcacacac tcacgggaca cagtgggaaa gtgctgtctg 1440

ctaagttcct gctggacaat gcgcggattg tctcaggaag tcacgaccgg actctcaaac 1500

tctgggatct acgcagcaaa gtctgcataa agacagtgtt tgcaggatcc agttgcaatg 1560

atattgtctg cacagagcaa tgtgtaatga gtggacattt tgacaagaaa attcgtttct 1620

gggacattcg atcagagagc atagttcgag agatggagct gttgggaaag attactgccc 1680

tggacttaaa cccagaaagg actgagctcc tgagctgctc ccgtgatgac ttgctaaaag 1740

ttattgatct ccgaacaaat gctatcaagc agacattcag tgcacctggg ttcaagtgcg 1800

gctctgactg gaccagagtt gtcttcagcc ctgatggcag ttacgtggcg gcaggctctg 1860

ctgagggctc tctgtatatc tggagtgtgc tcacagggaa agtggaaaag gttctttcaa 1920

agcagcacag ctcatccatc aatgcggtgg cgtggtcgcc ctctggctcg cacgttgtca 1980

gtgtggacaa aggatgcaaa gctgtgctgt gggcacagta ctgacggggc tctcagggct 2040

gggaggaccc cagtgccctc ctcagaagaa gcacatgggc tcctgcagcc ctgtcctggc 2100

aggtgatgtg ctgggtatag catggacctc ccagagaagc tcaagctatg tggcactgta 2160

gctttgccgt gaatgggatt tctgaagatt tgactgaggt ctctcttggc ctggaagaat 2220

aacactgaaa aaacctgacg ctgcggtcac ttagcagagg ctcaggttct tgccttggga 2280

aacactacta gctctgacct tccatacctc acttggggga gcacagggcc ccgctgggcc 2340

tcctcaccaa cggcagtgcc aaaatcagcc cccacatcaa ggtggtgttc tctgtgcttt 2400

ctctcgtcct tccaaagtcg gttctggcct aacgcatgtc ccaacacctt gggttcattt 2460

gcccggtgaa ctcactttaa gcattggatt aacggaaact cccgaactac agacccctcc 2520

ctggtgggtt gcatgaatgt gtctcattac tgctgaaatg tcctcacatc tctttcactg 2580

ttcttcagag ctttctggct ctctttcccc cacaaaattc gacatattta aaaatctccg 2640

tgtggcttta aaaaatggtt ttttgttttt ttgttttttt gaggtgggag aggatgtgtg 2700

aaaatctttt ccagggaaat gggttcgctg cagaggtaag gatgtgttcc tgtatcgatc 2760

tgcagacacc cagaaggtgg gtgcacactg catgcttggg ggtgccaagg gattcgagac 2820

ctccaacata cttgtctgaa ggtggtgatt ctggccatgg cccctctgcc aagcctgtgt 2880

gcgatgccct tggtgcttta gtgcaagaag cctaggctca gaagcacagc agcgccatct 2940

ttccgtttca ggggttgtga tgaaggccaa ggaaaaacat ttatctttac tattttacct 3000

acgtataaag ttttagttca ttgggtgtgc gaaacaccct ttttatcact tttaaatttg 3060

cactttattt tttttcttcc atgcttgttc tctggacatt tggggatgtg agtgttagag 3120

ctggtgagag aggagtcagg tggccttccc accgatggtc ctggcctcca cctgccctct 3180

cttccctgcc tgatcaccgc tttccaattt gcccttcaga gaacttaagt caaggagagt 3240

tgaaattcac aggccagggc acatctttta tttatttcat tatgttggcc aacagaactt 3300

gattgtaaat aataataaag aaatctgtta tatacttttc aaactccaaa aaaa 3354

<210> 10
<211> 3411
<212> DNA
<213> Homo sapiens

<400> 10
actagcgagc gccctgcgta ggcaccggct cctgagcccg tgcttcgggt gagggggcgg 60

gtcttccggc cctctcgaaa atcatttccg gcatgagccg gaagaccgtc ccggatggcc 120

tcggggactg ccagtgtgtg gaggtgagct ccgggattgc cggcattccc gcttctgctg 180

gttgcttcat gctgcaggct gcggccgtca gccctcgctc gcattggtgg cgctgaggtg 240

ccggggcagc aagtgacatg tcgtcgggcc tccgcgccgc tgacttcccc cgctggaagc 300

gccacatctc ggagcaactg aggcgccggg accggctgca gagacaggcg ttcgaggaga 360

tcatcctgca gtataacaaa ttgctggaaa agtcagatct tcattcagtg ttggcccaga 420

aactacaggc tgaaaagcat gacgtaccaa acaggcacga gataagtccc ggacatgatg 480

gcacatggaa tgacaatcag ctacaagaaa tggcccaact gaggattaag caccaagagg 540

aactgactga attacacaag aaacgtgggg agttagctca actggtgatt gacctgaata 600

accaaatgca gcggaaggac agggagatgc agatgaatga agcaaaaatt gcagaatgtt 660

tgcagactat ctctgacctg gagacggagt gcctagacct gcgcactaag ctttgtgacc 720

ttgaaagagc caaccagacc ctgaaggatg aatatgatgc cctgcagatc acttttactg 780

ccttggaggg aaaactgagg aaaactacgg aagagaacca ggagctggtc accagatgga 840

tggctgagaa agcccaggaa gccaatcggc ttaatgcaga gaatgaaaaa gactccagga 900

ggcggcaagc ccggctgcag aaagagcttg cagaagcagc aaaggaacct ctaccagtcg 960

aacaggatga tgacattgag gtcattgtgg atgaaacttc tgatcacaca gaagagacct 1020

ctcctgtgcg agccatcagc agagcagcca ctaagcgact ctcgcagcct gctggaggcc 1080

ttctggattc tatcactaat atctttggga gacgctctgt ctcttccttc ccagtccccc 1140

aggacaatgt ggatactcat cctggttctg gtaaagaagt gagggtacca gctactgcct 1200

tgtgtgtctt cgatgcacat gatggggaag tcaacgctgt gcagttcagt ccaggttccc 1260

ggttactggc cactggaggc atggaccgca gggttaagct ttgggaagta tttggagaaa 1320

aatgtgagtt caagggttcc ctatctggca gtaatgcagg aattacaagc attgaatttg 1380

atagtgctgg atcttacctc ttagcagctt caaatgattt tgcaagccga atctggactg 1440

tggatgatta tcgattacgg cacacactca cgggacacag tgggaaagtg ctgtctgcta 1500

agttcctgct ggacaatgcg cggattgtct caggaagtca cgaccggact ctcaaactct 1560

gggatctacg cagcaaagtc tgcataaaga cagtgtttgc aggatccagt tgcaatgata 1620

ttgtctgcac agagcaatgt gtaatgagtg gacattttga caagaaaatt cgtttctggg 1680

acattcgatc agagagcata gttcgagaga tggagctgtt gggaaagatt actgccctgg 1740

acttaaaccc agaaaggact gagctcctga gctgctcccg tgatgacttg ctaaaagtta 1800

ttgatctccg aacaaatgct atcaagcaga cattcagtgc acctgggttc aagtgcggct 1860

ctgactggac cagagttgtc ttcagccctg atggcagtta cgtggcggca ggctctgctg 1920

agggctctct gtatatctgg agtgtgctca cagggaaagt ggaaaaggtt ctttcaaagc 1980

agcacagctc atccatcaat gcggtggcgt ggtcgccctc tggctcgcac gttgtcagtg 2040

tggacaaagg atgcaaagct gtgctgtggg cacagtactg acggggctct cagggctggg 2100

aggaccccag tgccctcctc agaagaagca catgggctcc tgcagccctg tcctggcagg 2160

tgatgtgctg ggtatagcat ggacctccca gagaagctca agctatgtgg cactgtagct 2220

ttgccgtgaa tgggatttct gaagatttga ctgaggtctc tcttggcctg gaagaataac 2280

actgaaaaaa cctgacgctg cggtcactta gcagaggctc aggttcttgc cttgggaaac 2340

actactagct ctgaccttcc atacctcact tgggggagca cagggccccg ctgggcctcc 2400

tcaccaacgg cagtgccaaa atcagccccc acatcaaggt ggtgttctct gtgctttctc 2460

tcgtccttcc aaagtcggtt ctggcctaac gcatgtccca acaccttggg ttcatttgcc 2520

cggtgaactc actttaagca ttggattaac ggaaactccc gaactacaga cccctccctg 2580

gtgggttgca tgaatgtgtc tcattactgc tgaaatgtcc tcacatctct ttcactgttc 2640

ttcagagctt tctggctctc tttcccccac aaaattcgac atatttaaaa atctccgtgt 2700

ggctttaaaa aatggttttt tgtttttttg tttttttgag gtgggagagg atgtgtgaaa 2760

atcttttcca gggaaatggg ttcgctgcag aggtaaggat gtgttcctgt atcgatctgc 2820

agacacccag aaggtgggtg cacactgcat gcttgggggt gccaagggat tcgagacctc 2880

caacatactt gtctgaaggt ggtgattctg gccatggccc ctctgccaag cctgtgtgcg 2940

atgcccttgg tgctttagtg caagaagcct aggctcagaa gcacagcagc gccatctttc 3000

cgtttcaggg gttgtgatga aggccaagga aaaacattta tctttactat tttacctacg 3060

tataaagttt tagttcattg ggtgtgcgaa acaccctttt tatcactttt aaatttgcac 3120

tttatttttt ttcttccatg cttgttctct ggacatttgg ggatgtgagt gttagagctg 3180

gtgagagagg agtcaggtgg ccttcccacc gatggtcctg gcctccacct gccctctctt 3240

ccctgcctga tcaccgcttt ccaatttgcc cttcagagaa cttaagtcaa ggagagttga 3300

aattcacagg ccagggcaca tcttttattt atttcattat gttggccaac agaacttgat 3360

tgtaaataat aataaagaaa tctgttatat acttttcaaa ctccaaaaaa a 3411

<210>  11
<211>  588
<212>  PRT
<213>  Homo sapiens

<400>  11

Met Ser Ser Gly Leu Arg Ala Ala Asp Phe Pro Arg Trp Lys Arg His
1               5                   10                  15

Ile Ser Glu Gln Leu Arg Arg Arg Asp Arg Leu Gln Arg Gln Ala Phe
            20                  25                  30

Glu Glu Ile Ile Leu Gln Tyr Asn Lys Leu Leu Glu Lys Ser Asp Leu
        35                  40                  45

His Ser Val Leu Ala Gln Lys Leu Gln Ala Glu Lys His Asp Val Pro
    50                  55                  60

Asn Arg His Glu Ile Ser Pro Gly His Asp Gly Thr Trp Asn Asp Asn
65                  70                  75                  80

Gln Leu Gln Glu Met Ala Gln Leu Arg Ile Lys His Gln Glu Glu Leu
                85                  90                  95

Thr Glu Leu His Lys Lys Arg Gly Glu Leu Ala Gln Leu Val Ile Asp
            100                 105                 110

Leu Asn Asn Gln Met Gln Arg Lys Asp Arg Glu Met Gln Met Asn Glu
        115                 120                 125

Ala Lys Ile Ala Glu Cys Leu Gln Thr Ile Ser Asp Leu Glu Thr Glu
    130                 135                 140

Cys Leu Asp Leu Arg Thr Lys Leu Cys Asp Leu Glu Arg Ala Asn Gln
145                 150                 155                 160

Thr Leu Lys Asp Glu Tyr Asp Ala Leu Gln Ile Thr Phe Thr Ala Leu
                165                 170                 175

Glu Gly Lys Leu Arg Lys Thr Thr Glu Glu Asn Gln Glu Leu Val Thr
            180                 185                 190

Arg Trp Met Ala Glu Lys Ala Gln Glu Ala Asn Arg Leu Asn Ala Glu
        195                 200                 205

Asn Glu Lys Asp Ser Arg Arg Arg Gln Ala Arg Leu Gln Lys Glu Leu
    210                 215                 220

Ala Glu Ala Ala Lys Glu Pro Leu Pro Val Glu Gln Asp Asp Asp Ile
225                 230                 235                 240

Glu Val Ile Val Asp Glu Thr Ser Asp His Thr Glu Glu Thr Ser Pro
                245                 250                 255

Val Arg Ala Ile Ser Arg Ala Ala Thr Arg Arg Ser Val Ser Ser Phe
            260                 265                 270

Pro Val Pro Gln Asp Asn Val Asp Thr His Pro Gly Ser Gly Lys Glu
        275                 280                 285

Val Arg Val Pro Ala Thr Ala Leu Cys Val Phe Asp Ala His Asp Gly
    290                 295                 300

Glu Val Asn Ala Val Gln Phe Ser Pro Gly Ser Arg Leu Leu Ala Thr
305                 310                 315                 320

Gly Gly Met Asp Arg Arg Val Lys Leu Trp Glu Val Phe Gly Glu Lys
                325                 330                 335

Cys Glu Phe Lys Gly Ser Leu Ser Gly Ser Asn Ala Gly Ile Thr Ser
            340                 345                 350

Ile Glu Phe Asp Ser Ala Gly Ser Tyr Leu Leu Ala Ala Ser Asn Asp
        355                 360                 365

Phe Ala Ser Arg Ile Trp Thr Val Asp Asp Tyr Arg Leu Arg His Thr
    370                 375                 380

Leu Thr Gly His Ser Gly Lys Val Leu Ser Ala Lys Phe Leu Leu Asp
385                 390                 395                 400

Asn Ala Arg Ile Val Ser Gly Ser His Asp Arg Thr Leu Lys Leu Trp
                405                 410                 415

Asp Leu Arg Ser Lys Val Cys Ile Lys Thr Val Phe Ala Gly Ser Ser
            420                 425                 430

Cys Asn Asp Ile Val Cys Thr Glu Gln Cys Val Met Ser Gly His Phe
        435                 440                 445

Asp Lys Lys Ile Arg Phe Trp Asp Ile Arg Ser Glu Ser Ile Val Arg
    450                 455                 460

Glu Met Glu Leu Leu Gly Lys Ile Thr Ala Leu Asp Leu Asn Pro Glu
465                 470                 475                 480

Arg Thr Glu Leu Leu Ser Cys Ser Arg Asp Asp Leu Leu Lys Val Ile
                485                 490                 495

Asp Leu Arg Thr Asn Ala Ile Lys Gln Thr Phe Ser Ala Pro Gly Phe
            500                 505                 510

Lys Cys Gly Ser Asp Trp Thr Arg Val Val Phe Ser Pro Asp Gly Ser
        515                 520                 525

Tyr Val Ala Ala Gly Ser Ala Glu Gly Ser Leu Tyr Ile Trp Ser Val
    530                 535                 540

Leu Thr Gly Lys Val Glu Lys Val Leu Ser Lys Gln His Ser Ser Ser
545                 550                 555                 560

Ile Asn Ala Val Ala Trp Ser Pro Ser Gly Ser His Val Val Ser Val
                565                 570                 575

Asp Lys Gly Cys Lys Ala Val Leu Trp Ala Gln Tyr
            580                 585

<210>  12
<211>  607
<212>  PRT
<213>  Homo sapiens

<400>  12

Met Ser Ser Gly Leu Arg Ala Ala Asp Phe Pro Arg Trp Lys Arg His
1               5                   10                  15

Ile Ser Glu Gln Leu Arg Arg Arg Asp Arg Leu Gln Arg Gln Ala Phe
            20                  25                  30

Glu Glu Ile Ile Leu Gln Tyr Asn Lys Leu Leu Glu Lys Ser Asp Leu
        35                  40                  45

His Ser Val Leu Ala Gln Lys Leu Gln Ala Glu Lys His Asp Val Pro
    50                  55                  60

Asn Arg His Glu Ile Ser Pro Gly His Asp Gly Thr Trp Asn Asp Asn
65                  70                  75                  80

Gln Leu Gln Glu Met Ala Gln Leu Arg Ile Lys His Gln Glu Glu Leu
                85                  90                  95

Thr Glu Leu His Lys Lys Arg Gly Glu Leu Ala Gln Leu Val Ile Asp
            100                 105                 110

Leu Asn Asn Gln Met Gln Arg Lys Asp Arg Glu Met Gln Met Asn Glu
        115                 120                 125

Ala Lys Ile Ala Glu Cys Leu Gln Thr Ile Ser Asp Leu Glu Thr Glu
    130                 135                 140

Cys Leu Asp Leu Arg Thr Lys Leu Cys Asp Leu Glu Arg Ala Asn Gln
145                 150                 155                 160

Thr Leu Lys Asp Glu Tyr Asp Ala Leu Gln Ile Thr Phe Thr Ala Leu
                165                 170                 175

Glu Gly Lys Leu Arg Lys Thr Thr Glu Glu Asn Gln Glu Leu Val Thr
            180                 185                 190

Arg Trp Met Ala Glu Lys Ala Gln Glu Ala Asn Arg Leu Asn Ala Glu
        195                 200                 205

Asn Glu Lys Asp Ser Arg Arg Arg Gln Ala Arg Leu Gln Lys Glu Leu
    210                 215                 220

Ala Glu Ala Ala Lys Glu Pro Leu Pro Val Glu Gln Asp Asp Asp Ile
225                 230                 235                 240

Glu Val Ile Val Asp Glu Thr Ser Asp His Thr Glu Glu Thr Ser Pro
                245                 250                 255

Val Arg Ala Ile Ser Arg Ala Ala Thr Lys Arg Leu Ser Gln Pro Ala
            260                 265                 270

Gly Gly Leu Leu Asp Ser Ile Thr Asn Ile Phe Gly Arg Arg Ser Val
        275                 280                 285

Ser Ser Phe Pro Val Pro Gln Asp Asn Val Asp Thr His Pro Gly Ser
    290                 295                 300

Gly Lys Glu Val Arg Val Pro Ala Thr Ala Leu Cys Val Phe Asp Ala
305                 310                 315                 320

His Asp Gly Glu Val Asn Ala Val Gln Phe Ser Pro Gly Ser Arg Leu
                325                 330                 335

Leu Ala Thr Gly Gly Met Asp Arg Arg Val Lys Leu Trp Glu Val Phe
            340                 345                 350

Gly Glu Lys Cys Glu Phe Lys Gly Ser Leu Ser Gly Ser Asn Ala Gly
        355                 360                 365

Ile Thr Ser Ile Glu Phe Asp Ser Ala Gly Ser Tyr Leu Leu Ala Ala
    370                 375                 380

Ser Asn Asp Phe Ala Ser Arg Ile Trp Thr Val Asp Asp Tyr Arg Leu
385                 390                 395                 400

Arg His Thr Leu Thr Gly His Ser Gly Lys Val Leu Ser Ala Lys Phe
                405                 410                 415

Leu Leu Asp Asn Ala Arg Ile Val Ser Gly Ser His Asp Arg Thr Leu
            420                 425                 430

Lys Leu Trp Asp Leu Arg Ser Lys Val Cys Ile Lys Thr Val Phe Ala
        435                 440                 445

Gly Ser Ser Cys Asn Asp Ile Val Cys Thr Glu Gln Cys Val Met Ser
    450                 455                 460

Gly His Phe Asp Lys Lys Ile Arg Phe Trp Asp Ile Arg Ser Glu Ser
465                 470                 475                 480

Ile Val Arg Glu Met Glu Leu Leu Gly Lys Ile Thr Ala Leu Asp Leu
                485                 490                 495

Asn Pro Glu Arg Thr Glu Leu Leu Ser Cys Ser Arg Asp Asp Leu Leu
            500                 505                 510

Lys Val Ile Asp Leu Arg Thr Asn Ala Ile Lys Gln Thr Phe Ser Ala
        515                 520                 525

Pro Gly Phe Lys Cys Gly Ser Asp Trp Thr Arg Val Val Phe Ser Pro
    530                 535                 540

Asp Gly Ser Tyr Val Ala Ala Gly Ser Ala Glu Gly Ser Leu Tyr Ile
545                 550                 555                 560

Trp Ser Val Leu Thr Gly Lys Val Glu Lys Val Leu Ser Lys Gln His
                565                 570                 575

Ser Ser Ser Ile Asn Ala Val Ala Trp Ser Pro Ser Gly Ser His Val
            580                 585                 590

Val Ser Val Asp Lys Gly Cys Lys Ala Val Leu Trp Ala Gln Tyr
        595                 600                 605

<210>  13

<211>  1929
<212>  DNA
<213>  Homo sapiens

<400>  13
ctggggacgg gggccgagta gcgccttccc cgggccccgt gaaccggctg cgggtcgccc      60

ttggggggca gcggccgcag ccccccacct gggccctcgg tccgccctcc cggcgcgtcc      120

atgaactcag tgtcgccggc cgccgcgcag taccggagca gcagcccgga ggacgcgcgc      180

cgccggcccg aggcccgcag gccgcggggt cccagaggcc cagaccccaa cggcctgggg      240

ccttccggag ccagcggccc cgctcttggc tctcccgggg ctggcccgag tgagccggac      300

gaagtggaca agttcaaggc caagttcctg acagcctgga acaacgtcaa gtacggttgg      360

gtggttaaaa gccggaccag ctttagcaag atctccagca tccacctctg tggccgccgc      420

taccgtttcg agggcgaggg tgacatacag cgtttccagc gggactttgt gtcccgcctg      480

tggctcacat accgccggga cttcccgccc cttcctgggg gctgcctgac ctcggactgt      540

ggctgggggt gcatgttacg cagcggccag atgatgctgg cacagggcct tctgctgcat      600

ttcctgccca gagactggac atgggccgag ggcatgggcc tgggcccccc tgagctgtca      660

gggtcagcct ctcccagccg gtaccatggg cctgcccgct ggatgccccc acgctgggcc      720

cagggtgccc ctgagctgga gcaggaacgc cggcaccggc agattgtgtc ctggttcgcc      780

gaccaccccc gggccccctt tggcctacac cggctggtgg agcttgggca gagctcaggc      840

aagaaggcag gtgactggta tgggccatcg ctagtggcac acatcctcag gaaagccgtg      900

gagagctgct ccgacgtcac ccgcctggtg gtgtacgttt ctcaggactg cacagtgtac      960

aaggcggatg tggcacgcct ggtggccagg ccagacccca cagccgagtg gaagtctgtg      1020

gtcatcctgg tgcccgtgcg actgggtggc gagactctca accccgtgta tgtgccctgc      1080

gtgaaggaac tcctgcgttg cgagctgtgc ctgggcatca tgggtgggaa accgcgacac      1140

tcactgtact tcattggcta ccaagatgac ttcctgctgt acctggaccc tcactactgc      1200

cagcccactg tggatgtcag ccaggccgac ttccccctgg agtccttcca ctgcacctcg      1260

ccccgcaaga tggcctttgc caagatggac ccaagctgta ccgtgggctt ctatgctgga      1320

gacaggaagg agtttgagac actctgctca gagctgacca gggtcctcag ctcctcctca      1380

gccacagagc ggtaccccat gttcaccctg gccgagggcc atgctcagga ccacagcctg      1440

gacgacctct gctcccagct cgcccagccc acactccggc tccctcgcac agggcggctc      1500

ctcagggcca aacgccccag ctctgaggac tttgtgtttt tataaaggga ggggatgagg      1560

ggaaagatac aacactattt atttttttat ttatgtcatg tcgggtgtgg gatcttgagc      1620

tctggcagtg atgatggtac ttcctgttgt cagcccctca agcccagctg caaccagtct      1680

ggggccattc agccagggac agagcccaca gagcccatac acctgtctcc caccagcggg      1740

gccctcctgg cagggtaggg aaggaggacc ccgggcaccc ccctcagggc ctgactcacg     1800

tactgtagtt tgcactggac gcccgggccc tccctgtccc aaagccccct tgggggaact     1860

gtggctgctg ggggccaata aagctgtgta acttgaaaaa aaaaaaaaaa aaaaaaaaaa     1920

aaaaaaaaa                                                             1929

<210>  14
<211>  474
<212>  PRT
<213>  Homo sapiens

<400>  14

Met Asn Ser Val Ser Pro Ala Ala Ala Gln Tyr Arg Ser Ser Ser Pro
1               5                   10                  15

Glu Asp Ala Arg Arg Arg Pro Glu Ala Arg Arg Pro Arg Gly Pro Arg
            20                  25                  30

Gly Pro Asp Pro Asn Gly Leu Gly Pro Ser Gly Ala Ser Gly Pro Ala
        35                  40                  45

Leu Gly Ser Pro Gly Ala Gly Pro Ser Glu Pro Asp Glu Val Asp Lys
    50                  55                  60

Phe Lys Ala Lys Phe Leu Thr Ala Trp Asn Asn Val Lys Tyr Gly Trp
65                  70                  75                  80

Val Val Lys Ser Arg Thr Ser Phe Ser Lys Ile Ser Ser Ile His Leu
                85                  90                  95

Cys Gly Arg Arg Tyr Arg Phe Glu Gly Glu Gly Asp Ile Gln Arg Phe
            100                 105                 110

Gln Arg Asp Phe Val Ser Arg Leu Trp Leu Thr Tyr Arg Arg Asp Phe
        115                 120                 125

Pro Pro Leu Pro Gly Gly Cys Leu Thr Ser Asp Cys Gly Trp Gly Cys
    130                 135                 140

Met Leu Arg Ser Gly Gln Met Met Leu Ala Gln Gly Leu Leu Leu His
145                 150                 155                 160

Phe Leu Pro Arg Asp Trp Thr Trp Ala Glu Gly Met Gly Leu Gly Pro
                165                 170                 175

Pro Glu Leu Ser Gly Ser Ala Ser Pro Ser Arg Tyr His Gly Pro Ala
            180                 185                 190

Arg Trp Met Pro Pro Arg Trp Ala Gln Gly Ala Pro Glu Leu Glu Gln
        195                 200                 205

Glu Arg Arg His Arg Gln Ile Val Ser Trp Phe Ala Asp His Pro Arg
    210                 215                 220

Ala Pro Phe Gly Leu His Arg Leu Val Glu Leu Gly Gln Ser Ser Gly
225                 230                 235                 240

Lys Lys Ala Gly Asp Trp Tyr Gly Pro Ser Leu Val Ala His Ile Leu
                245                 250                 255

Arg Lys Ala Val Glu Ser Cys Ser Asp Val Thr Arg Leu Val Val Tyr
            260                 265                 270

Val Ser Gln Asp Cys Thr Val Tyr Lys Ala Asp Val Ala Arg Leu Val
        275                 280                 285

Ala Arg Pro Asp Pro Thr Ala Glu Trp Lys Ser Val Val Ile Leu Val
    290                 295                 300

Pro Val Arg Leu Gly Gly Glu Thr Leu Asn Pro Val Tyr Val Pro Cys
305                 310                 315                 320

Val Lys Glu Leu Leu Arg Cys Glu Leu Cys Leu Gly Ile Met Gly Gly
                325                 330                 335

Lys Pro Arg His Ser Leu Tyr Phe Ile Gly Tyr Gln Asp Asp Phe Leu
            340                 345                 350

Leu Tyr Leu Asp Pro His Tyr Cys Gln Pro Thr Val Asp Val Ser Gln
        355                 360                 365

Ala Asp Phe Pro Leu Glu Ser Phe His Cys Thr Ser Pro Arg Lys Met
    370                 375                 380

Ala Phe Ala Lys Met Asp Pro Ser Cys Thr Val Gly Phe Tyr Ala Gly
385                 390                 395                 400

Asp Arg Lys Glu Phe Glu Thr Leu Cys Ser Glu Leu Thr Arg Val Leu
                405                 410                 415

Ser Ser Ser Ser Ala Thr Glu Arg Tyr Pro Met Phe Thr Leu Ala Glu
            420                 425                 430

Gly His Ala Gln Asp His Ser Leu Asp Asp Leu Cys Ser Gln Leu Ala
        435                 440                 445

Gln Pro Thr Leu Arg Leu Pro Arg Thr Gly Arg Leu Leu Arg Ala Lys
    450                 455                 460

Arg Pro Ser Ser Glu Asp Phe Val Phe Leu
465                 470

<210>  15
<211>  1372
<212>  DNA
<213>  Homo sapiens

<400>  15
cgggtgctga tgcgagtcgg tggcagcgag gacattttct gactccctgg cccctgacac      60

ggctgcactt tccatcccgt cgcggggccg gccgctactc cggccccagg atgcagaatg     120

tgattaatac tgtgaaggga aaggcactgg aagtggctga gtacctgacc ccggtcctca     180

aggaatcaaa gtttaaggaa acaggtgtaa ttaccccaga agagtttgtg gcagctggag     240

atcacctagt ccaccactgt ccaacatggc aatgggctac aggggaagaa ttgaaagtga     300

aggcatacct accaacaggc aaacaatttt tggtaaccaa aaatgtgccg tgctataagc     360

ggtgcaaaca gatggaatat tcagatgaat tggaagctat cattgaagaa gatgatggtg     420

atggcggatg ggtagataca tatcacaaca caggtattac aggaataacg gaagccgtta     480

aagagatcac actggaaaat aaggacaata taaggcttca agattgctca gcactatgtg     540

aagaggaaga agatgaagat gaaggagaag ctgcagatat ggaagaatat gaagagagtg     600

gattgttgga aacagatgag gctaccctag atacaaggaa aatagtagaa gcttgtaaag     660

ccaaaactga tgctggcggt gaagatgcta ttttgcaaac cagaacttat gacctttaca     720

tcacttatga taaatattac cagactccac gattatggtt gtttggctat gatgagcaac     780

ggcagccttt aacagttgag cacatgtatg aagacatcag tcaggatcat gtgaagaaaa     840

cagtgaccat tgaaaatcac cctcatctgc caccacctcc catgtgttca gttcacccat     900

gcaggcatgc tgaggtgatg aagaaaatca ttgagactgt tgcagaagga gggggagaac     960

ttggagttca tatgtatctt cttattttct tgaaatttgt acaagctgtc attccaacaa    1020

tagaatatga ctacacaaga cacttcacaa tgtaatgaag agagcataaa atctatccta    1080

attattggtt ctgattttta aagaattaac ccatagatgt gaccattgac catattcatc    1140

aatatataca gtttctctaa taagggactt atatgtttat gcattaaata aaaatatgtt    1200

ccactaccag ccttacttgt ttaataaaaa tcagtgcaaa gaaaaaaaaa aaaaaaaaaa    1260

aaaaaaaaaa aaaaaaaaaa aaaaaaaaaa aaaaaaaaaa aaaaaaaaaa aaaaaaaaaa    1320

aaaaaaaaaa aaaaaaaaaa aaaaaaaaaa aaaaaaaaaa aaaaaaaaaa aa            1372

<210>  16
<211>  314
<212>  PRT
<213>  Homo sapiens

<400>  16

Met Gln Asn Val Ile Asn Thr Val Lys Gly Lys Ala Leu Glu Val Ala
1               5                   10                  15

Glu Tyr Leu Thr Pro Val Leu Lys Glu Ser Lys Phe Lys Glu Thr Gly
            20                  25                  30

Val Ile Thr Pro Glu Glu Phe Val Ala Ala Gly Asp His Leu Val His
        35                  40                  45

His Cys Pro Thr Trp Gln Trp Ala Thr Gly Glu Glu Leu Lys Val Lys
    50                  55                  60

Ala Tyr Leu Pro Thr Gly Lys Gln Phe Leu Val Thr Lys Asn Val Pro
65                  70                  75                  80

Cys Tyr Lys Arg Cys Lys Gln Met Glu Tyr Ser Asp Glu Leu Glu Ala
                85                  90                  95

Ile Ile Glu Glu Asp Asp Gly Asp Gly Gly Trp Val Asp Thr Tyr His
            100                 105                 110

Asn Thr Gly Ile Thr Gly Ile Thr Glu Ala Val Lys Glu Ile Thr Leu
        115                 120                 125

Glu Asn Lys Asp Asn Ile Arg Leu Gln Asp Cys Ser Ala Leu Cys Glu
    130                 135                 140

Glu Glu Glu Asp Glu Asp Glu Gly Glu Ala Ala Asp Met Glu Glu Tyr
145                 150                 155                 160

Glu Glu Ser Gly Leu Leu Glu Thr Asp Glu Ala Thr Leu Asp Thr Arg
                165                 170                 175

Lys Ile Val Glu Ala Cys Lys Ala Lys Thr Asp Ala Gly Gly Glu Asp
            180                 185                 190

Ala Ile Leu Gln Thr Arg Thr Tyr Asp Leu Tyr Ile Thr Tyr Asp Lys
        195                 200                 205

Tyr Tyr Gln Thr Pro Arg Leu Trp Leu Phe Gly Tyr Asp Glu Gln Arg
    210                 215                 220

Gln Pro Leu Thr Val Glu His Met Tyr Glu Asp Ile Ser Gln Asp His
225                 230                 235                 240

Val Lys Lys Thr Val Thr Ile Glu Asn His Pro His Leu Pro Pro Pro
                245                 250                 255

Pro Met Cys Ser Val His Pro Cys Arg His Ala Glu Val Met Lys Lys
            260                 265                 270

Ile Ile Glu Thr Val Ala Glu Gly Gly Gly Glu Leu Gly Val His Met
        275                 280                 285

Tyr Leu Leu Ile Phe Leu Lys Phe Val Gln Ala Val Ile Pro Thr Ile
    290                 295                 300

Glu Tyr Asp Tyr Thr Arg His Phe Thr Met
305                 310

<210>  17
<211>  2359
<212>  DNA
<213>  Homo sapiens

<400>  17
   cgaccgagca cagacacgtt gcccaccgct cctctcccga ggtctgtagt cgcggagaaa      60
   cacatgttgc gttactaacg ttcagaggtc tgcgacagct tcgatttgaa tgactagccg     120
   ggaacaccaa gtttcactgt gtaattgcgt ccccctactc cggcgcctcc tttgcgacgc     180
   tccctggaga aaagcacgcc cactgcacgc gctcagtcgc tacttccgct ctcgagtgtc     240
   tccaagcaag atggcggagg agccgcagtc tgtgttgcag cttcctactt caattgctgc     300
   tggaggggaa ggacttacgg atgtctcccc agaaacaacc accccggagc ccccgtcttc      360
   cgctgcagtt tccccgggaa cagaggaacc tgctggcgac accaagaaaa aaattgacat     420
   tttgctaaag gctgtgggag acactcctat tatgaaaaca aagaagtggg cagtagagcg     480
   aacacgaacc atccaaggac tcattgactt catcaaaaag tttcttaaac ttgtggcctc     540
   agaacagttg tttatttatg tgaatcagtc ctttgctcct tccccagacc aagaagttgg     600
   aactctctat gagtgttttg gcagtgatgg taaactggtt ttacattact gcaagtctca     660
   ggcgtgggga tgaaccacaa agaaaatcaa cttgctacta catgaaatgg attttcacgg     720
   aagagacagc tctgaaaagt tttgatgctt gtggcaagag acttaacaga tgtgatctat     780
   ttagtatgtg tctactctat gtttatgcat aagaaaacat ccatagcatg aatggactca     840
   gaaaaatgtg atttgtatta atgcaccagt catcataaaa gatggtcatg atagtacacc     900
   cattgctcct acttgttact attattgctg cagatctgcc tccaaggttg aaaaggagac     960
   taagactgta taaacatctt cattgtcagt tctcaaaatg actgaaattg ttttcatggt    1020
   aaaagttaat atactaaagg gttccttttt ttttaatgtt tacatttatc tctatgttta 1080
   cctttttagt cacattgacc tgctggctga atacctcaaa tagtccagta gagggcagtc 1140
   caccaggcag aaaaggttag gcgttttggt ttcacatctt tgctggggaa taatagggga 1200
   aatggctgtt tttgctaatt tttagctaat atctagccag gagagcaagc acataggaca 1260
   gactgaaaga ctgtaatttt acacaataca catggcttaa ttattttatt gggatacaga 1320
   aaaatataaa ttctggacaa ataagtcata tacctgtttt cagtcctaac atttaaggat 1380
   tcttgagtcc caatcacata actgtggtgt tactctgtca tttatatggt gtcaaaagca 1440
   cttgatgagt aaacccagta gcatcttttt gagtgtttca taatgcattt tccaacttga 1500
   aaacaataat tgaaaaatag ccttattgta tattttatgc catgactaaa agtgccattt 1560
   ttactgatgc tattagactg ataatttctt gaagtgaaat ttaacctttt tttctcttta 1620
   gtattatgtt tataatgcca tatttttaga aagcattcca gatcaggcat ggtggcttac 1680
   acctgtaatc ccagcacttt ggaaggctga ggtgtgggga ttgcgtgaag ccacaagttt 1740
   gagaccagcc tggttagcaa ggcaagatcc ccaactctac aaaaaaataa aaattaaaaa 1800
   aaaattatta ggctgcagag gcaagaggat cccctgagcc cagaagttca agggtatagt 1860
   gagtcgtgat tgtaccactg cattcctgct gagcaacaga gtgagacccc atctcaaaaa 1920
   agaaaaaaaa aggcattcta gtaaatcgaa tgtaatgtga atggaatttc aaaacaggat 1980
   ctaagatggt atgtagtaga attcaaagta atatcatttt aaagttaaat gagtatggaa 2040
   aaggtctgtt ctctagtttt gtccagttca gtttactgaa ggaatatatt taattatatt 2100
   catatattta acaaataaaa atatgttgaa ttttcgtatt gtttgccact gagggttcag 2160
   atgatagacc tcaaaaaatc gaaaatactg gttgaaattt gtagcatcca tttagttatt 2220
   ctttttgacc taaataactt aatagtttat taaatctaag gttagctaaa tatgtagcta 2280
   accttatttg ttttctttcc taacaactct gaagaataca taggactttg cacttttttt 2340
   tttttttttt ttttttaaa 2359

<210> 18
<211> 187
<212> PRT
<213> Homo sapiens

<400> 18

Met Thr Ser Arg Glu His Gln Val Ser Leu Cys Asn Cys Val Pro Leu
1               5                   10                  15

Leu Arg Arg Leu Leu Cys Asp Ala Pro Trp Arg Lys Ala Arg Pro Leu
            20                  25                  30

His Ala Leu Ser Arg Tyr Phe Arg Ser Arg Val Ser Pro Ser Lys Met
35                  40                  45

Ala Glu Glu Pro Gln Ser Val Leu Gln Leu Pro Thr Ser Ile Ala Ala
    50                  55                  60

Gly Gly Glu Gly Leu Thr Asp Val Ser Pro Glu Thr Thr Thr Pro Glu
65                  70                  75                  80

Pro Pro Ser Ser Ala Ala Val Ser Pro Gly Thr Glu Glu Pro Ala Gly
                85                  90                  95

Asp Thr Lys Lys Lys Ile Asp Ile Leu Leu Lys Ala Val Gly Asp Thr
            100                 105                 110

Pro Ile Met Lys Thr Lys Lys Trp Ala Val Glu Arg Thr Arg Thr Ile
        115                 120                 125

Gln Gly Leu Ile Asp Phe Ile Lys Lys Phe Leu Lys Leu Val Ala Ser
    130                 135                 140

Glu Gln Leu Phe Ile Tyr Val Asn Gln Ser Phe Ala Pro Ser Pro Asp
145                 150                 155                 160

Gln Glu Val Gly Thr Leu Tyr Glu Cys Phe Gly Ser Asp Gly Lys Leu
                165                 170                 175

Val Leu His Tyr Cys Lys Ser Gln Ala Trp Gly
            180                 185

<210>  19
<211>  2160
<212>  DNA
<213>  Homo sapiens

<400>  19
tgggcgggag gaacgcgccg ctaggcggga gagcgcggcc atggcggggc cgggcgtccc      60

cggtgccccc gcagcgcgct ggaaacgcca catcgtgcgg cagctgcggc ttcgggaccg     120

tacgcaaaag gcgcttttcc tggagctggt gccggcctat aaccatctct tagagaaggc     180

tgagctgctg gacaagttct caaagaagct gcagccggag ccaaacagtg tcactcccac     240

cacccaccag ggcccctggg aggagtcaga gcttgactca gaccaagtcc catcactggt     300

cgcactgagg gtgaagtggc aggaggagga ggaggggctc cggctggtct gtggtgagat     360

ggcctaccag gtggtggaga agggcgcggc cctgggcacg ctggagtcgg agctgcagca     420

gaggcaaagc aggctggcag ccctggaggc ccgcgtggcg cagctgcgag aggcgcgggc     480

gcagcaggcc cagcaggtgg aggagtggcg ggcgcagaat gcggtgcagc gggcagccta     540

cgaggcgctg cgcgcgcacg tcgggctccg ggaggcggca ctgcgcaggc tccaggaaga 600

ggcgcgcgac ctgctggaga ggctcgtgca gcgcaaggcg cgcgccgcgg ccgagcgcaa 660

cctgcgcaac gagcgccggg agcgggccaa gcaggcgcgg gtgtcccagg agctgaagaa 720

ggctgccaag cggaccgtga gcatcagcga gggcccggac accctaggcg atgggatgag 780

ggagagaagg gagactctgg ctctggcccc tgagccagag cccctggaga aggaagcttg 840

tgagaagtgg aagaggccct tcaggtctgc ctcagccacc tccctgacgc tgtcccactg 900

tgtggatgtg gtgaaggggc ttctggattt taagaagagg agaggtcact caattggggg 960

agcccctgag cagcgatacc agatcatccc tgtgtgtgtg gctgcccgac ttcctacccg 1020

ggctcaggat gtgctggatg cccacctctc tgaggtcaat gctgttcgtt ttggccccaa 1080

cagcagcctc ctggccactg gaggggctga ccgcctgatc cacctctgga atgttgtggg 1140

aagtcgcctg gaggccaacc agaccctgga gggagctggt ggcagcatca ccagtgtgga 1200

ctttgacccc tcgggctacc aggttttagc agcaacttac aaccaggctg cccagctctg 1260

gaaggtgggg gaggcacagt ccaaggagac actgtctgga cacaaggata aggtgacagc 1320

tgccaaattc aagctaacga ggcaccaggc agtgactggg agccgcgacc ggacagtgaa 1380

ggagtgggac ctcggccgtg cctattgctc caggaccatc aatgtccttt cctactgtaa 1440

tgacgtggtg tgtggggacc atatcatcat tagtggccac aatgaccaga agatccggtt 1500

ctgggacagc agggggcccc actgcaccca ggtcatccct gtgcagggcc gggtcacctc 1560

cctgagcctc agccacgacc aactgcacct gctcagctgt tcccgagaca acacactcaa 1620

ggtcatcgac ctgcgtgtca gcaacatccg ccaggtgttc agggccgatg gcttcaagtg 1680

tggttctgac tggaccaaag ctgtgttcag cccggacaga agctatgcac tggcaggctc 1740

ctgtgatggg gccctttaca tctgggatgt ggacaccggg aaactggaga gcagactaca 1800

gggaccccat tgcgctgccg tcaacgccgt ggcctggtgc tactccggga gccacatggt 1860

gagcgtggac cagggcagga aggttgtgct ctggcagtag ggccacgacc tgcctgcctg 1920

ggctggagct cttgcccgaa gcctgaagct tccttcggcg ccatgcaggg gttggggttg 1980

ggactggagc tggccttggg atttaatggg gaagaaggcc tggcaggacc tggcctgttt 2040

gtttaaaaat gaagtatggg ttgggggatt acgctagttt ttctttgtat ttttatctct 2100

atctcctcac tttttctccc aaagtagaaa aaaatgatat ctgaaaaaaa aaaaaaaaaa 2160

<210> 20
<211> 619
<212> PRT
<213> Homo sapiens

<400> 20

Met Ala Gly Pro Gly Val Pro Gly Ala Pro Ala Ala Arg Trp Lys Arg
1               5                   10                  15

His Ile Val Arg Gln Leu Arg Leu Arg Asp Arg Thr Gln Lys Ala Leu
            20                  25                  30

Phe Leu Glu Leu Val Pro Ala Tyr Asn His Leu Leu Glu Lys Ala Glu
        35                  40                  45

Leu Leu Asp Lys Phe Ser Lys Lys Leu Gln Pro Glu Pro Asn Ser Val
    50                  55                  60

Thr Pro Thr Thr His Gln Gly Pro Trp Glu Glu Ser Glu Leu Asp Ser
65                  70                  75                  80

Asp Gln Val Pro Ser Leu Val Ala Leu Arg Val Lys Trp Gln Glu Glu
                85                  90                  95

Glu Glu Gly Leu Arg Leu Val Cys Gly Glu Met Ala Tyr Gln Val Val
            100                 105                 110

Glu Lys Gly Ala Ala Leu Gly Thr Leu Glu Ser Glu Leu Gln Gln Arg
        115                 120                 125

Gln Ser Arg Leu Ala Ala Leu Glu Ala Arg Val Ala Gln Leu Arg Glu
    130                 135                 140

Ala Arg Ala Gln Gln Ala Gln Gln Val Glu Glu Trp Arg Ala Gln Asn
145                 150                 155                 160

Ala Val Gln Arg Ala Ala Tyr Glu Ala Leu Arg Ala His Val Gly Leu
                165                 170                 175

Arg Glu Ala Ala Leu Arg Arg Leu Gln Glu Glu Ala Arg Asp Leu Leu
            180                 185                 190

Glu Arg Leu Val Gln Arg Lys Ala Arg Ala Ala Ala Glu Arg Asn Leu
        195                 200                 205

Arg Asn Glu Arg Arg Glu Arg Ala Lys Gln Ala Arg Val Ser Gln Glu
    210                 215                 220

Leu Lys Lys Ala Ala Lys Arg Thr Val Ser Ile Ser Glu Gly Pro Asp
225                 230                 235                 240

Thr Leu Gly Asp Gly Met Arg Glu Arg Arg Glu Thr Leu Ala Leu Ala
                245                 250                 255

Pro Glu Pro Glu Pro Leu Glu Lys Glu Ala Cys Glu Lys Trp Lys Arg
        260                 265                 270

Pro Phe Arg Ser Ala Ser Ala Thr Ser Leu Thr Leu Ser His Cys Val
    275                 280                 285

Asp Val Val Lys Gly Leu Leu Asp Phe Lys Lys Arg Arg Gly His Ser
    290                 295                 300

Ile Gly Gly Ala Pro Glu Gln Arg Tyr Gln Ile Ile Pro Val Cys Val
305                 310                 315                 320

Ala Ala Arg Leu Pro Thr Arg Ala Gln Asp Val Leu Asp Ala His Leu
                325                 330                 335

Ser Glu Val Asn Ala Val Arg Phe Gly Pro Asn Ser Ser Leu Leu Ala
            340                 345                 350

Thr Gly Gly Ala Asp Arg Leu Ile His Leu Trp Asn Val Val Gly Ser
        355                 360                 365

Arg Leu Glu Ala Asn Gln Thr Leu Glu Gly Ala Gly Gly Ser Ile Thr
    370                 375                 380

Ser Val Asp Phe Asp Pro Ser Gly Tyr Gln Val Leu Ala Ala Thr Tyr
385                 390                 395                 400

Asn Gln Ala Ala Gln Leu Trp Lys Val Gly Glu Ala Gln Ser Lys Glu
                405                 410                 415

Thr Leu Ser Gly His Lys Asp Lys Val Thr Ala Ala Lys Phe Lys Leu
            420                 425                 430

Thr Arg His Gln Ala Val Thr Gly Ser Arg Asp Arg Thr Val Lys Glu
        435                 440                 445

Trp Asp Leu Gly Arg Ala Tyr Cys Ser Arg Thr Ile Asn Val Leu Ser
    450                 455                 460

Tyr Cys Asn Asp Val Val Cys Gly Asp His Ile Ile Ile Ser Gly His
465                 470                 475                 480

Asn Asp Gln Lys Ile Arg Phe Trp Asp Ser Arg Gly Pro His Cys Thr
                485                 490                 495

Gln Val Ile Pro Val Gln Gly Arg Val Thr Ser Leu Ser Leu Ser His
            500                 505                 510

Asp Gln Leu His Leu Leu Ser Cys Ser Arg Asp Asn Thr Leu Lys Val
        515                 520                 525

Ile Asp Leu Arg Val Ser Asn Ile Arg Gln Val Phe Arg Ala Asp Gly
    530                 535                 540

Phe Lys Cys Gly Ser Asp Trp Thr Lys Ala Val Phe Ser Pro Asp Arg
545                 550                 555                 560

Ser Tyr Ala Leu Ala Gly Ser Cys Asp Gly Ala Leu Tyr Ile Trp Asp
                565                 570                 575

Val Asp Thr Gly Lys Leu Glu Ser Arg Leu Gln Gly Pro His Cys Ala
            580                 585                 590

Ala Val Asn Ala Val Ala Trp Cys Tyr Ser Gly Ser His Met Val Ser
        595                 600                 605

Val Asp Gln Gly Arg Lys Val Val Leu Trp Gln
    610                 615

<210>  21
<211>  2304
<212>  DNA
<213>  Homo sapiens

<400>  21
acgctgcgtg ccgctgctgg gttccgccac gcccgtcatg gcggcggccc cggccggctc      60

tggccccgcc cctcggtgac gcgtcgcgag tcacctgacc aggctgcggg ctgaggagat     120

acaagggaag tggctatcgc cagagtcgga ttcgccgccg cagcagccgc cgcccccggg     180

agccgccggg accctcgcgt cgtcgccgcc gccgccgccc agatccctgc accatgccgt     240

cggagaagac cttcaagcag cgccgcacct tcgaacaaag agtagaagat gtccgactta     300

ttcgagagca gcatccaacc aaaatcccgg tgataataga acgatacaag ggtgagaagc     360

agcttcctgt tctggataaa acaaagttcc ttgtacctga ccatgtcaac atgagtgagc     420

tcatcaagat aattagaagg cgcttacagc tcaatgctaa tcaggccttc ttcctgttgg     480

tgaacggaca cagcatggtc agcgtctcca caccaatctc agaggtgtat gagagtgaga     540

aagatgaaga tggattcctg tacatggtct atgcctccca ggagacgttc gggatgaaat     600

tgtcagtgta aaaccagaaa aaatgcagct cttctagaat tgtttaaacc cttaccaagg     660

aaaaaaaagg gatgttacca actgagatcg atcagttcat ccaatcacag atcatgaaac     720

agtagtgttc ccacctagga gtgttaggaa gttgtgtttg tgtttcaagc agaaaaactg     780

agctccaagt gagcacattc agctttggaa actatattat ttaatgtagg ctagcttgtt     840

ttcaaatttt aaaagtttaa aaataaaata ctttgcattc taagttgcca ataaaataga 900

ccttcaagtt attttaatgc tcttttctca ctaataggaa cttgtaattc cagcagtaat 960

ttaaaggctt tcagagagac cctgagtctt ctcttcaggt tcacagaacc cgccgccttt 1020

ttgggtagaa gttttctact cagctagaga gatctcccta agaggatctt taggcctgag 1080

ttgtgaagcg caacccccgc aaaacgcatt tgccatcaca gttggcacaa acgcagggta 1140

aacgggctgt gtgagaaaac ggccctgact gtaaactgct gaaggtccct gactcctaag 1200

agaaccacac ccaaagtcct cactcttgca ggggtagaca tttctggttt ggtttgttct 1260

ctagatagtt acacacataa agacaccact caaaaggaaa cttgaataat ttataatttt 1320

gatcgagttt cttaaaagac cctggagaaa gagtggcatt tcttctgttt caggttttgt 1380

ctgagttcaa actagtgcct gtgttgttac ggaaagcagc agtgtaccag tgtcactctg 1440

gagtacagcg ggagaaacac aaaatagtat aactgaaaac attaacattc agacacactc 1500

ccttctgcct tccggcttaa agctgtggat gatccacgtt tttgtttttt taatgttaaa 1560

tgtgtaactc agtattactg aaaaggtacc cacattttga atagtagtta tcactcttag 1620

gtcagacagc catcagaatt ctcccacacc aagtgcatgt cagttgtgga gaaaacatag 1680

caaaaagagc cgtacgctct ttacagatac taatgtcaag agttaaacct cctcaggttc 1740

aacctgtgat aaaagactag tgcttcccag tacttgcatg gggttcacta tttatagttt 1800

tcttgggagt atcacaggaa aatcacaatt acaccacttt agaccctatg tgtagcaggt 1860

cacaacttac ccttgtgtgt ttagatgtgt atgaaatacc tgtatacgtt agtgaaagct 1920

gtttactgta acggggaaaa ccagattctt tgcatctggg ccctctactg attgttaaag 1980

gagttcctgt cacctgctcc ccccaccccc gcatgcgtct gtccacttgg ctaactttta 2040

atatgtgtat ttttacatta tgtatattct taactggact gtctcgttta gactgtatac 2100

atcatatctg acattattgt aactaccgtg tgatcagtaa gattcctgta agaaatactg 2160

ctttttaaga aaaaaaataa catgctgagg ggtgacctat atcccatgtg agtggtcact 2220

ttatttatag gatctttaaa acatttttaa tgaactaagt tgaataaagg cacaattaaa 2280

aactgtcaaa aaaaaaaaaa aaaa 2304

<210> 22
<211> 125
<212> PRT
<213> Homo sapiens

<400> 22

Met Pro Ser Glu Lys Thr Phe Lys Gln Arg Arg Thr Phe Glu Gln Arg
1               5                   10                  15

Val Glu Asp Val Arg Leu Ile Arg Glu Gln His Pro Thr Lys Ile Pro
            20                  25                  30

Val Ile Ile Glu Arg Tyr Lys Gly Glu Lys Gln Leu Pro Val Leu Asp
        35                  40                  45

Lys Thr Lys Phe Leu Val Pro Asp His Val Asn Met Ser Glu Leu Ile
    50                  55                  60

Lys Ile Ile Arg Arg Arg Leu Gln Leu Asn Ala Asn Gln Ala Phe Phe
65                  70                  75                  80

Leu Leu Val Asn Gly His Ser Met Val Ser Val Ser Thr Pro Ile Ser
                85                  90                  95

Glu Val Tyr Glu Ser Glu Lys Asp Glu Asp Gly Phe Leu Tyr Met Val
            100                 105                 110

Tyr Ala Ser Gln Glu Thr Phe Gly Met Lys Leu Ser Val
        115                 120                 125